(19) Europäisches Patentamt
European Patent Office
Office européen des brevets

(11) EP 4 524 142 A1

(12) EUROPEAN PATENT APPLICATION
published in accordance with Art. 153(4) EPC

(43) Date of publication:
**19.03.2025 Bulletin 2025/12**

(21) Application number: **23802817.9**

(22) Date of filing: **06.05.2023**

(51) International Patent Classification (IPC):
***C07D 498/14*** *(2006.01)* ***C07D 487/04*** *(2006.01)*
***C07D 471/04*** *(2006.01)* ***C07D 471/14*** *(2006.01)*
***C07D 401/14*** *(2006.01)* ***C07D 401/02*** *(2006.01)*
***A61K 31/498*** *(2006.01)* ***A61K 31/4985*** *(2006.01)*
***A61K 31/4375*** *(2006.01)* ***A61K 31/435*** *(2006.01)*
***A61P 35/00*** *(2006.01)*

(52) Cooperative Patent Classification (CPC):
**A61K 31/435; A61K 31/4375; A61K 31/498; A61K 31/4985; A61P 35/00; C07D 401/02; C07D 401/14; C07D 471/04; C07D 471/14; C07D 487/04; C07D 498/14**

(86) International application number:
**PCT/CN2023/092572**

(87) International publication number:
**WO 2023/217045 (16.11.2023 Gazette 2023/46)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:
**07.05.2022 CN 202210495459**
**17.06.2022 CN 202210693545**
**29.09.2022 CN 202211204558**
**31.10.2022 CN 202211351783**
**10.11.2022 CN 202211408423**
**09.12.2022 CN 202211593592**
**02.02.2023 CN 202310088997**
**15.02.2023 CN 202310123790**
**10.03.2023 CN 202310232367**
**23.03.2023 CN 202310303425**
**31.03.2023 CN 202310340861**
**12.04.2023 CN 202310388951**

(71) Applicant: **Medshine Discovery Inc.**
**Nanjing, Jiangsu 210032 (CN)**

(72) Inventors:
- **CHEN, Zhengxia**
  **Shanghai 200131 (CN)**
- **DAI, Meibi**
  **Shanghai 200131 (CN)**
- **ZHANG, Yang**
  **Shanghai 200131 (CN)**
- **CHEN, Shuhui**
  **Shanghai 200131 (CN)**

(74) Representative: **Dehns**
**10 Old Bailey**
**London EC4M 7NG (GB)**

# (54) FLUOROQUINOXALINONE DERIVATIVE FOR SELECTIVELY INHIBITING PARP1

(57) Disclosed is a series of fluorine-substituted heterocyclic compounds, and specifically, disclosed are a compound represented by formula (XII) and a pharmaceutically acceptable salt thereof.

EP 4 524 142 A1

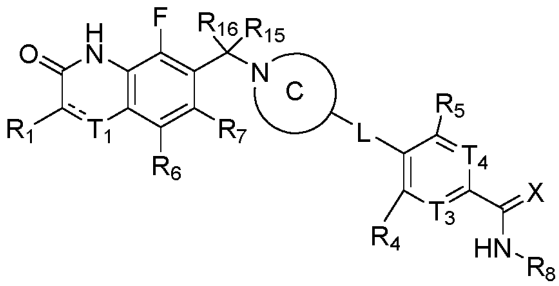

(XII)

## Description

The present application claims the right of the following priorities:

CN202210495459.8, application date: May 7, 2022;
CN202210693545.X, application date: June 17, 2022;
CN202211204558.2, application date: September 29, 2022;
CN202211351783.9, application date: October 31, 2022;
CN202211408423.8, application date: November 10, 2022;
CN202211593592.3, application date: December 9, 2022;
CN202310088997X, application date: February 2, 2023;
CN2023101237901, application date: February 15, 2023;
CN2023102323675, application date: March 10, 2023;
CN2023103034259, application date: March 23, 2023;
CN2023103408613, application date: March 31, 2023;
CN202310388951X, application date: April 12, 2023.

TECHNICAL FIELD

The present disclosure relates to a series of fluorine-substituted heterocyclic compounds, specifically to a compound of formula (XII) and a pharmaceutically acceptable salt thereof.

BACKGROUND

Poly-ADP-ribose polymerase (PARP) is a superfamily of proteases, currently consisting of 18 members. It plays a significant role in various cell cycle processes, such as replication, recombination, chromatin remodeling, and DNA damage repair.

Among them, PARP1 and PARP2 have been extensively studied due to their roles in DNA damage repair. PARP1 is activated by DNA damage breaks and catalyzes the addition of poly(ADP-ribose) (PAR) chains to target proteins. This post-translational modification, known as PARylation, mediates the recruitment of additional DNA repair factors to the site of DNA damage. After completing the recruitment of DNA repair factors, PARP undergoes auto-PARylation, which triggers the release of PARP bound to DNA, thereby allowing other DNA repair proteins to access and complete the repair.

Inhibiting PARP family enzymes has been developed as an anti-tumor strategy, which selectively kills cancer cells by inactivating complementary DNA repair pathways. Numerous preclinical and clinical studies have shown that tumor cells with BRCA1 or BRCA2 mutations, where BRCA is a key tumor suppressor protein involved in double-strand DNA break (DSB) repair through homologous recombination (HR), possess defective homologous recombination repair (HRD) pathways and rely on the function of PARP enzyme for survival. The growth of tumors with BRCA mutations becomes more dependent on the PARP pathway, making the tumors particularly sensitive to PARP1 inhibitors. In addition to being effective against BRCA-mutated cancers, clinical studies have also demonstrated certain efficacy of PARP inhibitors in non-BRCA-mutated tumors that exhibit homologous recombination defects.

Compared to other clinical PARP1/2 inhibitors, highly selective PARP1 inhibitors enhance selectivity for PARP1 over PARP2, which can reduce hematological toxicity caused by PARP2 inhibition, thereby achieving better clinical efficacy with lower toxicity. Therefore, there remains an unmet medical need for effective and safe PARP inhibitors, particularly for PARP inhibitors that selectively target PARP1.

PARP1-selective inhibitors can be used alone for solid tumors such as ovarian cancer, breast cancer, triple-negative breast cancer, and prostate cancer with BRCA1/2 mutations (synthetic lethality mechanism). They can also be used as combination therapies to enhance the effects of DNA-damage-based anti-cancer drugs (e.g., DNA alkylating agents, topoisomerase inhibitors, and platinum-based chemotherapeutics) and radiotherapy. Additionally, they can be used in combination with other targeted therapies, such as PD-1 inhibitors, androgen receptor (AR) inhibitors, cell cycle checkpoint kinase (Chk) inhibitors, and c-Met inhibitors, for the treatment of various BRCA-negative or BRCA-positive solid tumors.

CONTENT OF THE PRESENT INVENTION

The present disclosure provides a compound of formula (XII) or a pharmaceutically acceptable salt thereof,

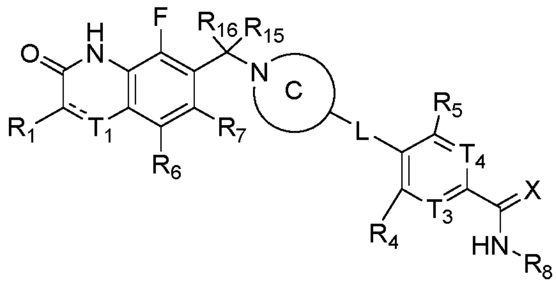

**(XII)**

,

wherein

X is selected from O and S;
the structural moiety

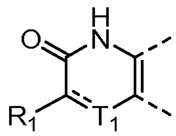

is selected from

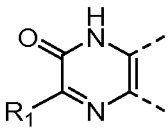

,

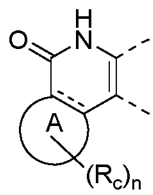

,

and

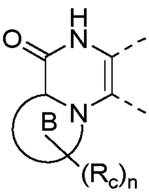

;

ring A is selected from phenyl and 6-membered heteroaryl;
ring B is selected from 6-membered heteroaryl;
L is selected from a single bond, and ring C is selected from

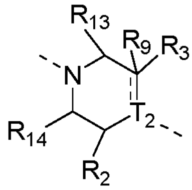

;

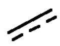

is selected from a single bond or a double bond;
$T_2$ is selected from C, N, and CH;
$T_3$ and $T_4$ are each independently selected from N and $CR_{10}$;

$R_1$ is selected from $C_{1-3}$ alkyl and $C_{3-5}$ cycloalkyl, and the $C_{1-3}$ alkyl and $C_{3-5}$ cycloalkyl are each independently and optionally substituted by 1, 2, or 3 halogens;
$R_2$ is selected from H and $C_{1-3}$ alkyl, and the $C_{1-3}$ alkyl is optionally substituted by 1, 2, or 3 halogens;
$R_3$ is selected from H and halogen;
$R_9$ is absent, or selected from H and halogen;
$R_4$ is selected from H, halogen, CN, $C_{1-3}$ alkyl, and $C_{1-3}$ alkoxy;
$R_5$ is selected from H, halogen, CN, $C_{1-3}$ alkyl, and $C_{1-3}$ alkoxy;
$R_6$ and $R_7$ are each independently selected from H, halogen, $C_{1-3}$ alkyl, and $C_{1-3}$ alkoxy, and the $C_{1-3}$ alkyl and $C_{1-3}$ alkoxy are each independently and optionally substituted by 1, 2, or 3 halogens;
$R_8$ is selected from $C_{1-3}$ alkyl and $C_{3-5}$ cycloalkyl, and the $C_{1-3}$ alkyl and $C_{3-5}$ cycloalkyl are each independently and optionally substituted by 1, 2, or 3 R;
$R_{10}$ is selected from H and halogen;
$R_{13}$ and $R_{14}$ are each independently selected from H, halogen, $C_{1-3}$ alkyl, $C_{1-3}$ alkoxy, and $C_{3-5}$ cycloalkyl, and the $C_{1-3}$ alkyl, $C_{1-3}$ alkoxy, and $C_{3-5}$ cycloalkyl are each independently and optionally substituted by 1, 2, or 3 halogens;
$R_{15}$ and $R_{16}$ are each independently selected from H, D, $C_{1-3}$ alkyl, and $C_{1-3}$ alkoxy, and the $C_{1-3}$ alkyl and $C_{1-3}$ alkoxy are each independently and optionally substituted by 1, 2, or 3 halogens;
or, $R_2$ and $R_4$ form a ring, so that the structural moiety

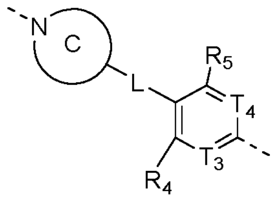 is

selected from

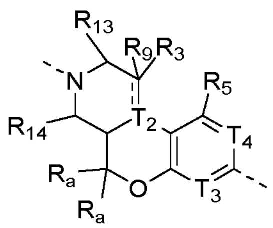 and 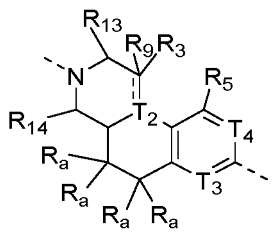;

or, $R_3$ and $R_5$ form a ring, so that the structural moiety

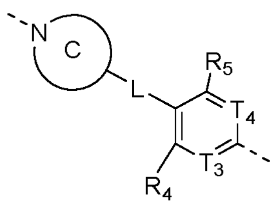 is

selected from

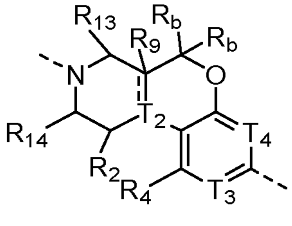 and 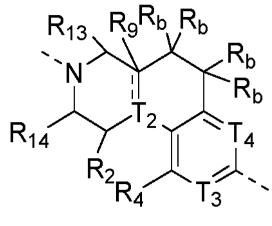;

each $R_a$ is independently selected from H, halogen, $C_{1-3}$ alkyl, $C_{1-3}$ alkoxy, and $C_{3-5}$ cycloalkyl, and the $C_{1-3}$ alkyl, $C_{1-3}$ alkoxy, and $C_{3-5}$ cycloalkyl are optionally substituted by 1, 2, or 3 halogens;

or, two $R_a$ on adjacent atoms, together with the atom to which they are attached, form a double bond or a cyclopropyl group;
each $R_b$ is independently selected from H, halogen, $C_{1-3}$ alkyl, $C_{1-3}$ alkoxy, and $C_{3-5}$ cycloalkyl, and the $C_{1-3}$ alkyl, $C_{1-3}$ alkoxy, and $C_{3-5}$ cycloalkyl are optionally substituted by 1, 2, or 3 halogens;
each $R_c$ is independently selected from H, halogen, $C_{1-3}$ alkyl, $C_{1-3}$ alkoxy, and $C_{3-5}$ cycloalkyl, and the $C_{1-3}$ alkyl, $C_{1-3}$ alkoxy, and $C_{3-5}$ cycloalkyl are optionally substituted by 1, 2, or 3 halogens;
each R is independently selected from halogen and D;
n is selected from 0, 1, 2, 3, and 4;
given that when the structural moiety

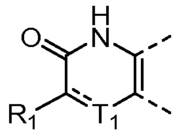

is selected from

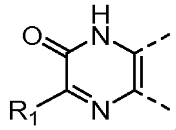,

L is selected from a single bond, and ring C is selected from

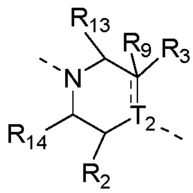,

then $R_2$ and $R_4$ form a ring, or $R_3$ and $R_5$ form a ring;
the halogen represents F, Cl, Br, and I atoms;
"hetero" in the 5- to 6-membered heteroaryl represents 1, 2, 3, or 4 heteroatoms or heteroatom groups, each independently selected from -O-, -S-, and -N-.

The present disclosure further provides a compound of formula (XII) or a pharmaceutically acceptable salt thereof,

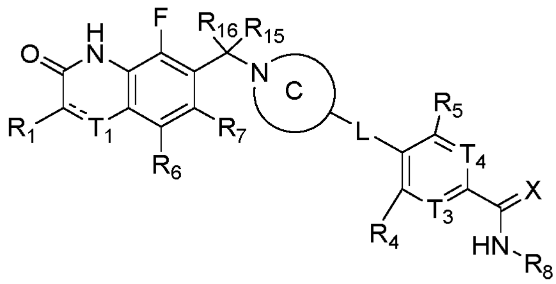

**(XII)** ,

wherein

X is selected from O, S, and N($R_d$);
the structural moiety

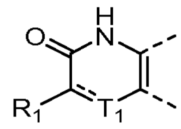

is selected from

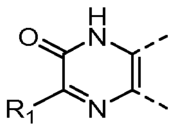, 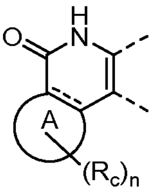,

and

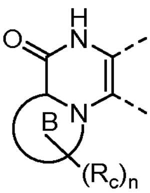;

ring A is selected from phenyl and 5- to 6-membered heteroaryl;
ring B is selected from 5- to 6-membered heteroaryl;
L is selected from a single bond, and ring C is selected from

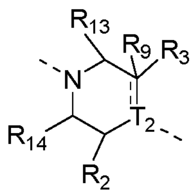;

or, L is selected from $N(R_{12})$, and ring C is selected from

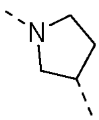;

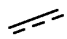

is selected from a single bond or a double bond;
$T_2$ is selected from C, N, and CH;
$T_3$ and $T_4$ are each independently selected from N and $CR_{10}$;
$R_1$ is selected from $C_{1-3}$ alkyl and $C_{3-5}$ cycloalkyl, and the $C_{1-3}$ alkyl and $C_{3-5}$ cycloalkyl are each independently and optionally substituted by 1, 2, or 3 halogens;
$R_2$ is selected from H and $C_{1-3}$ alkyl, and the $C_{1-3}$ alkyl is optionally substituted by 1, 2, or 3 halogens;
$R_3$ is selected from H and halogen;
$R_9$ is absent, or selected from H and halogen;
$R_4$ is selected from H, halogen, CN, $C_{1-3}$ alkyl, and $C_{1-3}$ alkoxy;
$R_5$ is selected from H, halogen, CN, $C_{1-3}$ alkyl, and $C_{1-3}$ alkoxy;
$R_6$ and $R_7$ are each independently selected from H, halogen, $C_{1-3}$ alkyl, and $C_{1-3}$ alkoxy, and the $C_{1-3}$ alkyl and $C_{1-3}$ alkoxy are each independently and optionally substituted by 1, 2, or 3 halogens;
$R_8$ is selected from $C_{1-3}$ alkyl and $C_{3-5}$ cycloalkyl, and the $C_{1-3}$ alkyl and $C_{3-5}$ cycloalkyl are each independently and

optionally substituted by 1, 2, or 3 R;

$R_{10}$ is selected from H and halogen;

$R_{12}$ is selected from H, $C_{1-3}$ alkyl, $C_{1-3}$ alkoxy, and $C_{3-5}$ cycloalkyl, and the $C_{1-3}$ alkyl, $C_{1-3}$ alkoxy, and $C_{3-5}$ cycloalkyl are each independently and optionally substituted by 1, 2, or 3 halogens;

$R_{13}$ and $R_{14}$ are each independently selected from H, halogen, $C_{1-3}$ alkyl, $C_{1-3}$ alkoxy, and $C_{3-5}$ cycloalkyl, and the $C_{1-3}$ alkyl, $C_{1-3}$ alkoxy, and $C_{3-5}$ cycloalkyl are each independently and optionally substituted by 1, 2, or 3 halogens;

$R_{15}$ and $R_{16}$ are each independently selected from H, D, $C_{1-3}$ alkyl, and $C_{1-3}$ alkoxy, and the $C_{1-3}$ alkyl and $C_{1-3}$ alkoxy are each independently and optionally substituted by 1, 2, or 3 halogens;

or, $R_2$ and $R_4$ form a ring, so that the structural moiety

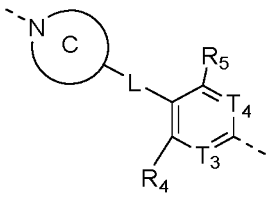

is selected from

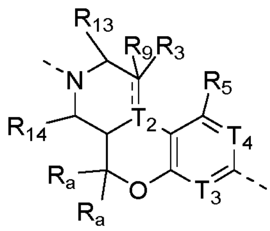

and

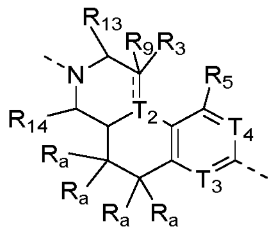

;

or, $R_3$ and $R_5$ form a ring, so that the structural moiety

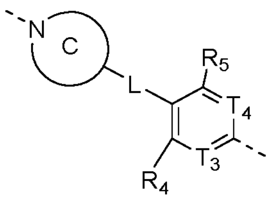

is selected from

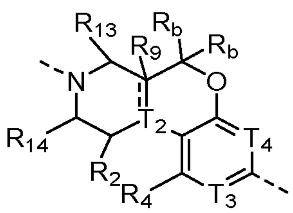

and

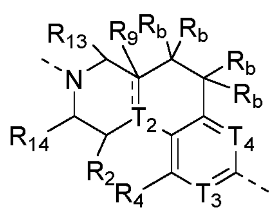

;

each $R_a$ is independently selected from H, halogen, $C_{1-3}$ alkyl, $C_{1-3}$ alkoxy, and $C_{3-5}$ cycloalkyl, and the $C_{1-3}$ alkyl, $C_{1-3}$ alkoxy, and $C_{3-5}$ cycloalkyl are optionally substituted by 1, 2, or 3 halogens;

or, two $R_a$ on adjacent atoms, together with the atom to which they are attached, form a double bond or a cyclopropyl group;

each $R_b$ is independently selected from H, halogen, $C_{1-3}$ alkyl, $C_{1-3}$ alkoxy, and $C_{3-5}$ cycloalkyl, and the $C_{1-3}$ alkyl, $C_{1-3}$ alkoxy, and $C_{3-5}$ cycloalkyl are optionally substituted by 1, 2, or 3 halogens;

each $R_c$ is independently selected from H, halogen, $C_{1-3}$ alkyl, $C_{1-3}$ alkoxy, and $C_{3-5}$ cycloalkyl, and the $C_{1-3}$ alkyl, $C_{1-3}$ alkoxy, and $C_{3-5}$ cycloalkyl are optionally substituted by 1, 2, or 3 halogens;

$R_d$ is selected from $C_{1-3}$ alkoxy, and the $C_{1-3}$ alkoxy is optionally substituted by 1, 2, or 3 halogens;

each R is independently selected from halogen and D;

n is selected from 0, 1, 2, 3, and 4;

given that

1) when the structural moiety

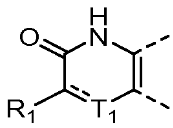

is selected from

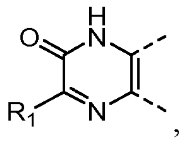

L is selected from a single bond, ring C is selected from

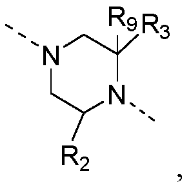

one of $T_3$ or $T_4$ is selected from N, and the other is selected from $CR_{10}$, then $R_2$, $R_3$, and $R_9$ are not simultaneously H; or

2) when the structural moiety

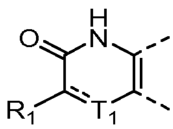

is selected from

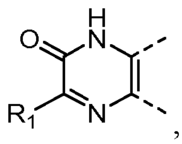

L is selected from a single bond, ring C is selected from

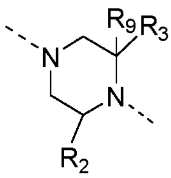

and

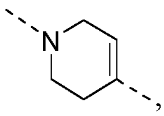

one of $T_3$ or $T_4$ is selected from N, and the other is selected from $CR_{10}$, then $R_6$ and $R_7$ are not simultaneously H; or

3) when the structural moiety

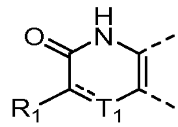

is selected from

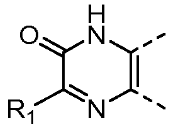
,

L is selected from a single bond, ring C is selected from

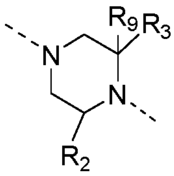
,

$T_3$ is selected from N, and $T_4$ is selected from $CR_{10}$, then $R_5$ is not H; or

4) when the structural moiety

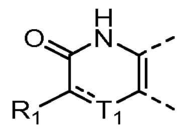

is selected from

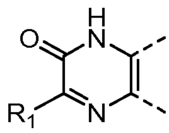
,

L is selected from a single bond, ring C is selected from

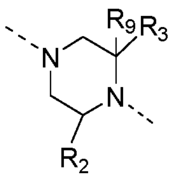
,

$T_4$ is selected from N, and $T_3$ is selected from $CR_{10}$, then $R_4$ is not H; or

5) when the structural moiety

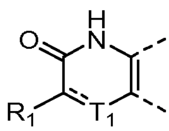

is selected from

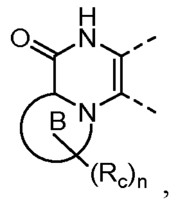

$R_{15}$ is selected from H, $R_{16}$ is selected from H, L is selected from a single bond, ring C is selected from

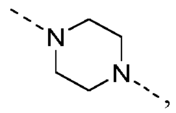

one of $T_3$ or $T_4$ is selected from N, and the other is selected from $CR_{10}$, then ring B is not pyrrolyl or pyrazolyl; or

6) when the structural moiety

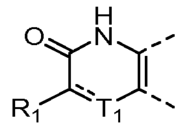

is selected from

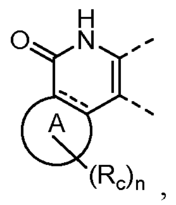

$R_{15}$ is selected from H, $R_{16}$ is selected from H, L is selected from a single bond, ring C is selected from

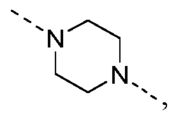

one of $T_3$ or $T_4$ is selected from N, and the other is selected from $CR_{10}$, then ring A is not furanyl.

The present disclosure further provides a compound of formula (XI) or a pharmaceutically acceptable salt thereof,

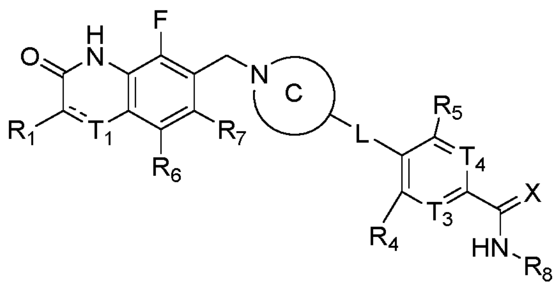

**(XI)**

,

wherein

X is selected from O, S, and $N(R_d)$;
the structural moiety

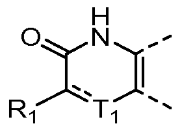

is selected from

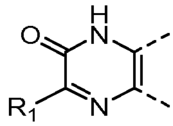
,
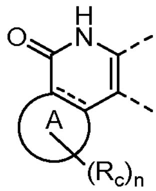
,

and

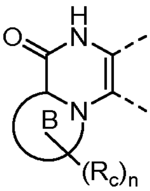
;

ring A is selected from phenyl and 5- to 6-membered heteroaryl;
ring B is selected from 5- to 6-membered heteroaryl;
L is selected from a single bond, and ring C is selected from

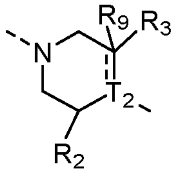
;

or, L is selected from $N(R_{12})$, and ring C is selected from

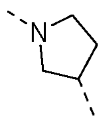
;

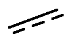

is selected from a single bond or a double bond;
$T_2$ is selected from C, N, and CH;
$T_3$ and $T_4$ are each independently selected from N and $CR_{10}$;
$R_1$ is selected from $C_{1-3}$ alkyl and $C_{3-5}$ cycloalkyl, and the $C_{1-3}$ alkyl and $C_{3-5}$ cycloalkyl are each independently and optionally substituted by 1, 2, or 3 halogens;
$R_2$ is selected from H and $C_{1-3}$ alkyl, and the $C_{1-3}$ alkyl is optionally substituted by 1, 2, or 3 halogens;
$R_3$ is selected from H and halogen;
$R_9$ is absent, or selected from H and halogen;
$R_4$ is selected from H and halogen;
$R_5$ is selected from H and halogen;

$R_6$ and $R_7$ are each independently selected from H and halogen;
$R_8$ is selected from $C_{1-3}$ alkyl and $C_{3-5}$ cycloalkyl, and the $C_{1-3}$ alkyl and $C_{3-5}$ cycloalkyl are each independently and optionally substituted by 1, 2, or 3 R;
$R_{10}$ is selected from H and halogen;
$R_{12}$ is selected from H, $C_{1-3}$ alkyl, $C_{1-3}$ alkoxy, and $C_{3-5}$ cycloalkyl, and the $C_{1-3}$ alkyl, $C_{1-3}$ alkoxy, and $C_{3-5}$ cycloalkyl are each independently and optionally substituted by 1, 2, or 3 halogens;
or, $R_2$ and $R_4$ form a ring, so that the structural moiety

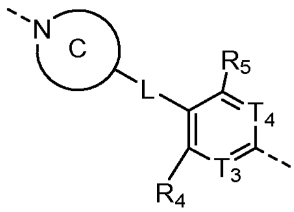

is selected from

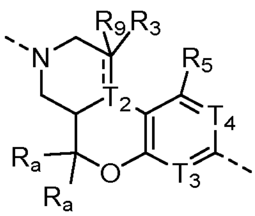
and
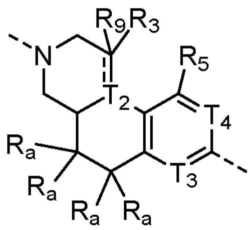
;

or, $R_3$ and $R_5$ form a ring, so that the structural moiety

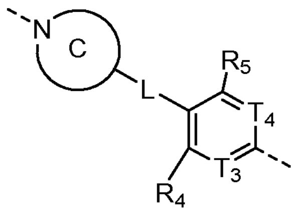

is selected from

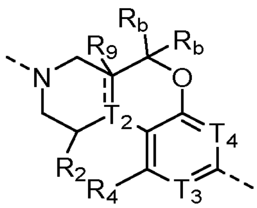
and
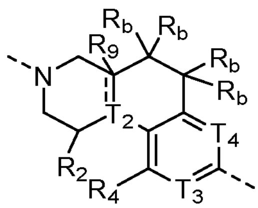
;

each $R_a$ is independently selected from H, halogen, and $C_{1-3}$ alkyl, and the $C_{1-3}$ alkyl is optionally substituted by 1, 2, or 3 halogens;
or, two $R_a$ on adjacent atoms, together with the atom to which they are attached, form a double bond or a cyclopropyl group;
each $R_b$ is independently selected from H, halogen, and $C_{1-3}$ alkyl, and the $C_{1-3}$ alkyl is optionally substituted by 1, 2, or 3 halogens;
each $R_c$ is independently selected from H, halogen, and $C_{1-3}$ alkyl, and the $C_{1-3}$ alkyl is optionally substituted by 1, 2, or 3 halogens;
$R_d$ is selected from $C_{1-3}$ alkoxy, and the $C_{1-3}$ alkoxy is optionally substituted by 1, 2, or 3 halogens;
each R is independently selected from halogen and D;
n is selected from 0, 1, 2, 3, and 4;
given that

1) when the structural moiety

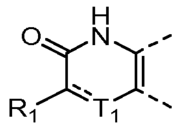

is selected from

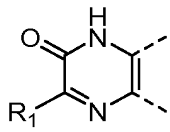,

L is selected from a single bond, ring C is selected from

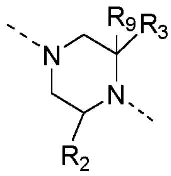,

and one of $T_3$ or $T_4$ is selected from N, then $R_2$, $R_3$, and $R_9$ are not simultaneously H; or
2) when the structural moiety

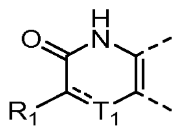

is selected from

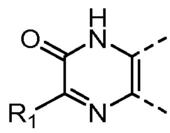,

L is selected from a single bond, ring C is selected from

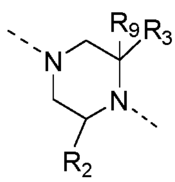 and 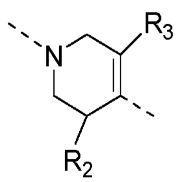,

and one of $T_3$ or $T_4$ is selected from N, then $R_6$ and $R_7$ are not simultaneously H; or
3) when the structural moiety

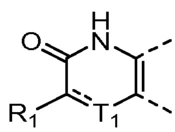

is selected from

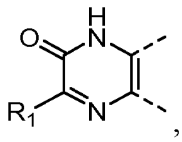

L is selected from a single bond, ring C is selected from

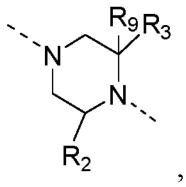

$T_3$ is selected from N, and $T_4$ is selected from $CR_{10}$, then $R_5$ is selected from halogen; or
4) when the structural moiety

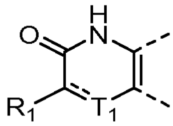

is selected from

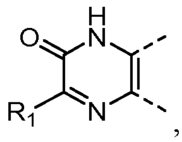

L is selected from a single bond, ring C is selected from

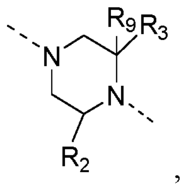

$T_4$ is selected from N, and $T_3$ is selected from $CR_{10}$, then $R_4$ is selected from halogen.

The present disclosure further provides a compound of formula (VIII) or a pharmaceutically acceptable salt thereof,

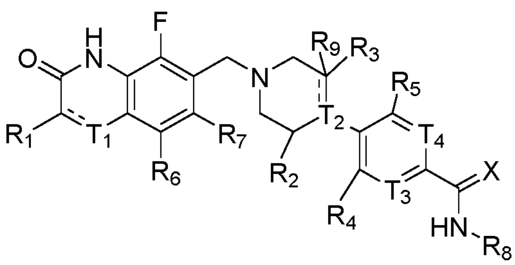

( VIII ) ,

wherein

X is selected from O, S, and $N(R_d)$;

the structural moiety

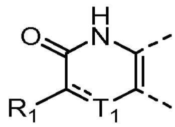

is selected from

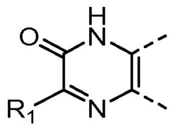, 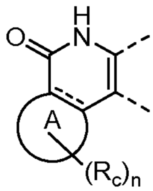,

and

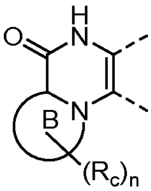;

ring A is selected from phenyl and 5- to 6-membered heteroaryl;
ring B is selected from 5- to 6-membered heteroaryl;

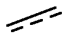

is selected from a single bond or a double bond;
$T_2$ is selected from C, N, and CH;
$T_3$ and $T_4$ are each independently selected from N and $CR_{10}$;
$R_1$ is selected from $C_{1-3}$ alkyl and $C_{3-5}$ cycloalkyl, and the $C_{1-3}$ alkyl and $C_{3-5}$ cycloalkyl are each independently and optionally substituted by 1, 2, or 3 halogens;
$R_2$ is selected from H and $C_{1-3}$ alkyl, and the $C_{1-3}$ alkyl is optionally substituted by 1, 2, or 3 halogens;
$R_3$ is selected from H and halogen;
$R_9$ is absent, or selected from H and halogen;
$R_4$ is selected from H and halogen;
$R_5$ is selected from H and halogen;
$R_6$ and $R_7$ are each independently selected from H and halogen;
$R_8$ is selected from $C_{1-3}$ alkyl and $C_{3-5}$ cycloalkyl, and the $C_{1-3}$ alkyl and $C_{3-5}$ cycloalkyl are each independently and optionally substituted by 1, 2, or 3 R;
$R_{10}$ is selected from H and halogen;
or, $R_2$ and $R_4$ form a ring, so that the structural moiety

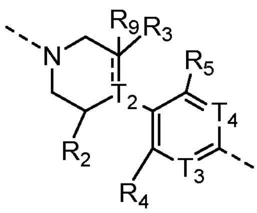

is selected from

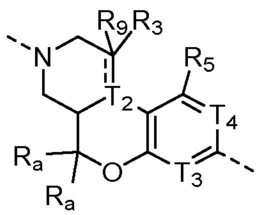
and
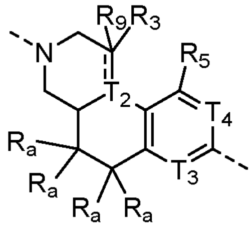
;

or, $R_3$ and $R_5$ form a ring, so that the structural moiety

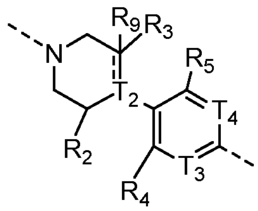

is selected from

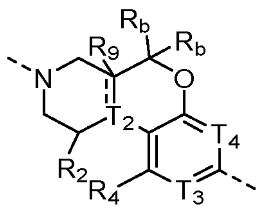
and
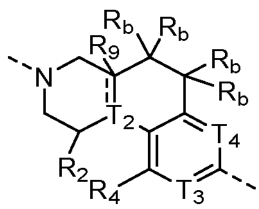
;

each $R_a$ is independently selected from H, halogen, and $C_{1-3}$ alkyl, and the $C_{1-3}$ alkyl is optionally substituted by 1, 2, or 3 halogens;

or, two $R_a$ on adjacent atoms, together with the atom to which they are attached, form a double bond or a cyclopropyl group;

each $R_b$ is independently selected from H, halogen, and $C_{1-3}$ alkyl, and the $C_{1-3}$ alkyl is optionally substituted by 1, 2, or 3 halogens;

each $R_c$ is independently selected from H, halogen, and $C_{1-3}$ alkyl, and the $C_{1-3}$ alkyl is optionally substituted by 1, 2, or 3 halogens;

$R_d$ is selected from $OCH_3$;

each R is independently selected from halogen and D;

n is selected from 0, 1, 2, 3, and 4;

given that

1) when the structural moiety

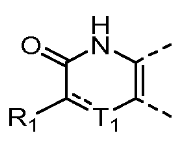

is selected from

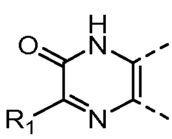
,

the structural moiety

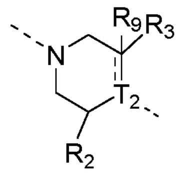

is selected from

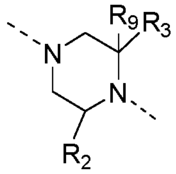

,

and one of $T_3$ or $T_4$ is selected from N, then $R_2$, $R_3$, and $R_9$ are not simultaneously H; or
2) when the structural moiety

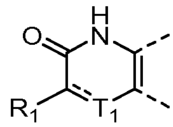

is selected from

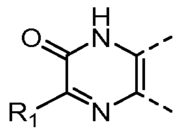

,

the structural moiety

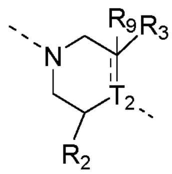

is selected from

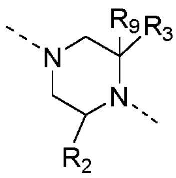

,

and one of $T_3$ or $T_4$ is selected from N, then $R_6$ and $R_7$ are not simultaneously H; or
3) when the structural moiety

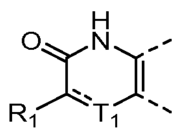

is selected from

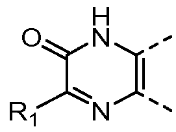,

the structural moiety

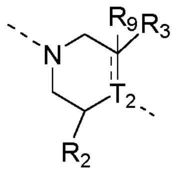

is selected from

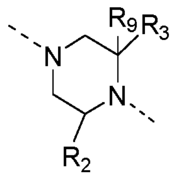,

$T_3$ is selected from N, and $T_4$ is selected from $CR_{10}$, then $R_5$ is selected from halogen; or 4) when the structural moiety

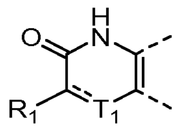

is selected from

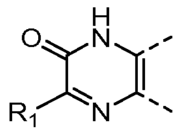,

the structural moiety

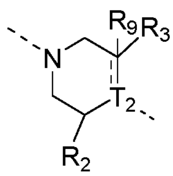

is selected from

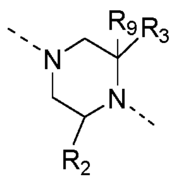,

$T_4$ is selected from N, and $T_3$ is selected from $CR_{10}$, then $R_4$ is selected from halogen; or 5) when the structural moiety

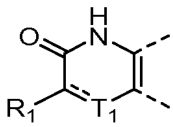

is selected from

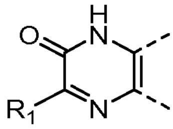

,

the structural moiety

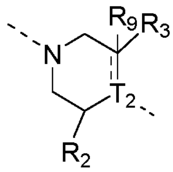

is selected from

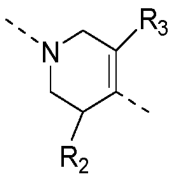

,

and one of $T_3$ or $T_4$ is selected from N, then $R_6$ and $R_7$ are not simultaneously H.

The present disclosure further provides a compound of formula (VIII) or a pharmaceutically acceptable salt thereof,

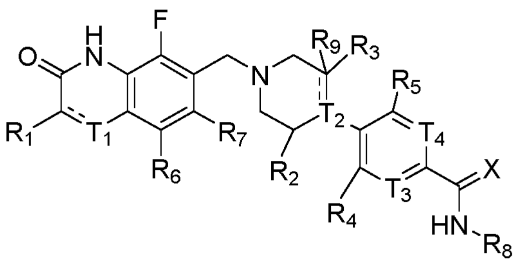

( VIII )

,

wherein

X is selected from O, S, and N($R_d$);
the structural moiety

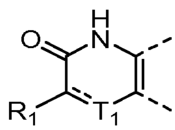

is selected from

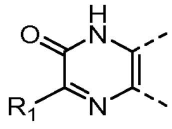, 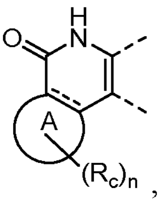,

and

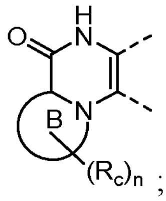;

ring A is selected from phenyl and 5- to 6-membered heteroaryl;
ring B is selected from 5- to 6-membered heteroaryl;

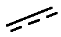

is selected from a single bond or a double bond;
the structural moiety

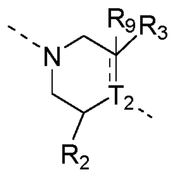

is selected from

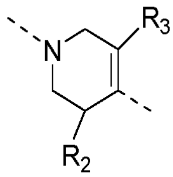, 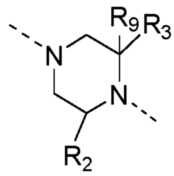,

and

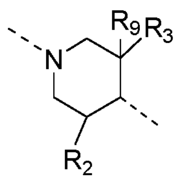;

$T_3$ and $T_4$ are each independently selected from N and $CR_{10}$;
$R_1$ is selected from $C_{1-3}$ alkyl and $C_{3-5}$ cycloalkyl, and the $C_{1-3}$ alkyl and $C_{3-5}$ cycloalkyl are each independently and optionally substituted by 1, 2, or 3 halogens;
$R_2$ is selected from H and $C_{1-3}$ alkyl, and the $C_{1-3}$ alkyl is optionally substituted by 1, 2, or 3 halogens;
$R_3$ and $R_9$ are each independently selected from H and halogen;
$R_4$ is selected from H and halogen;
$R_5$ is selected from H and halogen;
$R_6$ and $R_7$ are each independently selected from H and halogen;

$R_8$ is selected from $C_{1-3}$ alkyl and $C_{3-5}$ cycloalkyl, and the $C_{1-3}$ alkyl and $C_{3-5}$ cycloalkyl are each independently and optionally substituted by 1, 2, or 3 R;
$R_{10}$ is selected from H and halogen;
or, $R_2$ and $R_4$ form a ring, so that the structural moiety

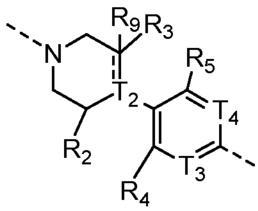

is selected from

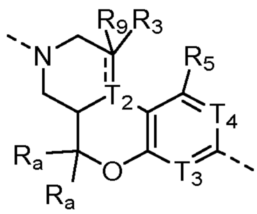
and
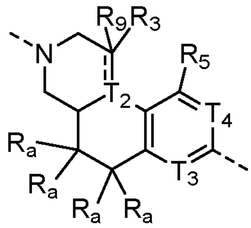
;

or, $R_3$ and $R_5$ form a ring, so that the structural moiety

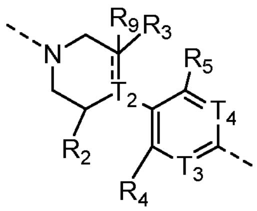

is selected from

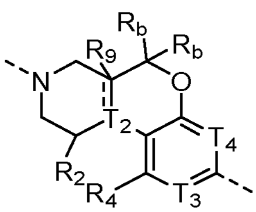
and
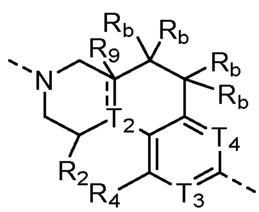
;

$T_2$ is selected from C, N, and CH;
each $R_a$ is independently selected from H, halogen, and $C_{1-3}$ alkyl, and the $C_{1-3}$ alkyl is optionally substituted by 1, 2, or 3 halogens;
or, two $R_a$ on adjacent atoms, together with the atom to which they are attached, form a double bond or a cyclopropyl group;
each $R_b$ is independently selected from H, halogen, and $C_{1-3}$ alkyl, and the $C_{1-3}$ alkyl is optionally substituted by 1, 2, or 3 halogens;
each $R_c$ is independently selected from H, halogen, and $C_{1-3}$ alkyl, and the $C_{1-3}$ alkyl is optionally substituted by 1, 2, or 3 halogens;
$R_d$ is selected from $OCH_3$;
each R is independently selected from halogen and D;
n is selected from 0, 1, 2, 3, and 4;
given that

1) when the structural moiety

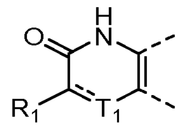

is selected from

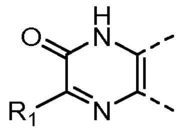
,

the structural moiety

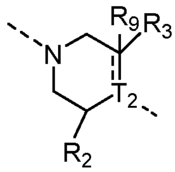

is selected from

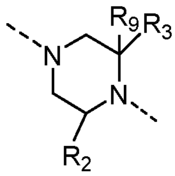
,

and one of $T_3$ or $T_4$ is selected from N, then $R_2$, $R_3$, and $R_9$ are not simultaneously H; or 2) when the structural moiety

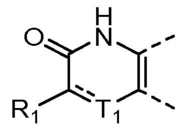

is selected from

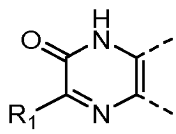
,

the structural moiety

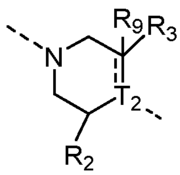

is selected from

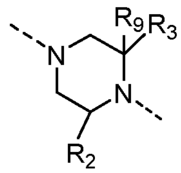

, and one of $T_3$ or $T_4$ is selected from N, then $R_6$ and $R_7$ are not simultaneously H; or
3) when the structural moiety

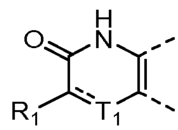

is selected from

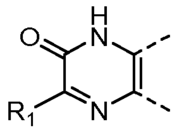

,

the structural moiety

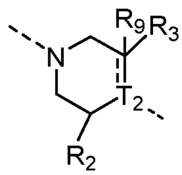

is selected from

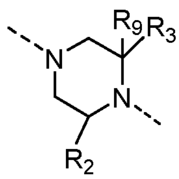

,

$T_3$ is selected from N, and $T_4$ is selected from $CR_{10}$, then $R_5$ is selected from halogen; or
4) when the structural moiety

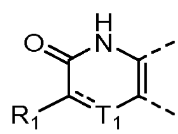

is selected from

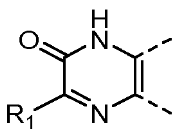

,

the structural moiety

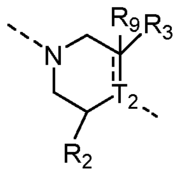

is selected from

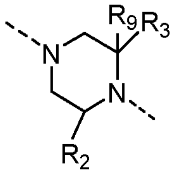

,

$T_4$ is selected from N, and $T_3$ is selected from $CR_{10}$, then $R_4$ is selected from halogen; or
5) when the structural moiety

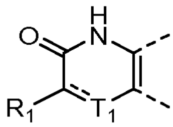

is selected from

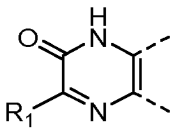

,

the structural moiety

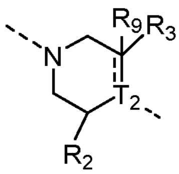

is selected from

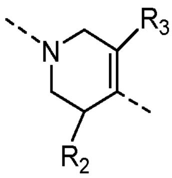

,

and one of $T_3$ or $T_4$ is selected from N, then $R_6$ and $R_7$ are not simultaneously H.

The present disclosure further provides a compound of formula (VII-1) or a pharmaceutically acceptable salt thereof,

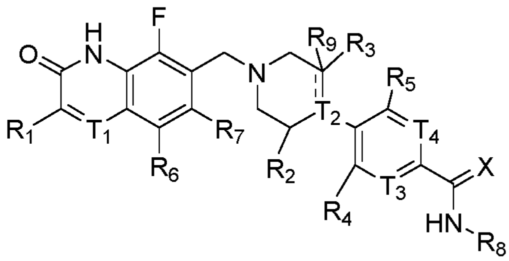

( VII-1 ) ,

wherein

when

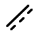

is selected from a double bond, then $T_2$ is selected from C, $R_9$ is absent, and $R_3$ is selected from H and halogen;
when

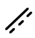

is selected from a single bond, then $T_2$ is selected from N and CH, $R_9$ and $R_3$ are each independently selected from H and halogen;
X is selected from O, S, and $N(R_d)$;
$T_1$ is selected from N;
or, $T_1$ and $R_1$ form a ring, so that the structural moiety

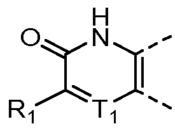

is selected from

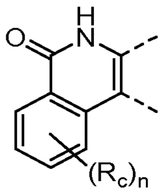 ;

$T_3$ and $T_4$ are each independently selected from N and $CR_{10}$;
$R_1$ is selected from $C_{1-3}$ alkyl and $C_{3-5}$ cycloalkyl, and the $C_{1-3}$ alkyl and $C_{3-5}$ cycloalkyl are each independently and optionally substituted by 1, 2, or 3 halogens;
$R_2$ is selected from H and $C_{1-3}$ alkyl, and the $C_{1-3}$ alkyl is optionally substituted by 1, 2, or 3 halogens;
$R_4$ is selected from H and halogen;
$R_5$ is selected from H and halogen;
$R_6$ and $R_7$ are each independently selected from H and halogen;
$R_8$ is selected from $C_{1-3}$ alkyl and $C_{3-5}$ cycloalkyl, and the $C_{1-3}$ alkyl and $C_{3-5}$ cycloalkyl are each independently and optionally substituted by 1, 2, or 3 R;
$R_{10}$ is selected from H and halogen;
or, $R_2$ and $R_4$ form a ring, so that the structural moiety

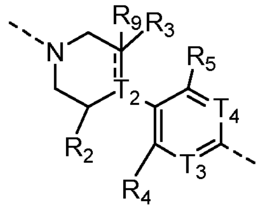

is selected from

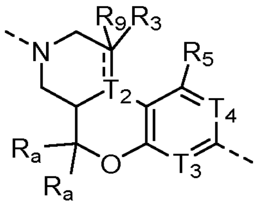

and

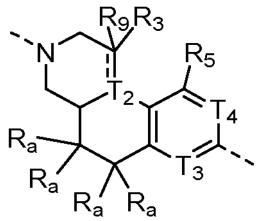

;

or, $R_3$ and $R_5$ form a ring, so that the structural moiety

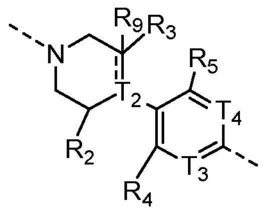

is selected from

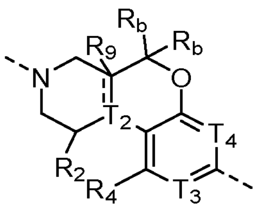

and

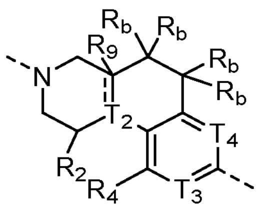

;

each $R_a$ is independently selected from H, halogen, and $C_{1-3}$ alkyl, and the $C_{1-3}$ alkyl is optionally substituted by 1, 2, or 3 halogens;
or, $R_a$ on two adjacent atoms, together with the atom to which they are attached, form a double bond;
each $R_b$ is independently selected from H, halogen, and $C_{1-3}$ alkyl, and the $C_{1-3}$ alkyl is optionally substituted by 1, 2, or 3 halogens;
each $R_c$ is independently selected from H, halogen, and $C_{1-3}$ alkyl, and the $C_{1-3}$ alkyl is optionally substituted by 1, 2, or 3 halogens;
$R_d$ is selected from $OCH_3$;
each R is independently selected from halogen and D;
n is selected from 0, 1, 2, 3, and 4;
given that

1) when $T_1$ is selected from N, $T_2$ is selected from N, and one of $T_3$ or $T_4$ is selected from N, then $R_2$, $R_3$, and $R_9$ are not simultaneously H; or
2) when $T_1$ is selected from N, $T_2$ is selected from N, and one of $T_3$ or $T_4$ is selected from N, then $R_6$ and $R_7$ are not simultaneously H; or
3) when $T_1$ is selected from N, $T_2$ is selected from N, $T_3$ is selected from N, and $T_4$ is selected from $CR_{10}$, then $R_5$ is selected from halogen; or
4) when $T_1$ is selected from N, $T_2$ is selected from N, $T_4$ is selected from N, and $T_3$ is selected from $CR_{10}$, then $R_4$ is selected from halogen; or

5) when $T_1$ is selected from N, the structural moiety

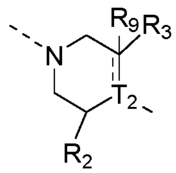

is selected from

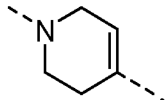
,

and one of $T_3$ or $T_4$ is selected from N, then $R_6$ and $R_7$ are not simultaneously H.

The present disclosure further provides a compound of formula (VI) or a pharmaceutically acceptable salt thereof,

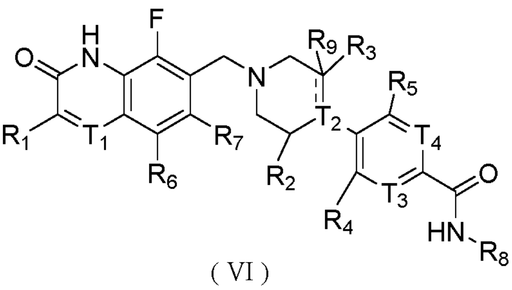

(VI)
,

wherein

when

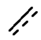

is selected from a double bond, then $T_2$ is selected from C, $R_9$ is absent, and $R_3$ is selected from H and halogen; when

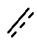

is selected from a single bond, then $T_2$ is selected from N and CH, $R_9$ and $R_3$ are each independently selected from H and halogen;
$T_1$ is selected from N and $CR_{11}$;
$T_3$ and $T_4$ are selected from N and $CR_{10}$;
$R_1$ is selected from $C_{1-3}$ alkyl and $C_{3-5}$ cycloalkyl, and the $C_{1-3}$ alkyl and $C_{3-5}$ cycloalkyl are each independently and optionally substituted by 1, 2, or 3 halogens;
$R_2$ is selected from H and $C_{1-3}$ alkyl, and the $C_{1-3}$ alkyl is optionally substituted by 1, 2, or 3 halogens;
$R_4$ is selected from H and halogen;
$R_5$ is selected from H and halogen;
or, $R_2$ and $R_4$ form a ring, so that the structural moiety

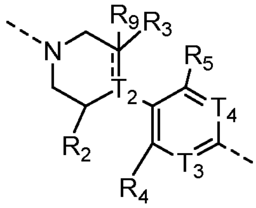

is selected from

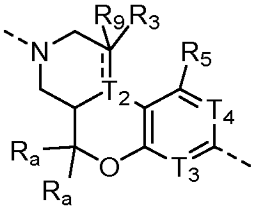 and 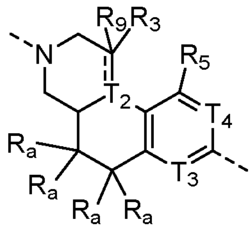 ;

or, $R_3$ and $R_5$ form a ring, so that the structural moiety

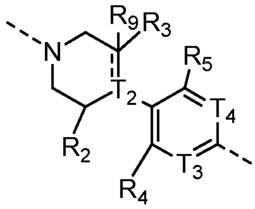

is selected from

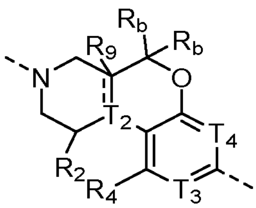 and 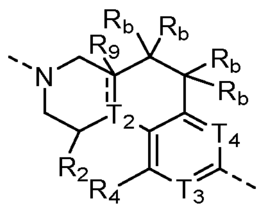 ;

$R_6$ and $R_7$ are each independently selected from H and halogen;
$R_8$ is selected from $C_{1-3}$ alkyl and $C_{3-5}$ cycloalkyl, and the $C_{1-3}$ alkyl and $C_{3-5}$ cycloalkyl are each independently and optionally substituted by 1, 2, or 3 halogens;
$R_{11}$ and $R_1$ form a benzene ring, so that the structural moiety

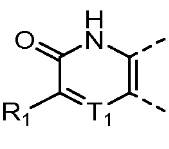

is selected from

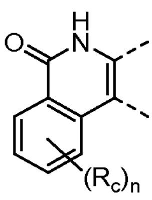 ;

$R_{10}$ is selected from H and halogen;
each $R_a$ is independently selected from H, halogen, and $C_{1-3}$ alkyl, and the $C_{1-3}$ alkyl is optionally substituted by 1, 2, or 3 halogens;
each $R_b$ is independently selected from H, halogen, and $C_{1-3}$ alkyl, and the $C_{1-3}$ alkyl is optionally substituted by 1, 2, or 3 halogens;
each $R_c$ is independently selected from H, halogen, and $C_{1-3}$ alkyl, and the $C_{1-3}$ alkyl is optionally substituted by 1, 2, or 3 halogens;
n is selected from 0, 1, 2, 3, and 4;

given that when $T_1$ is selected from N, $T_2$ is selected from N, and $T_3$ is selected from N, then

1) $R_2$, $R_3$, and $R_9$ are not simultaneously H; or
2) $R_6$ and $R_7$ are not simultaneously H; or
3) $R_5$ is selected from halogen.

The present disclosure further provides a compound of formula (I) or a pharmaceutically acceptable salt thereof,

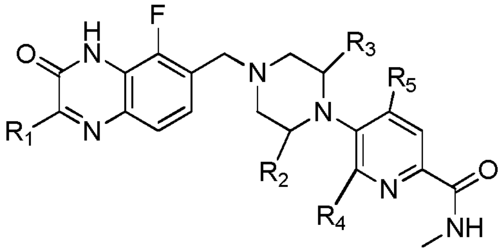

(I)

,

wherein

$R_1$ is selected from $C_{1-3}$ alkyl and $C_{3-5}$ cycloalkyl, and the $C_{1-3}$ alkyl and $C_{3-5}$ cycloalkyl are each independently and optionally substituted by 1, 2, or 3 halogens;
$R_2$ is selected from $C_{1-3}$ alkyl, and the $C_{1-3}$ alkyl is optionally substituted by 1, 2, or 3 halogens;
$R_3$ is selected from H;
$R_4$ is selected from halogen;
$R_5$ is selected from H;
or, $R_2$ and $R_4$ form a ring, so that the structural moiety

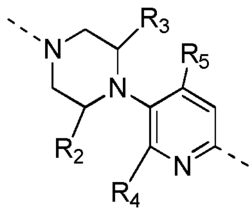

is selected from

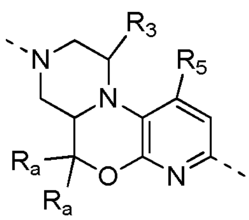

and

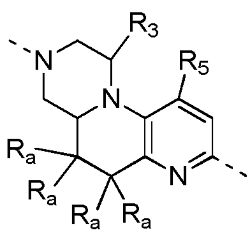

;

or, $R_3$ and $R_5$ form a ring, so that the structural moiety

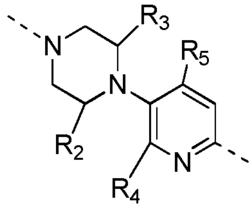

is selected from

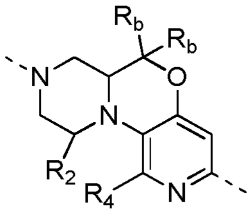 and 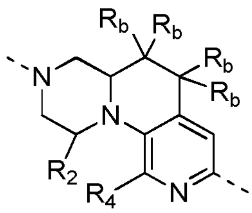 ;

each $R_a$ is independently selected from H, halogen, and $C_{1-3}$ alkyl, and the $C_{1-3}$ alkyl is optionally substituted by 1, 2, or 3 halogens;
each $R_b$ is independently selected from H, halogen, and $C_{1-3}$ alkyl, and the $C_{1-3}$ alkyl is optionally substituted by 1, 2, or 3 halogens.

The present disclosure further provides a compound of formula (VII-2) or a pharmaceutically acceptable salt thereof,

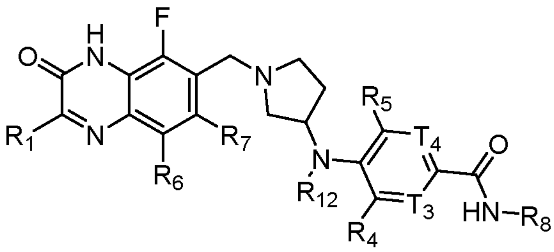

(VII-2)

,

wherein

$T_3$ and $T_4$ are each independently selected from N and $CR_{10}$;
$R_1$ is selected from $C_{1-3}$ alkyl and $C_{3-5}$ cycloalkyl, and the $C_{1-3}$ alkyl and $C_{3-5}$ cycloalkyl are each independently and optionally substituted by 1, 2, or 3 halogens;
$R_4$ is selected from H and halogen;
$R_5$ is selected from H and halogen;
$R_6$ and $R_7$ are each independently selected from H and halogen;
$R_8$ is selected from $C_{1-3}$ alkyl and $C_{3-5}$ cycloalkyl, and the $C_{1-3}$ alkyl and $C_{3-5}$ cycloalkyl are each independently and optionally substituted by 1, 2, or 3 R;
$R_{10}$ is selected from H and halogen;
$R_{12}$ is selected from H and $C_{1-3}$ alkyl;
each R is independently selected from halogen and D.

The present disclosure further provides a compound of formula (I) or a pharmaceutically acceptable salt

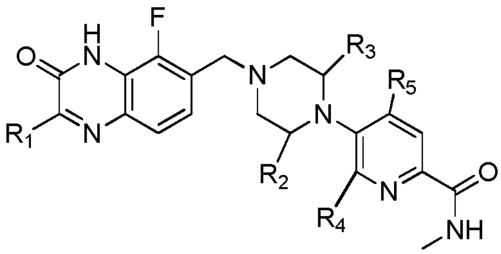

(I)

,

wherein

$R_1$ is selected from $C_{1-3}$ alkyl and $C_{3-5}$ cycloalkyl, and the $C_{1-3}$ alkyl and $C_{3-5}$ cycloalkyl are each independently and optionally substituted by 1, 2, or 3 halogens;
$R_2$ is selected from $C_{1-3}$ alkyl, and the $C_{1-3}$ alkyl is optionally substituted by 1, 2, or 3 halogens;
$R_3$ is selected from H;
$R_4$ is selected from halogen;
$R_5$ is selected from H;
or, $R_2$ and $R_4$ form a ring, so that the structural moiety

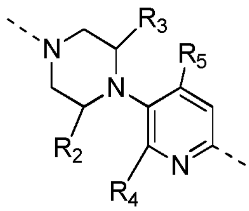

is selected from

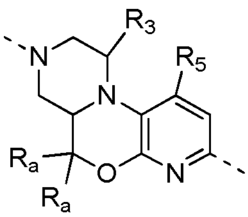

and

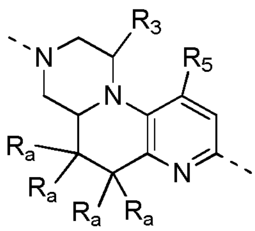

;

or, $R_3$ and $R_5$ form a ring, so that the structural moiety

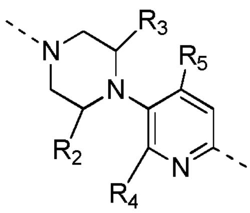

is selected from

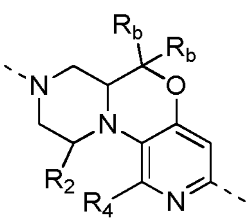

and

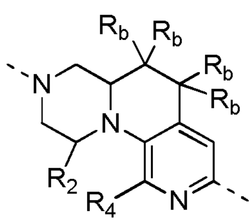

;

each $R_a$ is independently selected from H, halogen, and $C_{1-3}$ alkyl, and the $C_{1-3}$ alkyl is optionally substituted by 1, 2, or 3 halogens;
each $R_b$ is independently selected from H, halogen, and $C_{1-3}$ alkyl, and the $C_{1-3}$ alkyl is optionally substituted by 1, 2, or 3 halogens.

The present disclosure further provides a compound of formula (I) or a pharmaceutically acceptable salt thereof,

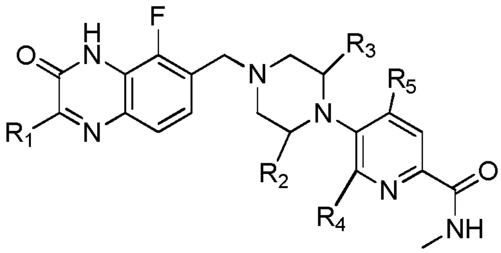

(I)

,

wherein

$R_1$ is selected from $C_{1-3}$ alkyl and $C_{3-5}$ cycloalkyl, and the $C_{1-3}$ alkyl and $C_{3-5}$ cycloalkyl are each independently and optionally substituted by 1, 2, or 3 halogens;
$R_2$ is selected from $C_{1-3}$ alkyl, and the $C_{1-3}$ alkyl is optionally substituted by 1, 2, or 3 halogens;
$R_3$ is selected from H;
$R_4$ is selected from halogen;
$R_5$ is selected from H;
or, $R_2$ and $R_4$ form a ring, so that the structural moiety

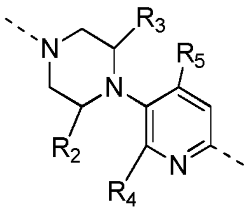

is selected from

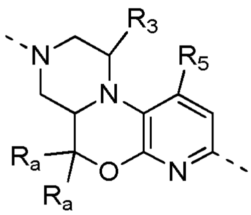

;

or, $R_3$ and $R_5$ form a ring, so that the structural moiety

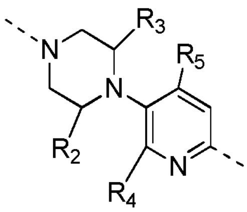

is selected from

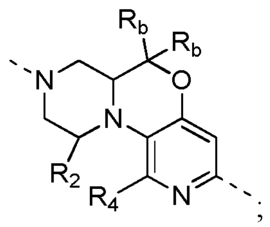

each $R_a$ is independently selected from H, halogen, and $C_{1-3}$ alkyl, and the $C_{1-3}$ alkyl is optionally substituted by 1, 2, or 3 halogens;
each $R_b$ is independently selected from H, halogen, and $C_{1-3}$ alkyl, and the $C_{1-3}$ alkyl is optionally substituted by 1, 2, or 3 halogens.

In some embodiments of the present disclosure, the X is selected from O, S, and $N(OCH_3)$, and other variables are as defined in the present disclosure.
In some embodiments of the present disclosure, the X is selected from O and S, and other variables are as defined in the present disclosure.
In some embodiments of the present disclosure, the X is selected from O, and other variables are as defined in the present disclosure.
In some embodiments of the present disclosure, each $R_a$ is independently selected from H, F, Cl, Br, I, $CH_3$, $CH_2CH_3$, and $CH_2CH_2CH_3$, and the $CH_3$, $CH_2CH_3$, and $CH_2CH_2CH_3$ are optionally substituted by 1, 2, or 3 halogens, and other variables are as defined in the present disclosure.
In some embodiments of the present disclosure, each $R_a$ is independently selected from H, F and $CH_3$, and other variables are as defined in the present disclosure.
In some embodiments of the present disclosure, each $R_a$ is independently selected from H and $CH_3$, and other variables are as defined in the present disclosure.
In some embodiments of the present disclosure, two $R_a$ on adjacent atoms, together with the atom to which they are attached, form a double bond or a cyclopropyl group, so that the structural moiety

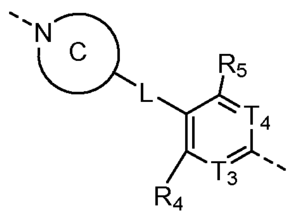

is selected from

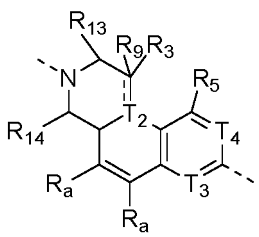

and

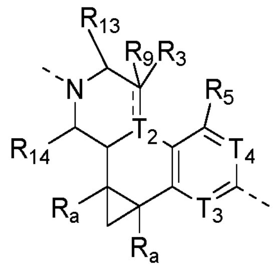

,

and other variables are as defined in the present disclosure.

In some embodiments of the present disclosure, two $R_a$ on adjacent atoms, together with the atom to which they are attached, form a double bond or a cyclopropyl group, so that the structural moiety

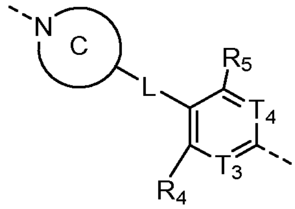

is selected from

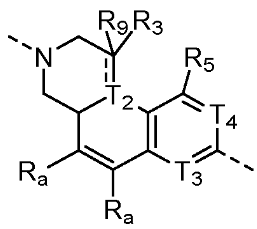

and

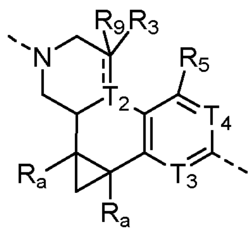

,

and other variables are as defined in the present disclosure.

In some embodiments of the present disclosure, two $R_a$ on adjacent atoms, together with the atom to which they are attached, form a double bond or a cyclopropyl group, so that the structural moiety

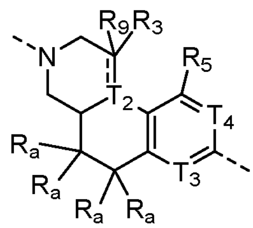

is selected from

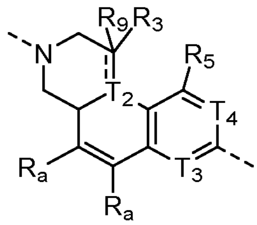

and

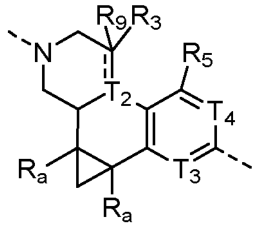

,

and other variables are as defined in the present disclosure.

In some embodiments of the present disclosure, $R_a$ on the two adjacent atoms, together with the neighboring atom, form a double bond, so that the structural moiety

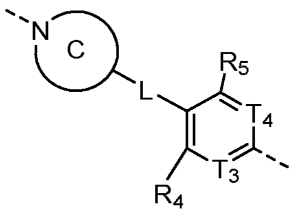

is selected from

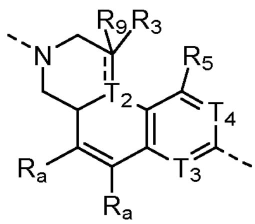

,

and other variables are as defined in the present disclosure.

In some embodiments of the present disclosure, $R_a$ on the two adjacent atoms, together with the neighboring atom, form a double bond, so that the structural moiety

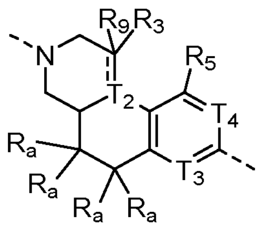

is selected from

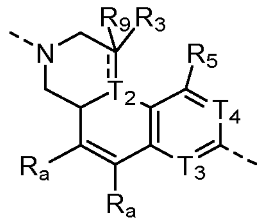

and other variables are as defined in the present disclosure.

In some embodiments of the present disclosure, each $R_b$ is independently selected from H, F, Cl, Br, I, $CH_3$, $CH_2CH_3$, and $CH_2CH_2CH_3$, and the $CH_3$, $CH_2CH_3$, and $CH_2CH_2CH_3$ are optionally substituted by 1, 2, or 3 halogens, and other variables are as defined in the present disclosure.

In some embodiments of the present disclosure, each $R_b$ is independently selected from H, F, and $CH_3$, and other variables are as defined in the present disclosure.

In some embodiments of the present disclosure, each $R_b$ is independently selected from H and $CH_3$, and other variables are as defined in the present disclosure.

In some embodiments of the present disclosure, each $R_c$ is independently selected from H, F, Cl, Br, I, $CH_3$, $CH_2CH_3$, and $CH_2CH_2CH_3$, and the $CH_3$, $CH_2CH_3$, and $CH_2CH_2CH_3$ are optionally substituted by 1, 2, or 3 halogens, and other variables are as defined in the present disclosure.

In some embodiments of the present disclosure, each $R_c$ is independently selected from H, F, and $CH_3$, and other variables are as defined in the present disclosure.

In some embodiments of the present disclosure, each $R_c$ is independently selected from H and F, and other variables are as defined in the present disclosure.

In some embodiments of the present disclosure, the $R_d$ is selected from $OCH_3$, and other variables are as defined in the present disclosure.

In some embodiments of the present disclosure, the $T_3$ is selected from N, CH, and CF, and other variables are as defined in the present disclosure.

In some embodiments of the present disclosure, the $T_3$ is selected from N and CF, and other variables are as defined in the present disclosure.

In some embodiments of the present disclosure, the $T_3$ is selected from N, and other variables are as defined in the present disclosure.

In some embodiments of the present disclosure, the $T_4$ is selected from N and CH, and other variables are as defined in the present disclosure.

In some embodiments of the present disclosure, the $R_1$ is selected from $CH_3$ and $CH_2CH_3$, and other variables are as defined in the present disclosure.

In some embodiments of the present disclosure, the $R_1$ is selected from $CH_3$, and other variables are as defined in the present disclosure.

In some embodiments of the present disclosure, the $R_2$ is selected from H and $CH_3$, and other variables are as defined in the present disclosure.

In some embodiments of the present disclosure, the $R_2$ is selected from $CH_3$, and other variables are as defined in the present disclosure.

In some embodiments of the present disclosure, the $R_4$, $R_5$, $R_6$, and $R_7$ are each independently selected from H, F, and $CH_3$, and other variables are as defined in the present disclosure.

In some embodiments of the present disclosure, the $R_3$ is selected from H and F, and other variables are as defined in the present disclosure.

In some embodiments of the present disclosure, the $R_4$ is selected from H, F, Cl, CN, and $CH_3$, and other variables are as defined in the present disclosure.

In some embodiments of the present disclosure, the $R_4$ is selected from H, F, Cl, and CN, and other variables are as defined in the present disclosure.

In some embodiments of the present disclosure, the $R_4$ is selected from H, F, and $CH_3$, and other variables are as defined in the present disclosure.

In some embodiments of the present disclosure, the $R_4$ is selected from H and F, and other variables are as defined in the present disclosure.

In some embodiments of the present disclosure, the $R_4$ is selected from F, and other variables are as defined in the present disclosure.

In some embodiments of the present disclosure, the $R_5$ is selected from H, F, Cl, and CN, and other variables are as defined in the present disclosure.

In some embodiments of the present disclosure, the $R_5$ is selected from H, F, CN, and $CH_3$, and other variables

are as defined in the present disclosure.

In some embodiments of the present disclosure, the $R_5$ is selected from H, F, and $CH_3$, and other variables are as defined in the present disclosure.

In some embodiments of the present disclosure, the $R_5$ is selected from H and F, and other variables are as defined in the present disclosure.

In some embodiments of the present disclosure, the $R_5$ is selected from H, and other variables are as defined in the present disclosure.

In some embodiments of the present disclosure, the $R_6$ and $R_7$ are each independently selected from H, F, and $CH_3$, and other variables are as defined in the present disclosure.

In some embodiments of the present disclosure, the $R_6$ is selected from H, F, and $CH_3$, and other variables are as defined in the present disclosure.

In some embodiments of the present disclosure, the $R_7$ is selected from H, F, and $CH_3$, and other variables are as defined in the present disclosure

In some embodiments of the present disclosure, the $R_6$ is selected from H and F, and other variables are as defined in the present disclosure.

In some embodiments of the present disclosure, the $R_7$ is selected from H and F, and other variables are as defined in the present disclosure.

In some embodiments of the present disclosure, the $R_8$ is selected from $CH_3$, $CH_2CH_3$, $CH_2CF_3$, cyclopropyl, and $CD_3$, and other variables are as defined in the present disclosure.

In some embodiments of the present disclosure, the $R_8$ is selected from $CH_3$, $CH_2CF_3$, cyclopropyl, and $CD_3$, and other variables are as defined in the present disclosure.

In some embodiments of the present disclosure, the $R_8$ is selected from $CH_3$, cyclopropyl, and $CD_3$, and other variables are as defined in the present disclosure.

In some embodiments of the present disclosure, the $R_8$ is selected from $CH_3$ and cyclopropyl, and other variables are as defined in the present disclosure.

In some embodiments of the present disclosure, the $R_9$ is selected from H and F, and other variables are as defined in the present disclosure.

In some embodiments of the present disclosure, the $R_{10}$ is selected from H and F, and other variables are as defined in the present disclosure.

In some embodiments of the present disclosure, the $R_{12}$ is selected from H and $CH_3$, and other variables are as defined in the present disclosure.

In some embodiments of the present disclosure, the $R_{13}$ is selected from H and $CH_3$, and other variables are as defined in the present disclosure.

In some embodiments of the present disclosure, the $R_{14}$ is selected from H and $CH_3$, and other variables are as defined in the present disclosure.

In some embodiments of the present disclosure, the $R_{15}$ is selected from H, D, and $CH_3$, and other variables are as defined in the present disclosure

In some embodiments of the present disclosure, the $R_{16}$ is selected from H, D, and $CH_3$, and other variables are as defined in the present disclosure

In some embodiments of the present disclosure, ring A is selected from phenyl and 6-membered heteroaryl ring, and other variables are as defined in the present disclosure.

In some embodiments of the present disclosure, ring A is selected from phenyl, pyrrolyl, pyrazolyl, imidazolyl, triazolyl, furanyl, thienyl, pyridyl, pyrazinyl, and pyrimidinyl, and other variables are as defined in the present disclosure.

In some embodiments of the present disclosure, ring A is selected from phenyl, pyridyl, pyrazinyl, and pyrimidinyl, and other variables are as defined in the present disclosure.

In some embodiments of the present disclosure, ring B is selected from 6-membered heteroaryl ring, and other variables are as defined in the present disclosure.

In some embodiments of the present disclosure, ring B is selected from pyrrolyl, pyrazolyl, imidazolyl, triazolyl, furanyl, thienyl, pyridyl, pyrazinyl, and pyrimidinyl, and other variables are as defined in the present disclosure.

In some embodiments of the present disclosure, ring B is selected from pyridyl, pyrazinyl, and pyrimidinyl, and other variables are as defined in the present disclosure.

In some embodiments of the present disclosure, the structural moiety

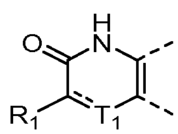

is selected from

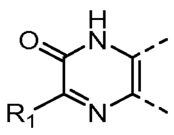, 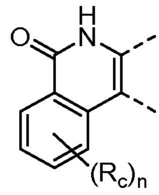, 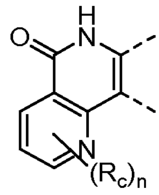, 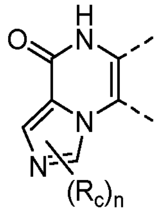, 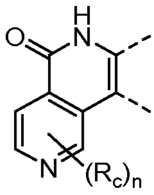,

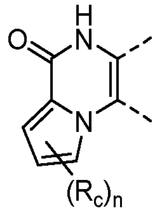, and 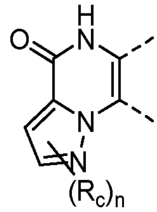,

and other variables are as defined in the present disclosure.

In some embodiments of the present disclosure, the structural moiety

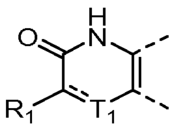

is selected from

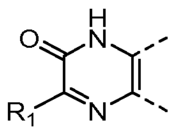, 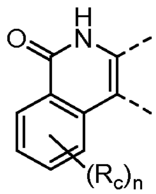, 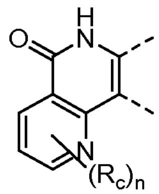, and 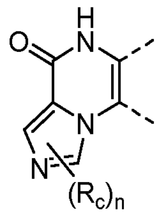,

and other variables are as defined in the present disclosure.

In some embodiments of the present disclosure, the structural moiety

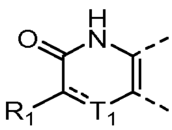

is selected from

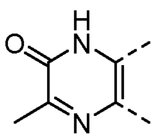, 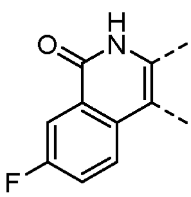, 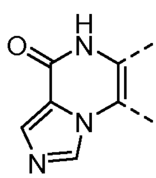, 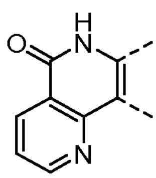, 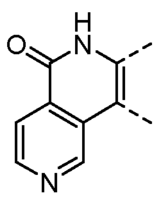,

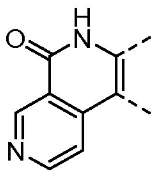, 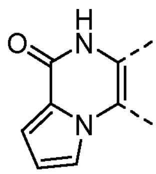, 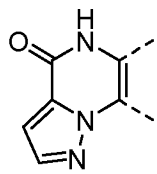, and 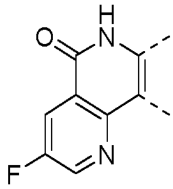,

and other variables are as defined in the present disclosure.

In some embodiments of the present disclosure, the structural moiety

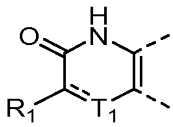

is selected from

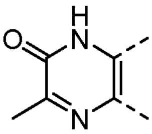, 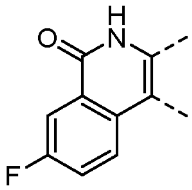, 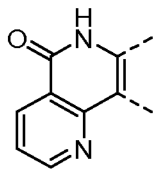, 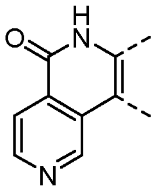, 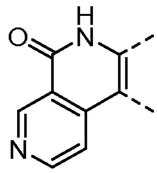,

and

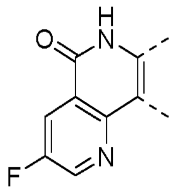,

and other variables are as defined in the present disclosure.

In some embodiments of the present disclosure, the structural moiety

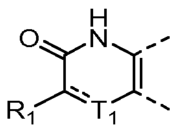

is selected from

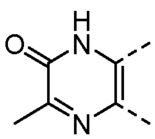, 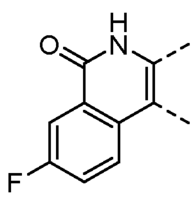, 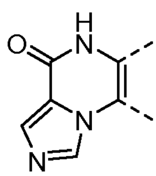, 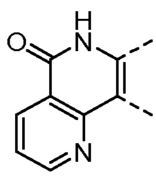, 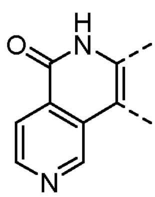,

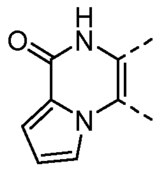 , and 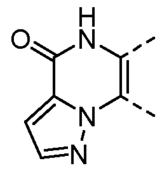 ,

and other variables are as defined in the present disclosure.

In some embodiments of the present disclosure, the structural moiety

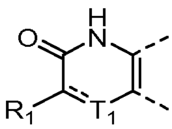

is selected from

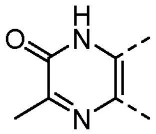 , 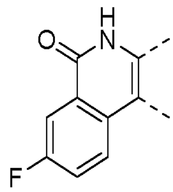 , 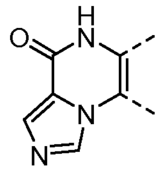 , and 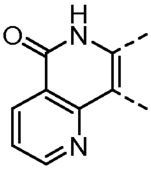 ,

and other variables are as defined in the present disclosure.

In some embodiments of the present disclosure, $R_{11}$ and $R_1$ form a benzene ring, so that the structural moiety

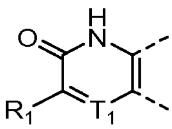

is selected from

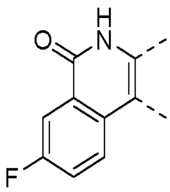 ,

and other variables are as defined in the present disclosure. In some embodiments of the present disclosure, the structural moiety

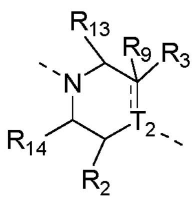

is selected from

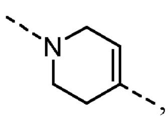 , 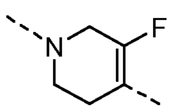 , 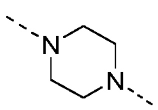 ,

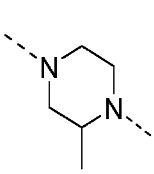, 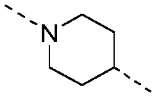, 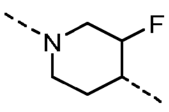, 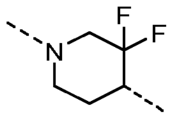, 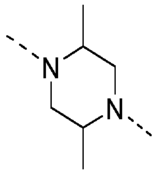, and 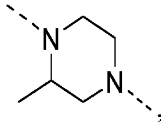;

and other variables are as defined in the present disclosure.

In some embodiments of the present disclosure, the structural moiety

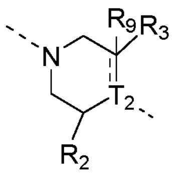

is selected from

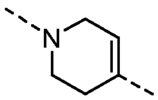, 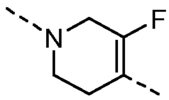, 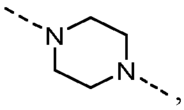, 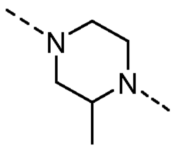, 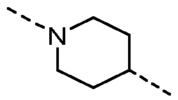,

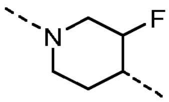, and 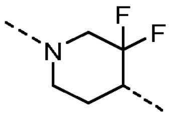,

and other variables are as defined in the present disclosure.

In some embodiments of the present disclosure, the structural moiety

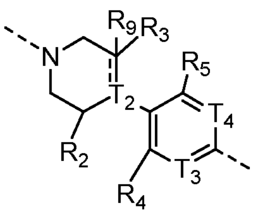

is selected from

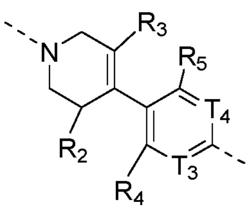, 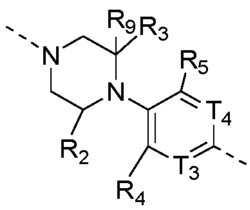,

and

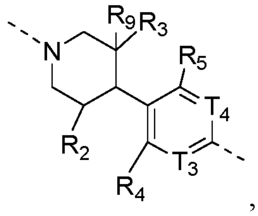

,

and other variables are as defined in the present disclosure.

In some embodiments of the present disclosure, the $R_2$ and $R_4$ form a ring, so that the structural moiety

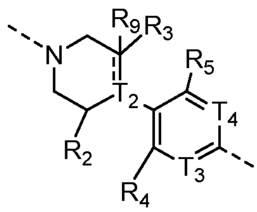

is selected from

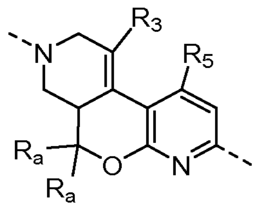

,

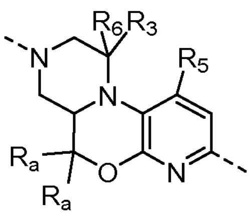

,

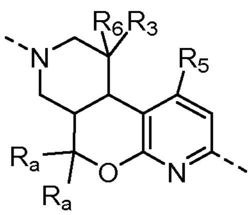

,

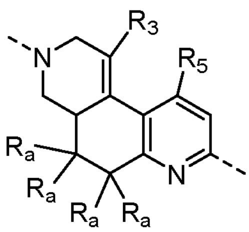

,

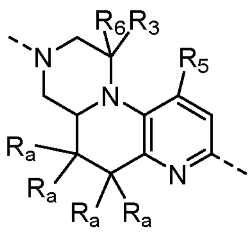

,

and

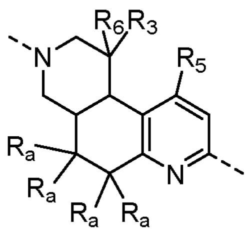

,

and other variables are as defined in the present disclosure.

In some embodiments of the present disclosure, the $R_3$ and $R_5$ form a ring, so that the structural moiety

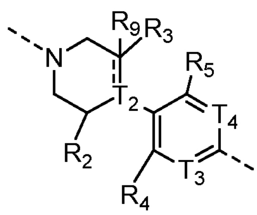

is selected from

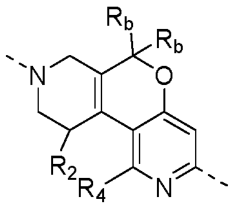, 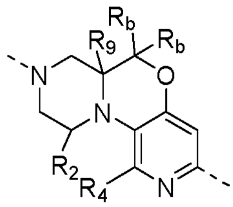,

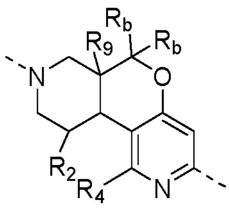, 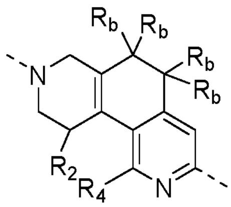, 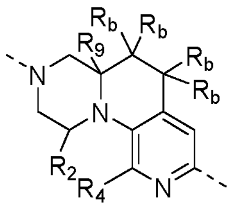, and 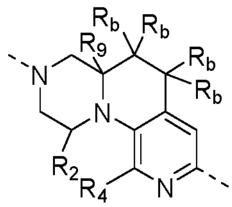,

and other variables are as defined in the present disclosure.

In some embodiments of the present disclosure, the $R_3$ and $R_5$ form a ring, so that the structural moiety

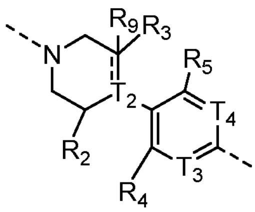

is selected from

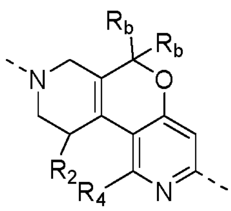, 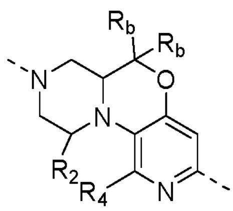,

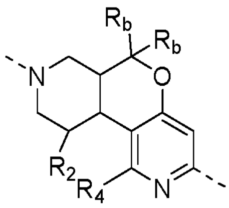, 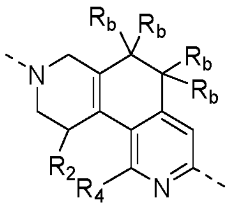, 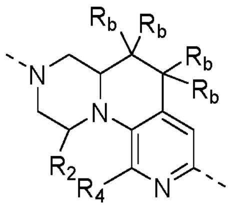, and 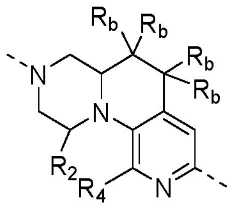,

and other variables are as defined in the present disclosure.

In some embodiments of the present disclosure, the $R_2$ and $R_4$ form a ring, so that the structural moiety

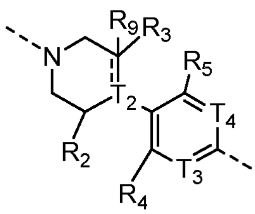

is selected from

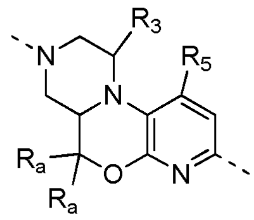

and

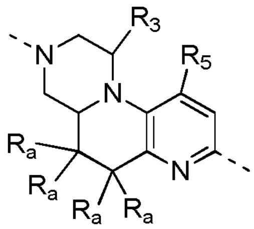

,

and other variables are as defined in the present disclosure.

In some embodiments of the present disclosure, the $R_3$ and $R_5$ form a ring, so that the structural moiety

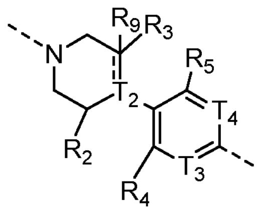

is selected from

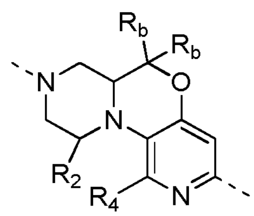

and

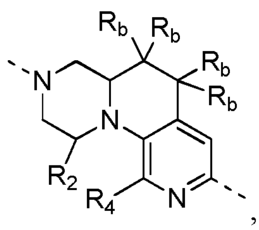

,

and other variables are as defined in the present disclosure.

In some embodiments of the present disclosure, the structural moiety

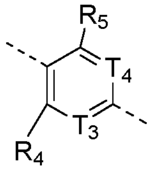

is selected from

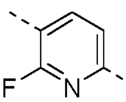, 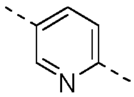, 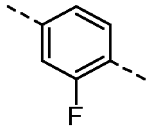, 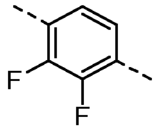, 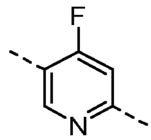, 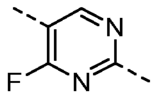,

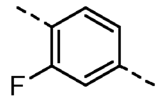, 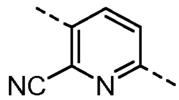, and 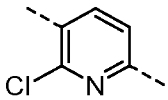,

and other variables are as defined in the present disclosure.

In some embodiments of the present disclosure, the $R_2$ and $R_4$ form a ring, so that the structural moiety

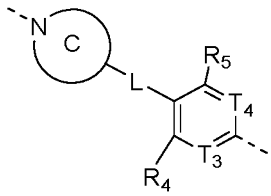

is selected from

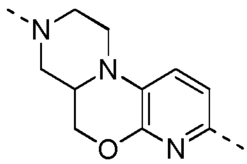,

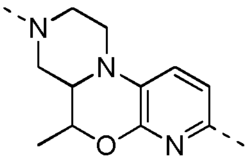, 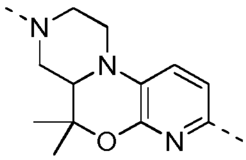, 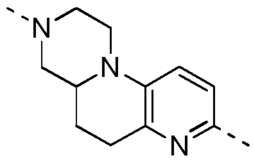, 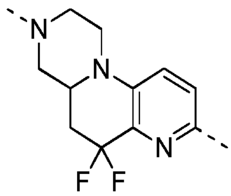,

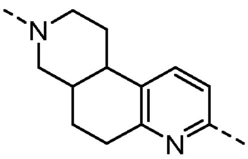, 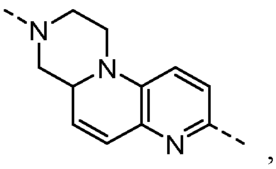, 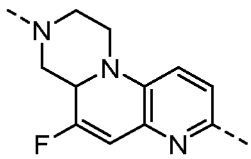, 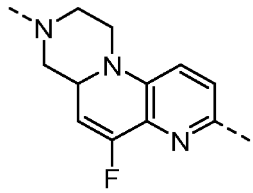,

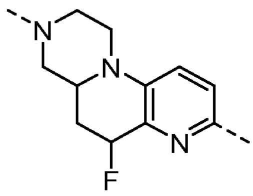, and 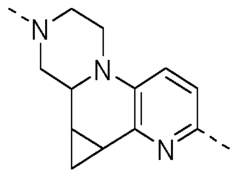,

and other variables are as defined in the present disclosure.

In some embodiments of the present disclosure, the $R_3$ and $R_5$ form a ring, so that the structural moiety

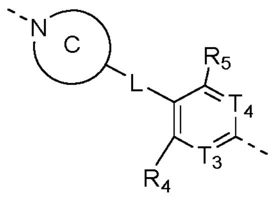

is selected from

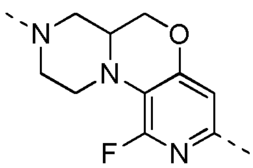,

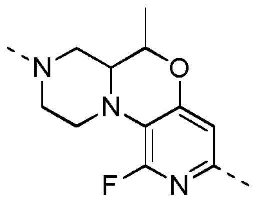, and 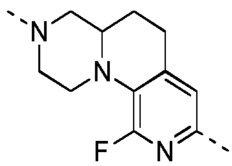,

and other variables are as defined in the present disclosure.

In some embodiments of the present disclosure, the L is selected from a single bond, the structural moiety

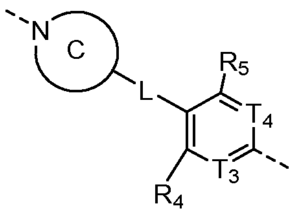

is selected from

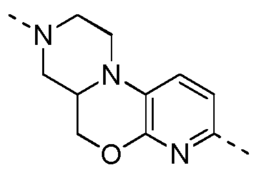,

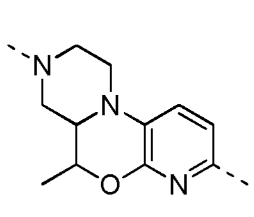 , 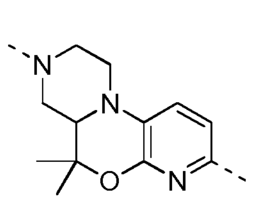 , 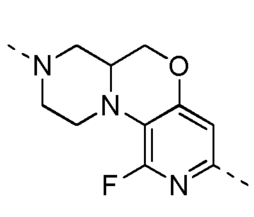 , 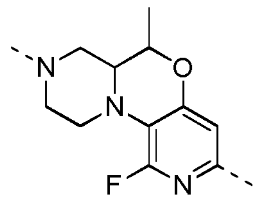 ,

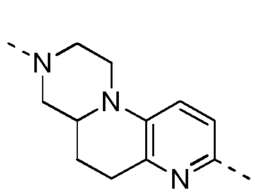 , 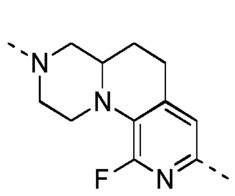 , 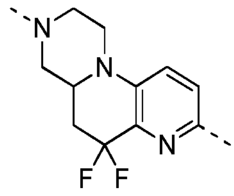 , 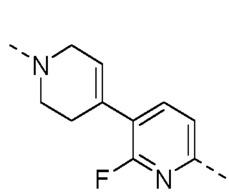 ,

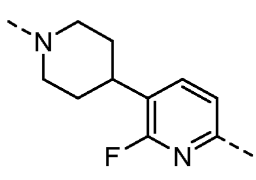 , 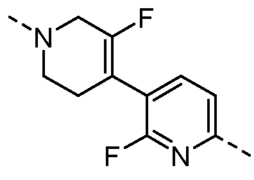 , 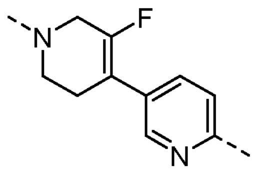 , 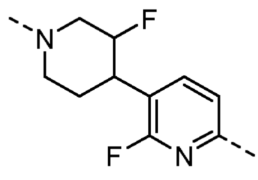 ,

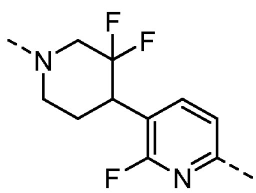 , 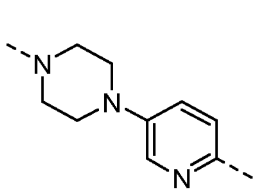 , 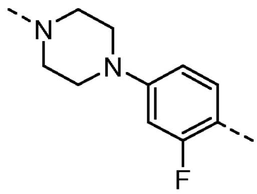 , 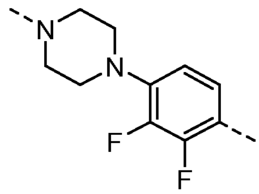 ,

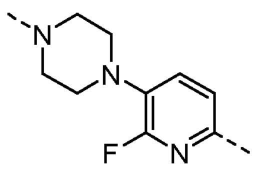 , 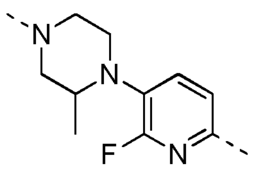 , 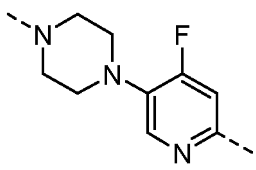 , 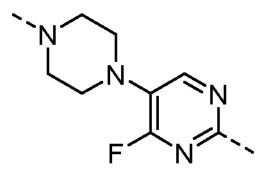 ,

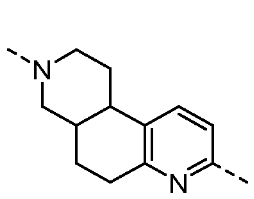 , 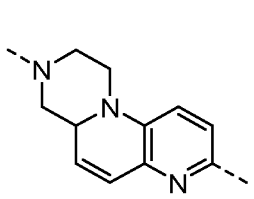 , 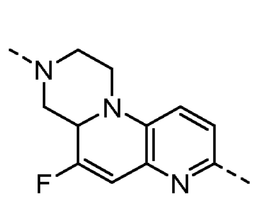 , 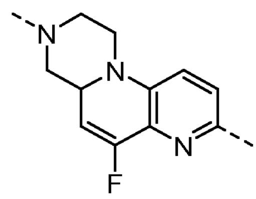 ,

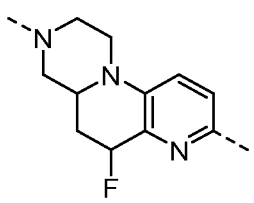 , 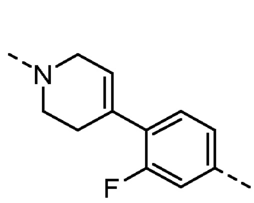 , 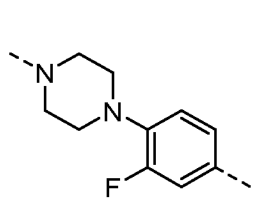 , 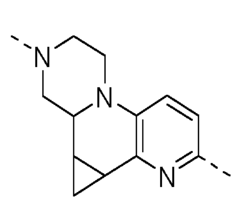 ,

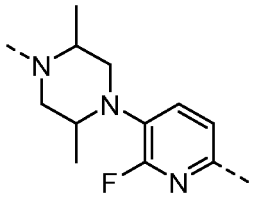 , 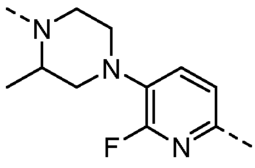 , 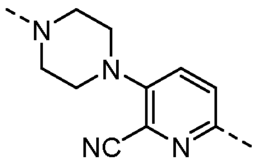 , 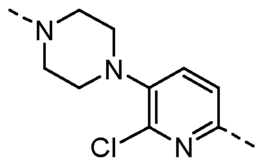 ,

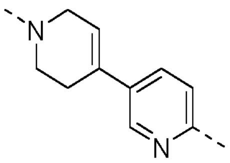 , 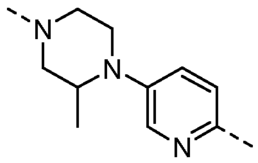 , 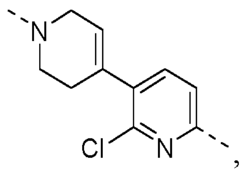 , and 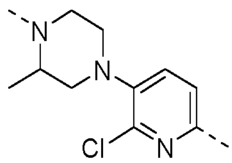 ,

and other variables are as defined in the present disclosure.

In some embodiments of the present disclosure, the L is selected from a single bond, the structural moiety

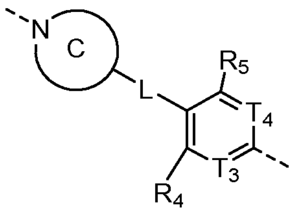

is selected from

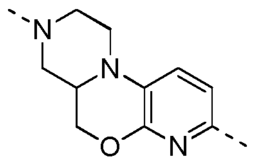 ,

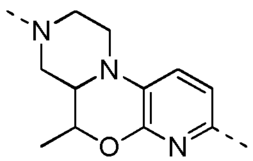 , 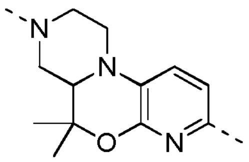 , 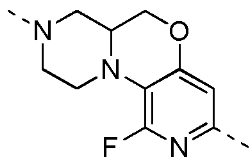 , 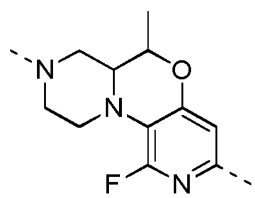 ,

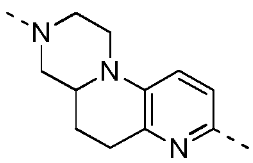 , 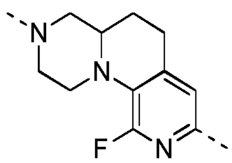 , 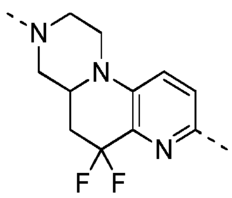 , 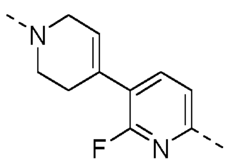 ,

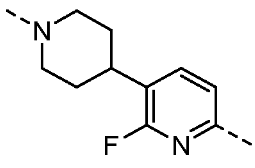 , 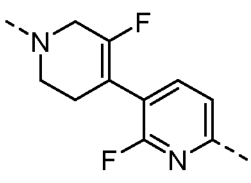 , 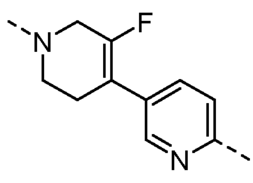 , 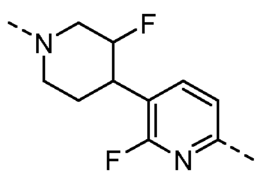 ,

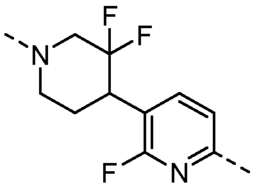, 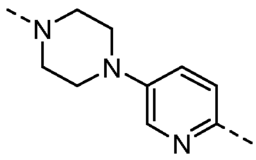, 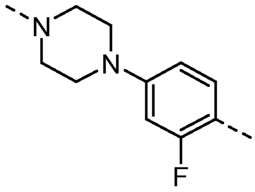, 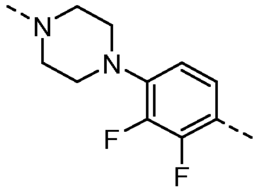,

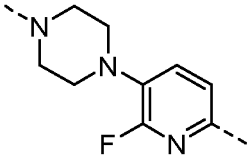, 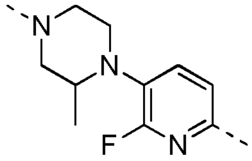, 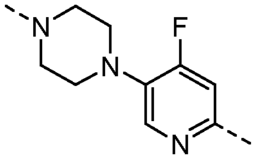, 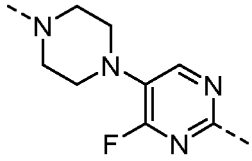,

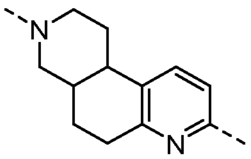, 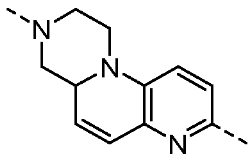, 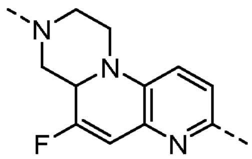, 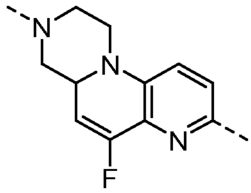,

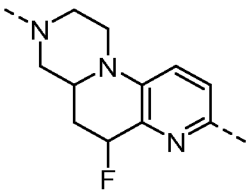, 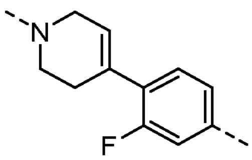, 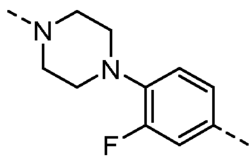, 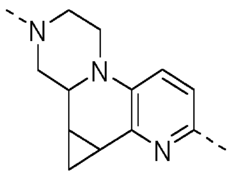,

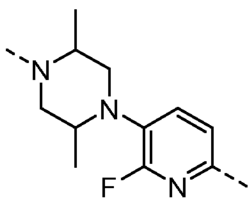, 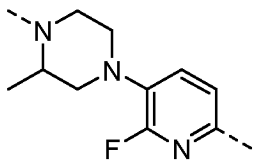, 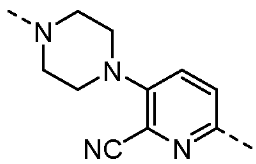, and 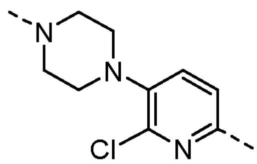,

and other variables are as defined in the present disclosure.

In some embodiments of the present disclosure, the L is selected from a single bond, the structural moiety

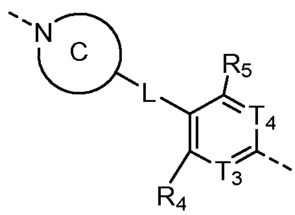

is selected from

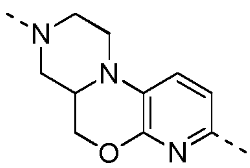,

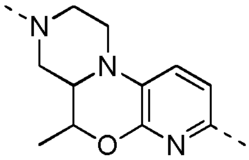, 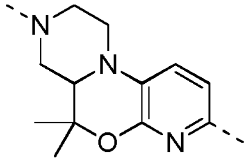, 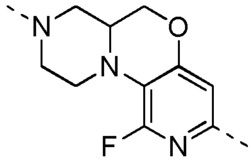, 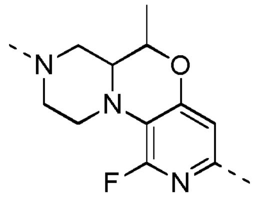,

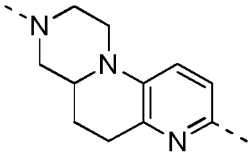, 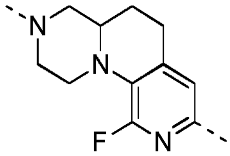, 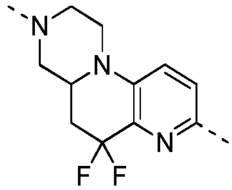, 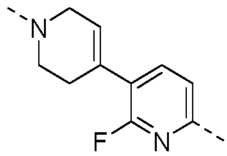,

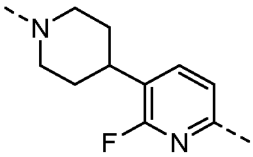, 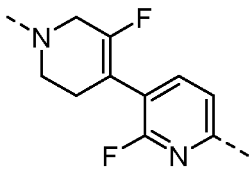, 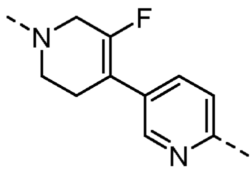, 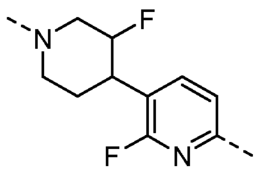,

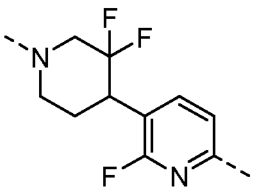, 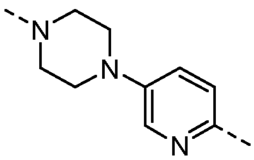, 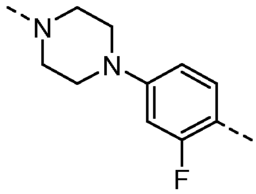, 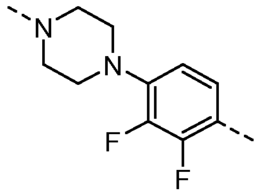,

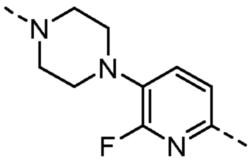, 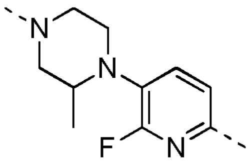, 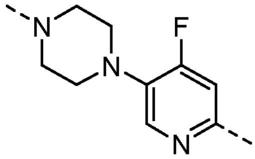, 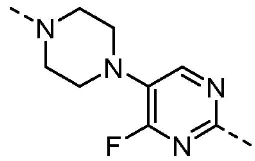,

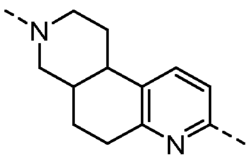, 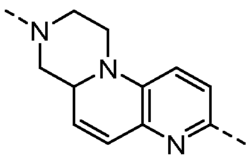, 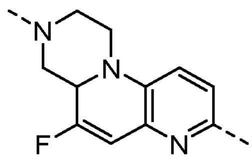, 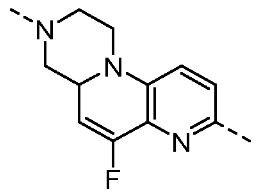,

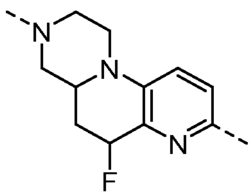, 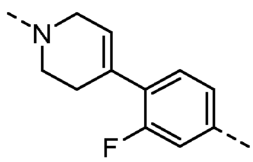, 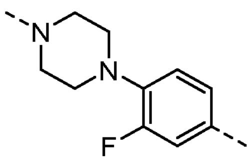, and 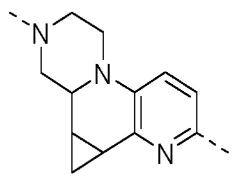,

and other variables are as defined in the present disclosure.

In some embodiments of the present disclosure, the structural moiety

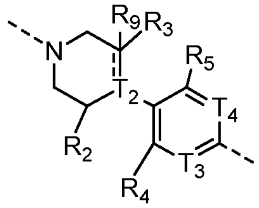

is selected from

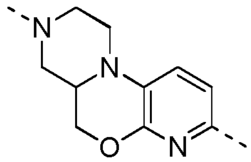 , 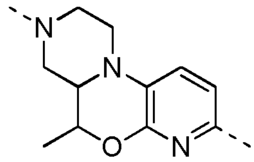 ,

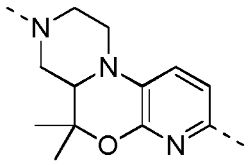 , 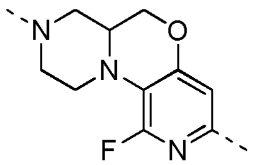 , 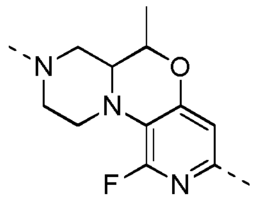 , 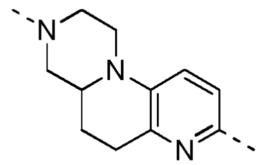 ,

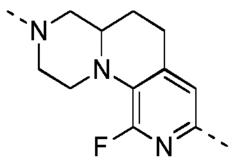 , 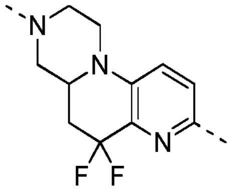 , 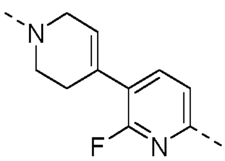 , 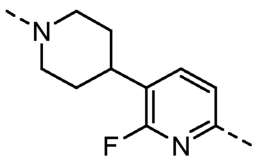 ,

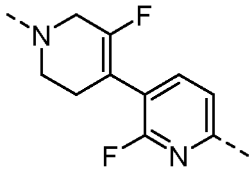 , 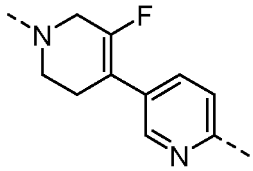 , 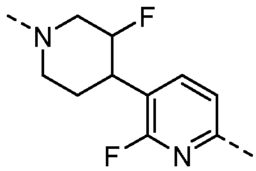 , 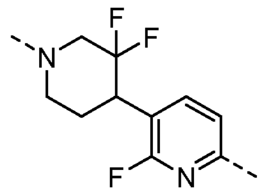 ,

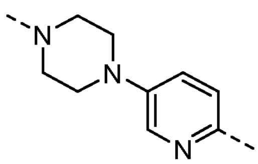 , 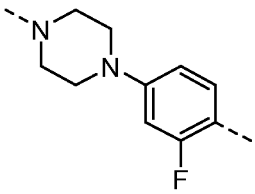 , 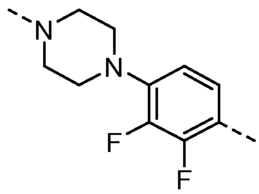 , 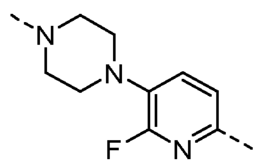 ,

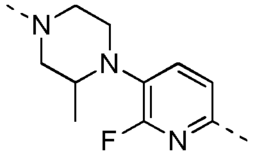 , 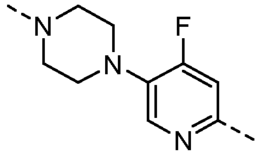 , 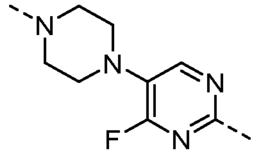 , 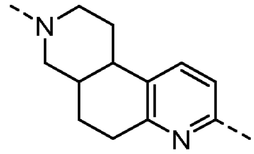 ,

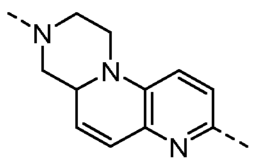 , 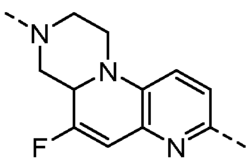 , 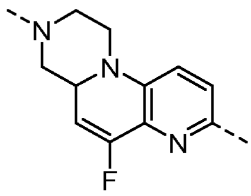 , 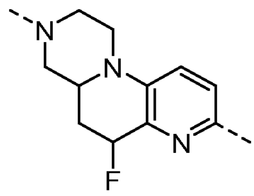 ,

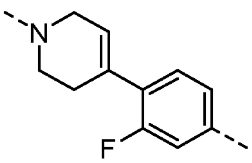 , 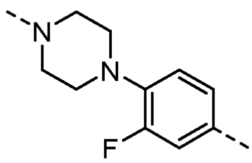 , and 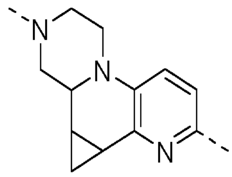 ,

and other variables are as defined in the present disclosure.

In some embodiments of the present disclosure, the structural moiety

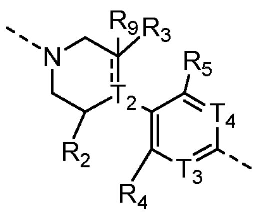

is selected from

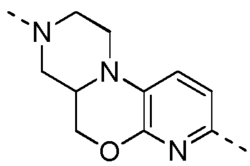 , 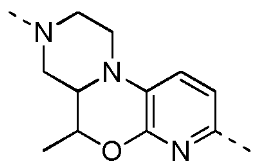 ,

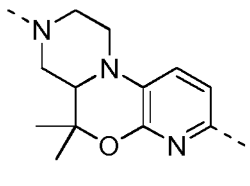 , 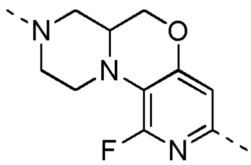 , 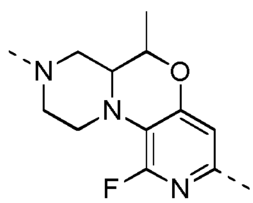 , 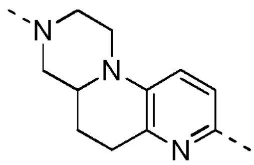 ,

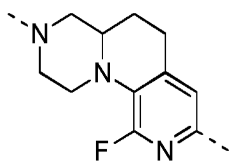 , 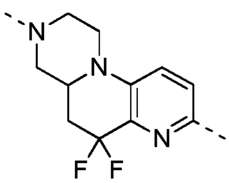 , 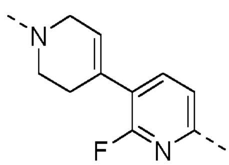 , 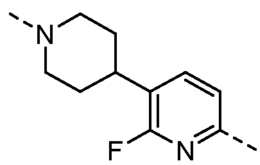 ,

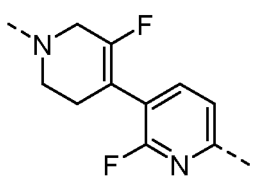 , 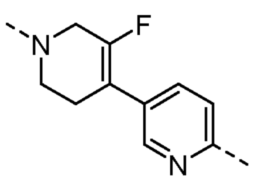 , 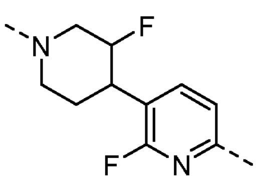 , 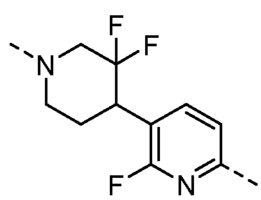 ,

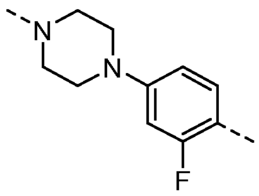 , 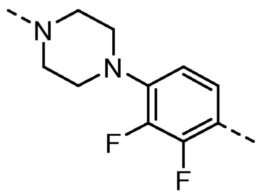 , 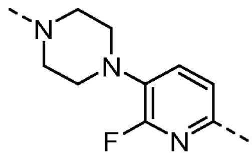 , 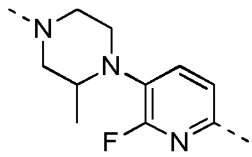 ,

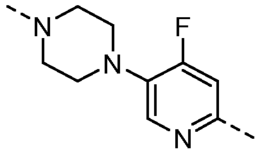 , 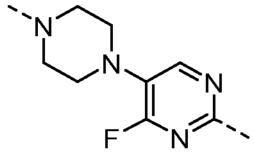 , 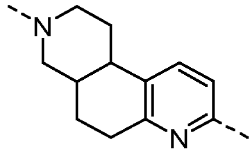 , 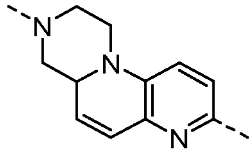 ,

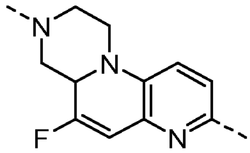 , 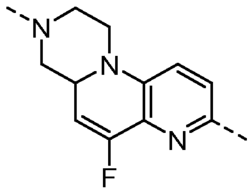 , 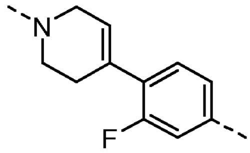 , 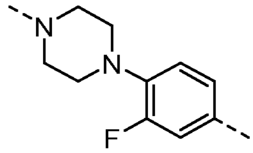 ,

and

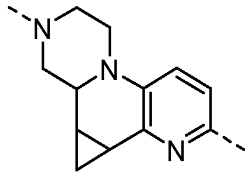 ,

and other variables are as defined in the present disclosure.

In some embodiments of the present disclosure, the structural moiety

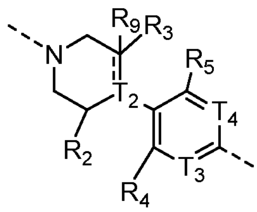

is selected from

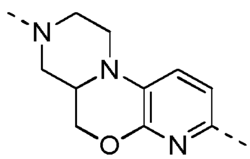 , 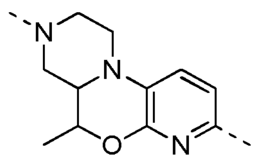 ,

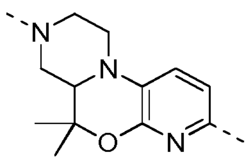 , 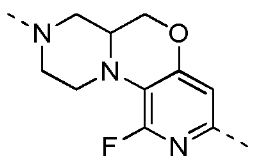 , 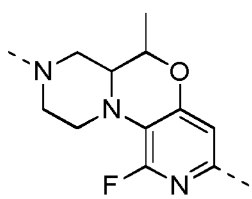 , 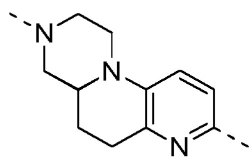 ,

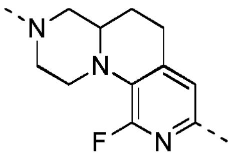 , 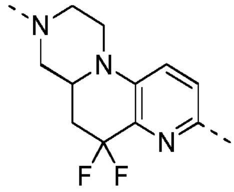 , 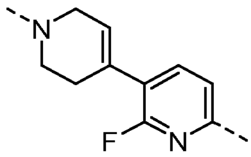 , 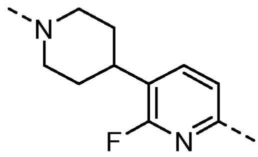 ,

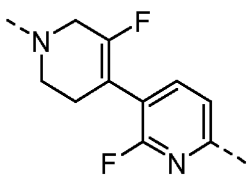 , 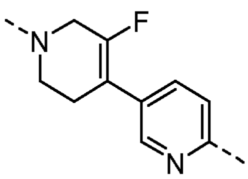 , 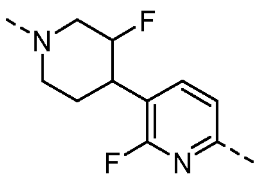 , 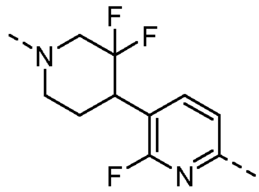 ,

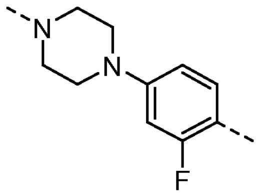 , 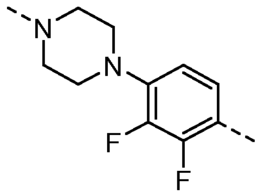 , 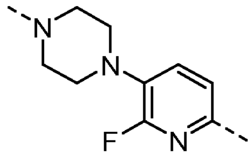 , 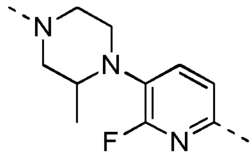 ,

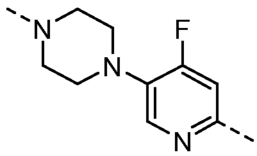 , 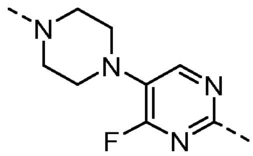 , 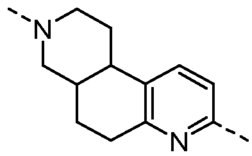 , 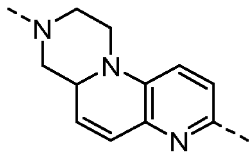 ,

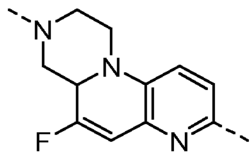 , 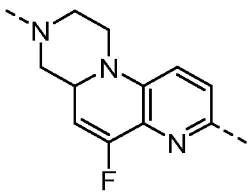 , 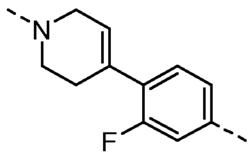 , and 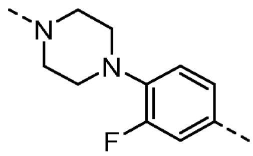 ,

and other variables are as defined in the present disclosure.

In some embodiments of the present disclosure, the structural moiety

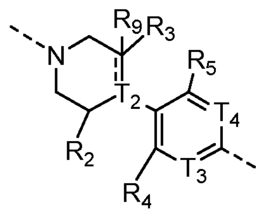

is selected from

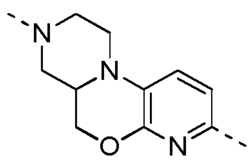 , 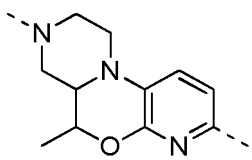 ,

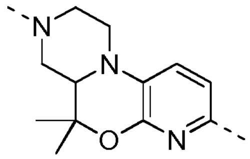 , 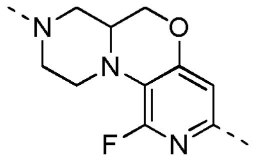 , 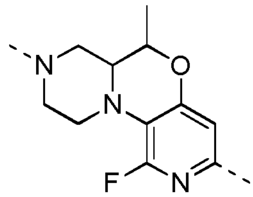 , 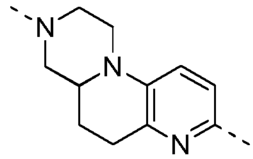 ,

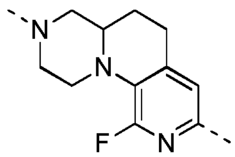 , 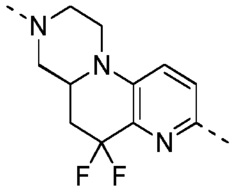 , 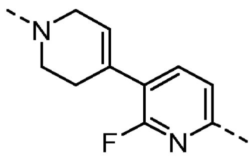 , 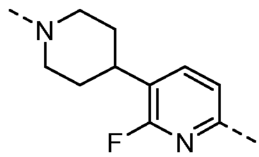 ,

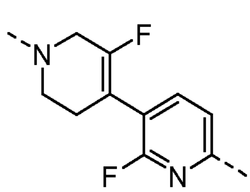 , 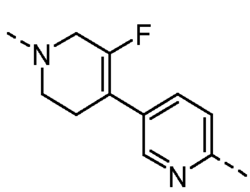 , 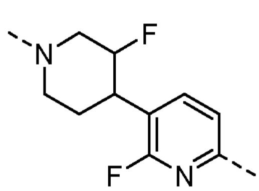 , 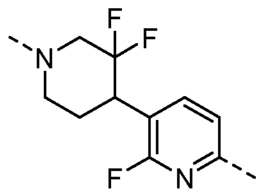 ,

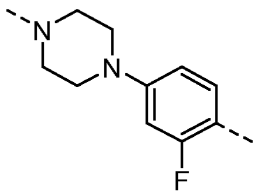 , 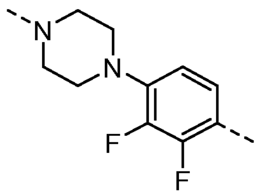 , 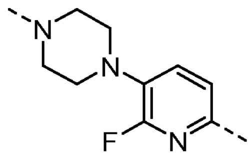 , 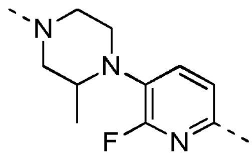 ,

and

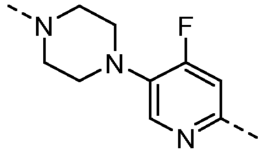 ,

and other variables are as defined in the present disclosure.

In some embodiments of the present disclosure, the structural moiety

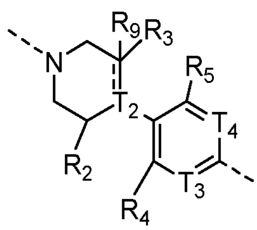

is selected from

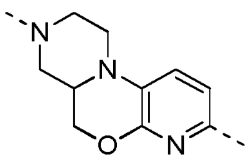 , 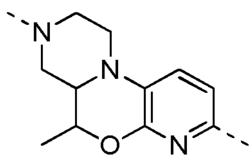 ,

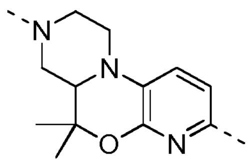 , 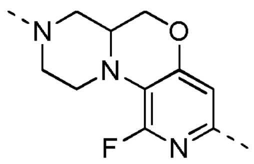 , 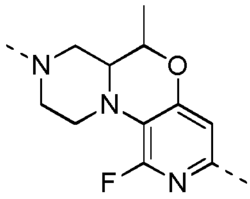 , 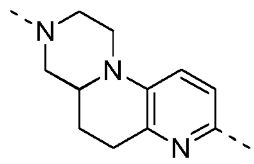 ,

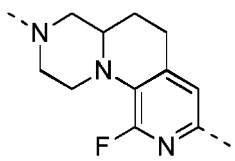 , 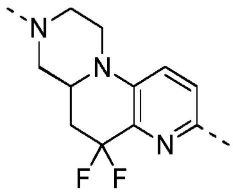 , 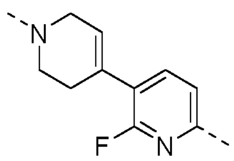 , 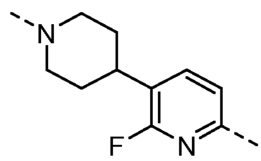 ,

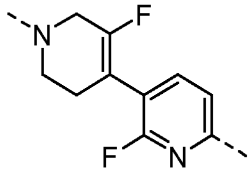 , 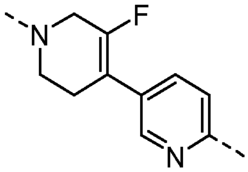 , 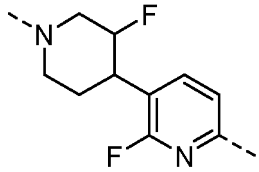 , 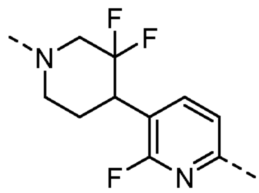 ,

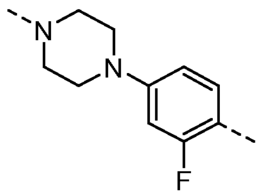 , 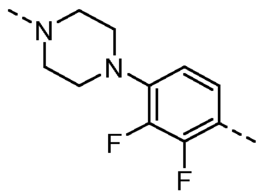 , 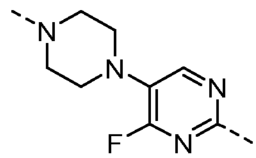 , 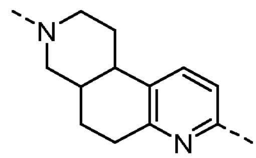 ,

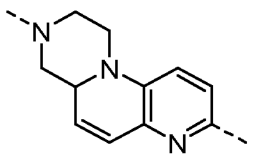 , 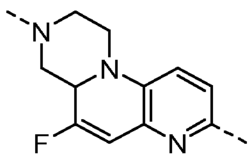 , 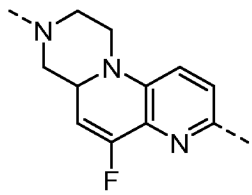 , 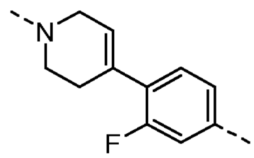 ,

and

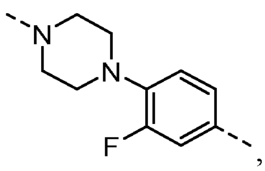 ,

and other variables are as defined in the present disclosure.

In some embodiments of the present disclosure, the structural moiety

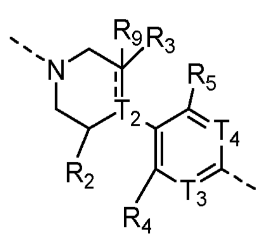

is selected from

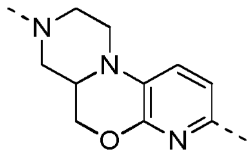, 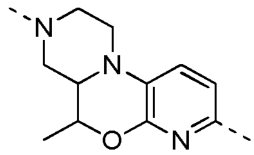,

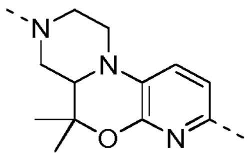, 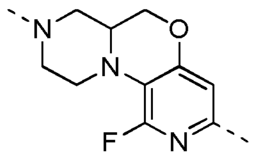, 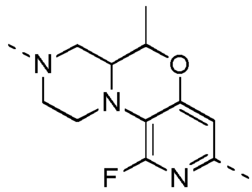, 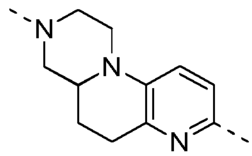,

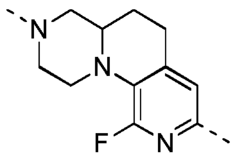, 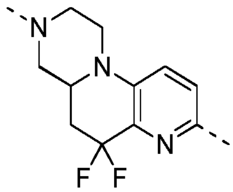, 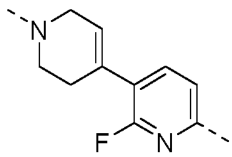, 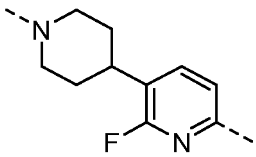,

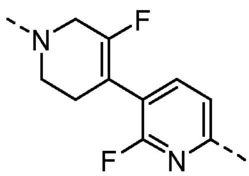, 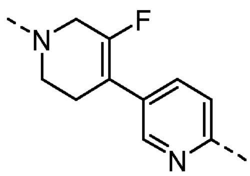, 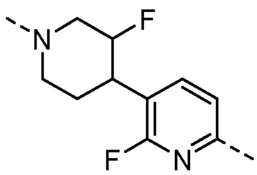, 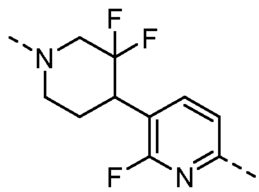,

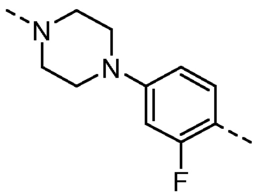, and 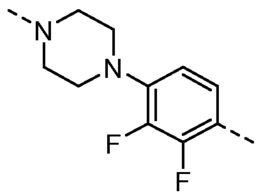,

and other variables are as defined in the present disclosure.

In some embodiments of the present disclosure, the structural moiety

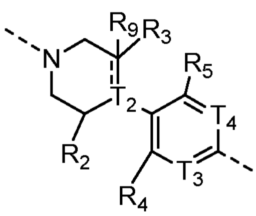

is selected from

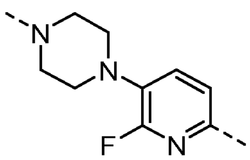, 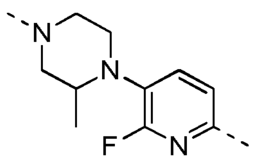,

and

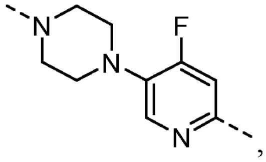

and other variables are as defined in the present disclosure.

In some embodiments of the present disclosure, the structural moiety

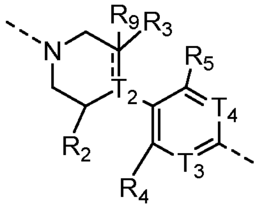

is selected from

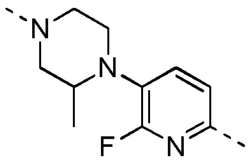
and
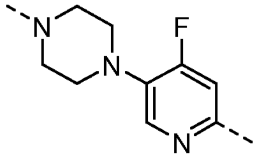

, and other variables are as defined in the present disclosure.

In some embodiments of the present disclosure, the structural moiety

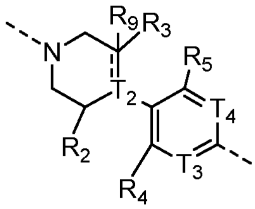

is selected from

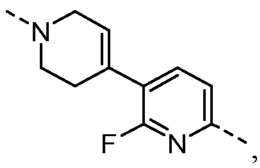

and other variables are as defined in the present disclosure.

In some embodiments of the present disclosure, when the structural moiety

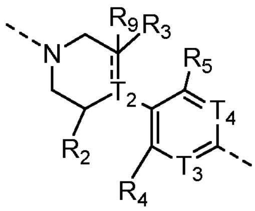

is selected from

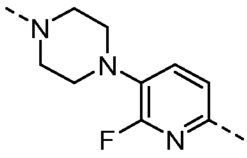
,

then $R_6$ and $R_7$ are not simultaneously H, and other variables are as defined in the present disclosure.

In some embodiments of the present disclosure, when the structural moiety

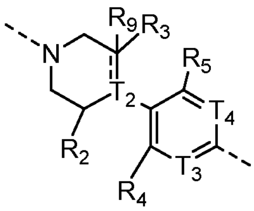

is selected from

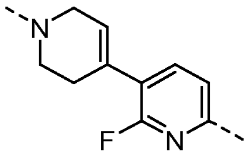
,

then $R_6$ and $R_7$ are not simultaneously H, and other variables are as defined in the present disclosure.

In some embodiments of the present disclosure, the structural moiety

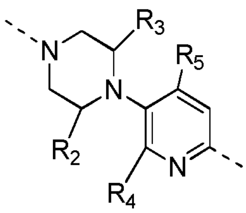

is selected from

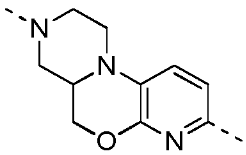
,
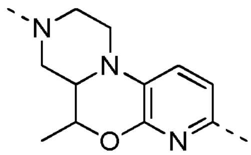
,
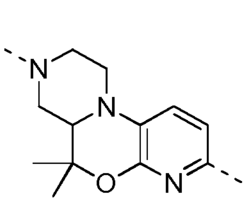
,
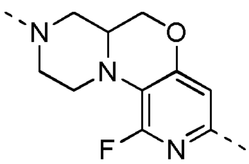
,
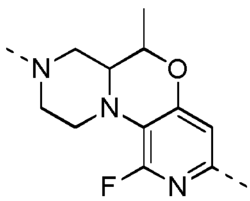
,
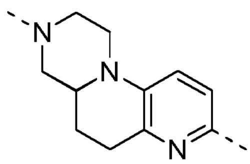
,

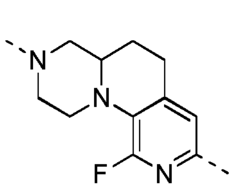, 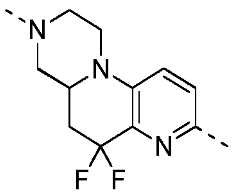, and 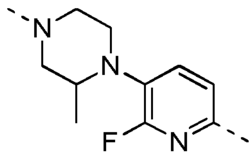,

and other variables are as defined in the present disclosure.

In some embodiments of the present disclosure, the structural moiety

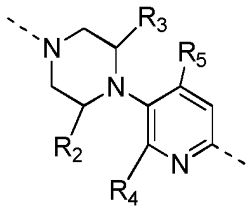

is selected from

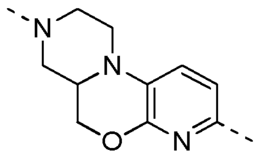, 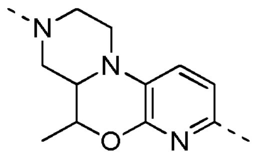,

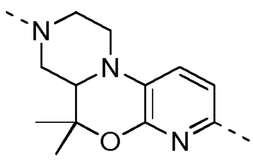, 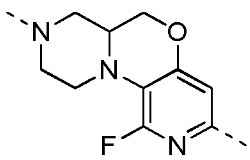, 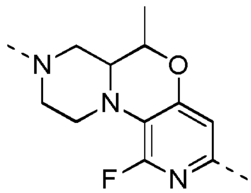, and 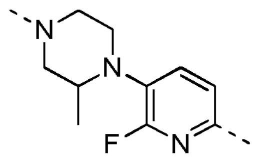,

and other variables are as defined in the present disclosure.

In some embodiments of the present disclosure, the compound or the pharmaceutically acceptable salt thereof is selected from:

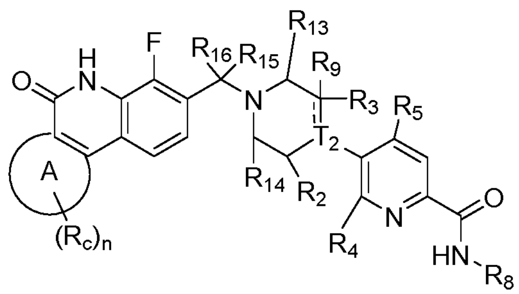

**(P-1)**

,

wherein ring A is selected from pyridyl;

$R_2$, $R_3$, $R_4$, $R_5$, $R_8$, $R_9$, $R_{13}$, $R_{14}$, $R_{15}$, $R_{16}$, $R_c$, $T_2$, n, and

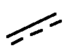

are as defined in the present disclosure.

In some embodiments of the present disclosure, the compound or the pharmaceutically acceptable salt thereof is selected from:

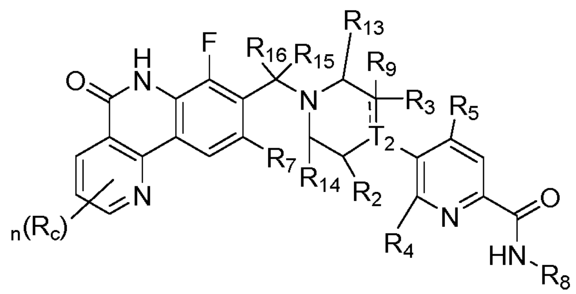

**(P-2)**

,

wherein

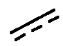

is selected from a single bond or a double bond;
$T_2$ is selected from C and N;
$R_2$ is selected from H and $C_{1-3}$ alkyl, and the $C_{1-3}$ alkyl is optionally substituted by 1, 2, or 3 halogens;
$R_3$ is selected from H and halogen;
$R_4$ is selected from H, halogen, CN, $C_{1-3}$ alkyl, and $C_{1-3}$ alkoxy;
$R_5$ is selected from H, halogen, CN, $C_{1-3}$ alkyl, and $C_{1-3}$ alkoxy;
$R_9$ is absent, or selected from H and halogen;
$R_7$, $R_8$, $R_{13}$, $R_{14}$, $R_{15}$, $R_{16}$, $R_c$, and n are as defined in the present disclosure.

In some embodiments of the present disclosure, the compound or the pharmaceutically acceptable salt thereof is selected from:

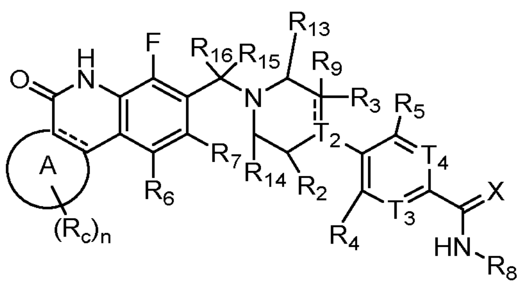

**(XII-1)**

and

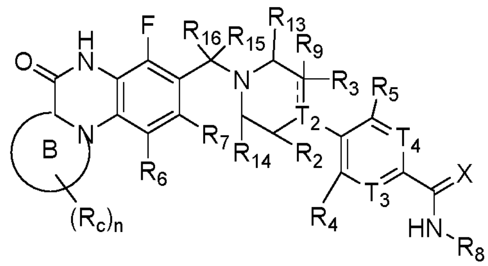

**(XII-2)**

,

wherein

ring A is selected from phenyl and 6-membered heteroaryl;
ring B is selected from 6-membered heteroaryl;
$R_2$, $R_3$, $R_4$, $R_5$, $R_6$, $R_7$, $R_8$, $R_9$, $R_{13}$, $R_{14}$, $R_{15}$, $R_{16}$, $R_c$, X, $T_2$, $T_3$, $T_4$, n, and

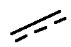

are as defined in the present disclosure.

In some embodiments of the present disclosure, the ring A is selected from a pyridine ring, and other variables are as defined in the present disclosure.

In some embodiments of the present disclosure, the compound or the pharmaceutically acceptable salt thereof is selected from:

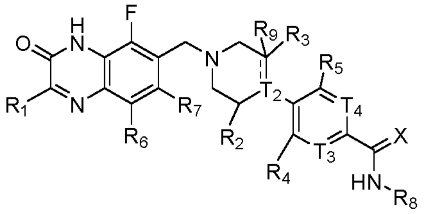

( VIII-1 ) ,

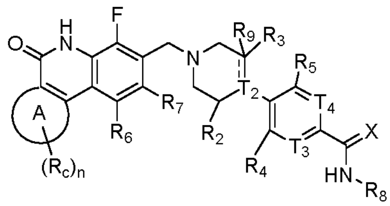

( VIII-2 ) ,

and

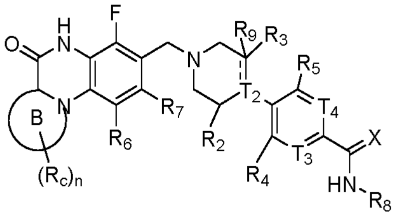

( VIII-3 ) ,

wherein ring A, ring B, $R_1$, $R_2$, $R_3$, $R_4$, $R_5$, $R_6$, $R_7$, $R_8$, $R_9$, X, $T_3$, $T_4$, and the structural moiety

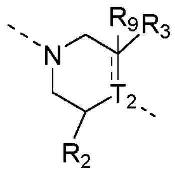

are as defined in the present disclosure.

In some embodiments of the present disclosure, the compound or the pharmaceutically acceptable salt thereof is selected from:

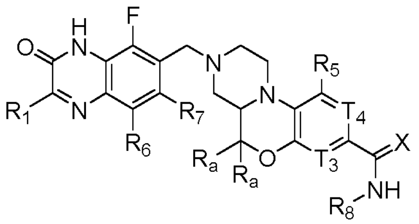

**(VIII-1-1)** ,

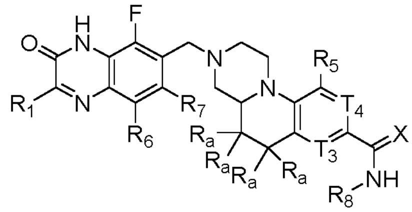

**(VIII-1-2)** ,

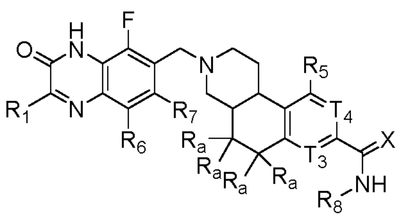

**(VIII-1-3)** ,

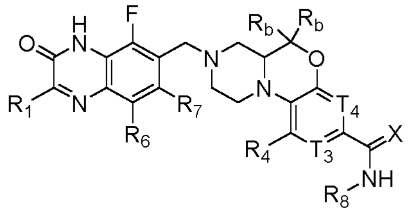

**(VIII-1-4)** ,

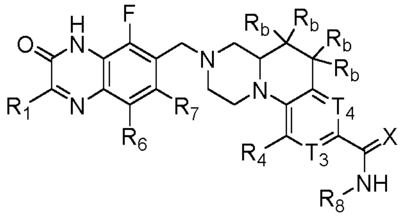

(VIII-1-5) ,

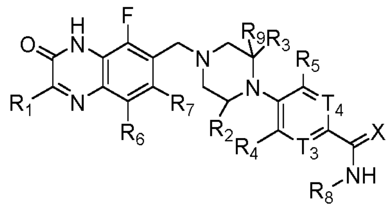

(VIII-1-6) ,

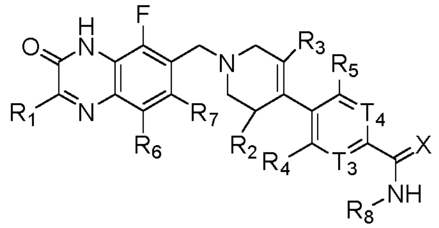

(VIII-1-7) , and

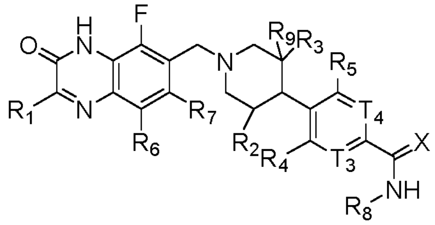

(VIII-1-8) ,

wherein $R_1$, $R_2$, $R_3$, $R_4$, $R_5$, $R_6$, $R_7$, $R_8$, $R_9$, $R_a$, $R_b$, X, $T_3$, and $T_4$ are as defined in the present disclosure.

In some embodiments of the present disclosure, the compound or the pharmaceutically acceptable salt thereof is selected from:

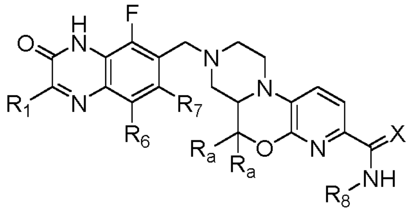

(VIII-1-1a) ,

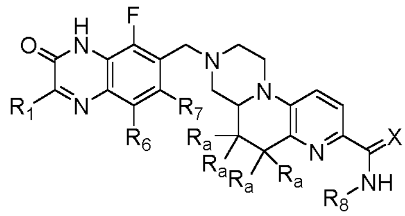

(VIII-1-2a) ,

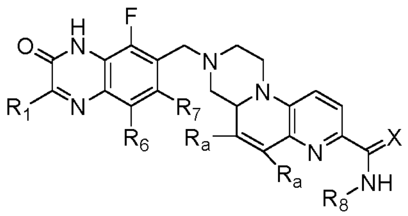

(VIII-1-2b) ,

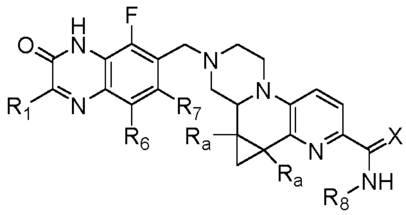

(VIII-1-2c) ,

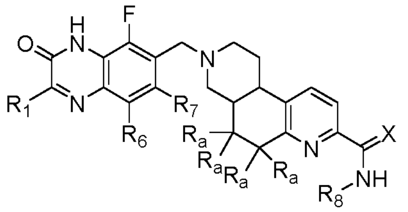

(VIII-1-3a) ,

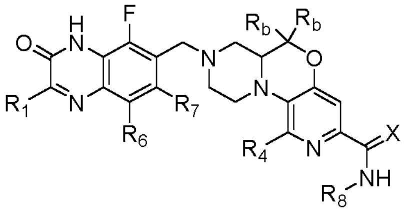

(VIII-1-4a) ,

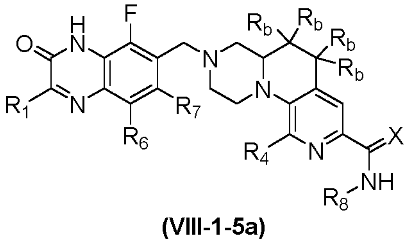

**(VIII-1-5a)** ,

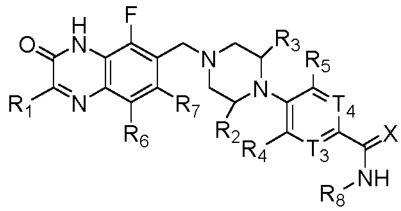

**(VIII-1-6a)** ,

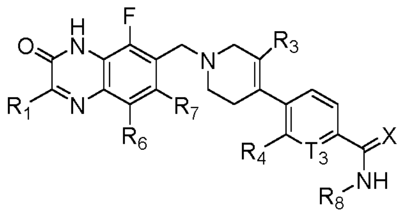

**(VIII-1-7a)** , and

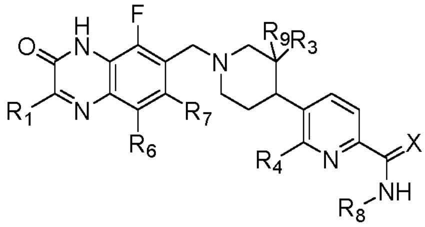

**(VIII-1-8a)** ,

wherein $R_1$, $R_2$, $R_3$, $R_4$, $R_5$, $R_6$, $R_7$, $R_8$, $R_9$, $R_a$, $R_b$, X, $T_3$, and $T_4$ are as defined in the present disclosure; given that

1) when the compound is selected from a compound of formula (VIII-6a), and one of $T_3$ or $T_4$ is selected from N, then $R_2$, $R_3$, and $R_9$ are not simultaneously H; or
2) when the compound is selected from a compound of formula (VIII-6a), and one of $T_3$ or $T_4$ is selected from N, then $R_6$ and $R_7$ are not simultaneously H; or
3) when the compound is selected from a compound of formula (VIII-6a), $T_3$ is selected from N, and $T_4$ is selected from $CR_{10}$, then $R_5$ is selected from halogen; or
4) when the compound is selected from a compound of formula (VIII-6a), $T_4$ is selected from N, and $T_3$ is selected from $CR_{10}$, then $R_4$ is selected from halogen; or
5) when the compound is selected from a compound of formula (VIII-7a), and $T_3$ is selected from N, then $R_6$ and $R_7$ are not simultaneously H.

There are still some embodiments of the present disclosure which are obtained by any combination of the above variables.

The present disclosure further provides a compound as follows or a pharmaceutically acceptable salt thereof,

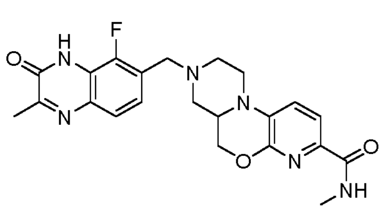

,

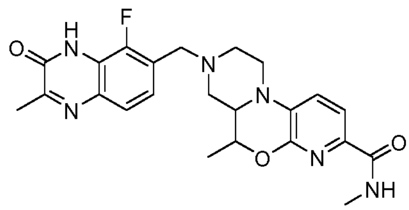

,

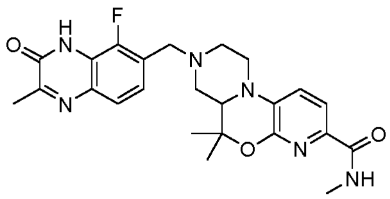

,

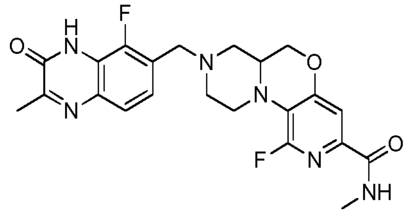

,

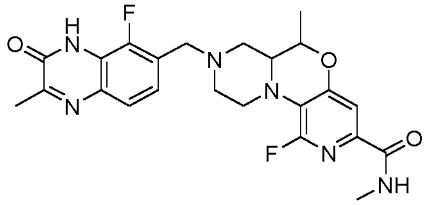,

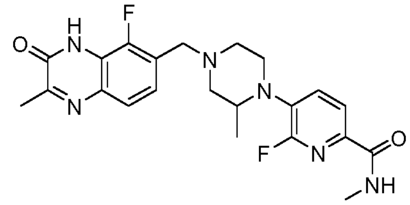,

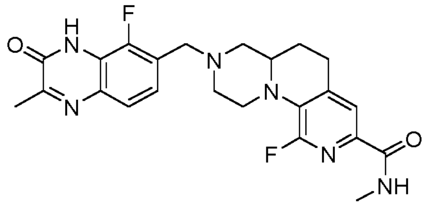,

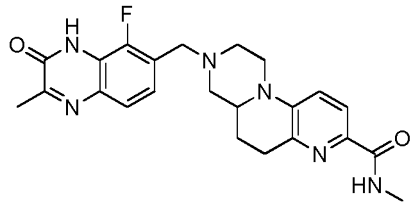,

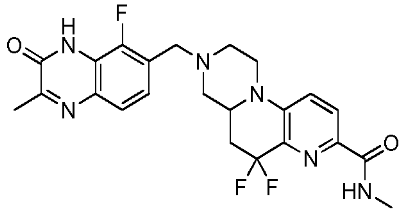,

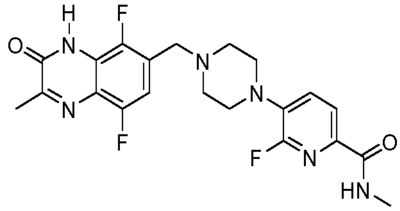,

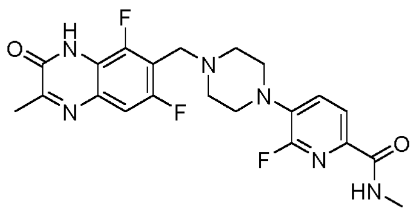,

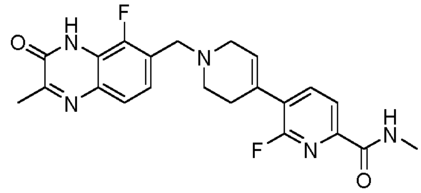,

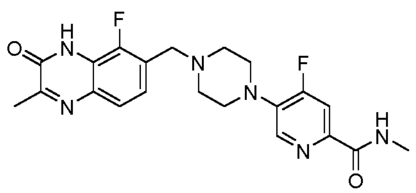,

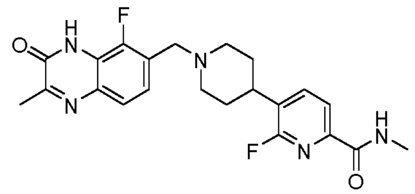,

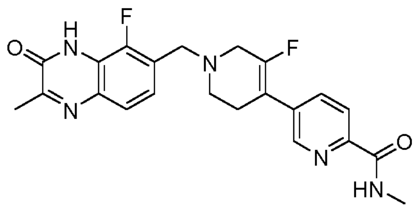,

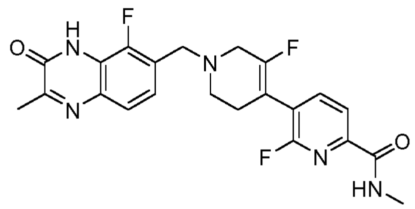,

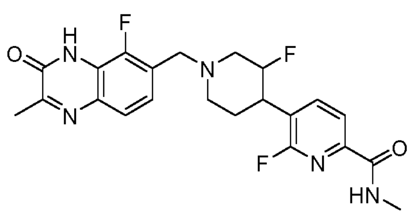,

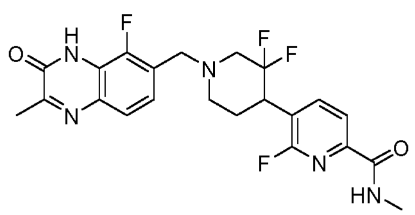,

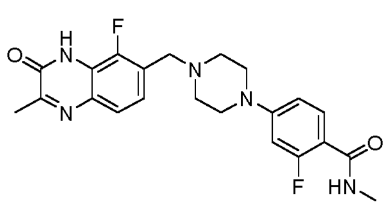
,

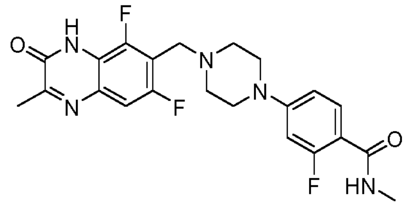
,

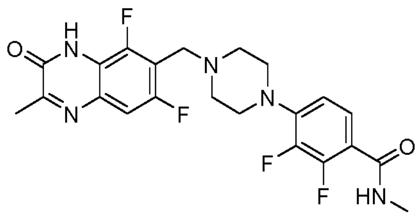
,

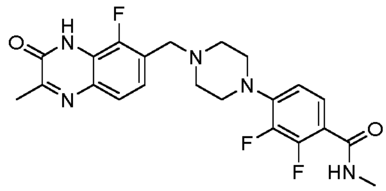
,

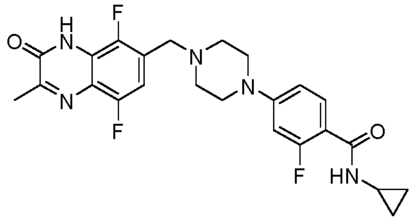
,

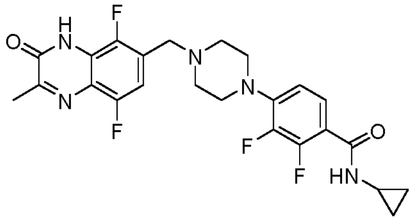
,

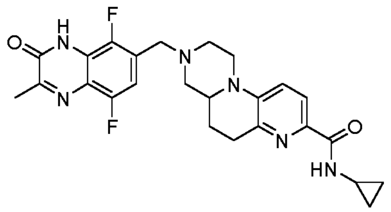
,

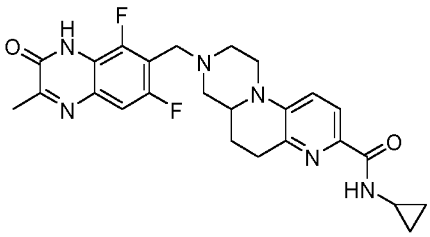
,

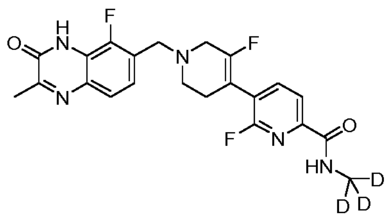
,

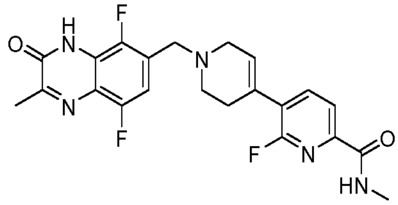
,

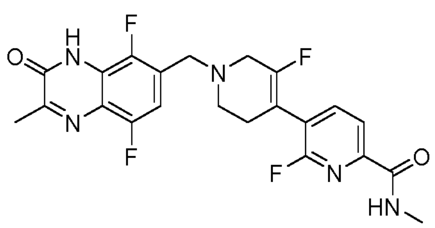
,

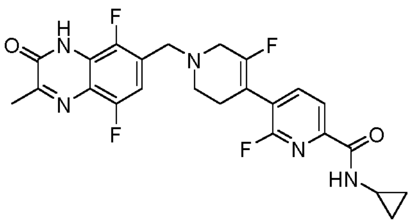
,

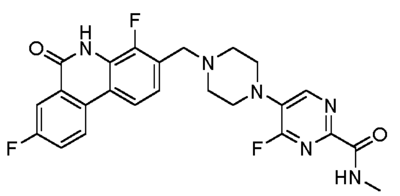
,
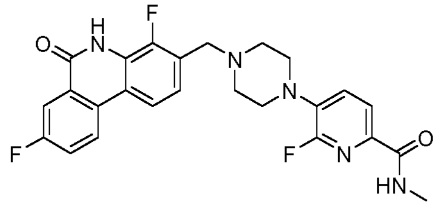
,
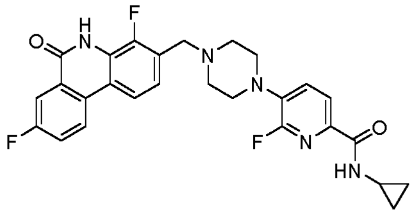
,
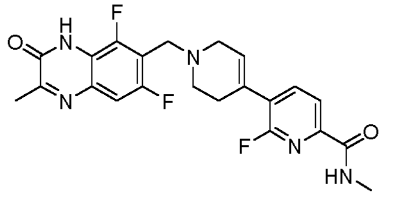
,
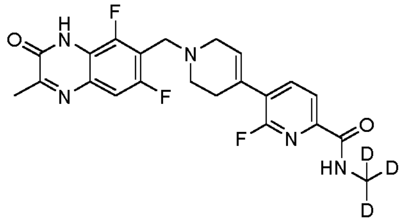
,
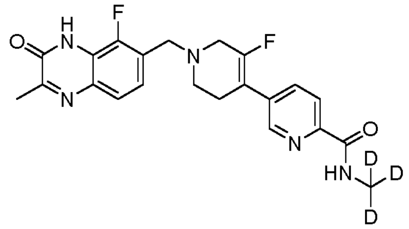
,
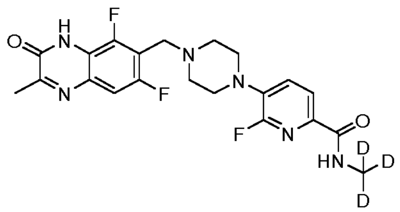
,
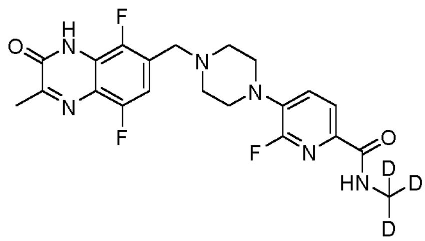
,
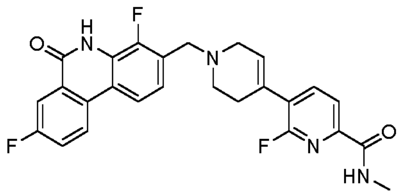
,
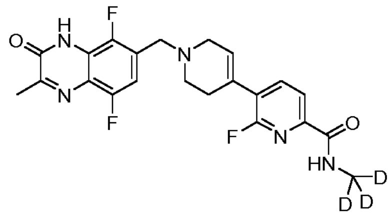
,
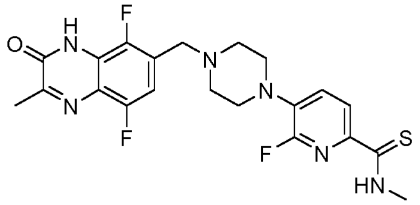
,
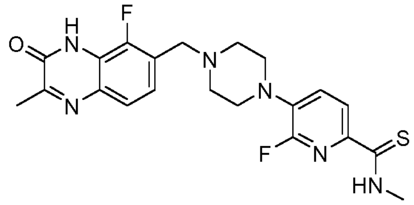
,

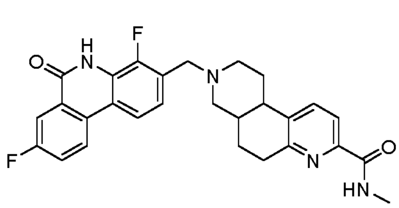,
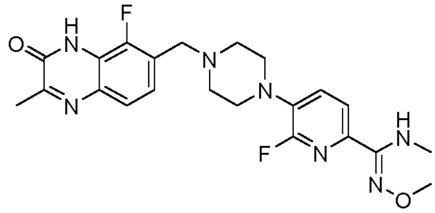,

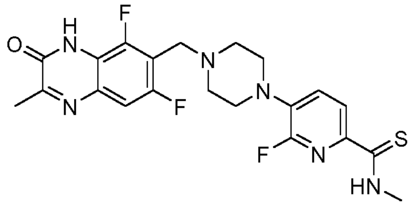,
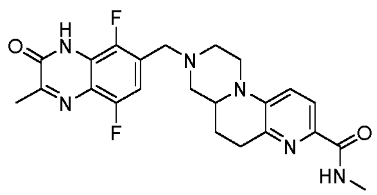,

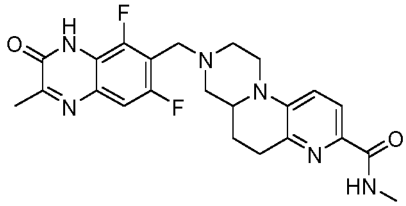,
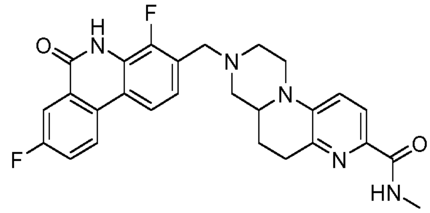,

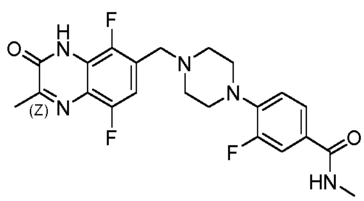,
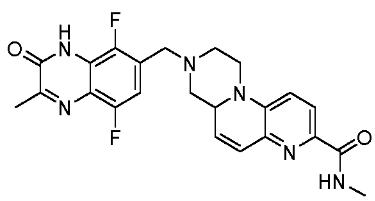,
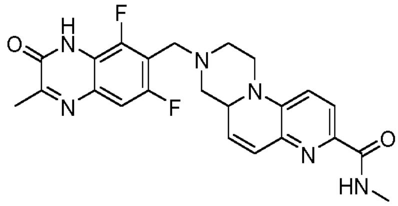,

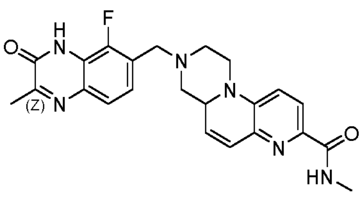,
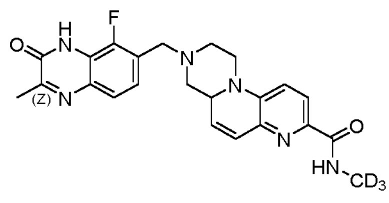,
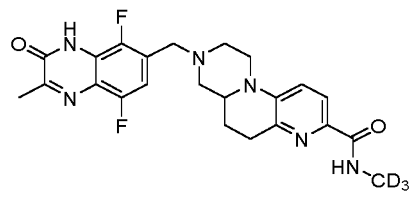,

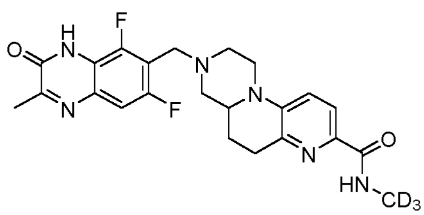,
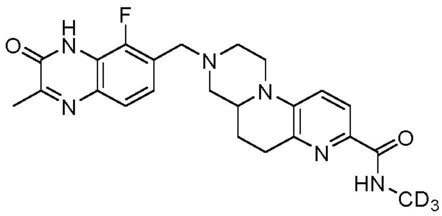,

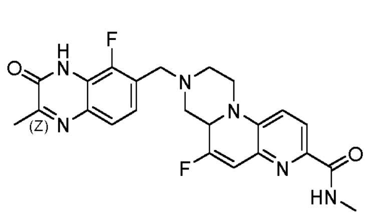,
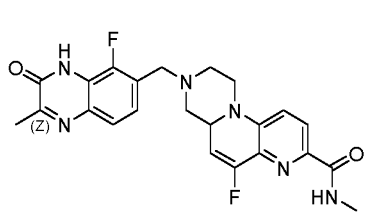,
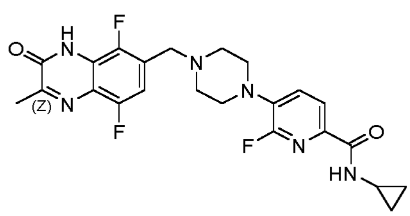,

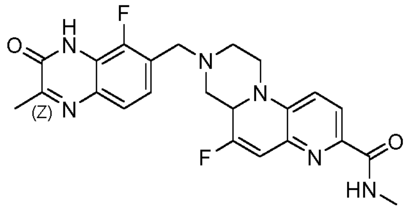
,
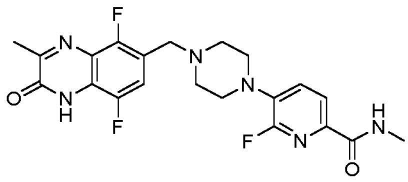
,
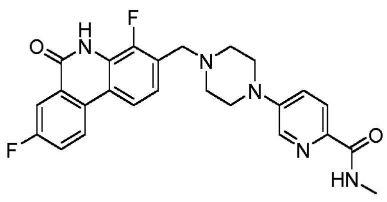
,
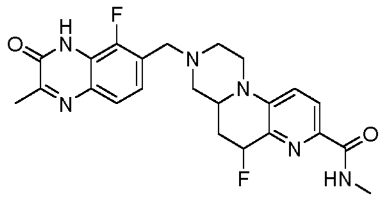
,
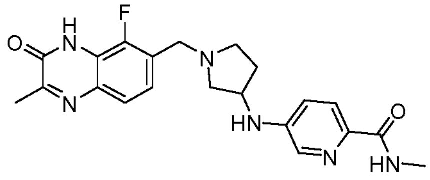
,
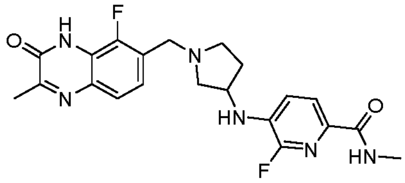
,
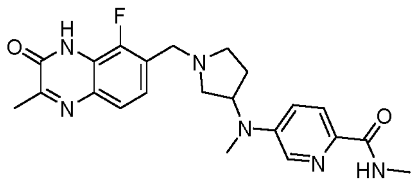
,
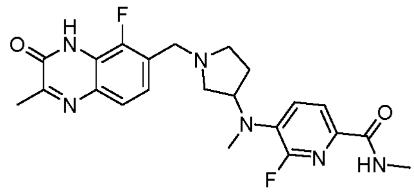
,
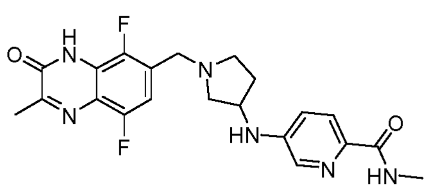
,
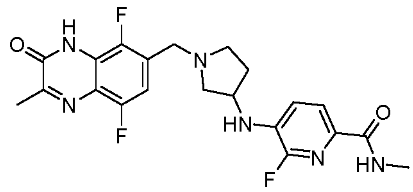
,
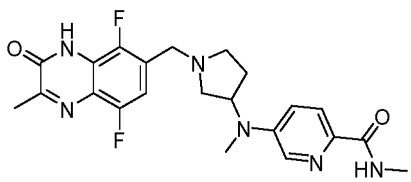
,
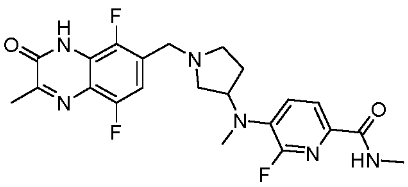
,
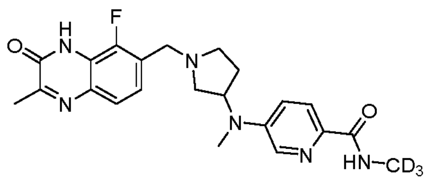
,
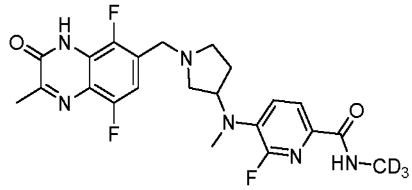
,

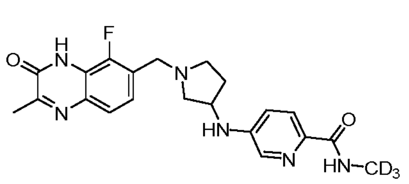,

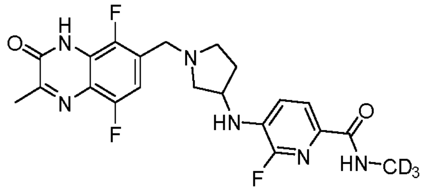,

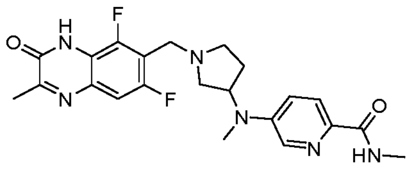,

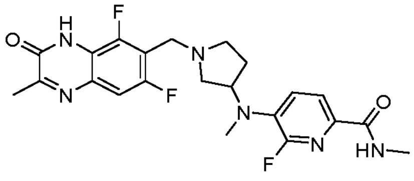,

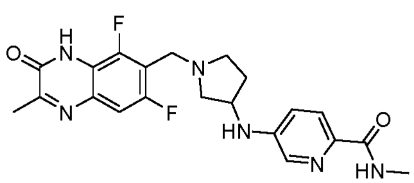,

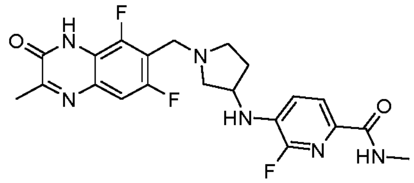,

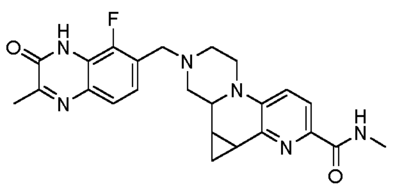,

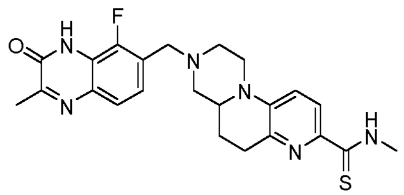,

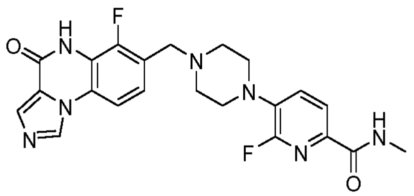,

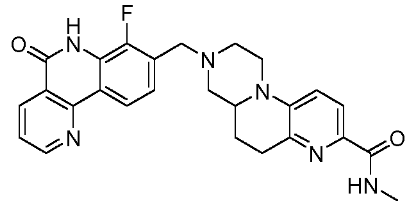,

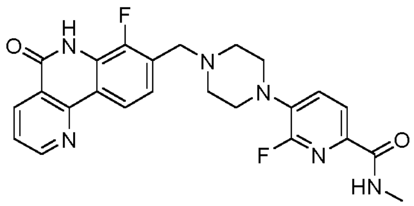,

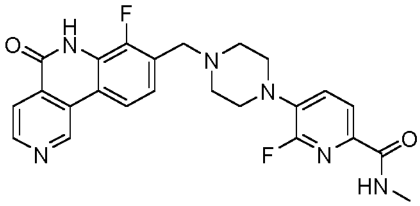,

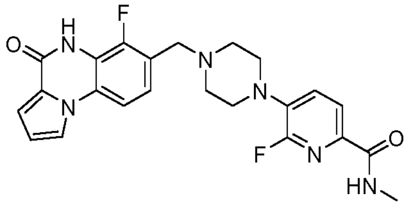,

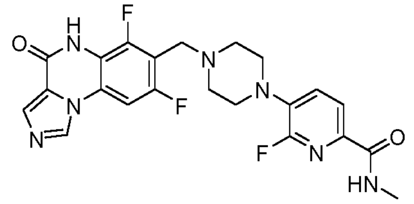,

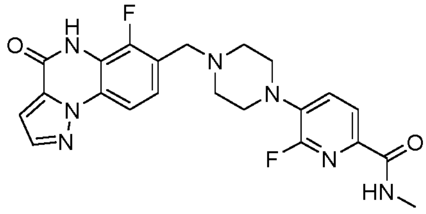
,

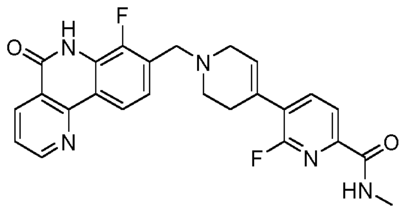
,

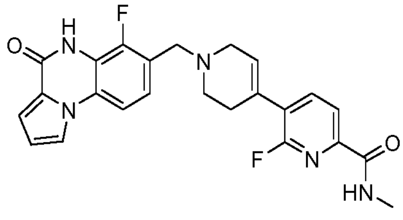
,

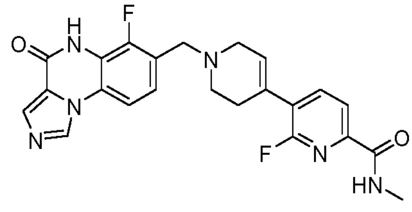
,

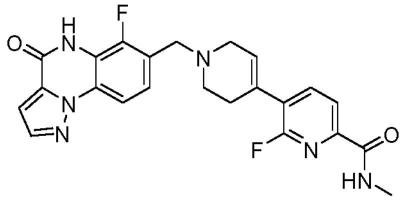
,

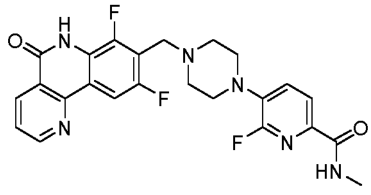
,

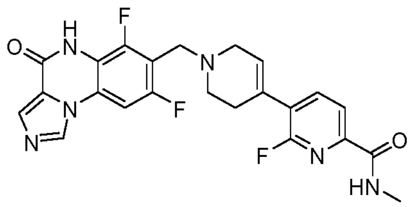
,

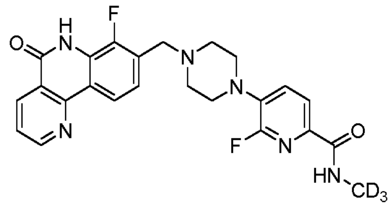
,

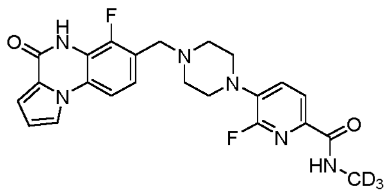
,

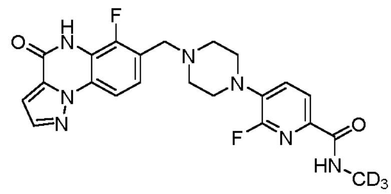
,

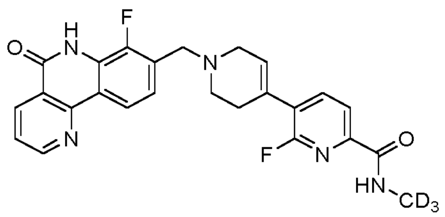
,

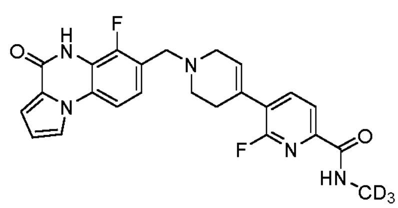
,

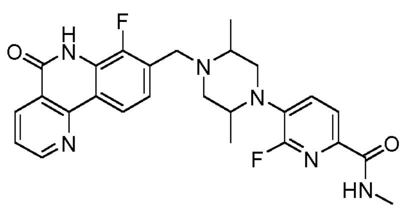
,

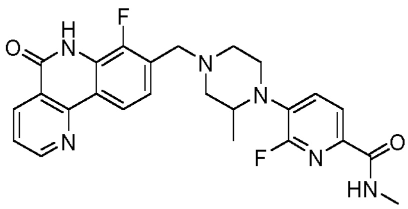
,

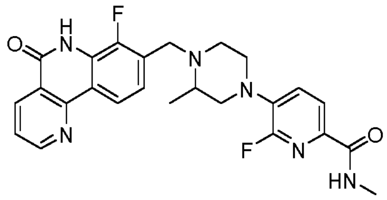,
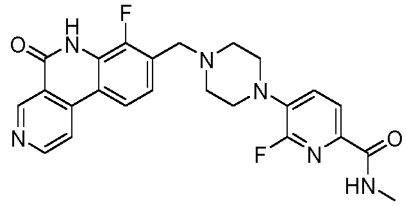,

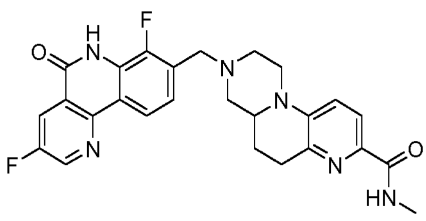,
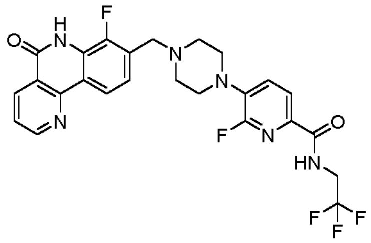,

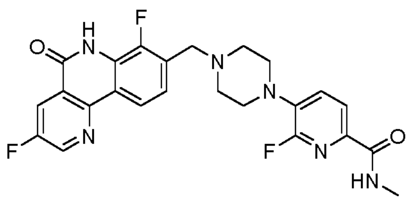,
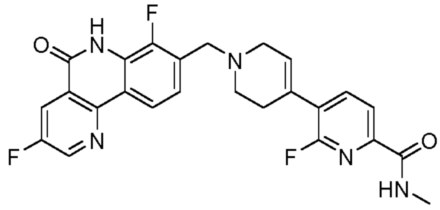,

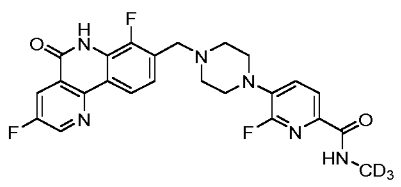,
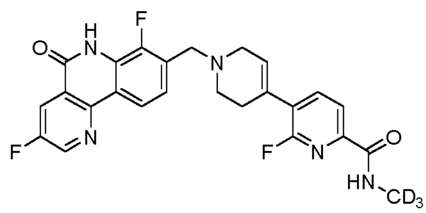,

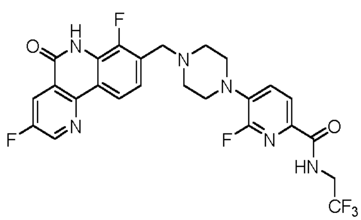,
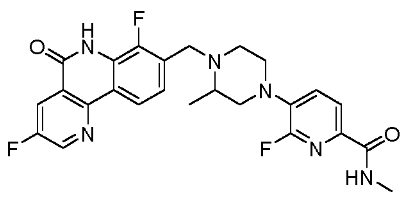,

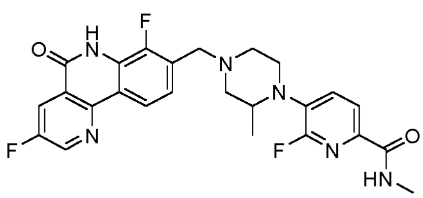,
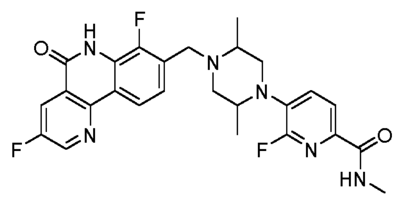,

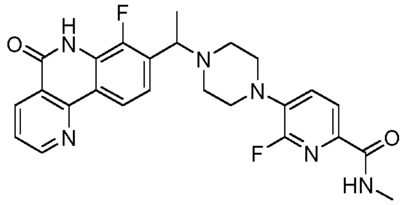,
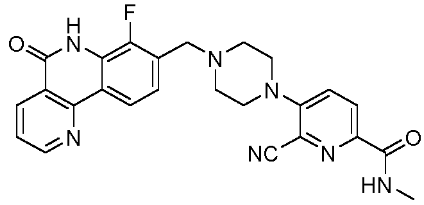,

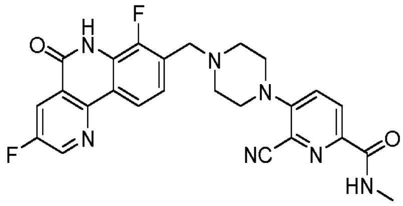,
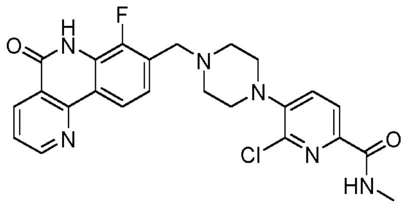,

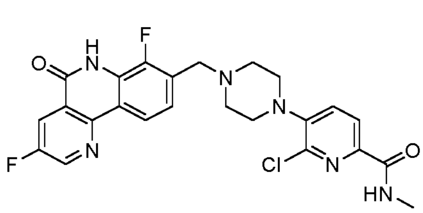,
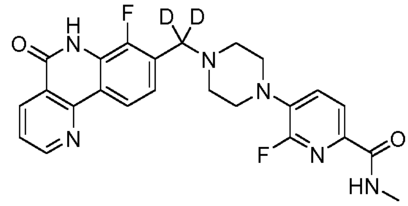,

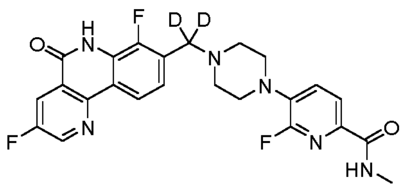,
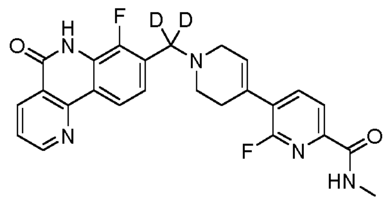,

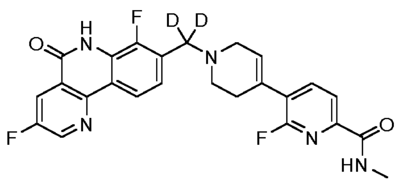,
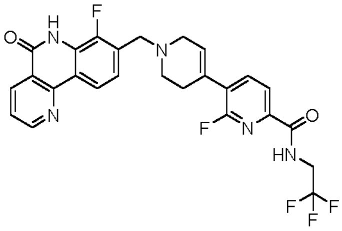,
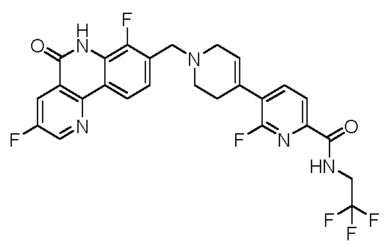,

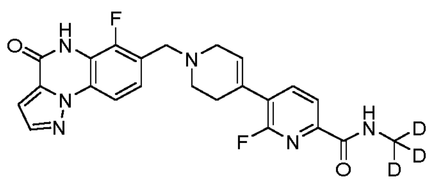,
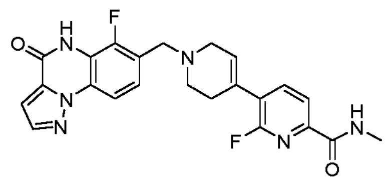,

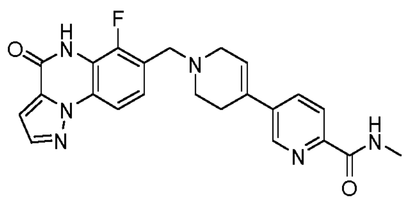,
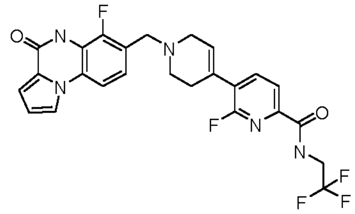,
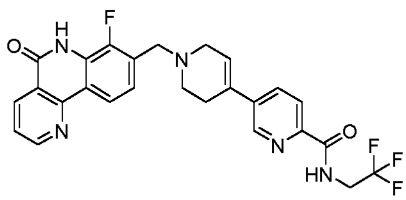,

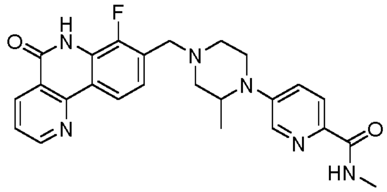
,

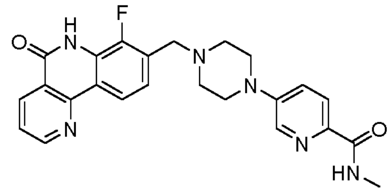
,

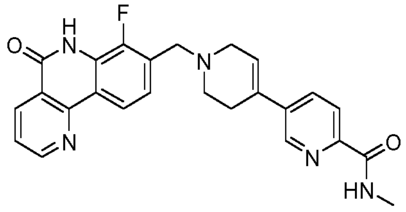
,

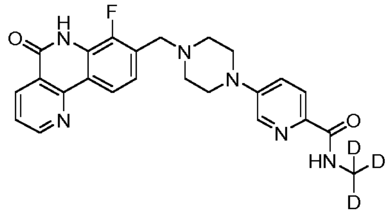
,

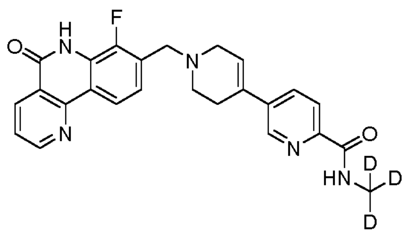
,

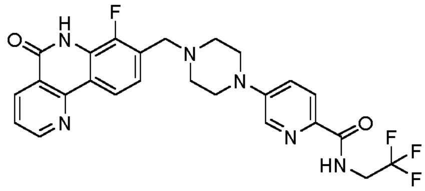
,

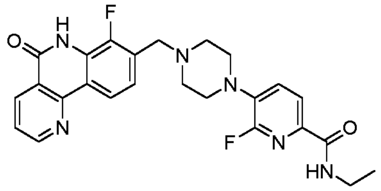
,

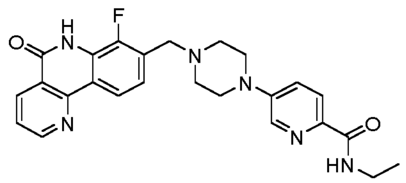
,

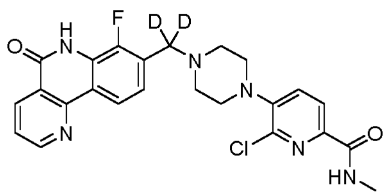
,

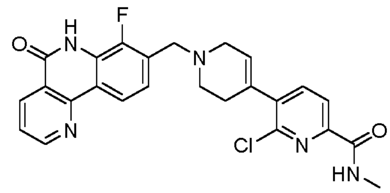
,

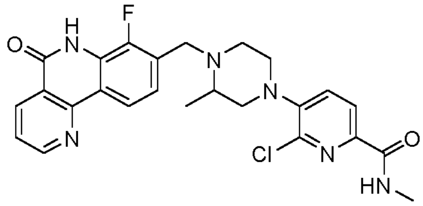
,

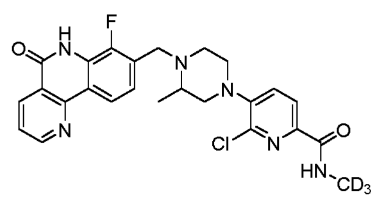
,

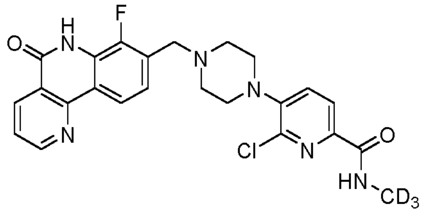
,

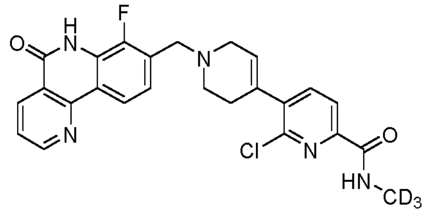
,

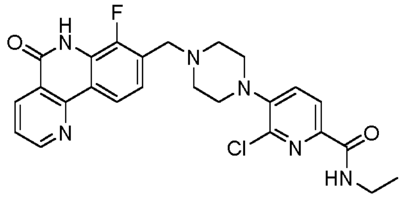,
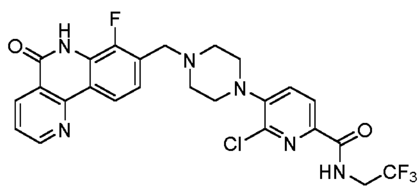,
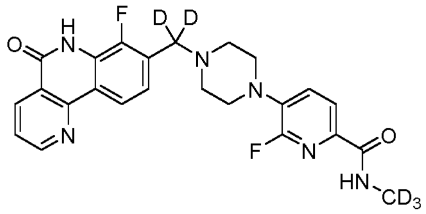,
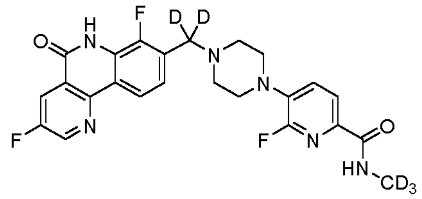,
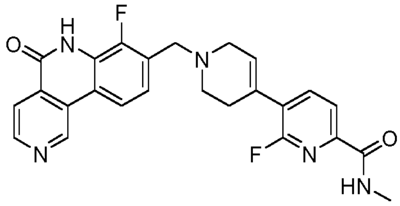, and
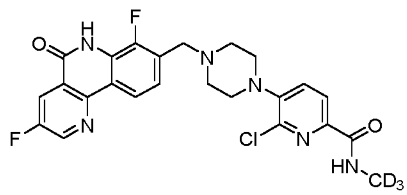.

In some embodiments of the present disclosure, the compound or the pharmaceutically acceptable salt thereof is provided, and the compound is selected from:

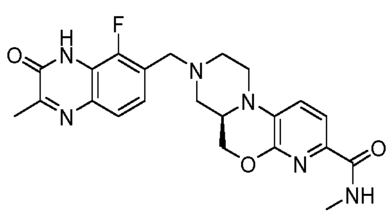,
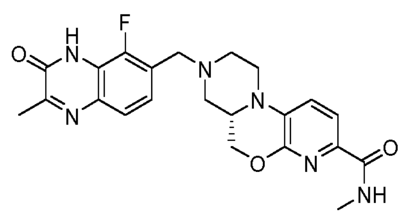,
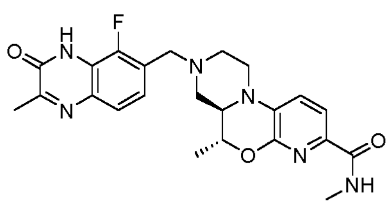,
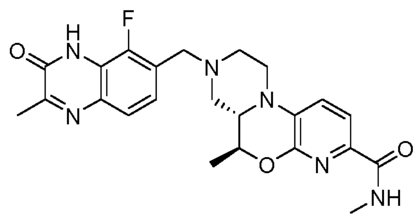,
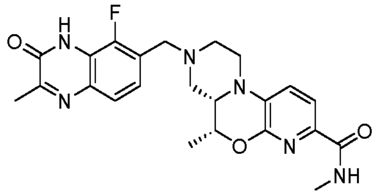,
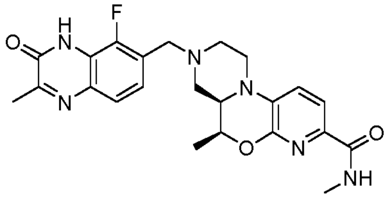,
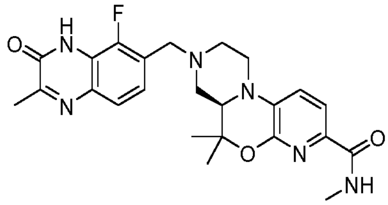,

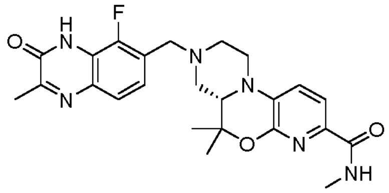,

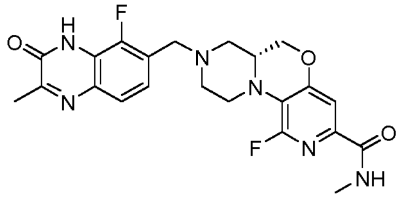,

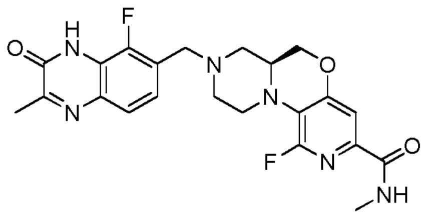,

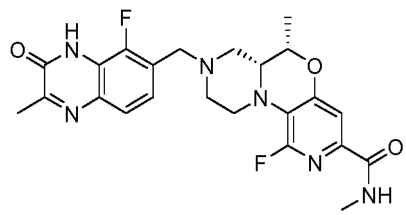,

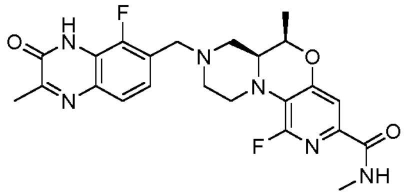,

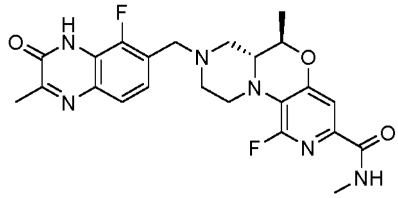,

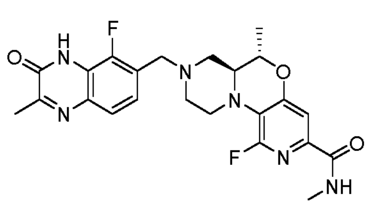, 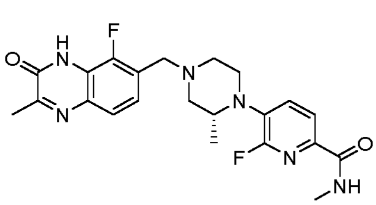, 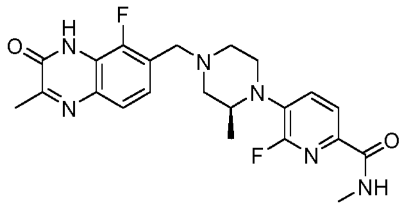,

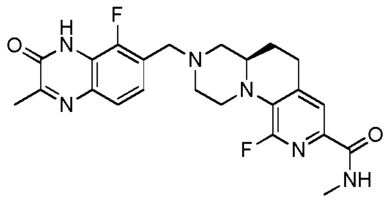, 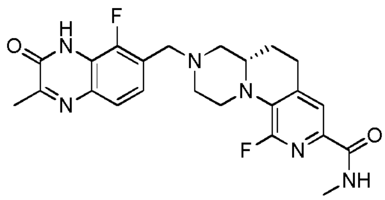, 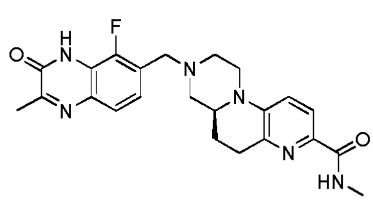,

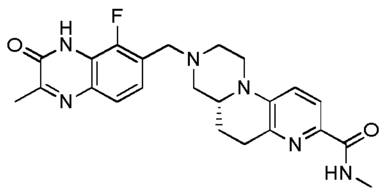,

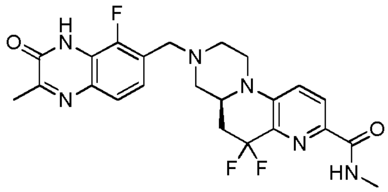,

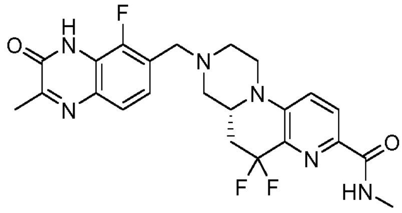,

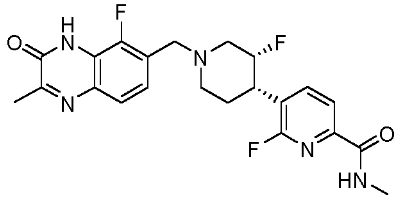,

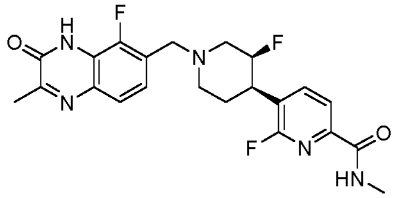 ,

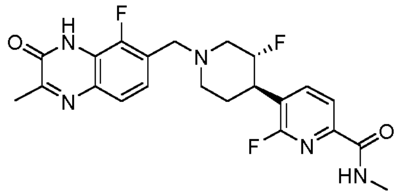 ,

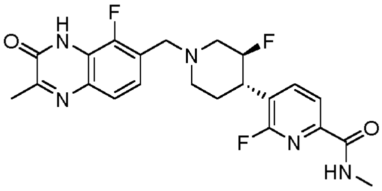 ,

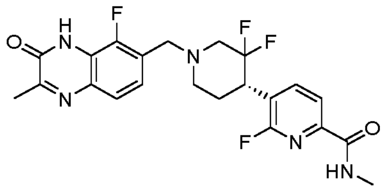 ,

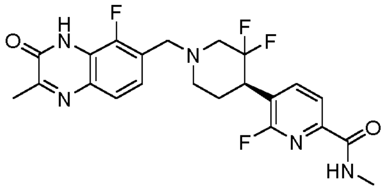 ,

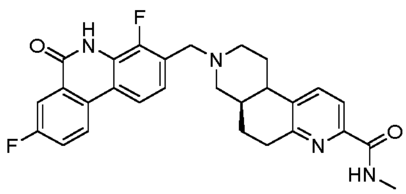 ,

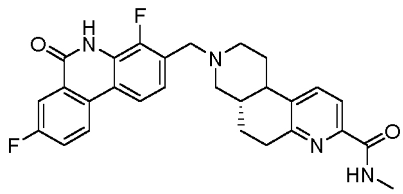 ,

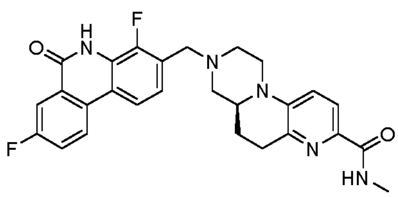 ,

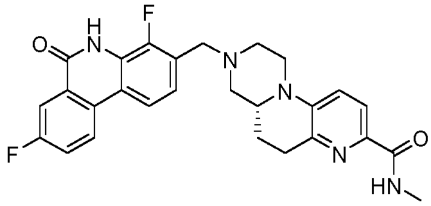 ,

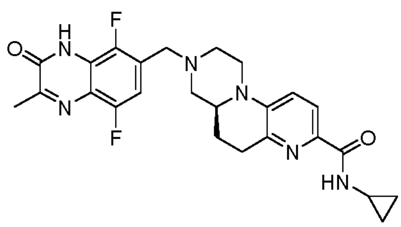 ,

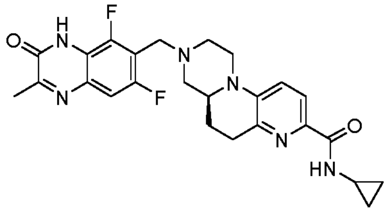 ,

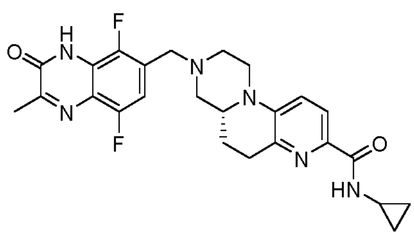 ,

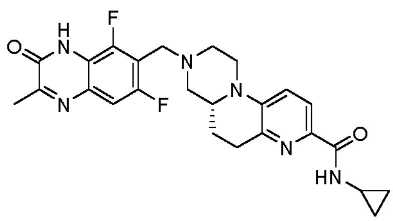 ,

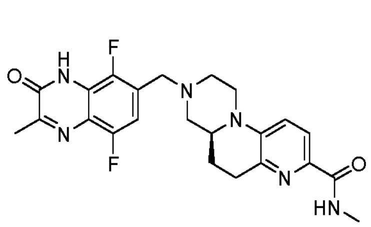 ,

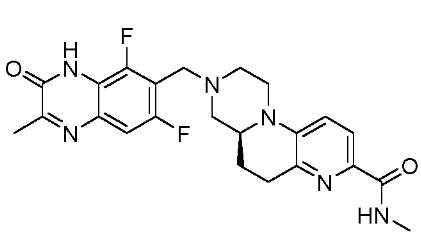 ,

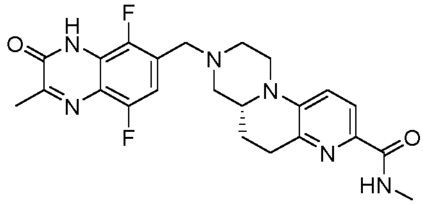,

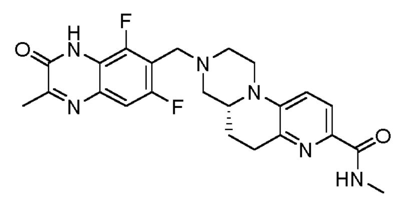,

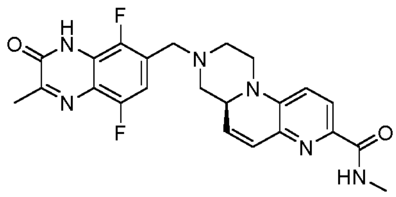,

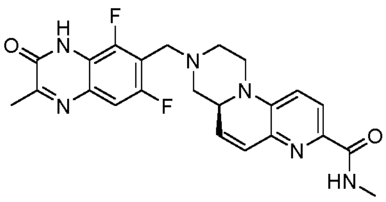,

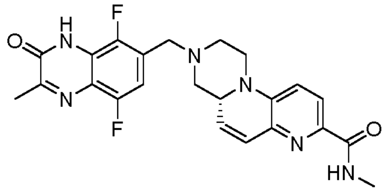,

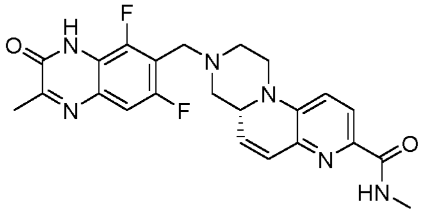,

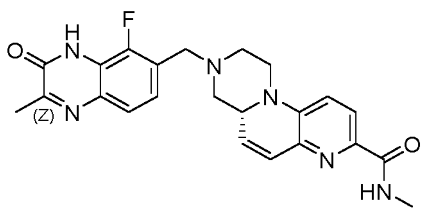,

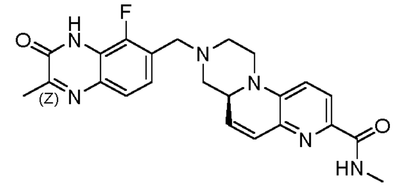,

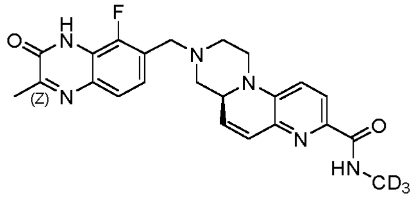,

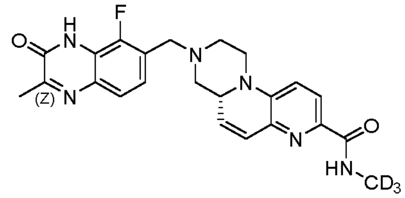,

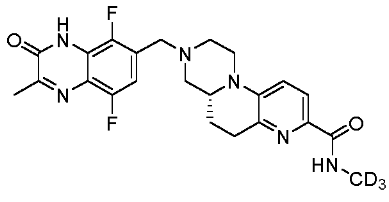,

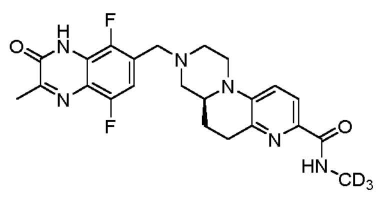,

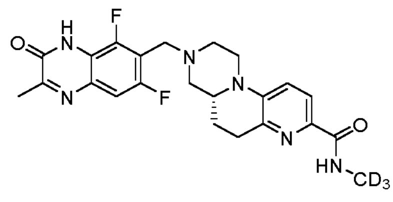,

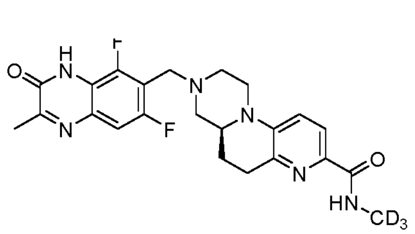,

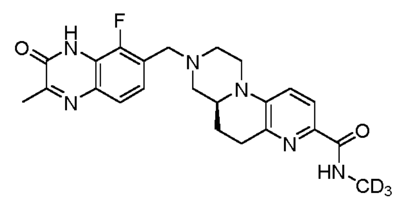,

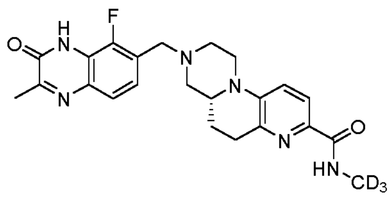

,

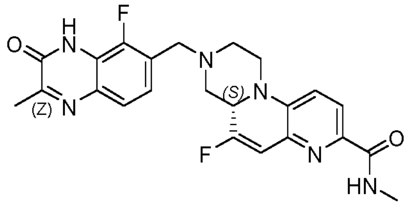

,

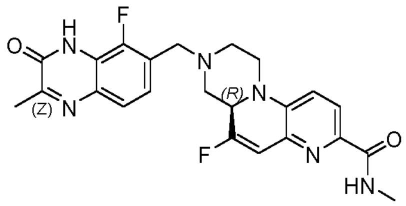

,

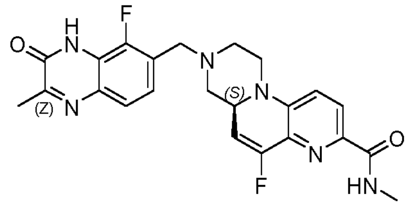

,

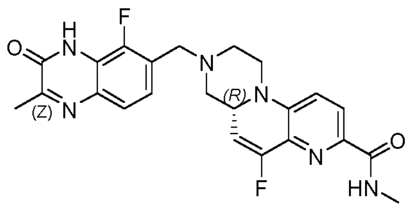

,

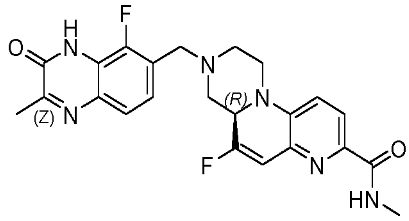

,

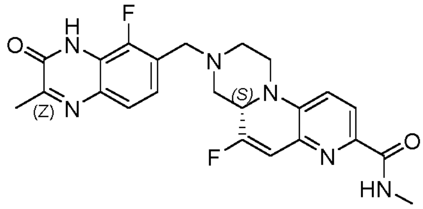

,

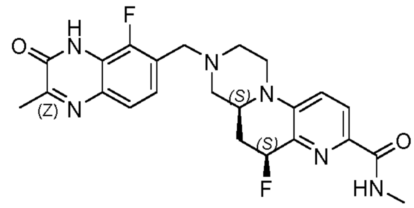

,

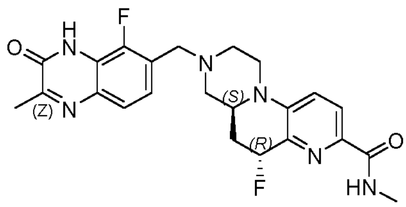

,

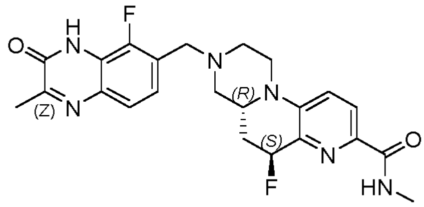

,

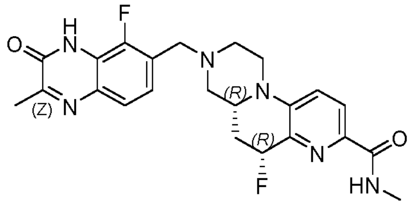

,

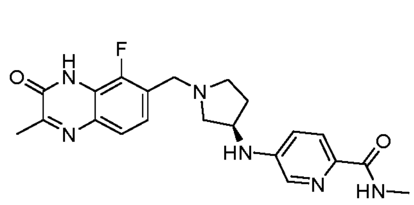

,

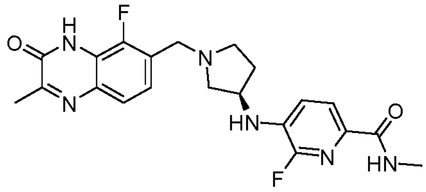

,

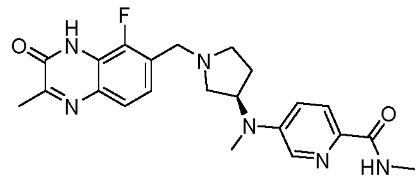

,

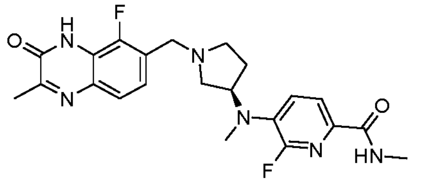
,
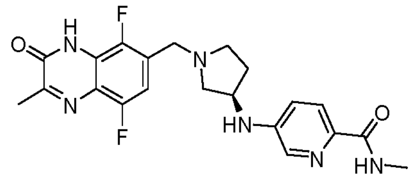
,

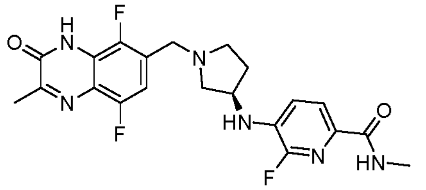
,
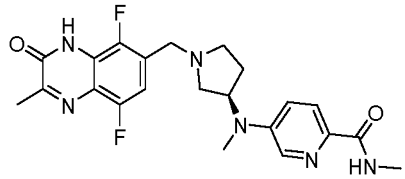
,

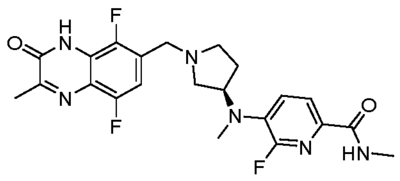
,
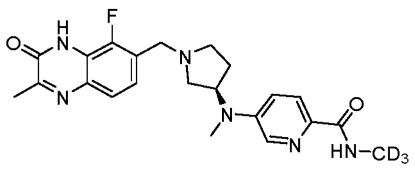
,

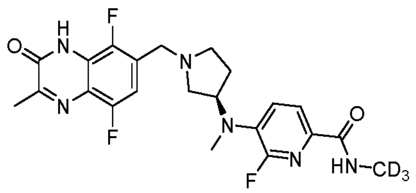
,
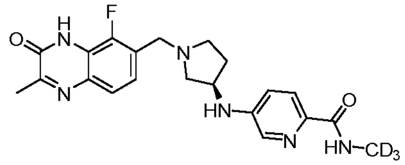
,

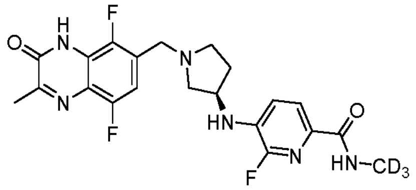
,
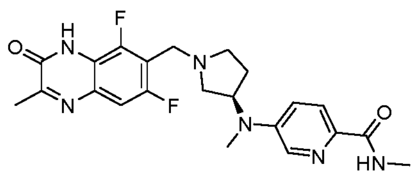
,

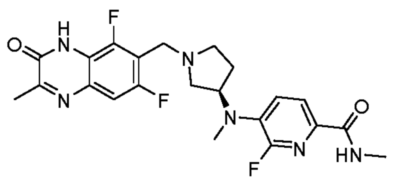
,
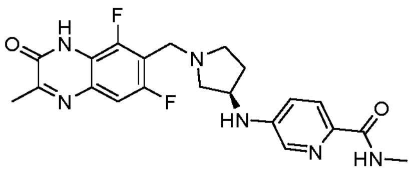
,

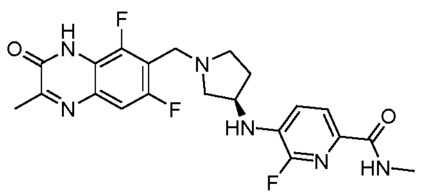
,
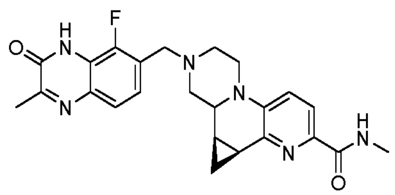
,

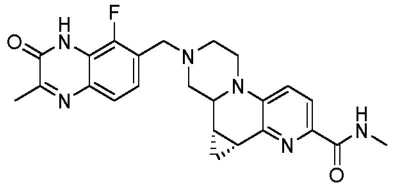
,
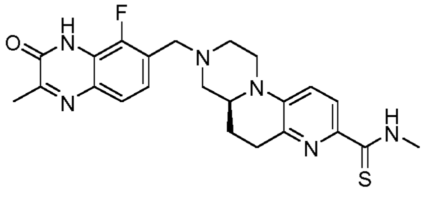
,
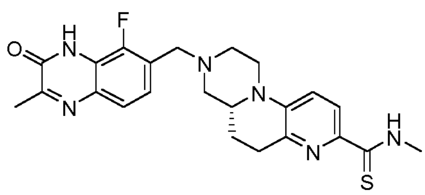
,
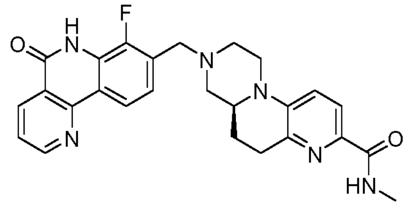
,
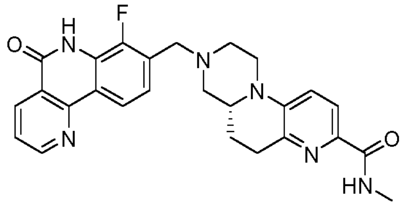
,
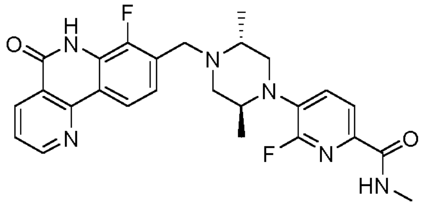
,
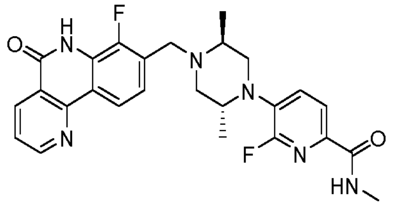
,
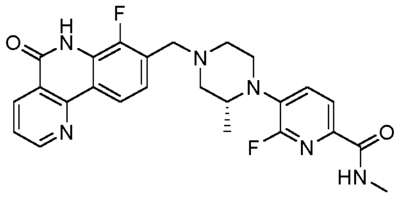
,
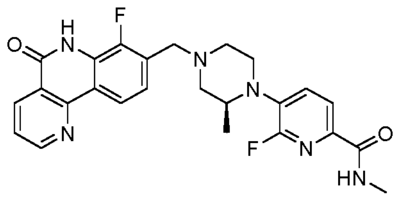
,
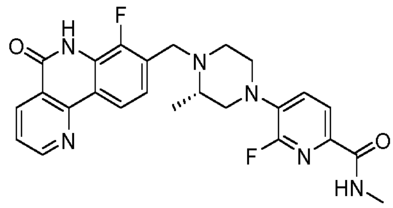
,
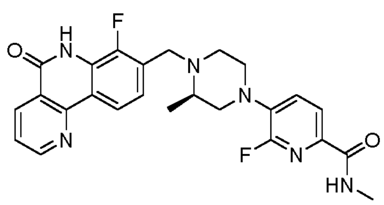
,
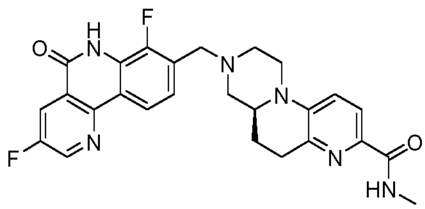
,
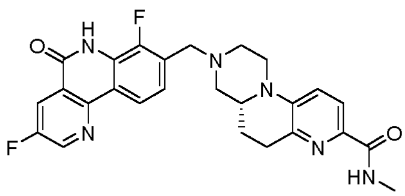
,
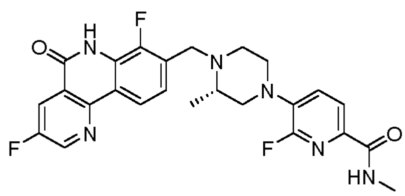
,

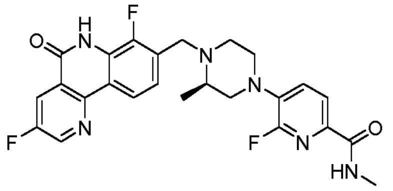
,
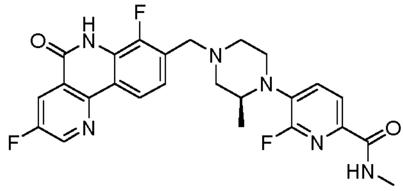
,

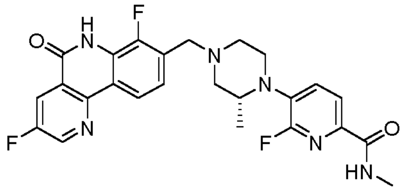
,
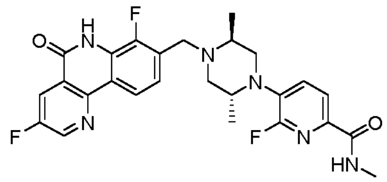
,

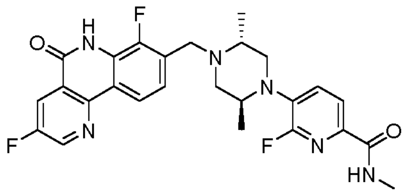
,
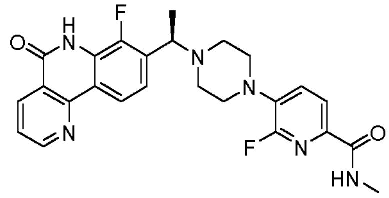
,

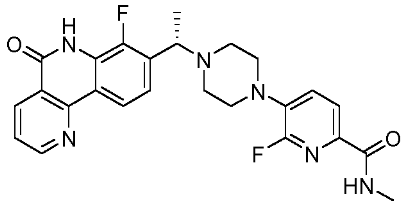
,
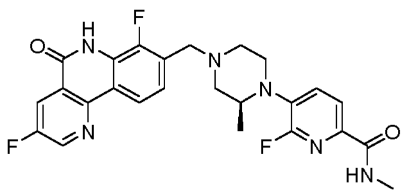
,

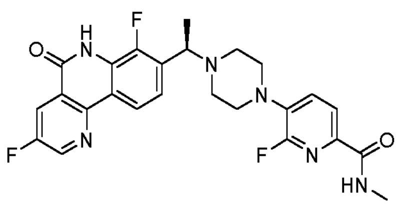
,
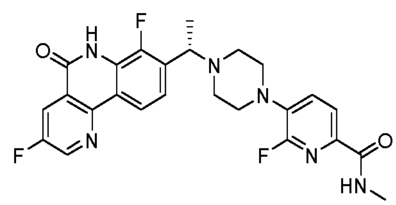
,

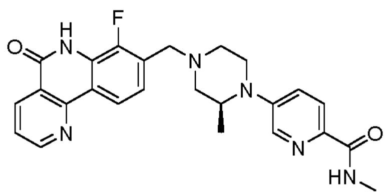
,
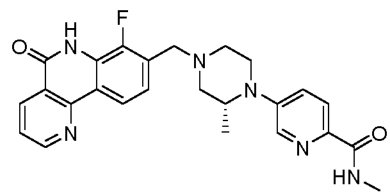
,

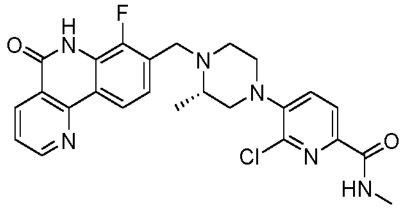
,
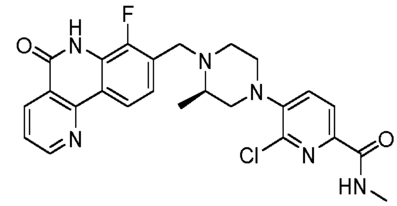
,

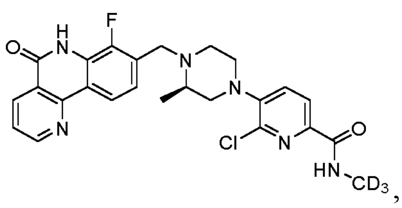, and 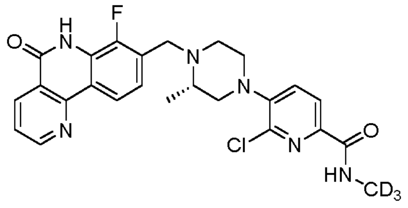.

The present disclosure further provides a pharmaceutical composition comprising a therapeutically effective amount of the compound or the pharmaceutically acceptable salt thereof as defined in the present disclosure, and a pharmaceutically acceptable carrier, diluent, or excipient.

The present disclosure further provides a use of the compound, the pharmaceutically acceptable salt thereof, or the composition in the manufacture of a medicament for treating solid tumors.

In some embodiments of the present disclosure, the solid tumors refer to solid tumors such as ovarian cancer, breast cancer, prostate cancer, and glioma.

The present disclosure further provides the following synthesis methods:

**Method 1**

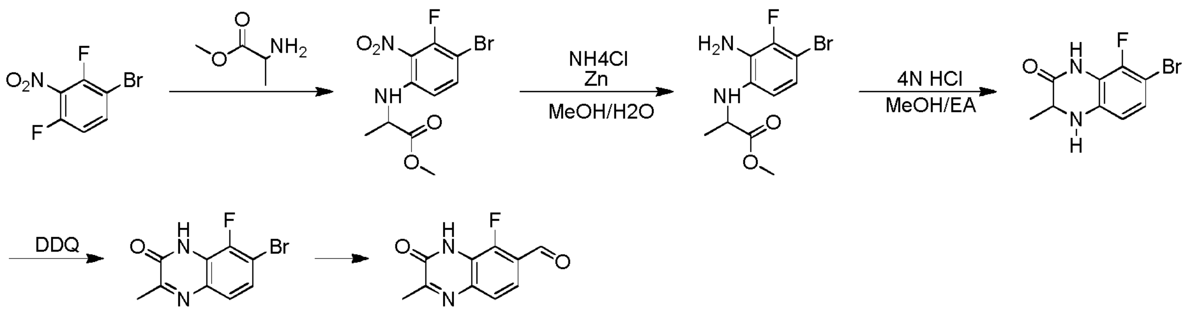


**Method 2**

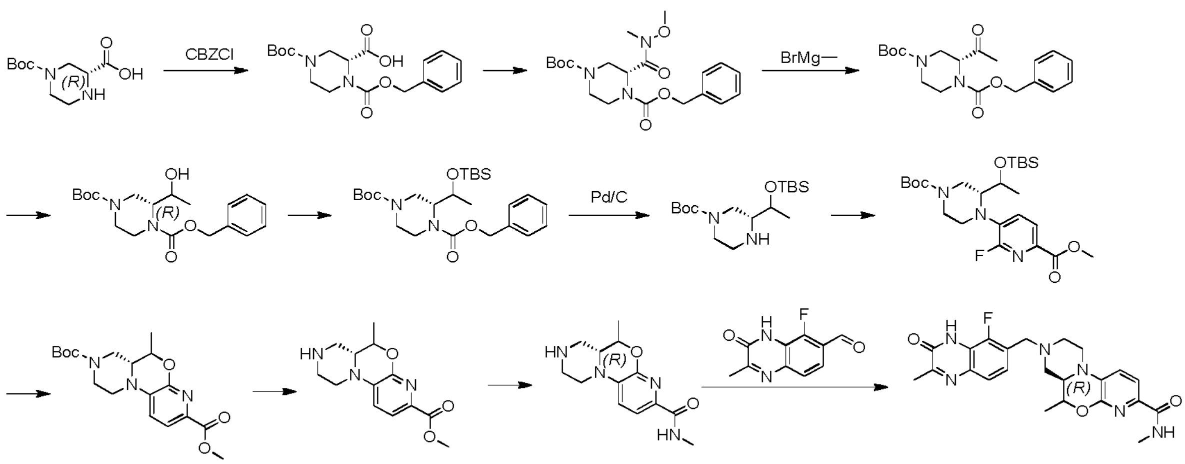


**Method 3**

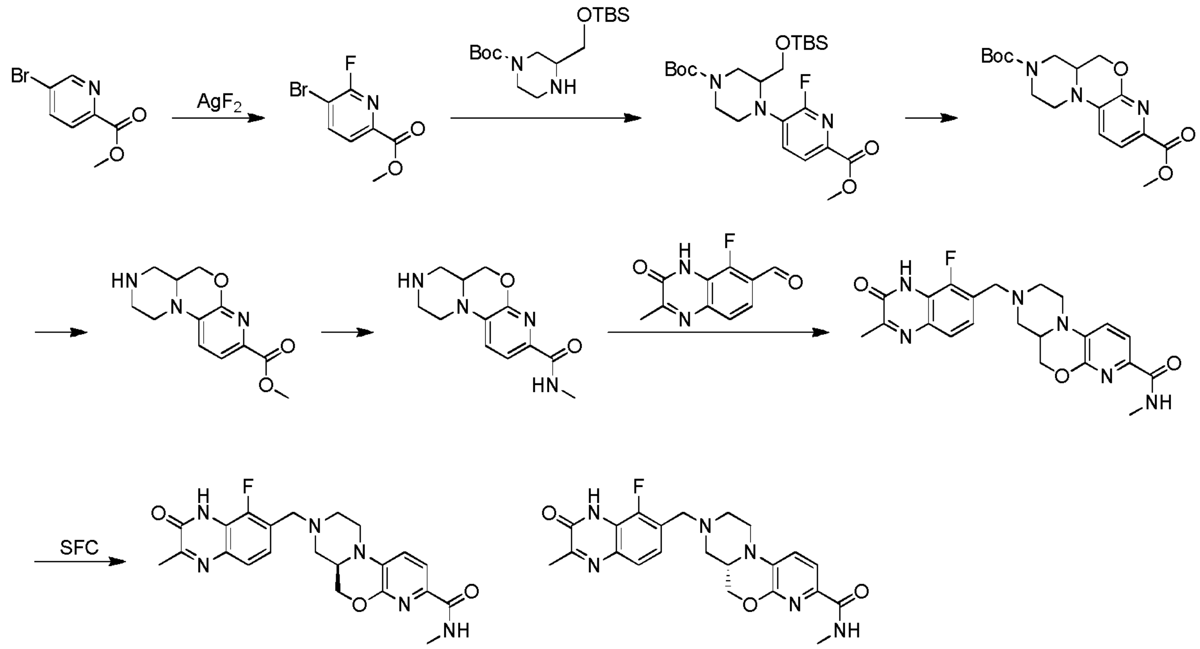


Method 4

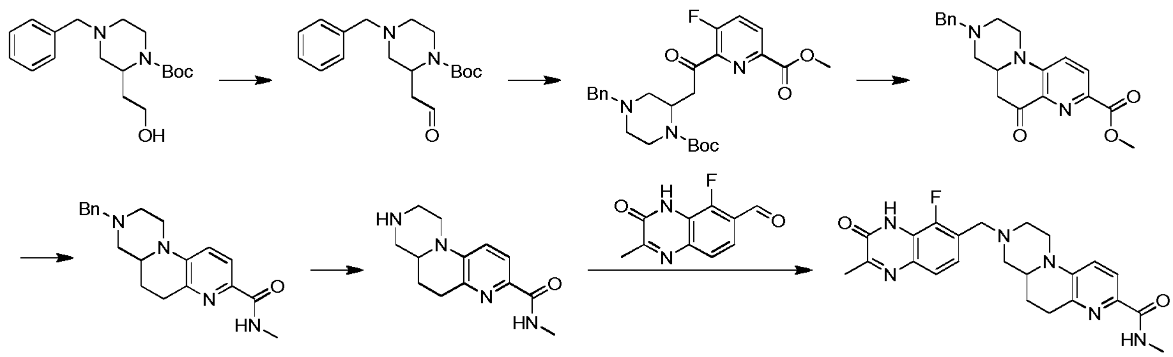

**Method 5**

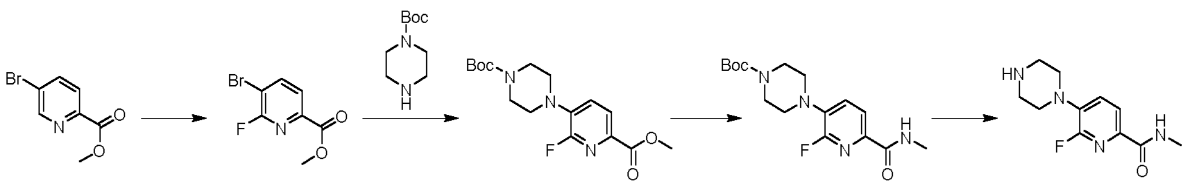

**Method 6**

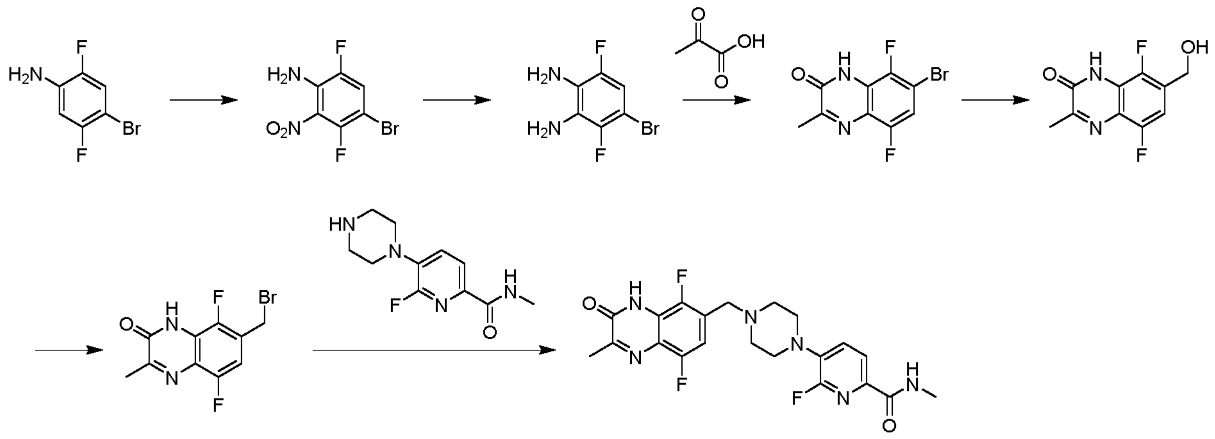

**Method 7**

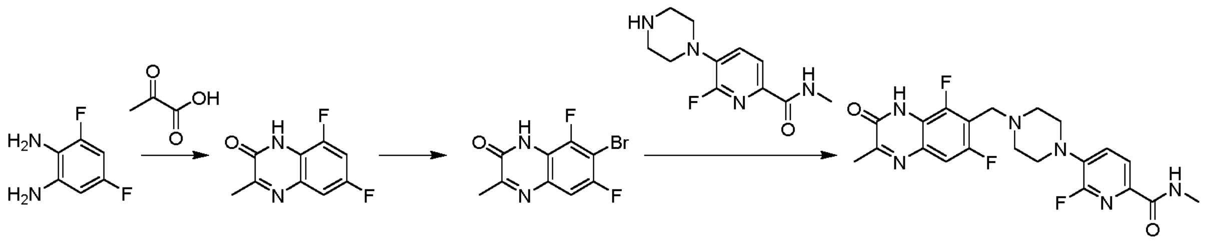

**Method 8**

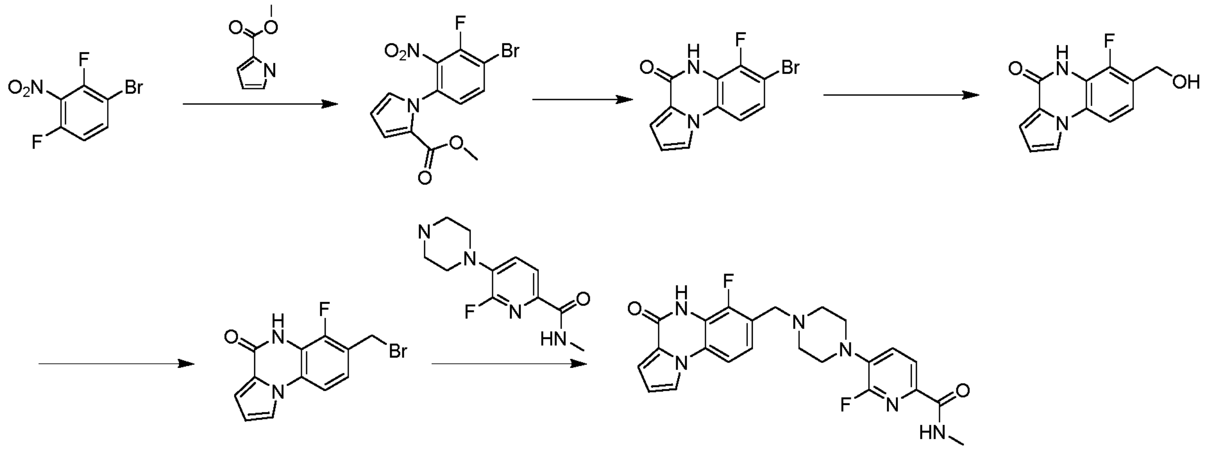

**Method 9**

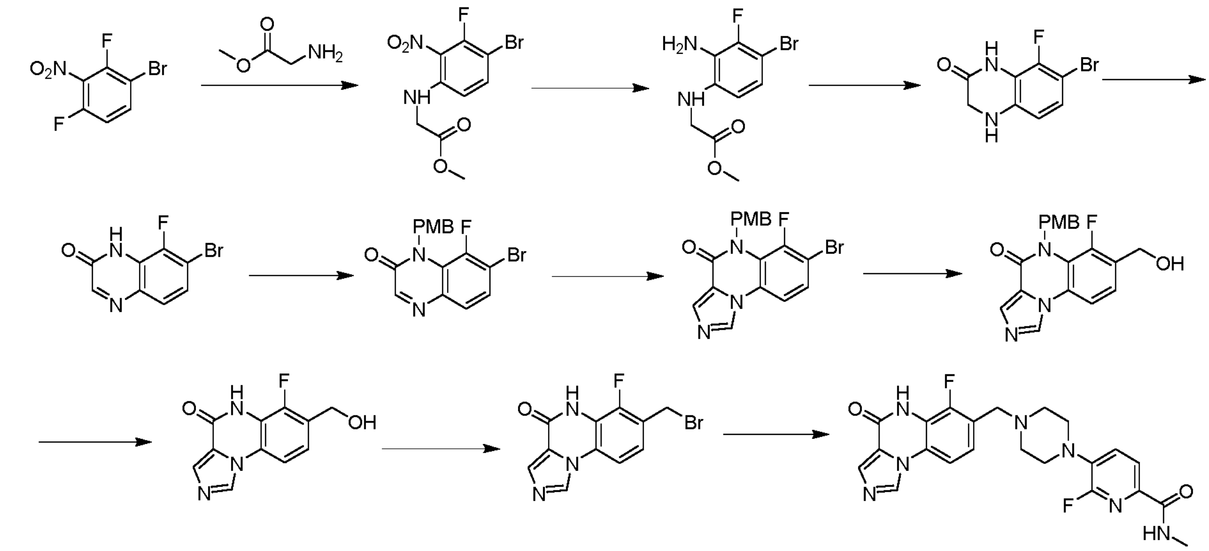

**Method 10**

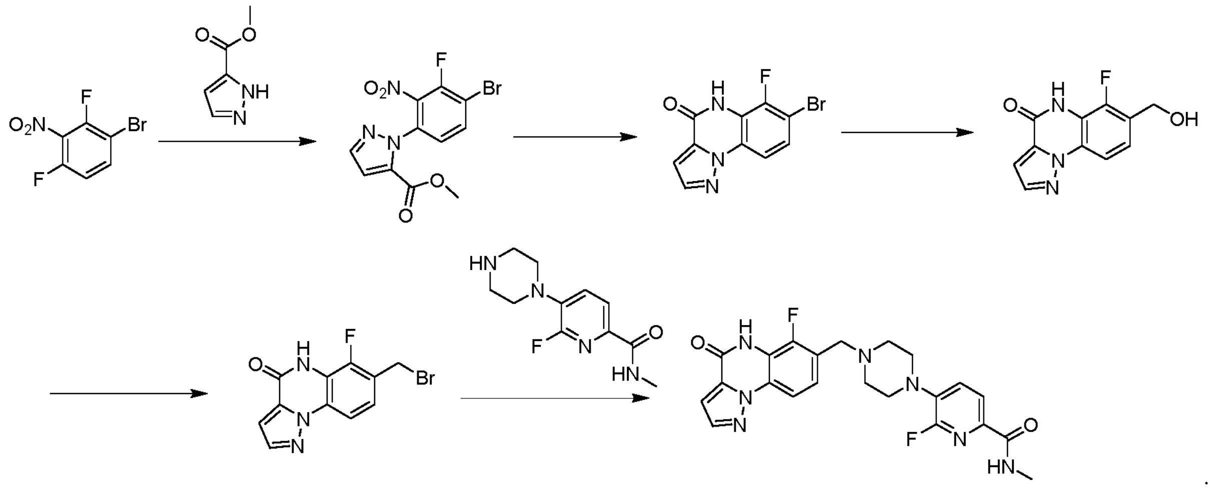

The present disclosure further provides the following bioassay methods:

**Test method 1:** PARP1 enzyme activity assay

1. Experimental materials:

PARP1 chemiluminescent assay kit purchased from BPS Bioscience; EnVision multimode microplate reader (PerkinElmer).

2. Experimental steps:

**Reagent preparation:**

Preparation of PBST buffer: 1X PBS containing 0.05% Tween-20 was prepared, that is, 5 μL of 100% Tween-20 added to 10 mL of PBS.

Preparation of 1X assay buffer: The 10X PARP assay buffer was diluted 10-fold with double-distilled water.

**Compound preparation:**

Preparation of compound solutions: The test compound was diluted 5-fold using 100% DMSO to the $8^{th}$ concentration, from 1000 μM to 12.8 nM. The internal control compound was diluted 5-fold using 100% DMSO to the $8^{th}$ concentration, from 200 μM to 2.56 nM. The test compound at each concentration gradient was further diluted using 1X assay buffer to prepare a working solution with a final DMSO concentration of 10%.

**Experimental methods:**

a) The histone solution from the kit was diluted 5-fold with 1X PBS. Then, 25 μL of the diluted solution was added to each well of the microplate, which was incubated overnight at 4°C.
b) After the incubation, the liquid in the wells was discarded. The wells were washed three times with 100 μL of PBST per well, and the remaining liquid in the wells was discarded.
c) 100 μL of blocking solution was added to each well of the microplate and incubated at 25°C for 90 minutes. After the incubation, the liquid in the wells was discarded. The wells were washed three times with 100 μL of PBST per well, and the remaining liquid in the wells was discarded.
d) 12.5 μL of substrate mixture solution (1.25 μL of 10X PARP assay buffer, 1.25 μL of 10X PARP assay mix, 2.5 μL of activated DNA, and 7.5 μL of double-distilled water) was added to each well of the microplate.
e) 2.5 μL of the compound working solution was added to each well of the microplate, with the experiment set up in duplicate wells.
f) PARP1 enzyme was diluted to a concentration of 2 ng/μL, and 10 μL of the diluted PARP1 enzyme solution was added to each well of the microplate. At this point, the final concentration gradient of the test compound ranged from 10 μM to 0.128 nM, while the final concentration gradient of the internal control compound ranged from 2 μM to 0.0256 nM. The reaction system, with PARP1 at 20 ng/well, was incubated at 25°C for 60 minutes.
g) After the incubation, the liquid in the wells was discarded. The wells were washed three times with 100 μL of PBST per well, and the remaining liquid in the wells was discarded.
h) Streptavidin-HRP was diluted 50-fold with blocking solution, and 25 μL of this diluted solution was added to each well of the microplate, which was incubated at 25°C for 30 minutes.
i) After the incubation, the liquid in the wells was discarded. The wells were washed three times with 100 μL of PBST per well, and the remaining liquid in the wells was discarded.
j) ELISA ECL substrate A and ELISA ECL substrate B were mixed at a ratio of 1:1 (v/v), and 50 μL of this mixture was added to each well of the microplate. The chemiluminescence values were then measured.

3. Method for processing experimental data

Using the equation (Sample - Min)/(Max - Min) × 100% to convert the raw data into enzyme activity, the $IC_{50}$ value might be obtained by curve fitting with four parameters (obtained by "log (inhibitor) vs. response -- Variable slope" mode in GraphPad Prism).

Max: containing 1% DMSO, PARP1, and the substrate mixture solution.
Min: without PARP1 enzyme.

**Test method 2: Study on the binding affinity of the compounds of the present disclosure to PARP1 and PARP2**

PARP1 experimental procedures:

The surface plasmon resonance (SPR) experiment was conducted on a Biacore 8K instrument (GE Healthcare).
First, under the condition of 25°C, the biotinylated PARP1 protein (sequence: 655-end) was coupled to a streptavidin-coated SA chip (Cytiva, 29699622). The specific steps were as follows: the chip surface was activated using 1 mM NaCl/50 mM NaOH. The PARP1 protein was diluted with a coupling buffer (50 mM Tris-HCl, pH 8.0, 150 mM NaCl, 10 mM $MgCl_2$, 0.05% P20) to prepare a ligand solution at a concentration of 10 μg/mL. This solution was injected over the chip surface (with an injection time of 50 seconds and an injection flow rate of 5 μL/min), coupling the PARP1 protein to the chip surface. Any excess active sites on the chip were blocked using a solution of 50% isopropanol/1 M NaCl/50 mM NaOH. The final coupling level in the experiment was 2000-3000 RU (Response Units).
Small molecule compounds were diluted by gradients in a buffer (50 mM Tris, pH 8.0, 150 mM NaCl, 10 mM

$MgCl_2$, 0.05% Tween 20) to obtain solutions with different compound concentrations. These solutions were injected over the chip surface, which was already coupled with the PARP1 protein. The injection flow rate was 50 μL/min, the injection time was 60 seconds, and the dissociation time was 20 minutes. The instrument detected the binding and dissociation curves of the protein-small molecule compounds.

The data from both the sample channel and the reference channel were analyzed using the Biacore 8K evaluation software to generate sensorgrams. The data were fitted based on a 1:1 binding model.

PARP2 experimental procedures:

The surface plasmon resonance (SPR) experiment was conducted on a Biacore 8K instrument (GE Healthcare).

First, under the condition of 25°C, the biotinylated PARP2 protein (sequence: 223-end) was coupled to a streptavidin-coated SA chip (Cytiva, 29699622). The specific steps were as follows: the chip surface was activated using 1 mM NaCl/60 mM NaOH. The PARP2 protein was diluted with a coupling buffer (50 mM Tris-HCl, pH 8.0, 150 mM NaCl, 10 mM $MgCl_2$, 0.05% P20) to prepare a ligand solution at a concentration of 10 μg/mL. This solution was injected over the chip surface (with an injection time of 50 seconds and an injection flow rate of 10 μL/min), coupling the PARP2 protein to the chip surface. Any excess active sites on the chip were blocked using a solution of 50% isopropanol/1 M NaCl/50 mM NaOH. The final coupling level in the experiment was 3000-4000 RU (Response Units).

Small molecule compounds were diluted by gradients in a buffer (50 mM Tris, pH 8.0, 150 mM NaCl, 10 mM $MgCl_2$, 0.05% Tween 20) to obtain solutions with different compound concentrations. These solutions were injected over the chip surface, which was already coupled with the PARP2 protein. The injection flow rate was 30 μL/min, the injection time was 60 seconds, and the dissociation time was 400 seconds. The instrument detected the binding and dissociation curves of the protein-small molecule compounds.

The data from both the sample channel and the reference channel were analyzed using the Biacore 8K evaluation software to generate sensorgrams. The data were fitted based on a 1:1 binding model.

**Technical effect**

The compounds of the present disclosure exhibit excellent binding activity to PARP1, selectively inhibiting the PARP1 protein while demonstrating weak binding activity to PARP2. These compounds also show superior antiproliferative activity against BRCA1-mutant MDA-MB-436 cells and BRCA2-knockout DLD 1 cells, along with excellent membrane permeability. *In vitro,* the compounds of the present disclosure demonstrate excellent stability in both human and mouse liver microsomes. The compounds of the present disclosure exhibit excellent metabolic stability *in vivo,* demonstrating excellent oral absorption and drug exposure. Oral administration also leads to high drug concentration in brain tissue, with a high brain-to-blood ratio, and displays relatively significant anti-tumor activity.

**Related definitions**

Unless otherwise specified, the following terms and phrases when used herein have the following meanings. A specific term or phrase should not be considered indefinite or unclear in the absence of a particular definition, but should be understood in the ordinary sense. When a trading name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction, or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by contacting such compounds with a sufficient amount of base in a pure solution or a suitable inert solvent. The pharmaceutically acceptable base addition salt includes a salt of sodium, potassium, calcium, ammonium, organic amine, magnesium, or similar salts. When the compound of the present disclosure contains a relatively basic functional group, an acid addition salt can be obtained by contacting the compound with a sufficient amount of acid in a pure solution or a suitable inert solvent. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including *cis* and *trans* isomers, (-)- and (+)-enantiomers, (R)- and (S)-enantiomers, diastereoisomers, *(D)*-isomers, (L)-isomers, racemic, and other mixtures thereof, such as enantiomers or diastereomer enriched mixtures, all of which are within the scope of the present disclosure. Additional asymmetric carbon atoms may be present in substituents such as alkyl. All these isomers and their mixtures are included within the scope of the present disclosure.

Unless otherwise specified, the term "enantiomer" or "optical isomer" refers to stereoisomers that are mirror images of each other.

Unless otherwise specified, the term *"cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and or "(±)" refers to racemic.

Unless otherwise specified, the absolute configuration of a stereogenic center is represented by a wedged solid bond ( ) and a wedged dashed bond ( ), and the relative configuration of a stereogenic center is represented by a straight solid bond ( ) and a straight dashed bond ( ), a wave line ( ) is used to represent a wedged solid bond ( ) or a wedged dashed bond ( ), or the wave line ( ) is used to represent a straight solid bond ( ) or a straight dashed bond ( ).

Unless otherwise specified, when a double bond structure, such as carbon-carbon double bond, carbon-nitrogen double bond, and nitrogen-nitrogen double bond, exists in the compound, and each of the atoms on the double bond is connected to two different substituents (including the condition where a double bond contains a nitrogen atom, the lone pair of electrons attached on the nitrogen atom is regarded as a substituent connected), if the atom on the double bond in the compound is connected to its substituent by a wave line ( ), this refers to the (Z) isomer, (E) isomer or a mixture of two isomers of the compound. For example, the following formula (A) means that the compound exists as a single isomer of formula (A-1) or formula (A-2) or as a mixture of two isomers of formula (A-1) and formula (A-2); the following formula (B) means that the compound exists in the form of a single isomer of formula (B-1) or formula (B-2) or in the form of a mixture of two isomers of formula (B-1) and formula (B-2). The following formula (C) means that the compound exists as a single isomer of formula (C-1) or formula (C-2) or as two a mixture of two isomers of formula (C-1) and formula (C-2).

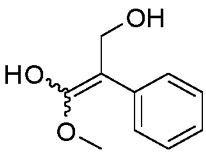 (A), 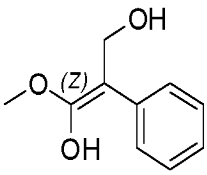 (A-1), 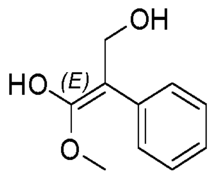 (A-2),

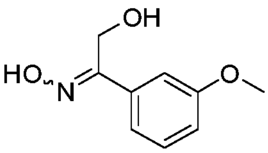 (B), 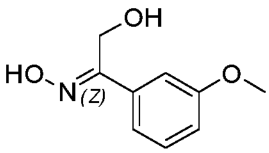 (B-1), 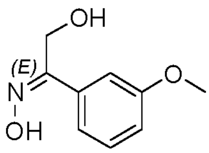 (B-2),

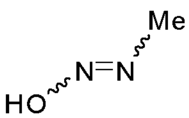 (C), 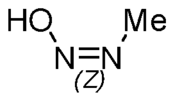 (C-1), 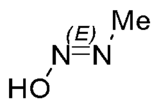 (C-2)

Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers can be reached. For example, proton tautomer (also called prototropic tautomer) includes interconversion through proton migration, such as keto-enol isomerization and imine-enamine isomerization. Valence tautomer includes some recombination of bonding electrons for mutual transformation. A specific example of keto-enol tautomerization is the tautomerism between two tautomers of

pentane-2,4-dione and 4-hydroxypent-3-en-2-one.

Unless otherwise specified, the terms "enriched in one isomer", "enriched in isomers", "enriched in one enantiomer" or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

The compound of the present disclosure may contain an unnatural proportion of atomic isotope at one or more than one atom that constitutes the compound. For example, the compound can be radiolabeled with a radioactive isotope, such as tritium ($^3H$), iodine-125 ($^{125}I$), or C-14 ($^{14}C$). For another example, deuterated drugs can be formed by replacing hydrogen with deuterium, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, *etc.* All isotopic variations of the compound of the present disclosure, whether radioactive or not, are encompassed within the scope of the present disclosure.

The term "optional" or "optionally" means that the subsequent event or condition may occur but not requisite, that the term includes the instance in which the event or condition occurs and the instance in which the event or condition does not occur.

The term "substituted" means one or more than one hydrogen atom on a specific atom is substituted by the substituent, including deuterium and hydrogen variables, as long as the valence of the specific atom is normal and the substituted compound is stable. When the substituent is an oxygen (*i.e.,* =O), it means two hydrogen atoms are substituted. The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted by 0 to -2 R, the group can be optionally substituted with up to two R, wherein the definition of R at each occurrence is independent. Moreover, a combination of the substituent and/or the variant thereof is allowed only when the combination results in a stable compound.

When the number of a linking group is 0, such as $-(CRR)_0-$, it means that the linking group is a single bond.

When one of the variables is selected from a single bond, it means that the two groups linked by the single bond are connected directly. For example, when L in A-L-Z represents a single bond, the structure of A-L-Z is actually A-Z.

Unless otherwise specified, when a group has one or more than one linkable site, any one or more than one site of the group can be linked to other groups through chemical bonds. When the linking site of the chemical bond is not positioned, and there is an H atom at the linkable site, then the number of H atoms at the site will decrease correspondingly with the number of the chemical bonds linking thereto so as to meet the corresponding valence. The chemical bond between the site and other groups can be represented by a straight solid bond

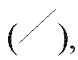

a straight dashed bond

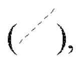

or a wavy line

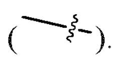

For example, the straight solid bond in $-OCH_3$ means that it is linked to other groups through the oxygen atom in the group; the straight dashed bond in

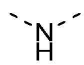

means that it is linked to other groups through the two ends of the nitrogen atom in the group; the wave lines in

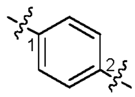

means that the phenyl group is linked to other groups through carbon atoms at position 1 and position 2.

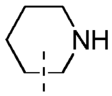

means that it can be linked to other groups through any linkable sites on the piperidinyl by one chemical bond, including at least four types of linkage, including

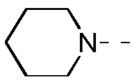,
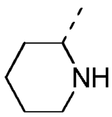,

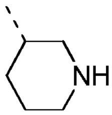,
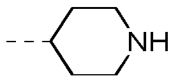.

Even though the H atom is drawn on the -N-,

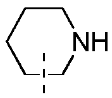

still includes the linkage of

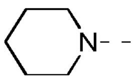,

merely when one chemical bond was connected, the H of this site will be reduced by one to the corresponding monovalent piperidinyl.

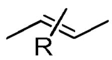

indicates that R can be connected to either end of the double bond, meaning it represents both

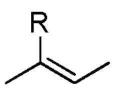 and 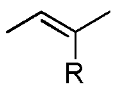.

Unless otherwise specified, the phrase "two $R_a$ on adjacent atoms, together with the atom to which they are attached, form a double bond" in the general formula means that the two $R_a$ on adjacent carbon atoms form a double bond along with the single bond between the two carbon atoms. For example, in the structural moiety

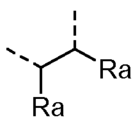,

the two $R_a$ form a double bond with the single bond between the two carbon atoms, resulting in the formation of the structural moiety

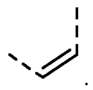

Unless otherwise specified, the term "halogen element" or "halogen" by itself or as part of another substituent refers to fluorine, chlorine, bromine, or iodine atom.

Unless otherwise specified, the term "$C_{1-3}$ alkyl" refers to a linear or branched saturated hydrocarbon group consisting of 1 to 3 carbon atoms. The $C_{1-3}$ alkyl includes $C_{1-2}$ and $C_{2-3}$ alkyl, *etc.;* it can be monovalent (such as methyl), divalent (such as methylene), or multivalent (such as methine). Examples of $C_{1--3}$ alkyl include, but are not limited to, methyl (Me), ethyl (Et), propyl (including n-propyl and isopropyl), *etc.*

Unless otherwise specified, the term "$C_{1-3}$ alkoxy" refers to an alkyl group containing 1 to 3 carbon atoms that are connected to the rest of the molecule through an oxygen atom. The $C_{1-3}$ alkoxy includes $C_{1-2}$, $C_{2-3}$, $C_3$, $C_2$ alkoxy, *etc.* Examples of $C_{1--3}$ alkoxy include, but are not limited to, methoxy, ethoxy, propoxy (including n-propoxy and isopropoxy), *etc.*

Unless otherwise specified, "$C_{3-5}$ cycloalkyl" refers to a saturated cyclic hydrocarbon group consisting of 3 to 5 carbon atoms, which is a monocyclic system, and the $C_{3-5}$ cycloalkyl includes $C_{3-4}$, $C_{4-5}$ cycloalkyl, *etc.;* it can be monovalent, divalent, or multivalent. Examples of $C_{3-5}$ cycloalkyl include, but are not limited to, cyclopropyl, cyclobutyl, cyclopentyl, *etc.*

Unless otherwise specified, the terms "5- to 6-membered heteroaryl ring" and "5- to 6- membered heteroaryl" are used interchangeably. The term "5- to 6-membered heteroaryl" refers to a monocyclic group consisting of 5 to 6 ring atoms with a conjugated $\pi$-electron system, in which 1, 2, 3, or 4 ring atoms are heteroatoms independently selected from O, S, and N, and the rest are carbon atoms, Here, the nitrogen atom is optionally quaternized, and nitrogen and sulfur heteroatoms can be optionally oxidized (i.e., NO and $S(O)_p$, wherein p is 1 or 2). The 5- to 6-membered heteroaryl can be linked to the rest of the molecule through a heteroatom or a carbon atom. The 5- to 6-membered heteroaryl includes 5-membered and 6-membered heteroaryl. Examples of the 5- to 6-membered heteroaryl include, but are not limited to, pyrrolyl (including N-pyrrolyl, 2-pyrrolyl, and 3-pyrrolyl, *etc.),* pyrazolyl (including 2-pyrazolyl and 3-pyrazolyl, *etc.),* imidazolyl (including N-imidazolyl, 2-imidazolyl, 4-imidazolyl, and 5-imidazolyl, *etc.),* oxazolyl (including 2-oxazolyl, 4-oxazolyl, and 5-oxazolyl, *etc.),* triazolyl (including 1H-1,2,3-triazolyl, 2H-1,2,3-triazolyl, 1H-1,2,4-triazolyl, and 4H-1,2,4-triazolyl, *etc.),* tetrazolyl, isoxazolyl (including 3-isoxazolyl, 4-isoxazolyl, and 5-isoxazolyl, *etc.),* thiazolyl (including 2-thiazolyl, 4-thiazolyl, and 5-thiazolyl, *etc.),* furyl (including 2-furyl and 3-furyl, *etc.),* thienyl (including 2-thienyll and 3-thienyl, *etc.),* pyridyl (including 2-pyridyl, 3-pyridyl, and 4-pyridyl, *etc.),* pyrazinyl, pyrimidinyl (including 2-pyrimidinyl, 4-pyrimidinyl, *etc.).*

Unless otherwise specified, the terms "6-membered heteroaryl ring" and "6-membered heteroaryl" in the present disclosure can be used interchangeably, and the term "6-membered heteroaryl" refers to a monocyclic group consisting of 6 ring atoms with a conjugated $\pi$-electron system, wherein 1, 2, 3, or 4 ring atoms are heteroatoms independently selected from O, S, and N, and the rest are carbon atoms. Here, the nitrogen atom is optionally quaternized, and nitrogen and sulfur heteroatoms can be optionally oxidized (i.e., NO and $S(O)_p$, wherein p is 1 or 2). The 5- to 6-membered heteroaryl can be linked to the rest of the molecule through a heteroatom or a carbon atom.

Unless otherwise specified, $C_{n-n+m}$ or $C_n$-$C_{n+m}$ includes any specific case of n to n+m carbons, for example, $C_{1-12}$ includes $C_1$, $C_2$, $C_3$, $C_4$, $C_5$, $C_6$, $C_7$, $C_8$, $C_9$, $C_{10}$, $C_{11}$, and $C_{12}$, and any range from n to n+m is also included, for example $C_{1-12}$ includes $C_{1-3}$, $C_{1-6}$, $C_{1-9}$, $C_{3-6}$, $C_{3-9}$, $C_{3-12}$, $C_{6-9}$, $C_{6-12}$, $C_{9-12}$, *etc.;* similarly, n-membered to n+m-membered means that the number of atoms on the ring is from n to n+m, for example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring, and any range from n to n+m is also included, for example, 3- to 12-membered ring includes 3- to 6-membered ring, 3- to 9-membered ring, 5- to 6-membered ring, 5- to 7-membered ring, 6- to 7-membered ring, 6- to 8-membered ring, 6- to 10-membered ring, *etc.*

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, and preferred embodiments include, but are not limited to, the examples of the present disclosure.

The structure of the compounds of the present disclosure can be confirmed by conventional methods known to those skilled in the art, and if the present disclosure involves an absolute configuration of a compound, then the absolute configuration can be confirmed by means of conventional techniques in the art. For example, in the case of single crystal X-ray diffraction (SXRD), the absolute configuration can be confirmed by collecting diffraction intensity data from the cultured single crystal using a Bruker D8 venture diffractometer with CuK$\alpha$ radiation as the light source and scanning mode: $\varphi/\omega$

scan, and after collecting the relevant data, the crystal structure can be further analyzed by the direct method (Shelxs97).

The solvents used in the present disclosure are commercially available. The present disclosure uses the following abbreviations: hr represents hours; min represents minutes; DIEA represents N,N-diisopropylethylamine; DDQ represents 2,3-dichloro-5,6-dicyanobenzoquinone; XPHOS-PD-G2 represents chloro(2-dicyclohexylphosphino-2,4,6-triisopropyl-1,1'-biphenyl)[2-(2-amino-1,1'-biphenyl)]palladium(II); TBSCl represents tertbutyldimethylsilyl chloride; DMAP represents 4-dimethylaminopyridine; $Cs_2CO_3$ represents cesium carbonate; RuPhos represents 2-dicyclohexylphosphino-2,6-diisopropoxy-1,1'-biphenyl; $Pd_2(dba)_3$ represents tris(dibenzylideneacetone)dipalladium; TBAF represents tetrabutylammonium fluoride; $Et_3N$ represents triethylamine; $Na_2CO_3$ represents sodium carbonate; $NaHCO_3$ represents sodium bicarbonate; $H_2O$ represents water; PE represents petroleum ether; EA represents ethyl acetate; DMF represents *N*,*N*-dimethylformamide; MeOH represents methanol; EtOH represents ethanol; THF represents tetrahydrofuran; DCM represents dichloromethane; NBS represents *N*-bromosuccinimide; Dess-Martin represents Dess-Martin periodinane, CAS No. 87413-09-0; KHMDS represents potassium bis(trimethylsilyl)amide; $Pd(OAc)_2$ represents palladium acetate; $PPh_3$ represents triphenylphosphine; Pd/C represents palladium on carbon; HATU represents 2-(7-azabenzotriazol-1-yl)-N,N,N',N'-tetramethyluronium hexafluorophosphate; $(Boc)_2O$ represents di-tert-butyl dicarbonate; $LiAlH_4$ represents lithium aluminum hydride; N-Boc-piperazine represents 1-tert-butoxycarbonylpiperazine; Lawesson's reagent represents the compound with CAS No. 19172-47-5; DMSO represents dimethyl sulfoxide; $NaBH(OAc)_3$ represents sodium triacetoxyborohydride; KI represents potassium iodide; $NH_4Cl$ represents ammonium chloride; HCl represents hydrochloric acid; dioxane represents 1,4-dioxane; $Pd(dppf)Cl_2$ represents [1,1'-bis(diphenylphosphino)ferrocene]dichloropalladium.

## BRIEF DESCRIPTION OF THE DRAWINGS

FIG. 1: Diagram showing tumor growth volumes.
FIG. 2: Diagram showing body weight changes of mice during drug administration.

## DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific examples have also been disclosed; for those skilled in the art, it is obvious to make various modifications and improvements to the specific examples of the present disclosure without departing from the spirit and scope of the present disclosure.

### Example 1

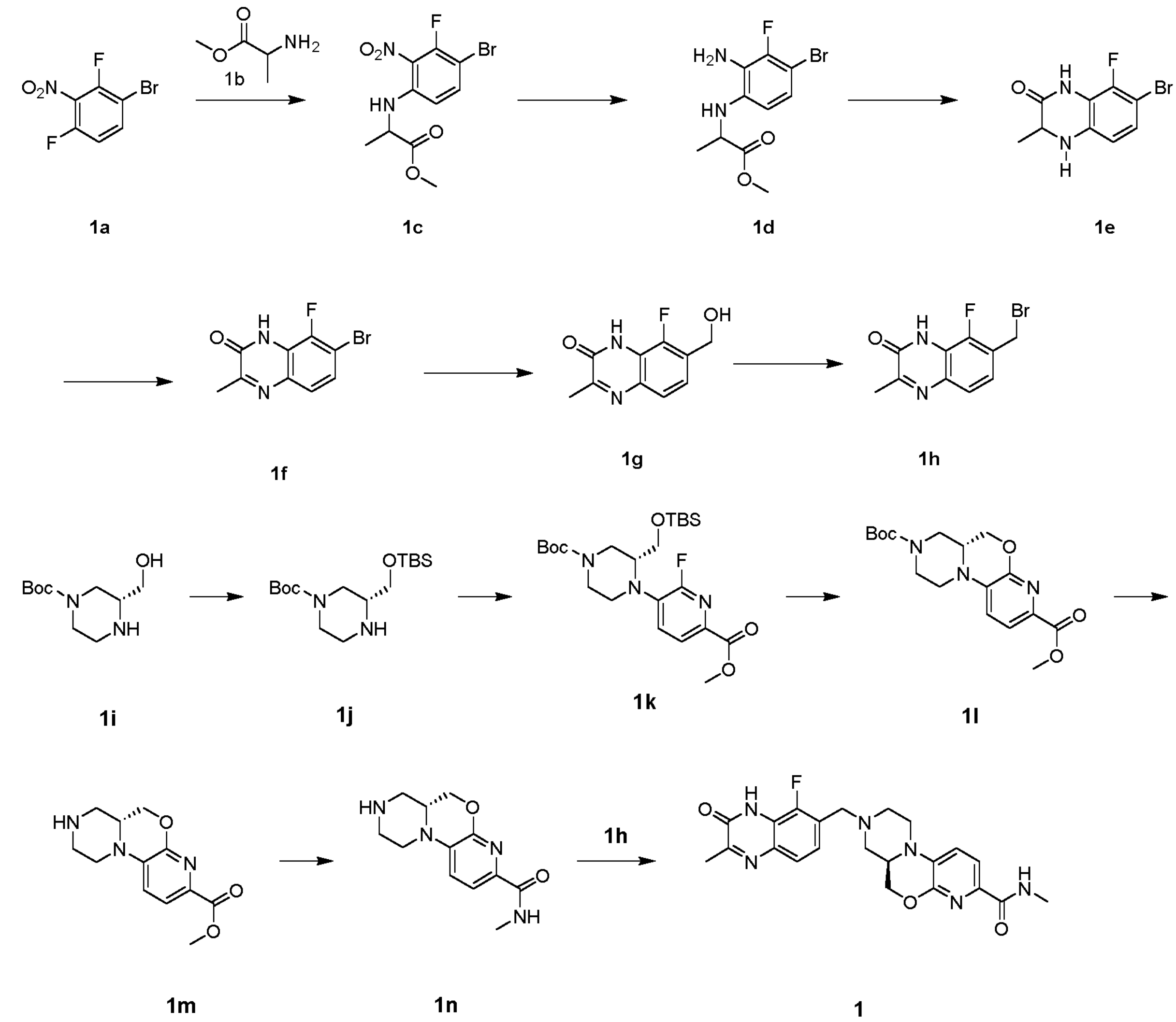


1a 1c 1d 1e

1f 1g 1h

1i 1j 1k 1l

1m 1n 1

Step 1: Synthesis of intermediate **1c**

Compound **1a** (9.5 g, 39.92 mmol), DIEA (15.48 g, 119.75 mmol, 20.86 mL), and compound **1b** (6.13 g, 43.91 mmol, HCl) were added to DMF (100 mL) and the reaction mixture was stirred at 25°C for 18 hours. Water (1000 mL) was then added to the reaction mixture. The mixture was extracted with ethyl acetate (3 × 100 mL). The organic phases were combined, dried, and concentrated to obtain a crude product. The crude product was subjected to column chromatography (eluent: PE:EA = 4:1, v/v) to obtain intermediate **1c.** MS m/z: 320.8, 322.8 $[M+H]^+$.

Step 2: Synthesis of intermediate **1d**

At 0°C, intermediate **1c** (7.4 g, 23.05 mmol), $NH_4Cl$ (9.86 g, 184.37 mmol), and zinc powder (7.7 g, 117.76 mmol) were added to a mixture of MeOH (100 mL) and $H_2O$ (2 mL). The reaction mixture was stirred for 2 hours. The reaction mixture was then filtered, and the filtrate was evaporated under reduced pressure to remove methanol. Water (100 mL) was added thereto, and the mixture was extracted with ethyl acetate (2 × 30 mL). The organic phases were combined, dried, and filtered. The filtrate was concentrated under reduced pressure to obtain intermediate **1d,** which was used directly in the next step. MS m/z: 290.7, 292.7 $[M+H]^+$.

Step 3: Synthesis of intermediate **1e**

At 0°C, intermediate **1d** (6.7 g, 23.01 mmol) was added to a mixed solvent of 4 M HCl in dioxane (1.81 mL), ethyl acetate (70 mL), and methanol (70 mL). The reaction mixture was stirred for 2 hours. The reaction mixture was then filtered, and the filtrate was concentrated under reduced pressure to obtain intermediate **1e** (5.5 g, 21.23 mmol). $^1H$ NMR (400 MHz, DMSO-$d_6$) δ ppm 10.46 (s, 1 H) 7.42 - 7.57 (m, 1 H) 7.00 (dd, *J*=8.53, 7.28 Hz, 1 H) 6.49 (d, *J*=8.03 Hz, 1 H) 3.82 - 3.86

(m, 1 H) 1.25 (d, *J*=6.53 Hz, 3 H).

Step 4: Synthesis of intermediate **1f**

At 0°C, DDQ (5.26 g, 23.16 mmol) was added in batches to a solution of intermediate **1e** (5.0 g, 19.30 mmol) in DCM (250 mL). The temperature was then raised to 25°C, and the reaction mixture was stirred for 2 hours and evaporated under reduced pressure to remove the solvent. A saturated $NaHCO_3$ aqueous solution (200 mL) was slowly added to quench the reaction, and the mixture was stirred overnight. The reaction mixture was extracted with ethyl acetate (3 × 50 mL), and the organic phases were combined and concentrated to obtain the target product, which was subjected to column chromatography (eluent: DCM:MeOH = 100:1, v/v) to obtain intermediate **1f.** MS m/z: 256.7, 258.7 $[M+H]^+$, $^1H$ NMR (400 MHz, DMSO-$d_6$) δ ppm 12.61 (br s, 1 H) 7.45 - 7.55 (m, 2 H) 2.40 (s, 3 H).

Step 5: Synthesis of intermediate **1g**

Intermediate **1f** (1.0 g, 3.89 mmol), 1-(tributylstannyl)methanol (1.50 g, 4.67 mmol), and XPHOS-PD-G2 (153.04 mg, 194.51 μmol) were added to 1,4-dioxane (20 mL). The reaction mixture was stirred at 80°C under a nitrogen atmosphere for 4 hours. The reaction mixture was then concentrated under reduced pressure to obtain a crude product. The crude product was purified by column chromatography (eluent: DCM/MeOH = 20/1, v/v) to obtain intermediate **1g.** MS m/z: 209.0 $[M+1]^+$.

Step 6: Synthesis of intermediate **1h**

Under a nitrogen atmosphere at 0°C, tributylphosphine (388.72 mg, 1.92 mmol, 474.05 μL) was slowly added dropwise to a solution of 1,2-dibromotetrachloroethane (688.23 mg, 2.11 mmol, 253.96 μL) and intermediate **1g** (200 mg, 960.67 μmol) in DCM (5 mL). The reaction mixture was stirred at 20°C for 3 hours. The reaction mixture was then concentrated under reduced pressure to obtain a crude product. A mixed solvent of DCM and MeOH (20:1, 5 mL) was added to the crude product. The mixture was stirred for 30 minutes and filtered. The filter cake was concentrated under reduced pressure to obtain intermediate **1h.** $^1H$ NMR (400 MHz, DMSO-$d_6$) δ ppm 12.55 (s, 1 H) 7.47 - 7.58 (m, 1 H) 7.31 - 7.43 (m, 1 H) 4.80 - 4.90 (m, 2 H) 2.42 (s, 3 H).

Step 7: Synthesis of intermediate **1j**

Triethylamine (1.87 g, 18.49 mmol) was added to a solution of intermediate **1i** (2 g, 9.25 mmol), TBSCl (2.09 g, 13.87 mmol), and DMAP (112.97 mg, 924.74 μmol) in DCM (40 mL). The reaction mixture was stirred at 20°C for 16 hours. The reaction mixture was then concentrated under reduced pressure to obtain a crude product. The crude product was purified by column chromatography (eluent: DCM/MeOH = 20/1 to 10/1, v/v) to obtain intermediate 1j. $^1H$ NMR (400 MHz, $CD_3OD$) δ ppm 3.88 - 4.12 (m, 2 H) 3.63 (d, J=5.52 Hz, 2 H) 3.01 (d, J=12.30 Hz, 1 H) 2.54 - 2.94 (m, 4 H) 1.48 (s, 9 H) 0.95 (s, 9 H) 0.12 (s, 6 H).

Step 8: Synthesis of intermediate **1k**

$Cs_2CO_3$ (197.15 mg, 605.08 μmol), RuPhos (14.12 mg, 30.25 μmol), intermediate 1j (0.1 g, 302.54 μmol), methyl 5-bromo-6-fluoro-pyridine-2-carboxylate (70.80 mg, 302.54 μmol), and $Pd_2(dba)_3$ (55.41 mg, 60.51 μmol) were added to a toluene solution (4 mL). The reaction mixture was stirred at 100°C for 24 hours. The reaction mixture was subjected to suction filtration under reduced pressure, and the filtrate was concentrated under reduced pressure to obtain a crude product. The crude product was separated by column chromatography (eluent: PE:EA= 1:1, v/v) to obtain intermediate **1k.** MS m/z: 484.3 $[M+H]^+$.

Step 9: Synthesis of intermediate **11**

TBAF (1 M in THF, 258.45 μL, 2.5 eq) and intermediate **1k** (50 mg, 103.38 μmol) were added to a THF solution (4 mL). The reaction mixture was stirred at 25°C for 16 hours. The reaction mixture was then concentrated under reduced pressure to obtain intermediate **11.** MS m/z: 350.2 $[M+H]^+$.

Step 10: Synthesis of hydrochloride of intermediate **1m**

Intermediate **11** (30 mg, 85.87 μmol) was added to an EA solution (2 mL). HCl/EA (4 M, 85.87 μL) was added dropwise to the reaction mixture. The mixture was stirred at 25°C for 2 hours. The reaction mixture was concentrated under

reduced pressure to obtain the hydrochloride of intermediate **1m.** MS m/z: 250.2 $[M+H]^+$.

Step 11: Synthesis of intermediate **1n**

Under a nitrogen atmosphere, the hydrochloride of intermediate **1m** (20 mg) was added to an EtOH solution (2 mL). Methylamine (2.26 mg, 72.84 μmol) was then added thereto. The mixture was stirred at 20°C for 1 hour. The reaction mixture was concentrated under reduced pressure to obtain intermediate **1n.** MS m/z: 249.1 $[M+H]^+$.

Step 12: Synthesis of trifluoroacetate of compound **1**

Intermediate **1n** (10.50 mg, 36.89 μmol) was added to DMF (2 mL). $Et_3N$ (7.47 mg, 73.78 μmol, 10.27 μL) was then added thereto. The mixture was stirred at 25°C for 30 minutes. KI (2.70 mg, 16.24 μmol, 5.68 μL) and intermediate **1h** (10 mg, 36.89 μmol) were then added to the reaction mixture, and the mixture was stirred at 50°C for 2 hours. The reaction mixture was concentrated under reduced pressure using an oil pump at 60°C to obtain a crude product. The crude product was purified by preparative HPLC (column: Welch Xtimate C18, 100 × 40 mm × 3 μm; mobile phase: [water (trifluoroacetic acid)-acetonitrile]; acetonitrile%: 0%-23% over 8 minutes) to obtain the trifluoroacetate of compound 1. MS m/z: 439.2 $[M+H]^+$, $^1$H NMR (400 MHz, $CD_3OD$) δ ppm 7.63 - 7.73 (m, 2 H) 7.36 - 7.50 (m, 2 H) 4.44 - 4.54 (m, 3 H) 4.22 (dd, *J=11.26,* 7.63 Hz, 1 H) 4.13 (d, *J=11.01* Hz, 1 H) 3.51 - 3.64 (m, 3 H) 3.11 - 3.22 (m, 2 H) 2.92 (s, 3 H) 2.85 - 2.91 (m, 1 H) 2.56 (s, 3 H).

## Example 2

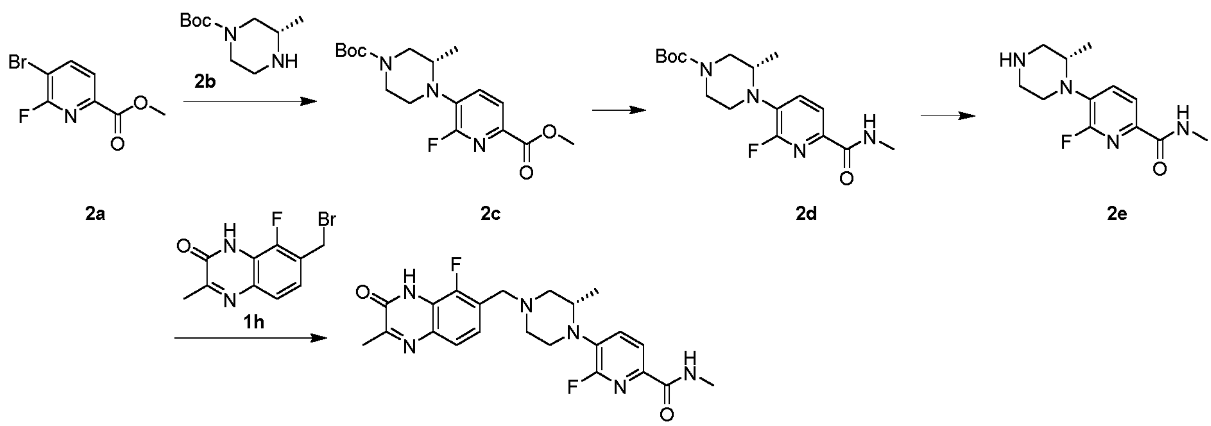


2a 2c 2d 2e

2

Step 1: Synthesis of intermediate 2c

Compound **2a** (0.5 g, 2.14 mmol) and compound 2b (642.89 mg, 3.21 mmol) were added to toluene (10 mL). $Cs_2CO_3$ (2.09 g, 6.42 mmol), RuPhos (199.72 mg, 428.00 μmol), and $Pd_2(dba)_3$ (195.96 mg, 214.00 μmol) were then added thereto. The reaction mixture was purged with nitrogen three times and stirred at 100°C for 16 hours. The reaction mixture was subjected to suction filtration under reduced pressure, and the filtrate was concentrated under reduced pressure to obtain a crude product. The crude product was purified by column chromatography (eluent: PE/EA = 4/1 to 2/1, v/v) to obtain intermediate 2c. MS m/z: 354.2 $[M+1]^+$, $^1$H NMR (400 MHz, $CDCl_3$) δ ppm 7.99 (d, J=8.03 Hz, 1 H) 7.22 - 7.29 (m, 1 H) 4.01 (d, *J*=6.53 Hz, 1 H) 4.00 - 4.24 (m, 1 H) 3.98 (s, 3 H) 3.81 (d, *J*=13.30 Hz, 1 H) 3.29 - 3.44 (m, 2 H) 3.07 - 3.25 (m, 2 H) 1.50 (s, 9 H) 1.08 (d, *J*=6.53 Hz, 3 H).

Step 2: Synthesis of intermediate **2d**

Intermediate **2c** (0.4 g, 1.13 mmol) and methylamine in THF (2 M, 7.27 mL) were added to EtOH (10 mL), and the reaction mixture was stirred at 20°C for 16 hours. The reaction mixture was concentrated under reduced pressure to obtain the crude intermediate **2d,** which was used directly in the next step. MS m/z: 353.0 $[M+H]^+$.

Step 3: Synthesis of hydrochloride of intermediate **2e**

Intermediate **2d** (0.4 g, 1.14 mmol) was added to EA (5 mL). HCl in ethyl acetate (4 M, 1.42 mL) was then added thereto. The reaction mixture was stirred at 20°C for 16 hours. The reaction mixture was concentrated under reduced pressure to obtain the hydrochloride of the crude intermediate **2e,** which was used directly in the next step. MS m/z: 253.0 $[M+H]^+$.

Step 4: Synthesis of trifluoroacetate of compound **2**

Intermediate **2e** (21.30 mg) was added to DMF (2 mL). Then, $Et_3N$ (14.93 mg, 147.56 μmol, 20.54 μL) was added thereto. The reaction mixture was stirred at 25°C for 30 minutes. KI (5.39 mg, 32.49 μmol) and intermediate **1h** (20 mg, 73.78 μmol) were then thereto. The reaction mixture was stirred at 50°C for 2 hours. The reaction mixture was concentrated under reduced pressure to obtain a crude product. The crude product was purified by preparative HPLC (column: Welch Xtimate C18, 100 × 40 mm × 3 μm; mobile phase: [water (trifluoroacetic acid)-acetonitrile]; acetonitrile%: 0%-26% over 8 minutes) to obtain the trifluoroacetate of compound **2.** MS m/z: 443.1 $[M+H]^+$, $^1H$ NMR (400 MHz, DMSO-$d_6$) δ ppm 7.19 (d, *J*=7.28 Hz, 1 H) 6.99 (s, 1 H) 6.90 (d, *J*=8.78 Hz, 1 H) 6.67 (d, J=6.78 Hz, 1 H) 3.80 (s, 2 H) 2.78 (s, 1 H) 2.70 (s, 3 H) 2.39 - 2.48 (m, 3 H) 2.14 (s, 3 H) 1.76 (s, 3 H) 0.32 (s, 3 H).

**Example 3**

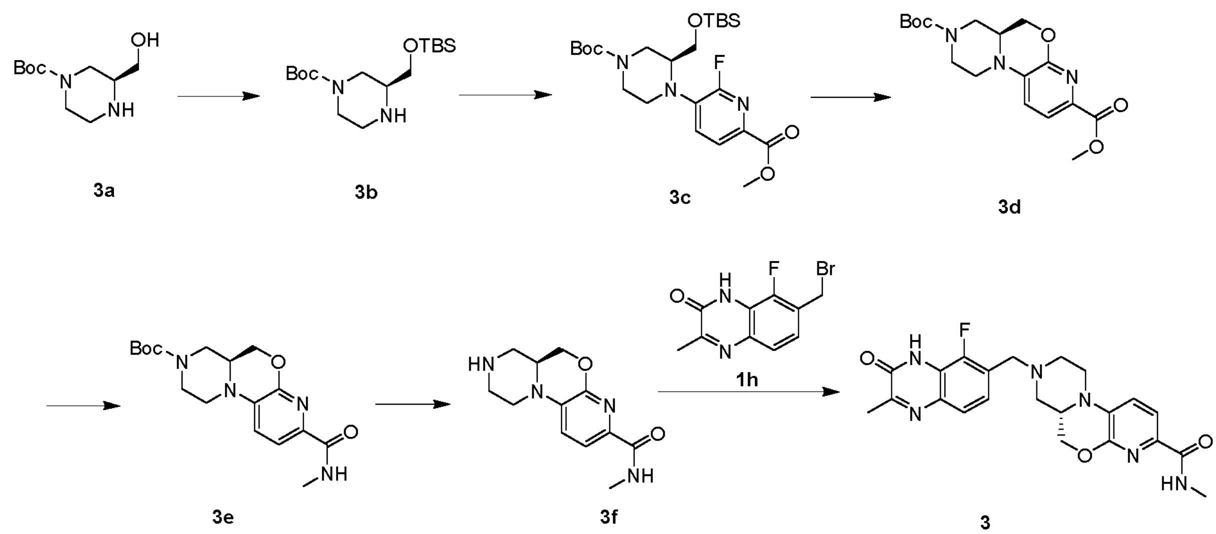

3a
3b
3c
3d
3e
3f
1h
3

Step 1: Synthesis of intermediate **3b**

Compound **3a** (1 g, 4.62 mmol) was added to DCM (20 mL), and the reaction mixture was cooled to 0°C. $Et_3N$ (935.74 mg, 9.25 mmol, 1.29 mL), TBSCl (1.05 g, 6.94 mmol, 849.87 μL), and DMAP (56.49 mg, 462.37 μmol) were then added thereto. The reaction mixture was stirred at 20°C for 16 hours. The reaction mixture was concentrated under reduced pressure to obtain a crude product. The crude product was purified by column chromatography (eluent: DCM/MeOH = 20/1 to 10/1, v/v) to obtain intermediate **3b.** $^1H$ NMR (400 MHz, $CDCl_3$) δ ppm 3.86 ( s, 1 H) 3.52 ( s, 1 H) 3.41 (dd, *J*=9.79, 7.28 Hz, 1 H) 2.92 (d, *J=11.29* Hz, 1 H) 2.63 - 2.84 (m, 3 H) 2.48 (s, 1 H) 1.90 (s, 1 H) 1.28 - 1.48 (m, 9 H) 0.72 - 0.97 (m, 9 H) -0.08 - 0.08 (m, 6 H).

Step 2: Synthesis of intermediate **3c**

Methyl 5-bromo-6-fluoro-pyridine-2-carboxylate (1 g, 4.27 mmol) and intermediate 3b (987.97 mg, 2.99 mmol) were added to toluene (20 mL). RuPhos (398.80 mg, 854.00 μmol), $Pd_2(dba)_3$ (391.30 mg, 427.00 μmol), and $Cs_2CO_3$ (4.17 g, 12.81 mmol) were then added thereto. The reaction mixture was purged with nitrogen three times and stirred at 100°C for 2 hours. The reaction mixture was subjected to suction filtration under reduced pressure, and the filtrate was concentrated under reduced pressure to obtain a crude product. The crude product was purified by column chromatography (eluent: PE/EA = 4/1 to 2/1, v/v) to obtain intermediate **3c.** MS m/z: 484.3 $[M+H]^+$.

Step **3:** Synthesis of intermediate **3d**

Compound **3c** (300 mg, 620.29 μmol) was added to THF (2 mL), and TBAF (1 M in THF, 1.86 mL) was added thereto. The reaction mixture was stirred at 20°C for 2 hours. The reaction mixture was subjected to suction filtration under reduced pressure, and the filtrate was concentrated under reduced pressure to obtain a crude product **3d,** which was used directly in the next step. MS m/z: 350.1 $[M+H]^+$.

Step 4: Synthesis of intermediate **3e**

Compound **3d** (200.00 mg) was added to EtOH (5 mL), and a solution of methylamine in ethanol (1 mL, 40%) was added thereto. The reaction mixture was stirred at 20°C for 32 hours. The reaction mixture was concentrated under reduced pressure to obtain a crude product. The crude product was used directly in the next step, yielding intermediate **3e.** MS m/z: 349.1 $[M+H]^+$.

Step 5: Synthesis of hydrochloride of intermediate **3f**

Compound **3e** (0.1 g) was added to EA (5 mL), and HCl/ethyl acetate (4 M, 358.79 μL) was added thereto. The reaction mixture was stirred at 20°C for 8 hours. The reaction mixture was concentrated under reduced pressure to obtain a crude hydrochloride of compound **3f,** which was used directly in the next step. MS m/z: 249.1 $[M+H]^+$.

Step 6: Synthesis of trifluoroacetate of compound **3**

Compound **3f** (10.50 mg, HCl) was added to DMF (2 mL). Then, $Et_3N$ (7.47 mg, 73.78 μmol, 10.27 μL) was added thereto. The reaction mixture was stirred at 25°C for 30 minutes. KI (2.70 mg, 16.24 μmol, 5.68 μL) and compound **1h** (10 mg, 36.89 μmol) were then added thereto. The reaction mixture was stirred at 50°C for 2 hours. The reaction mixture was concentrated using an oil pump at 60°C to obtain a crude product. The crude product was purified by preparative HPLC (column: Xtimate C18, 150 × 40 mm × 5 μm; mobile phase: [water (trifluoroacetic acid)-acetonitrile]; acetonitrile%: 1%-30% over 10 minutes) to obtain the trifluoroacetate of compound **3.** MS m/z: 439.0 $[M+H]^+$, $^1H$ NMR (400 MHz, $CD_3OD$) δ ppm 7.66 - 7.71 (m, 2 H) 7.42 - 7.48 (m, 1 H) 7.36 - 7.42 (m, 1 H) 4.50 (dd, J=11.19, 2.56 Hz, 1 H) 4.43 (s, 2 H) 4.22 (dd, J=11.13, 7.75 Hz, 1 H) 4.12 (d, J=10.01 Hz, 1 H) 3.56 (br s, 1 H) 3.51 (d, J=9.38 Hz, 2 H) 3.14 (s, 2 H) 2.92 (s, 3 H) 2.85 (s, 1 H) 2.56 (s, 3 H).

**Example 4**

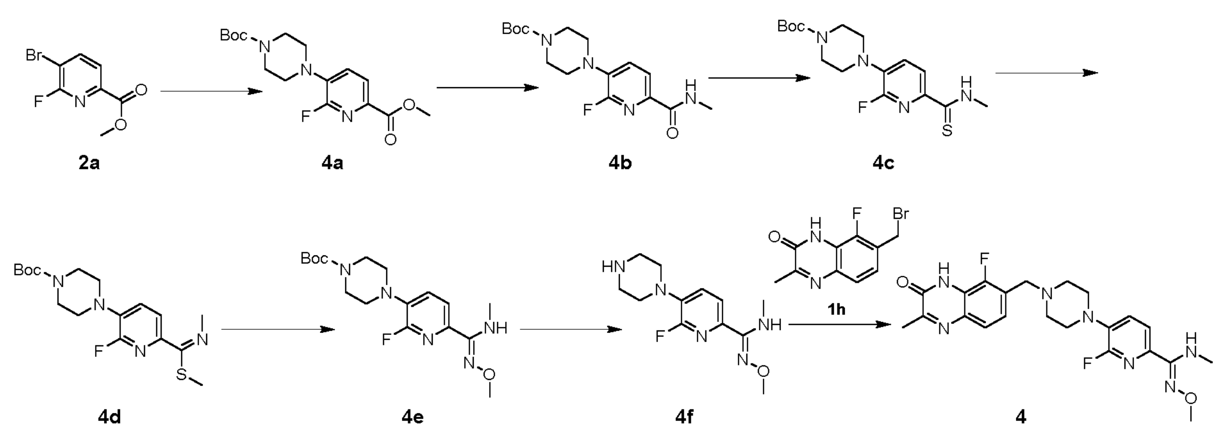


**2a** **4a** **4b** **4c**

**4d** **4e** **4f** **4**

Step 1: Synthesis of intermediate **4a**

Toluene (5 mL) was added to a reaction flask containing compound 2a (500 mg, 2.14 mmol) and N-Boc-piperazine (397.93 mg, 2.14 mmol). RuPhos (199.40 mg, 427.31 μmol), cesium carbonate (1.39 g, 4.27 mmol), and $Pd_2(dba)_3$ (195.65 mg, 213.65 μmol) were then sequentially added thereto. Under a nitrogen atmosphere, the reaction mixture was heated to 100°C and stirred for 16 hours. The reaction mixture was filtered directly, and the filtrate was concentrated under reduced pressure to obtain a crude product. The crude product was purified by silica gel column

chromatography (gradient elution: PE:EA = 100:0 to 80:20) to obtain intermediate **4a.** MS m/z: 340.1 $[M+H]^+$. $^1H$ NMR (400 MHz, $CDCl_3$) δ ppm 7.98 - 7.98 (m, 1 H) 7.91 - 8.06 (m, 1 H) 3.98 (s, 3 H) 3.57 - 3.68 (m, 4 H) 3.16 - 3.31 (m, 4 H) 1.51 (s, 9 H).

Step 2: Synthesis of intermediate **4b**

Compound **4a** (200 mg, 589.34 μmol) was dissolved in ethanol (8 mL), and a solution of methylamine in ethanol (1.19 g, 17.68 mmol) was added. The reaction mixture was stirred at 25°C for 16 hours. The reaction mixture was concentrated to obtain intermediate **4b,** which was used directly in the next reaction without purification. MS m/z: 339.1 $[M+H]^+$. $^1H$ NMR (400 MHz, $CDCl_3$) δ ppm 1.42 (s, 9 H) 2.93 (d, *J*=5.13 Hz, 3 H) 3.03 - 3.16 (m, 4 H) 3.44 - 3.63 (m, 4 H) 7.24 (dd, *J=10.01,* 8.13 Hz, 1 H) 7.44 (br d, *J*=4.13 Hz, 1 H) 7.93 (dd, *J*=8.00, 1.00 Hz, 1 H).

Step 3: Synthesis of intermediate **4c**

Intermediate **4b** (50 mg, 147.76 μmol) was dissolved in toluene (2.5 mL), and Lawesson's reagent (71.72 mg, 177.32 μmol) was added. The reaction mixture was stirred at 120°C for 16 hours. The reaction mixture was extracted with water (1 mL) and ethyl acetate (3 mL). The organic phase was dried over anhydrous sodium sulfate. The reaction mixture was filtered and concentrated under reduced pressure, and the residue was purified by prep-TLC (PE:EA = 5:1) to obtain intermediate **4c.** MS m/z: 355.1 $[M+H]^+$.

Step 4: Synthesis of intermediate **4d**

Intermediate **4c** (10 mg, 28.21 μmol) was dissolved in DMF (0.5 mL), and sodium hydride (2.82 mg, 70.53 μmol, purity of 60%) was added. The mixture was stirred at 0°C for 0.5 hours. Methyl iodide (8.01 mg, 56.43 μmol, 3.51 μL) was then added thereto, and the mixture was stirred at 25°C for 2 hours. The reaction was quenched by adding two drops of ammonium chloride solution. The reaction mixture was extracted with ethyl acetate (5 mL), and the organic phase was washed with saturated brine (2 mL), then dried over anhydrous sodium sulfate, filtered, and concentrated to obtain intermediate **4d,** which was used directly in the next step without purification. MS m/z: 369.2 $[M+H]^+$.

Step 5: Synthesis of intermediate **4e**

Intermediate **4d** (5 mg, 13.57 μmol) was dissolved in EtOH (0.5 mL). Triethylamine (6.87 mg, 67.85 μmol, 9.44 μL) and methoxyamine hydrochloride (3.40 mg, 40.71 μmol) were added, and the reaction mixture was stirred at 60°C for 1.5 hours. The reaction mixture was extracted with ethyl acetate (5 mL). The organic phase was washed with water (2 mL), dried over anhydrous sodium sulfate, then filtered and concentrated. The resulting product was purified by prep-TLC (PE:EA = 1:1), and the reaction mixture was filtered and concentrated to obtain intermediate **4e.** MS m/z: 368.1 $[M+H]^+$. $^1H$ NMR (400 MHz, $CDCl_3$) δ ppm 7.48 - 7.56 (m, 1 H) 7.15 - 7.18 (m, 1 H) 3.79 (s, 3 H) 3.51 - 3.56 (m, 4 H) 3.00 - 3.05 (m, 4 H) 2.92 (s, 3 H)1.42 (s, 9 H) .

Step 6: Synthesis of hydrochloride of intermediate **4f**

Intermediate **4e** (10 mg, 27.22 μmol) was dissolved in MeOH (0.5 mL). HCl/dioxane (4 M, 36.74 μL) was added, and the reaction mixture was stirred at 25°C for 1.5 hours. LCMS analysis indicated that most of the starting material remained. An additional 0.2 mL of HCl/dioxane (4 M, 36.74 μL) was added, and the mixture was stirred for another 1.5 hours. The reaction mixture was then concentrated to obtain the hydrochloride of intermediate **4f,** which was used directly in the next step without purification.

Step 7: Synthesis of trifluoroacetate of compound **4**

Intermediate **4f** (10 mg, hydrochloride) was dissolved in DMF (1 mL). Triethylamine (6.66 mg, 65.84 μmol, 9.16 μL) was added, and the reaction mixture was stirred at 25°C for 0.5 hours. Intermediate **1h** (8.92 mg, 32.92 μmol) and potassium iodide (2.73 mg, 16.46 μmol) were then added. The mixture was stirred at 50°C for 2 hours. The reaction mixture was filtered to obtain a crude product. The crude product was purified by preparative HPLC (column: Welch Xtimate C18, 100 × 40 mm × 3 μm; mobile phase: [water (trifluoroacetic acid)-acetonitrile]; acetonitrile%: 0%-30% over 8 minutes) to obtain the trifluoroacetate of compound **4.** MS m/z: 458.2 $[M+H]^+$. $^1H$ NMR (400 MHz, $CD_3OD$) δ ppm 2.56 (s, 3 H) 3.03 (s, 3 H) 3.59 (br s, 8 H) 3.83 - 3.94 (m, 3 H) 4.64 (s, 2 H) 7.46 - 7.52 (m, 1 H) 7.60 - 7.76 (m, 3 H).

**Example 5**

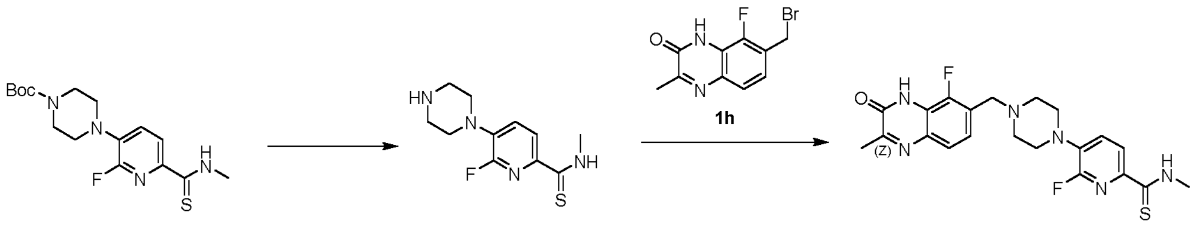


**4c** **5a** **5**

Step 1: Synthesis of hydrochloride of intermediate **5a**

Intermediate 4c (19.4 mg, 54.73 μmol) was dissolved in MeOH (1 mL). HCl/dioxane (4 M, 73.89 μL) was added, and the reaction mixture was stirred at 25°C for 16 hours. The reaction mixture was concentrated to obtain the hydrochloride of intermediate **5a,** which was used directly in the next step without purification. MS m/z: 255.0 $[M+H]^+$.

Step 2: Synthesis of trifluoroacetate of compound **5**

Intermediate **5a** (15 mg, hydrochloride) was dissolved in DMF (1 mL). Triethylamine (10.44 mg, 103.17 μmol, 14.36 μL) was added, and the reaction mixture was stirred at 25°C for 0.5 hours. Methyl iodide (4.28 mg, 25.79 μmol) and intermediate **1h** (13.98 mg, 51.58 μmol) were then added, and the mixture was stirred at 50°C for 2 hours. The reaction mixture was filtered to obtain a crude product. The crude product was purified by preparative HPLC (column: Welch Xtimate C18, 100 × 40 mm × 3 μm; mobile phase: [water (trifluoroacetic acid)-acetonitrile]; gradient: 3%-33% acetonitrile over 8 minutes) to obtain the trifluoroacetate of compound **5.** MS m/z: 445.1 $[M+H]^+$. $^1$H NMR (400 MHz, $CD_3OD$) δ ppm 2.56 (s, 3 H) 3.27 - 3.29 (m, 3 H) 3.54 (br d, *J*=3.76 Hz, 8 H) 4.58 (s, 2 H) 7.47 (t, *J*=7.91 Hz, 1 H) 7.59 (dd, *J=10.16,* 8.41 Hz, 1 H) 7.70 (d, *J*=8.28 Hz, 1 H) 8.47 (d, *J*=8.03 Hz, 1 H) 10.18 - 10.34 (m, 1 H).

**Example 6**

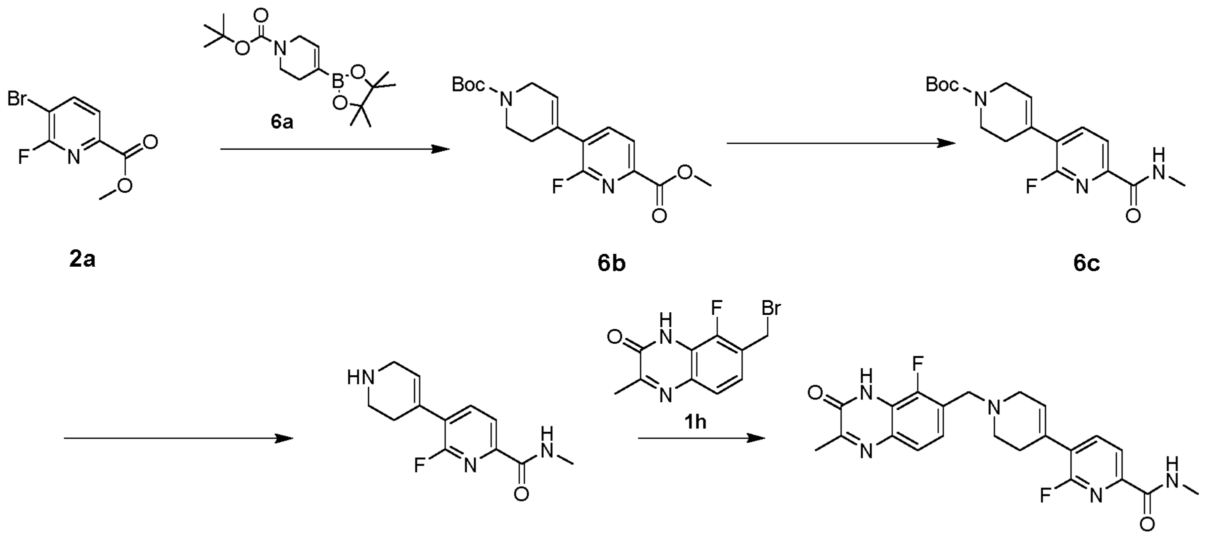


**2a** **6b** **6c**

**6d** **6**

Step 1: Synthesis of intermediate 6b

Dioxane (1 mL), water (0.2 mL), intermediate **2a** (30 mg, 128.19 μmol), and compound 6a (43.60 mg, 141.01

μmol) were added to a 10 mL vial and stirred. After nitrogen purging, potassium phosphate (54.42 mg, 256.39 μmol) and [1,1'-bis(diphenylphosphino)ferrocene]dichloropalladium(II) (9.38 mg, 12.82 μmol) were added. The reaction mixture was heated to 80°C and reacted for 2 hours. The reaction mixture was filtered, and the filtrate was concentrated under reduced pressure to obtain a crude product. The crude product was purified by preparative TLC (eluent: PE:EA = 3:1) to obtain intermediate **6b.** MS m/z: 337.1 $[M+H]^+$.

Step 2: Synthesis of intermediate **6c**

Intermediate **6b** (37 mg, 110.00 μmol) was dissolved in EtOH (1.5 mL). A solution of methylamine in ethanol (222.81 mg, 3.30 mmol) was added, and the reaction mixture was stirred at 25°C for 6 hours. The reaction mixture was directly concentrated to obtain intermediate **6c,** which was used directly in the next step without purification. MS m/z: 336.0 $[M+H]^+$.

Step 3: Synthesis of hydrochloride of intermediate **6d**

Intermediate **6c** (17 mg, 50.69 μmol) was dissolved in MeOH (0.5 mL). HCl/dioxane (4 M, 68.43 μL) was added, and the reaction mixture was stirred at 25°C for 2 hours. The reaction mixture was directly concentrated to obtain the hydrochloride of intermediate **6d,** which was used directly in the next step without purification. MS m/z: 236.1 $[M+H]^+$.

Step 4: Synthesis of trifluoroacetate of compound **6**

Intermediate **6d** (10 mg, 36.80 μmol, hydrochloride) was dissolved in DMF (1 mL). Triethylamine (7.45 mg, 73.61 μmol, 10.24 μL) was added, and the reaction mixture was stirred at 25°C for 0.5 hours. Intermediate **1h** (9.98 mg, 36.80 μmol) and potassium iodide (3.05 mg, 18.40 μmol) were then added. The mixture was stirred at 50°C for 2 hours. The reaction mixture was filtered to obtain a crude product. The crude product was separated by preparative HPLC (column: Welch Xtimate C18, 100 × 40 mm × 3 μm; mobile phase: [water (trifluoroacetic acid)-acetonitrile]; gradient: 0%-30% acetonitrile over 8 minutes) to obtain the trifluoroacetate of compound **6.** MS m/z: 426.2 $[M+H]^+$. $^1H$ NMR (400 MHz, $CDCl_3$) δ ppm 8.12 (dd, *J*=7.78, 1.51 Hz, 1 H) 7.83 - 7.91 (m, 1 H) 7.70 - 7.79 (m, 1 H) 7.57 - 7.68 (m, 2 H) 6.13 (br s, 1 H) 4.53 (s, 2 H) 3.74 - 4.00 (m, 2 H) 3.36 - 3.66 (m, 2 H) 3.04 - 3.09 (m, 3 H) 2.91 - 2.99 (m, 1 H)2.65 (s, 3 H).

**Example 7**

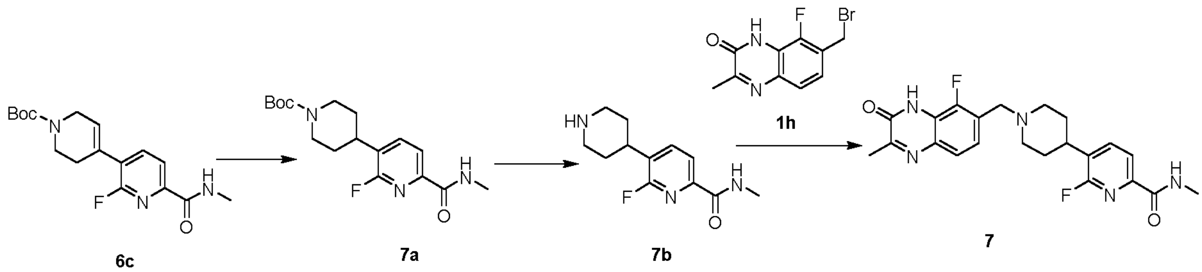

6c 7a 7b 7

Step 1: Synthesis of intermediate **7a**

Intermediate **6c** (10 mg, 29.82 μmol) was added to a mixture of MeOH (3 mL) and ethyl acetate (1 mL). Palladium on carbon (2 mg, 29.82 μmol, 10%) was also added thereto. The reaction mixture was purged with argon three times and then with hydrogen three times. The reaction mixture was stirred under a hydrogen atmosphere at 16 psi and 30°C for 16 hours. The reaction mixture was filtered through diatomite, and the organic phase was concentrated under reduced pressure to obtain intermediate **7a,** which was used directly in the next step. MS m/z: 360.2 $[M+23]^+$.

Step 2: Synthesis of hydrochloride of intermediate **7b**

Intermediate **7a** (10 mg, 29.64 μmol) was dissolved in MeOH (0.5 mL). HCl/dioxane (4 M, 37.05 μL) was added thereto. The reaction mixture was stirred at 25°C for 2 hours. The reaction mixture was directly concentrated under reduced pressure to obtain the hydrochloride of intermediate **7b,** which was used directly in the next step. MS m/z: 238.0 $[M+1]^+$.

Step 3: Synthesis of trifluoroacetate of compound 7

Triethylamine (6.94 mg, 68.56 μmol) was added to a solution of intermediate **7b** (9.38 mg, 34.28 μmol, crude hydrochloride) in DMF (1 mL). The reaction mixture was stirred at 20°C for 0.5 hours. Intermediate **1h** (9.29 mg, 34.28 μmol) and potassium iodide (569.03 μg, 3.43 μmol) were then added thereto. The reaction mixture was stirred at 50°C for 2 hours. The reaction mixture was concentrated under reduced pressure to obtain a crude product. The crude product was purified by preparative HPLC (column: Welch Xtimate C18, 100 × 40 mm × 3 μm; mobile phase: [water (trifluoroacetic acid)-acetonitrile]; gradient: 0%-30% acetonitrile over 8 minutes) to obtain the trifluoroacetate of compound 7. MS m/z: 428.3 $[M+1]^+$. $^1$H NMR (400 MHz, $CD_3OD$) δ ppm 8.75 ( s, 1 H) 8.01 (s, 2 H) 7.71 (d, *J*=8.28 Hz, 1 H) 7.47 (t, *J*=7.65 Hz, 1 H) 4.58 (s, 2 H) 3.72 (br s, 2 H) 3.18 - 3.30 (m, 3 H) 2.95 (d, *J*=5.02 Hz, 3 H) 2.56 (s, 3 H) 2.15 - 2.26 (m, 2 H) 2.07 (d, J=12.80 Hz, 2 H).

**Example 8**

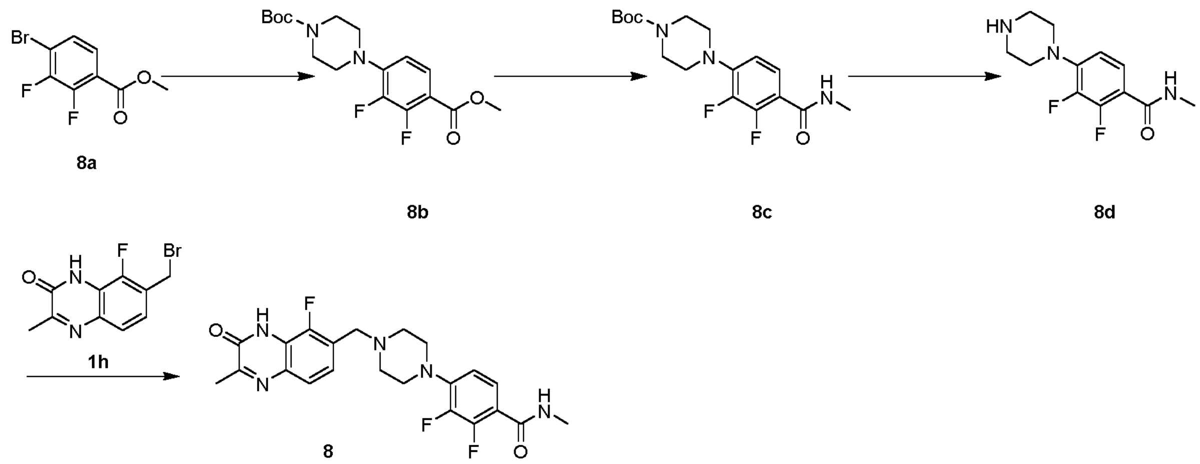

8a

8b

8c

8d

1h

8

Step 1: Synthesis of intermediate **8b**

Compound **8a** (245.05 mg, 976.20 μmol) and N-Boc-piperazine (200 mg, 1.07 mmol) were added to toluene (5 mL). $Pd_2(dba)_3$ (8.94 mg, 9.76 μmol), RuPhos (9.11 mg, 19.52 μmol), and $Cs_2CO_3$ (636.13 mg, 1.95 mmol) were added thereto. The reaction mixture was purged with nitrogen three times and stirred at 100°C for 16 hours. The reaction mixture was filtered through diatomite, and the organic phase was concentrated under reduced pressure to obtain a crude product. A mixture of ethyl acetate and n-heptane (ethyl acetate/n-heptane = 5/1, 10 mL) was added to the crude product, and the resulting mixture was stirred at 25°C for 0.5 hours and subjected to suction filtration under reduced pressure, and the filter cake was concentrated to obtain intermediate **8b.** MS m/z: 301.2 $[M-56+1]^+$. $^1$H NMR (400 MHz, DMSO-$d_6$) δ ppm 7.61 (t, *J*=8.19 Hz, 1 H) 6.93 (t, *J*=8.38 Hz, 1 H) 3.83 (s, 3 H) 3.50-3.46 (m, 4 H) 3.13 - 3.23 (m, 4 H) 1.43 (s, 9 H).

Step 2: Synthesis of intermediate **8c**

Compound 2 (100 mg, 280.61 μmol) and a solution of methylamine in ethanol (87.15 mg, 1.29 mmol) were added to EtOH (2 mL). The mixture was stirred at 25°C for 16 hours, and then the reaction temperature was increased to 50°C. The mixture was stirred at 50°C for 16 hours. The reaction mixture was directly concentrated under reduced pressure to obtain a crude product. The crude product was purified by preparative TLC (PE/EA = 2/1, $R_f$ = 0.1) to obtain intermediate **8c.** MS m/z: 378.2 $[M+23]^+$.

Step 3: Synthesis of hydrochloride of intermediate **8d**

Intermediate **8c** (50 mg, 140.69 μmol) was dissolved in MeOH (0.5 mL). HCl/dioxane (4 M, 37.05 μL) was added thereto. The reaction mixture was stirred at 25°C for 2 hours, then directly concentrated under reduced pressure to obtain the hydrochloride of intermediate **8d,** which was used directly in the next step. MS m/z: 256.0 $[M+1]^+$.

Step 4: Synthesis of trifluoroacetate of compound **8**

Triethylamine (10.41 mg, 102.84 μmol) was added to a solution of intermediate **8d** (15 mg, 51.42 μmol, crude hydrochloride) in DMF (1 mL). The reaction mixture was stirred at 20°C for 0.5 hours. Intermediate **1h** (13.94 mg, 51.42 μmol) and potassium iodide (853.55 μg, 5.14 μmol) were then added thereto. The reaction mixture was stirred at 50°C for 2 hours. The reaction mixture was concentrated using an oil pump under reduced pressure to obtain a crude product. The crude product was purified by preparative HPLC (column: Welch Xtimate C18, 100 × 40 mm × 3 μm; mobile phase: [water (trifluoroacetic acid)-acetonitrile]; gradient: 0%-30% acetonitrile over 8 minutes) to obtain the trifluoroacetate of compound **8.** MS m/z: 468.1 $[M+23]^+$. $^1$H NMR (400 MHz, $CD_3OD$) δ ppm 8.15 (s, 1 H) 7.70 (d, *J*=8.28 Hz, 1 H) 7.39 - 7.57 (m, 2 H) 6.89 - 7.03 (m, 1 H) 4.55 (s, 2 H) 3.50 (s, 8 H) 2.89 - 2.98 (m, 3 H) 2.56 (s, 3 H).

**Example 9**

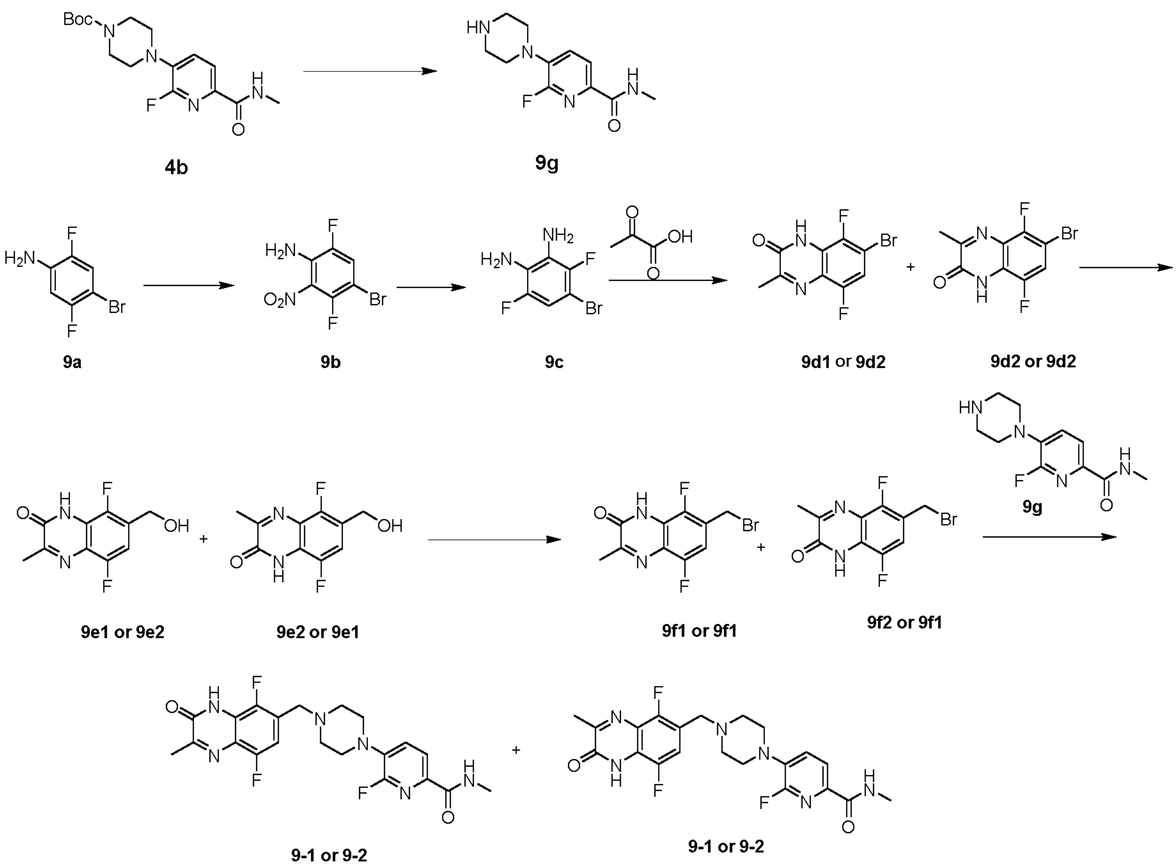

4b

9g

9a

9b

9c

9d1 or 9d2

9d2 or 9d2

9e1 or 9e2

9e2 or 9e1

9f1 or 9f1

9f2 or 9f1

9g

9-1 or 9-2

9-1 or 9-2

Step 1: Synthesis of hydrochloride of intermediate **9g**

Intermediate **4b** (50 mg, 147.76 μmol) was dissolved in methanol (0.2 mL). HCl/dioxane (4 M, 199.48 μL) was added, and the reaction mixture was stirred at 25°C for 2 hours. The reaction mixture was concentrated under reduced pressure without further purification to obtain the hydrochloride of intermediate **9g.** MS m/z: 238.9 $[M+1]^+$.

Step 2: Synthesis of intermediate **9b**

At 0°C, compound **9a** (1 g, 4.81 mmol) was added to trifluoroacetic acid (10 mL). Potassium nitrate (760 mg, 7.52 mmol) was slowly added in batches. The reaction mixture was then stirred at 20°C for 16 hours. The reaction mixture was poured into 90 mL of saturated sodium bicarbonate solution, and then extracted with ethyl acetate (100 mL × 3). The organic phases was washed with saturated sodium chloride solution (100 mL × 2), filtered, and concentrated. The crude product was purified by silica gel column chromatography (EA:PE = 0%-10%) to obtain intermediate **9b.** $^1$H NMR (400 MHz, DMSO-$d_6$) δ ppm 7.68 - 7.92 (m, 1 H), 7.08 (br s, 2 H).

Step 3: Synthesis of intermediate **9c**

Intermediate **9b** (100 mg, 395.26 μmol) was dissolved in EtOH (1 mL) and cooled to 0°C. Acetic acid (237.36 mg, 3.95 mmol, 226.27 μL) was first added, then zinc (240 mg, 3.67 mmol) was added in batches. The reaction was carried out for 6 hours. The reaction mixture was filtered and concentrated to obtain a crude product. The crude product was purified by preparative TLC (PE:EA = 3:1) to obtain intermediate **9c.** MS m/z: 224.9, 226.9 $[M+1]^+$, $[M+3]^+$. $^1H$ NMR (400 MHz, $CDCl_3$) δ ppm 6.69 - 6.80 (m, 1 H), 3.52 (s, 4 H).

Step 4: Synthesis of mixture of **9d1** and **9d2**

Intermediate **9c** (25 mg, 112.10 μmol) was added to EtOH (0.32 mL), then pyruvic acid (11.85 mg, 134.52 μmol) was added. The reaction mixture was reacted at 100°C for 2 hours, then slowly cooled to 25°C to crystallize over 14 hours. The reaction mixture was filtered, and the solid was washed once with 0.5 mL of ethanol. A mixture of intermediates **9d1** and **9d2** was obtained without further purification. MS m/z: 274.9 $[M+1]^+$.

Step 5: Synthesis of mixture of **9e1** and **9e2**

The mixture of intermediates **9d1** and **9d2** (95 mg) and XPHOS-PD-G2 (13.59 mg, 17.27 μmol) was dissolved in dioxane (2 mL). 1-(Tributylstannyl)methanol (133.08 mg, 414.47 μmol) was added, and the mixture was purged with nitrogen and reacted at 80°C for 16 hours. The reaction mixture was purified by preparative TLC (PE:EA = 1:1) to obtain a mixture of intermediates **9e1** and **9e2.** MS m/z: 227.0 $[M+1]^+$.

Step 6: Synthesis of mixture of **9f1** and **9f2**

Under a nitrogen atmosphere and at 0°C, tributylphosphine (80.50 mg, 397.91 μmol, 98.18 μL) was slowly added dropwise to a solution of intermediates **9f1** and **9f2** (45 mg) and 1,2-dibromotetrachloroethane (142.54 mg, 437.71 μmol) in dichloromethane (1.2 mL). The reaction mixture was reacted at 25°C for 2 hours. The reaction mixture was purified by preparative TLC (dichloromethane:methanol = 15:1) to obtain a mixture of intermediates **9f1** and **9f2.**

Step 7: Synthesis of mixture of trifluoroacetates of compounds **9-1** and **9-2**

The mixture of intermediates **9f1** and **9f2** (43 mg) was added to DMF (2 mL). Triethylamine (29.92 mg, 295.65 μmol) was added, and the reaction mixture was stirred at 25°C for 15 minutes. Potassium iodide (11.04 mg, 66.52 μmol) and intermediate **9g** (46 mg, 147.82 μmol) were then added thereto. The mixture was stirred at 50°C for 2 hours. The reaction mixture was separated by preparative HPLC (column: Welch Xtimate C18, 100 × 40 mm × 3 μm; mobile phase: [water (trifluoroacetic acid)-acetonitrile]; acetonitrile%: 0%-30% acetonitrile over 8 minutes). Thus a mixture of trifluoroacetates of compounds **9-1** and **9-2** was obtained. MS m/z: 447.1 $[M+1]^+$.

**Example 10**

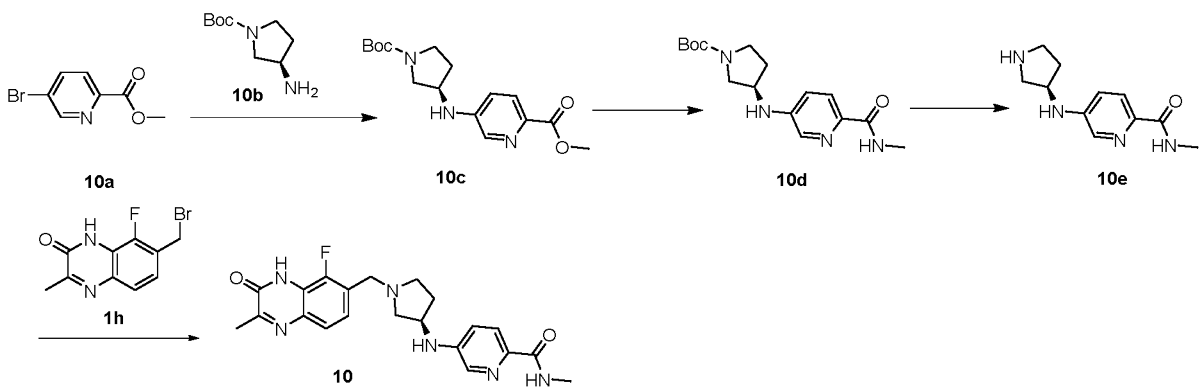

**10a** **10b** **10c** **10d** **10e** **1h** **10**

Step 1: Synthesis of intermediate **10c**

Under a nitrogen atmosphere, **10a** (50 mg, 231.45 μmol), compound **10b** (51.73 mg, 277.74 μmol), $Cs_2CO_3$ (150.82 mg, 462.89 μmol), RuPhos (21.60 mg, 46.29 μmol), and $Pd_2(dba)_3$ (21.19 mg, 23.14 μmol) were added to toluene (1 mL). The mixture was heated to 100°C and stirred for 16 hours. The reaction mixture was filtered through diatomite and washed with DCM (5 mL). The filtrate was concentrated under reduced pressure to obtain a crude product. The crude product was purified by preparative TLC (PE:EA = 0:1) to obtain intermediate **10c.** MS m/z: 322.1 $[M+1]^+$, $^1$H NMR (400 MHz, DMSO-$d_6$) δ ppm 8.06 (d, *J*=2.76 Hz, 1H), 7.82 (d, *J*=8.53 Hz, 1H), 6.93 - 7.02 (m, 2H), 4.04 - 4.13 (m, 1H), 3.74 - 3.80 (m, 3 H), 3.53 - 3.61 (m, 1H), 3.42 - 3.47 (m, 1H), 3.32 - 3.35 (m, 1H), 3.04 - 3.21 (m, 1H), 2.09 - 2.20 (m, 1H), 1.74 - 1.86 (m, 1H), 1.40 (br d, J=6.53 Hz, 9H).

Step 2: Synthesis of intermediate 10d

Intermediate **10c** (60 mg, 186.70 μmol) was added to EtOH (1 mL), then a solution of methylamine in ethanol (1.23 g, 11.88 mmol) was added thereto. The reaction mixture was stirred at 25°C for 16 hours. The reaction mixture was concentrated under reduced pressure to obtain intermediate **10d,** which was used directly in the next step. MS m/z: 321.2 $[M+1]^+$.

Step 3: Synthesis of hydrochloride of intermediate **10e**

Intermediate **10d** (25 mg, 78.03 μmol) was added to MeOH (0.5 mL), then HCl/dioxane (4 M, 105.34 μL) was added. The reaction mixture was stirred at 25°C for 2 hours. The reaction mixture was concentrated under reduced pressure to obtain the hydrochloride of intermediate 10e, which was used directly in the next step. MS m/z: 221.1 $[M+1]^+$, $^1$H NMR (400 MHz, DMSO-$d_6$) δ 9.35 (br s, 3H), 8.01 (d, J=2.51 Hz, 1H), 7.92 (dd, J=8.66, 4.39 Hz, 1H), 7.21 (dd, J=8.41, 2.38 Hz, 1H), 4.20 - 4.30 (m, 1H), 3.42 -3.51 (m, 1H), 3.24 - 3.37 (m, 2H), 3.04 - 3.13 (m, 1H), 2.79 (d, J=4.52 Hz, 3H), 2.19 - 2.31 (m, 1H), 1.86 - 1.98 (m, 1H).

Step 4: Synthesis of compound 10

Triethylamine (15.77 mg, 155.80 μmol, 21.69 μL) was added to a solution of intermediate 10e (20 mg, hydrochloride) in DMF (1 mL). The reaction mixture was stirred at 20°C for 0.5 hours. Compound **1h** (21.12 mg, 77.90 μmol) and potassium iodide (1.29 mg, 7.79 μmol) were then added thereto. The reaction mixture was heated to 50°C and stirred for 2 hours. The reaction mixture was washed with water (2 mL), extracted three times with a mixture of DCM and MeOH (DCM:MeOH = 10:1, 3 mL). The organic phases were combined, dried over anhydrous sodium sulfate, and filtered. The filtrate was concentrated under reduced pressure to obtain a crude product. The crude product was purified by preparative TLC (DCM:MeOH = 10:1) to obtain compound 10. MS m/z: 411.1 $[M+1]^+$, $^1$H NMR (400 MHz, DMSO-$d_6$) δ ppm 12.44 (s, 1H), 8.27 (br d, J=4.77 Hz, 1H), 7.91 (d, J=2.51 Hz, 1H), 7.72 (d, J=8.53 Hz, 1H), 7.50 (d, J=7.78 Hz, 1H), 7.24 - 7.32 (m, 1H), 6.94 (dd, J=8.16, 2.64 Hz, 1H), 6.65 (d, J=6.53 Hz, 1H), 3.94 - 4.02 (m, 1H), 3.75 (br s, 2H), 3.37 - 3.42 (m, 1H), 2.86 (br t, *J*=8.66 Hz, 1H), 2.75 (d, *J*=4.77 Hz, 3H), 2.65 - 2.71 (m, 1H), 2.43 - 2.47 (m, 1H), 2.41 (s, 3H), 2.20 - 2.28 (m, 1H), 1.54 - 1.65 (m, 1H).

**Example 11**

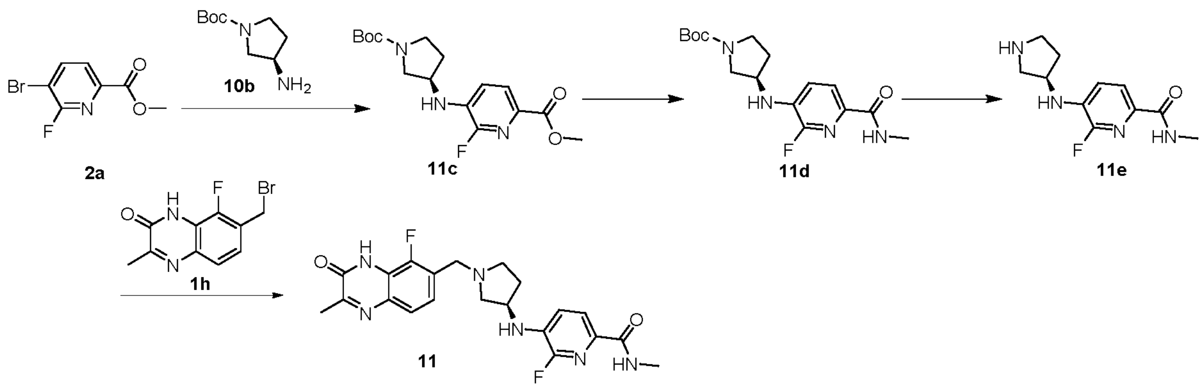
2a
10b
11c
11d
11e
1h
11

Step **1:** Synthesis of intermediate **11c**

Under a nitrogen atmosphere, **2a** (50 mg, 231.45 μmol), compound **10b** (51.73 mg, 277.74 μmol), $Cs_2CO_3$ (150.82 mg, 462.89 μmol), RuPhos (21.60 mg, 46.29 μmol), and $Pd_2(dba)_3$ (21.19 mg, 23.14 μmol) were added to toluene (1 mL). The reaction mixture was heated to 100°C and stirred for 16 hours. The reaction mixture was filtered through diatomite, washed with DCM (5 mL). The filtrate was concentrated under reduced pressure to obtain a crude product. The crude product was purified by preparative TLC (PE:EA = 0:1) to obtain intermediate **11c.** MS m/z: 340.1 $[M+1]^+$, $^1H$ NMR (400 MHz, DMSO-$d_6$) δ ppm 7.84 (d, J=8.00 Hz, 1H), 7.24 (br t, J=9.26 Hz, 1H), 6.88 (br d, J=6.38 Hz, 1H), 4.07 - 4.18 (m, 1H), 3.79 (s, 3H), 3.56 - 3.64 (m, 1H), 3.38 - 3.49 (m, 2H), 3.20 (br dd, J=11.01, 4.75 Hz, 1H), 2.11 - 2.22 (m, 1H), 1.86 - 1.96 (m, 1H), 1.40 (br d, J=5.38 Hz, 9H).

Step 2: Synthesis of intermediate **11d**

Intermediate **11c** (60 mg, 186.70 μmol) was added to EtOH (1 mL), then a solution of methylamine in ethanol (1.23 g, 11.88 mmol) was added thereto. The reaction mixture was stirred at 25°C for 16 hours. The reaction mixture was concentrated under reduced pressure to obtain intermediate **11d,** which was used directly in the next step. MS m/z: 339.1 $[M+1]^+$. $^1H$ NMR (400 MHz, DMSO-$d_6$) δ ppm 8.21 (br d, J=4.63 Hz, 1H), 7.75 (d, J=8.25 Hz, 1H), 7.24 - 7.31 (m, 1H), 6.56 (br d, J=6.50 Hz, 1H), 4.04 - 4.18 (m, 1H), 3.56 - 3.66 (m, 1H), 3.38 - 3.47 (m, 2H), 3.16 - 3.24 (m, 1H), 2.75 (d, J=4.75 Hz, 3H), 2.08 - 2.24 (m, 1H), 1.84 - 1.97 (m, 1H) 1.40 (br d, J=6.13 Hz, 9H).

Step 3: Synthesis of hydrochloride of intermediate **11e**

Intermediate **11d** (25 mg, 78.03 μmol) was added to MeOH (0.5 mL), then HCl/dioxane (4 M, 105.34 μL) was added. The reaction mixture was stirred at 25°C for 4 hours. The reaction mixture was concentrated under reduced pressure to obtain the hydrochloride of intermediate **11e,** which was used directly in the next step. MS m/z: 239.1 $[M+1]^+$.

Step 4: Synthesis of compound **11**

Triethylamine (11.05 mg, 109.20 μmol, 15.20 μL) was added to a solution of the hydrochloride of **11e** (obtained from step 3, 15 mg) in DMF (1 mL). The reaction mixture was stirred at 20°C for 0.5 hours. Compound **1h** (14.80 mg, 54.60 μmol) and potassium iodide (906.37 μg, 5.46 μmol) were thereto. The reaction mixture was stirred at 50°C for 2 hours. The reaction mixture was washed with water (2 mL), extracted three times with a mixture of DCM and MeOH (DCM:MeOH = 10:1, 3 mL). The organic phases were combined, dried over anhydrous sodium sulfate, and concentrated under reduced pressure to obtain a crude product. The crude product was purified by preparative TLC (DCM:MeOH = 10:1) to obtain compound **11.** MS m/z: 429.2 $[M+1]^+$, $^1H$ NMR (400 MHz, DMSO-$d_6$) δ ppm 12.43 (s, 1H), 8.15 (br d, *J*=4.63 Hz, 1H), 7.72 (d, J=8.00 Hz, 1H), 7.50 (d, J=8.25 Hz, 1H), 7.28 (t, *J*=7.63 Hz, 1H), 7.16 (dd, *J*=10.44, 8.32 Hz, 1H), 6.41 (br d, *J*=5.88 Hz, 1H), 3.96 - 4.04 (m, 1H), 3.76 (br s, 2H), 3.29 (br s, 1H), 2.92 (br t, *J*=8.00 Hz, 1H), 2.74 (d, J=4.75 Hz, 3H), 2.64 - 2.70 (m, 1H), 2.53 - 2.58 (m, 1H), 2.41 (s, 3H), 2.18 - 2.27 (m, 1H), 1.73 - 1.81 (m, 1H).

## Example 12

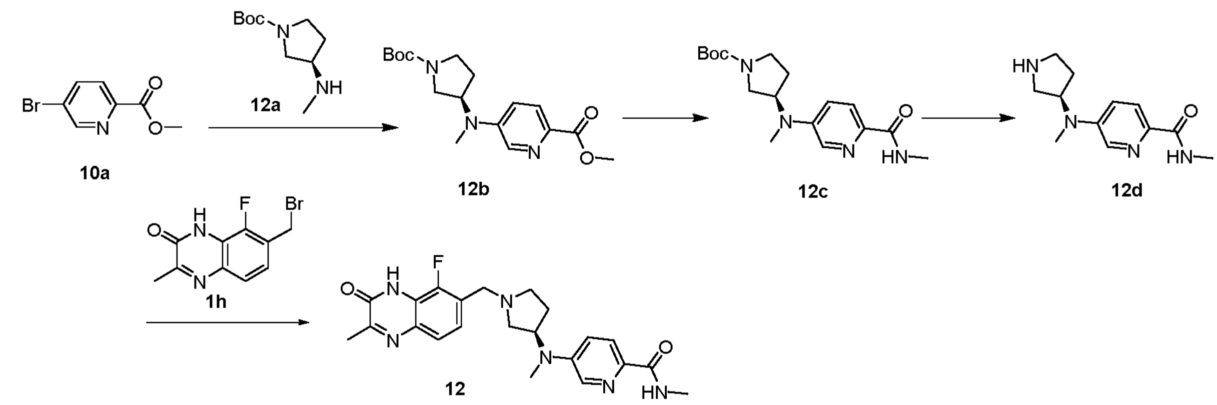
10a
12a
12b
12c
12d
1h
12

Step 1: Synthesis of intermediate **12b**

Under a nitrogen atmosphere, **10a** (50 mg, 231.45 μmol), compound **12a** (51.73 mg, 277.74 μmol), $Cs_2CO_3$ (150.82 mg, 462.89 μmol), RuPhos (21.60 mg, 46.29 μmol), and $Pd_2(dba)_3$ (21.19 mg, 23.14 μmol) were added to toluene (1 mL). The reaction mixture was heated to 100°C and stirred for 16 hours. The reaction mixture was filtered through diatomite, washed with DCM (5 mL). The filtrate was concentrated under reduced pressure to obtain a crude product. The crude product was purified by preparative TLC (PE:EA = 0:1) to obtain intermediate **12b.** MS m/z: 336.2 $[M+1]^+$.

Step **2:** Synthesis of intermediate **12c**

Intermediate **12b** (45 mg, 134.17 μmol) was added to EtOH (1 mL), then a solution of methylamine in ethanol (694.48 mg, 6.71 mmol) was added thereto. The reaction mixture was stirred at 25°C for 16 hours. The reaction mixture was concentrated under reduced pressure to obtain compound **12c.** MS m/z: 335.2 $[M+1]^+$. $^1H$ NMR (400 MHz, DMSO-$d_6$) δ ppm 8.34 (br d, J=4.75 Hz, 1H), 8.18 (d, J=2.88 Hz, 1H), 7.81 (d, J=8.88 Hz, 1H), 7.31 (dd, J=8.88, 3.00 Hz, 1H), 4.61 - 4.70 (m, 1H), 3.53 (br t, J=8.88 Hz, 1H), 3.41 - 3.46 (m, 1H), 3.30 - 3.33 (m, 3H), 3.21 - 3.25 (m, 1H), 2.78 (d, J=4.88 Hz, 3H), 1.95 - 2.11 (m, 3H), 1.41 (s, 9H).

Step 3: Synthesis of hydrochloride of intermediate **12d**

Intermediate **12c** (44 mg, 131.57 μmol) was added to MeOH (0.5 mL), and HCl/dioxane (4 M, 105.34 μL) was then added. The reaction mixture was stirred at 25°C for 2 hours. The reaction mixture was concentrated under reduced pressure to obtain the hydrochloride of intermediate **12d,** which was used directly in the next step. MS m/z: 235.1 $[M+1]^+$.

Step 4: Synthesis of compound **12**

Triethylamine (14.95 mg, 147.73 μmol, 20.56 μL) was added to a solution of the hydrochloride of **12d** (20 mg) in DMF (1 mL). The reaction mixture was stirred at 20°C for 0.5 hours. Compound **1h** (20.02 mg, 73.87 μmol) and potassium iodide (1.23 mg, 7.39 μmol) were then added thereto. The reaction mixture was stirred at 50°C for 2 hours. The reaction mixture was washed with water (2 mL), extracted three times with a mixture of DCM and MeOH (DCM:MeOH = 10:1, 3 mL). The organic phases were combined, dried over anhydrous sodium sulfate, and concentrated under reduced pressure to obtain a crude product. The crude product was purified by preparative TLC (DCM:MeOH=10:1) to obtain compound **12.** MS m/z: 425.2 $[M+1]^+$, $^1H$ NMR (400 MHz, DMSO-$d_6$) δ ppm 12.45 (br s, 1H), 8.31 (br d, *J*=5.02 Hz, 1H), 8.11 (d, *J=3.01* Hz, 1H), 7.77 (d, *J*=8.78 Hz, 1H), 7.52 (d, *J*=8.28 Hz, 1H), 7.30 (t, *J*=7.65 Hz, 1H), 7.22 (dd, *J*=8.78, 3.01 Hz, 1H), 4.56 - 4.67 (m, 1H), 3.75 (s, 2H), 3.32 (s, 3H), 2.87 (td, *J*=8.34, 2.89 Hz, 1H), 2.77 (d, *J*=5.02 Hz, 3H), 2.73 (dd, *J*=10.04, 3.26 Hz, 1H), 2.62 (t, *J*=8.78 Hz, 1H), 2.41 (s, 3H), 2.31 - 2.37 (m, 1H), 2.17 - 2.28 (m, 1H), 1.66 - 1.76 (m, 1H).

**Example 14**

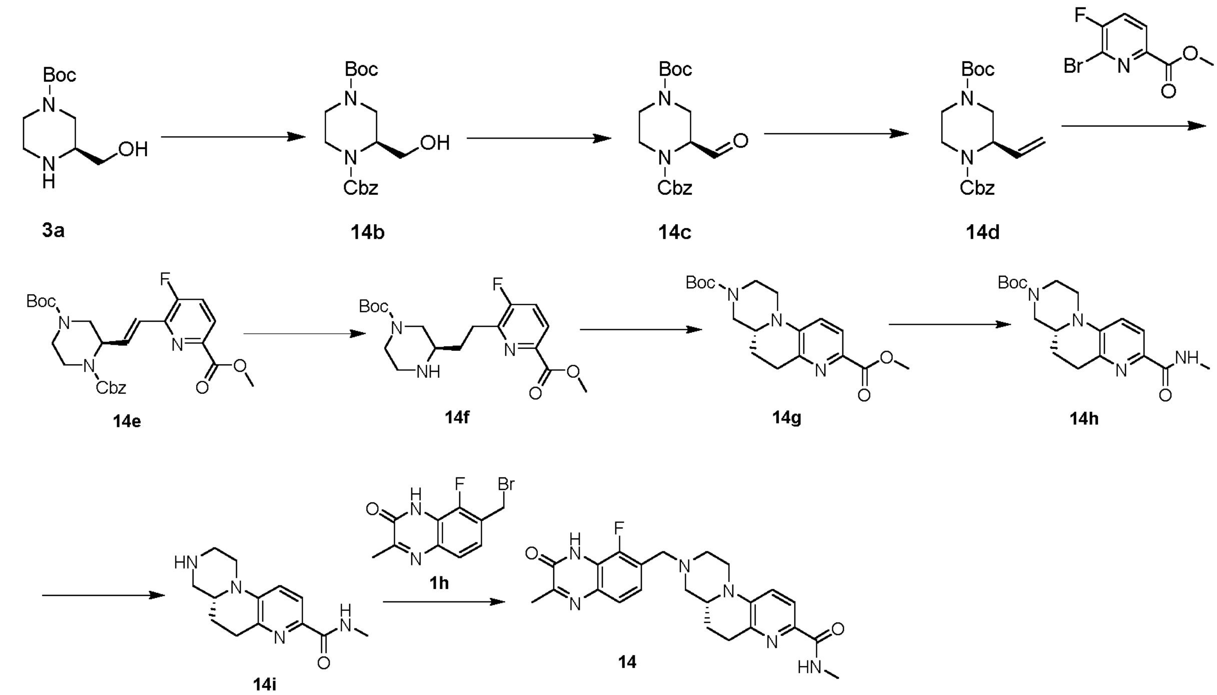

3a 14b 14c 14d

14e 14f 14g 14h

1h

14i 14

Step 1: Synthesis of intermediate **14b**

Compound **3a** (8 g, 36.99 mmol) was added to a mixture of DCM (80 mL) and $H_2O$ (80 mL). $NaHCO_3$ (9.32 g, 110.97 mmol) was then added thereto. The reaction mixture was cooled to 0°C, and benzyl chloroformate (9.47 g, 55.48 mmol) was added thereto. The reaction mixture was stirred at 20°C for 16 hours. An additional 80 mL of DCM was added to the reaction mixture. The mixture was extracted to allow phase separation. The organic phase was dried over anhydrous sodium sulfate, and filtered. The filtrate was concentrated under reduced pressure at 50°C to obtain a crude product. The crude product was purified by column chromatography (eluent: PE/EA= 5/1 to 2/1, v/v) to obtain intermediate **14b.**

Step 2: Synthesis of intermediate **14c**

Intermediate **14b** (5 g, 14.27 mmol) was added to DCM (100 mL). At 0°C, Dess-Martin periodinane (9.08 g, 21.40 mmol, 6.63 mL) was added thereto in batches, The reaction mixture was stirred at 0°C for 3 hours. Calcium hydroxide (6 g) was added to the reaction mixture, and the mixture was stirred at 0°C for 1 hour. The mixture was filtered, and the filter cake was washed with DCM (100 mL × 3). The organic phase was dried over anhydrous sodium sulfate and concentrated under reduced pressure at 50°C to obtain a crude product. The crude product was purified by flash silica gel column chromatography (eluent: PE:EA = 90:10 to 80:20, v/v) to obtain intermediate **14c.**

Step 3: Synthesis of intermediate **14d**

Under a nitrogen atmosphere, KHMDS (1 M, 3.44 mL) was added dropwise to a solution of methyltriphenylphosphonium bromide (1.23 g, 3.44 mmol) in THF (4 mL) at 20°C. The reaction mixture was stirred at 20°C for 1 hour. The temperature was then lowered to - 78°C, and a solution of intermediate **14c** (0.4 g, 1.15 mmol) in THF (4 mL) was slowly added dropwise to the reaction mixture with stirring over 10 minutes. The mixture was warmed to 20°C and stirred for 1 hour at that temperature. Methanol (72 mL) was added to the reaction mixture, then a saturated potassium sodium tartrate solution (36 mL) was added. The mixture was extracted with ethyl acetate (180 mL × 3). The organic phases was dried over anhydrous sodium sulfate, and filtered. The filtrate was concentrated under reduced pressure at 45°C to obtain a crude product. The crude product was purified by silica gel column chromatography (eluent: PE/EA = 90:10 to 80:20, v/v) to obtain intermediate **14d.**

Step 4: Synthesis of intermediate **14e**

Methyl 6-bromo-5-fluoropicolinate (100 mg, 427.31 μmol), intermediate **14d** (148.03 mg, 427.31 μmol), $Pd(OAc)_2$ (9.59 mg, 42.73 μmol), $PPh_3$ (22.42 mg, 85.46 μmol), and $Na_2CO_3$ (90.58 mg, 854.62 μmol) were added to DMF (5 mL). The reaction mixture was purged with nitrogen and then heated to 130°C and stirred for 6 hours. 5 mL of

water was added to the reaction mixture, and the mixture was extracted with ethyl acetate (10 mL) three times. The organic phases were combined, dried over anhydrous sodium sulfate, and concentrated under reduced pressure to obtain a crude product. The crude product was purified by preparative TLC (PE:EA = 2:1) to obtain intermediate **14e.** MS m/z: 500.2 $[M+1]^+$.

Step 5: Synthesis of intermediate **14f**

Intermediate **14e** (20 mg, 40.04 μmol) was added to methanol (4 mL), then Pd/C (42.61 mg) was added to the reaction mixture. The reaction mixture was purged with argon three times, then with hydrogen three times. The reaction mixture was stirred at 30°C under a hydrogen pressure of 30 psi for 16 hours. The reaction mixture was cooled to 20°C, and subjected to suction filtration under reduced pressure. The filtrate was concentrated under reduced pressure to obtain intermediate **14f,** which was used directly in the next step. MS m/z: 368.2 $[M+1]^+$.

Step 6: Synthesis of intermediate **14g**

Potassium carbonate (11.28 mg, 81.65 μmol) was added to a solution of intermediate **14f** (15 mg, 40.83 μmol) in DMF (1 mL). The reaction mixture was heated to 50°C and stirred for 6 hours. Water (5 mL) was then added to the reaction mixture, and the mixture was extracted with EA (10 mL) three times. The organic phases were combined, dried over anhydrous sodium sulfate, and concentrated under reduced pressure to obtain a crude product. The crude product was purified by preparative TLC (PE:EA = 1:1) to obtain intermediate **14g.** MS m/z: 348.2 $[M+1]^+$; $^1H$ NMR (400 MHz, $CDCl_3$) δ ppm 7.82 (d, *J*=8.63 Hz, 1H), 6.94 (d, *J*=8.76 Hz, 1H), 3.98 - 4.13 (m, 2H), 3.87 (s, 3H), 3.62 - 3.74 (m, 1H), 3.14 (tt, *J*=10.33, 3.42 Hz, 1H), 2.94 - 3.06 (m, 2H), 2.84 - 2.92 (m, 1H), 2.57 - 2.70 (m, 1H), 2.04 (dq, *J=13.35,* 4.39 Hz, 1H), 1.67 - 1.82 (m, 1H), 1.52 - 1.63 (m, 1H), 1.42 (s, 9H).

Step 7: Synthesis of intermediate **14h**

A solution of methylamine in ethanol (0.41 g, 3.96 mmol) was added to a solution of intermediate **14g** (20 mg, 57.57 μmol) in EtOH (1 mL). The reaction mixture was stirred at 25°C for 16 hours. The reaction mixture was concentrated under reduced pressure to obtain intermediate **14h.** MS m/z: 347.2 $[M+1]^+$.

Step 8: Synthesis of hydrochloride of intermediate **14i**

Intermediate **14h** (18 mg, 51.96 μmol) was dissolved in MeOH (0.5 mL). HCl/dioxane (4 M, 64.95 μL) was then added thereto, and the reaction mixture was stirred at 25°C for 2 hours. The reaction mixture was concentrated under reduced pressure to obtain the hydrochloride of intermediate **14i.** MS m/z: 247.2 $[M+1]^+$.

Step 9: Synthesis of compound **14**

Triethylamine (70.73 μmol, 9.84 μL) was added to a solution of the hydrochloride of intermediate **14i** (10 mg) in DMF (1 mL). The reaction mixture was stirred at 20°C for 0.5 hours. Then, intermediate **1h** (9.59 mg, 35.36 μmol) and potassium iodide (587.06 μg, 3.54 μmol) were added, and the reaction mixture was stirred at 50°C for 2 hours. The reaction mixture was filtered, and the residue was separated by preparative HPLC (column: Welch Xtimate C18, 100 × 40 mm × 3 μm; mobile phase: [water (trifluoroacetic acid)-acetonitrile]; gradient: 0%-30% acetonitrile over 8 minutes) to obtain a crude product. A solution of methylamine in ethanol (0.30 g) was added thereto. The reaction mixture was stirred at 25°C for 24 hours, then concentrated to obtain compound **14.** MS m/z: 437.2 $[M+1]^+$, $^1H$ NMR (400 MHz, $CD_3OD$) δ ppm 7.63 (d, J=8.53 Hz, 1H), 7.45 (d, *J*=8.53 Hz, 1H), 7.29 (t, *J*=7.65 Hz, 1H), 7.10 (d, *J*=8.78 Hz, 1H), 4.50 (br s, 1H), 3.75 (br d, *J=11.29* Hz, 1H), 3.67 (s, 2H), 3.03 - 3.14 (m, 1H), 2.87 - 2.95 (m, 2H), 2.81 (s, 3H), 2.37 - 2.43 (m, 2H), 2.16 - 2.27 (m, 1H), 2.09 (t, *J*=7.53 Hz, 1H), 1.88 - 1.95 (m, 3H), 1.65 - 1.76 (m, 1H), 1.46 - 1.54 (m, 1H).

**Example 15**

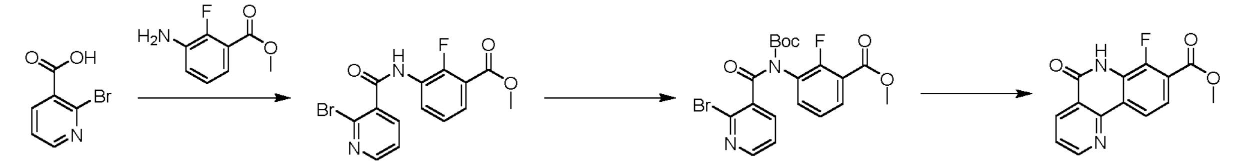

15a 15b 15c 15d

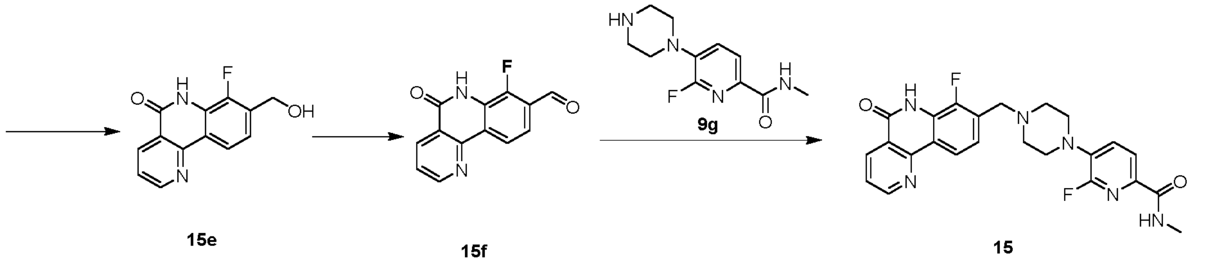



15e 15f 15

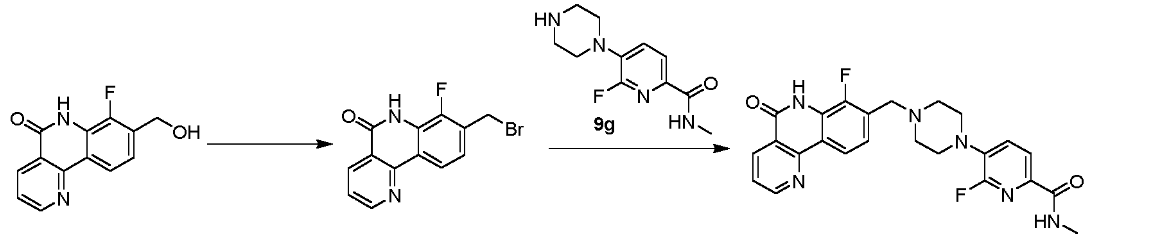



15e 15g 15

Step 1: Synthesis of intermediate **15b**

Compound **15a** (0.8 g, 3.96 mmol) and methyl 3-amino-2-fluorobenzoate (803.87 mg, 4.75 mmol) were added to DMF (10 mL). HATU (1.81 g, 4.75 mmol) and triethylamine (1.20 g, 11.88 mmol, 1.65 mL) were then added to the reaction mixture. The reaction mixture was stirred at 25°C for 16 hours. The reaction mixture was added to 5 mL of water, and the mixture was extracted with ethyl acetate (10 mL × 3). The organic phases were combined, dried over anhydrous sodium sulfate, and filtered. The filtrate was concentrated under reduced pressure at 45°C to obtain a crude product. The crude product was purified by preparative TLC (PE/EA= 2/1, $R_f$ = 0.14) to obtain intermediate **15b.** MS m/z: 352.7, 354.7 $[M+1]^+$; $^1H$ NMR (400 MHz, $CDCl_3$) δ ppm 8.53 - 8.62 (m, 1 H) 8.43 (dd, *J*=4.75, 1.88 Hz, 2 H) 8.00 (dd, *J*=7.63, 1.88 Hz, 1 H) 7.65 (td, *J*=7.41, 1.69 Hz, 1 H) 7.36 (dd, *J*=7.63, 4.75 Hz, 1 H) 7.13 - 7.24 (m, 1 H) 3.84 - 3.91 (m, 3 H).

Step 2: Synthesis of intermediate **15c**

Intermediate **15b** (700 mg, 1.98 mmol) was added to DCM (15 mL). $Et_3N$ (5.95 mmol, 827.70 μL), $(Boc)_2O$ (519.14 mg, 2.38 mmol), and DMAP (48.43 mg, 396.44 μmol) were then added to the reaction mixture. The reaction mixture was stirred at 25°C for 4 hours. The reaction mixture was added to 5 mL of water, and the mixture was extracted with ethyl acetate (10 mL × 3). The organic phases were combined, dried over anhydrous sodium sulfate, and concentrated under reduced pressure at 45°C to obtain a crude product. The crude product was purified by column chromatography (eluent: PE/EA = 5/1 to 2/1, v/v) to obtain intermediate **15c.** MS m/z: 452.9, 454.9$[M+1]^+$.

Step 3: Synthesis of intermediate **15d**

Intermediate **15c** (20 mg, 44.12 μmol) was added to DMF (3 mL). Tributylphosphine (8.93 mg, 44.12 μmol), 1,3-bis(diphenylphosphino)propane (7.28 mg, 17.65 μmol), $Pd(OAc)_2$ (3.96 mg, 17.65 μmol), and potassium carbonate (18.30 mg, 132.37 μmol) were then added to the reaction mixture. The reaction was carried out under microwave irradiation at 140°C for 10 minutes. The reaction mixture was concentrated under reduced pressure at 60°C to obtain a crude product. The crude product was purified by preparative TLC (DCM/MeOH = 20/1) to obtain intermediate **15d.** MS m/z: 272.8 $[M+1]^+$.

Step **4:** Synthesis of intermediate **15e**

Intermediate **15d** (20 mg, 73.47 μmol) was added to THF (2 mL). At 0°C, $LiAlH_4$ (2.79 mg, 73.47 μmol) was added

to the reaction mixture. The mixture was stirred at 0°C for 30 minutes. The reaction mixture was added with saturated potassium sodium tartrate solution (2 mL), and extracted with ethyl acetate (10 mL × 3). The organic phases were combined, dried over anhydrous sodium sulfate, and concentrated under reduced pressure at 45°C to obtain a crude product. The crude product was purified by preparative TLC (PE/EA = 2/1) to obtain intermediate **15e.** MS m/z: 244.8 $[M+1]^+$.

Step 5: Synthesis of intermediate **15f**

Intermediate **15e** (11.5 mg, 47.09 μmol) was added to THF (4 mL). Manganese dioxide (40.94 mg, 470.89 μmol) was then added to the reaction mixture at 20°C. The mixture was reacted at 20°C for 2 hours, then filtered. The filtrate was concentrated under reduced pressure at 45°C to obtain a crude product **15f.** MS m/z: 243.0 $[M+1]^+$.

Step 6: Synthesis of trifluoroacetate of compound **15**

Intermediate **9g** (12.26 mg, hydrochloride) was added to DMSO (2 mL), then $Et_3N$ (9.03 mg, 89.22 μmol) was added to the reaction mixture. The reaction mixture was stirred at 25°C for 0.5 hours. Intermediate **15f** (10 mg, 41.29 μmol) was then added to the reaction mixture, and the pH of the mixture was adjusted to 6 using acetic acid. The mixture was stirred at 25°C for 2 hours. $NaBH(OAc)_3$ (18.91 mg, 89.22 μmol) was added to the reaction mixture, which was stirred at 25°C for 1 hour. The reaction mixture was added with water (2 mL), and extracted with dichloromethane (5 mL × 3). The organic phases were combined, dried over anhydrous sodium sulfate, and concentrated under reduced pressure at 45°C to obtain a crude product. The crude product was purified by preparative HPLC (column: Welch Xtimate C18, 100 × 40 mm × 3 μm; mobile phase: [water (trifluoroacetic acid)-acetonitrile]; gradient: 2%-32% acetonitrile over 8 minutes) to obtain the trifluoroacetate of compound **15.** MS m/z: 487.2 $[M+Na]^+$. $^1H$ NMR (400 MHz, DMSO-$d_6$) δ ppm 12.19 (br s, 1H), 10.03-10.15 (m, 1H), 9.16 (d, *J*=2.40 Hz, 1H), 8.72 (d, *J*=3.20 Hz, 1H), 8.43-8.62 (m,2H), 7.92 (d, *J*=4.42 Hz, 1H), 7.71-7.84 (m, 2H), 7.45-7.52 (m, 1H), 4.66 (s, 2H), 3.70-3.81 (m, 4H), 3.19-3.25 (m, 4H), 2.81 (d, *J*=2.40 Hz, 3H).

Step 7: Synthesis of intermediate **15g**

Intermediate **15e** (50 mg, 204.73 μmol) was dissolved in DCM (3 mL). 1,2-Dibromotetrachloroethane (146.67 mg, 450.41 μmol) was added, and at 0°C, tributylphosphine (82.84 mg, 409.47 μmol) was added. The reaction mixture was stirred at 25°C for 3 hours. The reaction mixture was filtered to obtain the filter cake, which was intermediate **15g.** $^1H$ NMR (400 MHz, DMSO-$d_6$) δ ppm 12.02 (s, 1H), 9.10 (d, *J*=4.52 Hz, 1H), 8.65 (d, *J*=8.03 Hz, 1H), 8.39 - 8.52 (m, 1H), 7.69 - 7.80 (m, 1H), 7.34 - 7.54 (m, 1H), 4.78 - 5.01 (m, 2H).

Step 8: Synthesis of Compound **15**

Intermediate **9g** (38.02 mg, hydrochloride) was dissolved in DMF (1 mL). Triethylamine (32.95 mg, 325.61 μmol) was added, and the reaction mixture was stirred at 25°C for 0.5 hours. Intermediate **15g** (50 mg, 162.80 μmol) and KI (2.70 mg, 16.28 μmol) were then added. The reaction mixture was stirred at 50°C for 3 hours. The reaction mixture was added with water (2 mL), and stirred for 10 minutes, resulting in the precipitation of a solid. The reaction mixture was filtered to obtain the filter cake, which was compound **15g.** MS m/z: 465.1 $[M+1]^+$. $^1H$ NMR (400 MHz, DMSO-$d_6$) δ ppm 11.90 (s, 1H), 9.09 (dd, *J*=4.63, 1.75 Hz, 1H), 8.65 (dd, *J*=7.94, 1.81 Hz, 1H), 8.39 - 8.49 (m, 2H), 7.84 (dd, *J*=8.13, 1.25 Hz, 1H), 7.72 (dd, *J*=8.00, 4.50 Hz, 1H), 7.57 (dd, *J*=10.76, 8.13 Hz, 1H), 7.37 (dd, *J*=7.94, 6.44 Hz, 1H), 3.75 (s, 2H), 3.15 - 3.24 (m, 4H), 2.76 (d, *J*=4.75 Hz, 3H), 2.61 - 2.65 (m, 4H).

**Example 16**

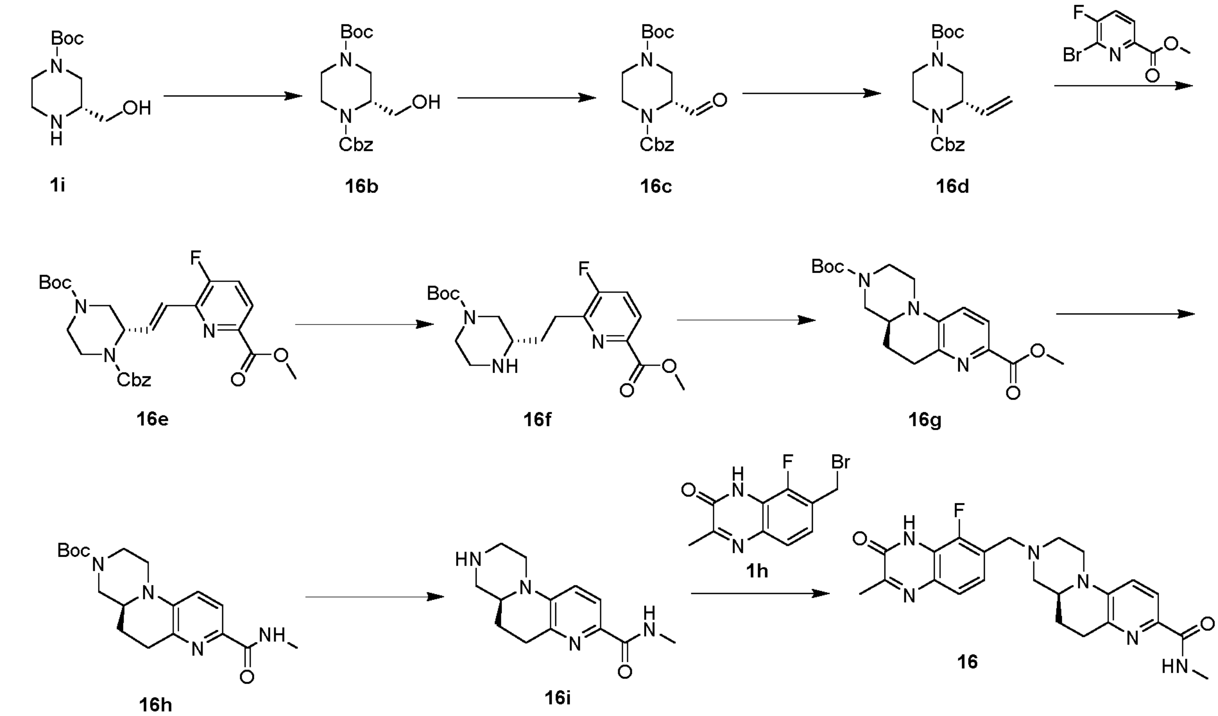

1i 16b 16c 16d 16e 16f 16g 1h 16h 16i 16

Step 1: Synthesis of intermediate **16b**

Compound **1i** (5 g, 23.12 mmol) was added to a mixture of $H_2O$ (40 mL) and DCM (40 mL). $NaHCO_3$ (5.83 g, 69.36 mmol) was then added to the reaction mixture. The mixture was cooled to 0°C, and benzyl chloroformate (5.92 g, 34.68 mmol, 4.95 mL) was added to the reaction mixture. The reaction mixture was stirred at 20°C for 16 hours. DCM (40 mL) was added to the reaction mixture, resulting in separation of phases. The organic phase was dried over anhydrous sodium sulfate, and concentrated under reduced pressure at 50°C to obtain a crude product. The crude product was purified by column chromatography (eluent: PE/EA = 5:1 to 2:1, v/v) to obtain intermediate **16b.**

Step 2: Synthesis of intermediate **16c**

Intermediate **16b** (8 g, 22.83 mmol) was added to DCM (120 mL). At 0°C, Dess-Martin periodinane (14.52 g, 34.25 mmol, 10.61 mL) was added to the reaction mixture in batches. The mixture was stirred at 0°C for 3 hours. Calcium hydroxide (10 g) was added to the reaction mixture, and the mixture was stirred at 0°C for 1 hour. The mixture was then filtered, and the filter cake was washed with dichloromethane (60 mL × 3). The filtrate was dried over anhydrous sodium sulfate and concentrated under reduced pressure at 50°C to obtain a crude product. The crude product was purified by column chromatography (eluent: ethyl acetate/n-heptane = 5/1 to 2/1, v/v) to obtain intermediate **16c.**

Step 3: Synthesis of intermediate **16d**

Under a nitrogen atmosphere, KHMDS (1 M, 51.67 mL) was added dropwise to a solution of methyltriphenylphosphonium bromide (18.46 g, 51.67 mmol) in THF (50 mL) at 20°C. The reaction mixture was stirred at 20°C for 1 hour. The temperature was then lowered to -70°C, and a solution of intermediate **16c** (6 g, 17.22 mmol) in THF (50 mL) was slowly added dropwise to the reaction mixture with stirring over 10 minutes. The reaction mixture was warmed to 20°C and stirred for 3 hours at that temperature. The reaction mixture was added with 50 mL of methanol, then 50 mL of saturated potassium sodium tartrate solution was added thereto. The mixture was extracted with ethyl acetate (100 mL × 3). The organic phase was dried over anhydrous sodium sulfate and filtered. The filtrate was concentrated under reduced pressure at 45°C to obtain a crude product. The crude product was purified by column chromatography (eluent: PE/EA = 5/1 to 1/1, v/v) to obtain intermediate **16d.** $^1H$ NMR (400 MHz, $CDCl_3$) δ ppm 7.14 - 7.28 (m, 5 H) 5.61 (ddd, *J*=17.39, 10.76, 4.50 Hz, 1 H) 5.02 - 5.11 (m, 2 H) 5.00 (d, J=2.63 Hz, 2 H) 4.58 (br s, 1 H) 3.67 - 3.96 (m, 3 H) 2.58 - 3.04 (m, 3 H) 1.30 (s, 9 H).

Step 4: Synthesis of intermediate **16e**

Intermediate **16d** (500.00 mg, 1.44 mmol), methyl 6-bromo-5-fluoropicolinate (337.77 mg, 1.44 mmol), $PPh_3$

(75.71 mg, 288.67 μmol), $Na_2CO_3$ (458.93 mg, 4.33 mmol), and $Pd(OAc)_2$ (32.40 mg, 144.33 μmol) were added to DMF (5 mL). The reaction mixture was purged with nitrogen three times and stirred at 130°C for 12 hours. The reaction mixture was added with 10 mL of water, then extracted with ethyl acetate (10 mL × 3). The organic phase was dried over anhydrous sodium sulfate, and concentrated under reduced pressure at 50°C to obtain a crude product. The crude product was purified by column chromatography (eluent: PE/EA = 4/1 to 2/1, v/v) to obtain intermediate **16e.** MS m/z: 522.1 $[M+23]^+$.

Step 5: Synthesis of intermediate **16f**

Intermediate **16e** (0.2 g, 400.38 μmol) was added to a hydrogenation flask containing methanol (10 mL). The flask was purged with argon once, and Pd/C (200 mg) was added into the reaction flask. The reaction mixture was purged with hydrogen three times and stirred under a hydrogen atmosphere (30 psi) at 30°C for 16 hours. The reaction mixture was subjected to suction filtration under reduced pressure through diatomite, and the filtrate was concentrated under reduced pressure at 40°C to obtain intermediate **16f.** MS m/z: 368.1 $[M+1]^+$.

Step 6: Synthesis of intermediate **16g**

Intermediate **16f (100** mg, 272.17 μmol) was added to DMF (4 mL), then potassium carbonate (75.23 mg, 544.34 μmol) was added to the reaction mixture. The reaction mixture was stirred at 50°C for 2 hours, and subjected to suction filtration under reduced pressure. The filtrate was concentrated under reduced pressure to obtain intermediate **16g.**

MS m/z: 348.2 $[M+1]^+$.

Step 7: Synthesis of intermediate **16h**

Intermediate **16g** (80 mg, 230.28 μmol) was added to ethanol (2 mL). Then a solution of methylamine in ethanol (2.53 g, 24.44 mmol) was added thereto. The reaction mixture was stirred at 30°C for 16 hours, and concentrated under reduced pressure to obtain intermediate **16h.** MS m/z: 347.1 $[M+1]^+$

Step 8: Synthesis of hydrochloride of intermediate **16i**

Intermediate **16h** (40 mg, 115.47 μmol) was added to MeOH (2 mL). HCl/dioxane solution (4 M, 144.33 μL) was then added thereto. The reaction mixture was stirred at 30°C for 4 hours, and concentrated under reduced pressure to obtain the hydrochloride of intermediate **16i.** MS m/z: 269.1 $[M+23]^+$.

Step 9: Synthesis of trifluoroacetate of compound **16**

$Et_3N$ (10.74 mg, 106.09 μmol, 14.77 μL) was added to a solution of the hydrochloride of intermediate **16i** (15 mg) in DMF (1 mL). The reaction mixture was stirred at 20°C for 0.5 hours. Intermediate **1h** (14.38 mg, 53.05 μmol) and potassium iodide (880.58 μg, 5.30 μmol) were then added thereto. The reaction mixture was stirred at 50°C for 2 hours, and concentrated under reduced pressure to obtain a crude product. The crude product was purified by preparative HPLC (column: Welch Xtimate C18, 100 × 40 mm × 3 μm; mobile phase: [water (trifluoroacetic acid)-acetonitrile]; gradient: 24%-54% acetonitrile) to obtain the trifluoroacetate of compound 16. MS m/z: 437.2 $[M+1]^+$; $^1H$ NMR (400 MHz, $CD_3OD$) δ ppm 7.82 (d, J=8.53 Hz, 1 H) 7.71 (d, J=8.28 Hz, 1 H) 7.44 - 7.51 (m, 1 H) 7.37 (d, J=8.78 Hz, 1 H) 4.59 (s, 2 H) 4.23 (br d, J=14.56 Hz, 1 H) 3.61 - 3.75 (m, 2 H) 3.38 - 3.54 (m, 1 H) 3.08 - 3.23 (m, 3 H) 2.99 - 3.06 (m, 2 H) 2.94 (s, 3 H) 2.56 (s, 3 H) 2.14 - 2.24 (m, 1 H) 1.85 - 1.98 (m, 1 H).

**Example 17**

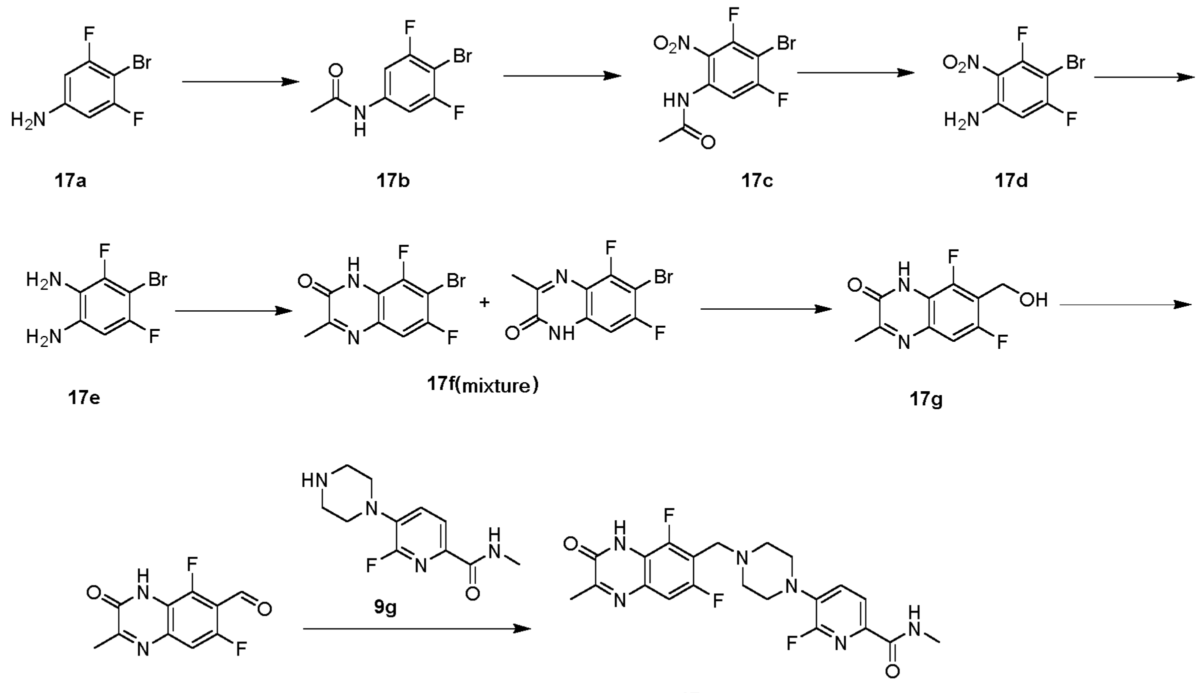

17a 17b 17c 17d

17e 17f(mixture) 17g

9g

17h 17

Step 1: Synthesis of intermediate 17b

At 0°C, triethylamine (19.46 g, 192.30 mmol) was added dropwise to a solution of compound **17a** (10 g, 48.08 mmol) in DCM (50 mL). The mixture was stirred for 30 minutes. Subsequently, acetyl chloride (6.04 g, 76.92 mmol, 5.47 mL) was slowly added dropwise thereto. The reaction mixture was then warmed to 25°C and reacted for 3 hours. The reaction mixture was added to water (100 mL). The mixture was extracted with dichloromethane (40 mL × 5). The organic phases were combined, washed with saturated brine (100 mL). The organic phase was dried over anhydrous sodium sulfate, and filtered. The filtrate was concentrated under reduced pressure to obtain a crude product. The crude product was purified by column chromatography (eluent: EA/PE = 0%-10%, v/v) to obtain intermediate **17b.** MS m/z: 249.9, 251.9 $[M+1]^+$; $^1$H NMR (400 MHz, DMSO-$d_6$) δ ppm 10.41 (s, 1 H) 7.43-7.45 (m, 2 H) 2.07 (s, 3 H).

Step 2: Synthesis of intermediate **17c**

Fuming nitric acid (71.99 mmol, 3.24 mL) was added to a three-neck flask and cooled to 0°C. Intermediate **17b** was added in batches, then sulfuric acid (334.75 mmol, 17.84 mL) was slowly added dropwise thereto while maintaining the temperature at 0°C. The dropwise addition was completed within 1 hour, and the mixture was then warmed to 25°C and reacted for 1 hour. The reaction mixture was poured into 100 mL of 3M sodium hydroxide solution at 0°C, and the pH was adjusted to approximately 7. The mixture was extracted with ethyl acetate (30 mL × 5). The organic phases were combined, washed with saturated brine (100 mL × 2), dried over anhydrous sodium sulfate, filtered, and concentrated under reduced pressure to obtain intermediate **17c.** $^1$H NMR (400 MHz, DMSO-$d_6$) δ ppm 10.55 (s, 1 H) 7.50-7.53 (m, 1 H) 2.08 (s, 3 H).

Step 3: Synthesis of intermediate **17d**

Intermediate **17c** (9.50 g, 32.20 mmol) was dissolved in sulfuric acid (90 mL) and reacted at 50°C for 2 hours. The reaction mixture was poured into 100 mL of ice water, and the pH was adjusted to 7-8 using 3 M NaOH solution. The mixture was filtered, and the filter cake was washed with 10 mL of water, dried under vacuum to obtain intermediate **17d.** $^1$H NMR (400 MHz, DMSO-*d*6) δ ppm 7.45 (br, s, 2 H) 6.74-6.77 (m, 1 H).

Step 4: Synthesis of intermediate **17e**

Intermediate **17d** (6.2 g, 24.51 mmol) was dissolved in EtOH (70 mL) and cooled to 0°C. Acetic acid (14.72 g, 245.06 mmol, 14.03 mL) was added, then zinc (14.88 g, 227.56 mmol) was added in batches. The reaction was carried out for 1 hour. The reaction mixture was poured into 500 mL of water and extracted with ethyl acetate (80 mL × 5). The organic

phase was washed with 300 mL of saturated brine, dried over anhydrous sodium sulfate, and concentrated under reduced pressure to obtain intermediate **17e.** $^1$H NMR (400 MHz, DMSO-*d6)* δ ppm 6.37-6.40 (m, 1 H) 5.21 (br, s, 2 H) 4.65 (br, s, 2 H).

Step 5: Synthesis of intermediate **17f**

Intermediate **17e** (3.15 g, 14.12 mmol) was added to EtOH (30 mL). Then pyruvic acid (1.49 g, 16.95 mmol, 1.19 mL) was dropwise added. The reaction mixture was reacted at 100°C for 2 hours and concentrated under reduced pressure. 10 mL of ethanol was added, and the mixture was stirred for 20 minutes. The mixture was filtered, and the filter cake was collected to obtain a mixture of intermediate **17f.** MS m/z: 274.9, 276.9 $[M+1]^+$.

Step 6: Synthesis of intermediate **17g**

The mixture of intermediate **17f** (3.05 g, 11.09 mmol) and 1-(tributylstannyl)methanol (4.27 g, 13.31 mmol) were dissolved in dioxane (30 mL). Chloro(2-dicyclohexylphosphino-2',4',6'-triisoporpyl-1,1'-biphenyl)[2-(2'-amino-1,1'-biphenyl)] palladium (II) (436.24 mg, 554.45 μmol) was then added thereto. The reaction mixture was purged with nitrogen and reacted at 110°C for 3 hours. The reaction mixture was concentrated under reduced pressure. The reaction mixture was purified by flash silica gel chromatography (EA/PE = 0% to 30%, MeOH/DCM = 0% to 5%) to obtain a crude product. The crude product was further separated by preparative HPLC (column: Welch Xtimate C18, 100 × 40 mm × 3 μm; mobile phase: [water (trifluoroacetic acid)-acetonitrile]; gradient: 0%-26% acetonitrile over 8 minutes) to obtain intermediate **17g.** MS m/z: 227.1 $[M+1]^+$; $^1$H NMR (400 MHz, DMSO-*d*6) δ ppm 12.51(s, 1 H) 7.35-7.37 (m, 1 H) 5.25 (s, 1 H) 4.57 (s, 2 H) 2.41 (s, 3 H).

Step 7: Synthesis of intermediate **17h**

At 25°C, Dess-Martin periodinane (84.39 mg, 198.96 μmol) was added to a solution of intermediate **17g** (30 mg, 132.64 μmol) in DCM (2 mL). The reaction mixture was stirred for 2 hours. The reaction mixture was added with calcium hydroxide (0.057 g), and stirred at 25°C for 1 hour. The mixture was filtered, and the filter cake was washed with dichloromethane (10 mL × 3). The filtrate was concentrated under reduced pressure at 50°C to obtain a crude product. The crude product was purified by preparative TLC (PE:EA= 1:1) to obtain intermediate **17h.** $^1$H NMR (400 MHz, DMSO-$d_6$) δ ppm 12.72 (s, 1H), 10.27 (s, 1H), 7.53 - 7.63 (m, 1H), 2.47 (s, 3H).

Step 8: Synthesis of compound **17**

Intermediate **9g** (21.86 mg, hydrochloride) was dissolved in DMSO (1 mL). Triethylamine (13.54 mg, 133.83 μmol) was added to the reaction mixture with stirring, and the mixture was stirred at 25°C for 30 minutes. Intermediate **17h** (15 mg, 66.92 μmol) was then added thereto, and the pH was adjusted to 6-7 using acetic acid. The reaction mixture was stirred at 25°C for 2 hours. $NaBH(OAc)_3$ (28.36 mg, 133.83 μmol) was added thereto, and the mixture was stirred at 25°C for 16 hours. The reaction mixture was added with water (2 mL) and extracted with EA (5 mL) three times. The organic phases were combined, dried over anhydrous sodium sulfate, and concentrated under reduced pressure to obtain a crude product. The crude product was purified by preparative HPLC (column: Welch Xtimate C18, 100 × 40 mm × 3 μm; mobile phase: [water (trifluoroacetic acid)-acetonitrile]; gradient: 0%-30% acetonitrile over 8 minutes). The resulting product was basified with triethylamine (3 μL) and further purified by preparative TLC (100% EA) to obtain compound **17.** MS m/z: 447.1 $[M+1]^+$; $^1$H NMR (400 MHz, $CDCl_3$) δ ppm 7.88 (dd, J=8.16, 1.13 Hz, 1H), 7.49 (dd, J=10.29, 8.03 Hz, 1H), 7.37 (dd, J=9.79, 1.76 Hz, 1H), 3.87 (s, 2H), 3.22 - 3.25 (m, 4H), 2.91 (s, 3H), 2.71 - 2.77 (m, 4H), 2.53 (s, 3H).

**Example 18**

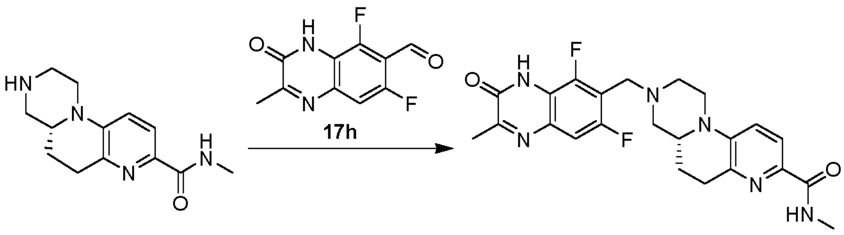


14i 18

Step 1: Synthesis of compound **18**

Intermediate **14i** (19.87 mg, hydrochloride) was dissolved in DMSO (1 mL). Triethylamine (133.83 μmol, 18.63 μL) was added to the reaction mixture with stirring, and the mixture was stirred at 25°C for 30 minutes. Intermediate **17h** (15.00 mg, 66.92 μmol) was then added thereto, and the pH was adjusted to 6-7 using acetic acid. The mixture was stirred at 25°C for 2 hours. $NaBH(OAc)_3$ (28.36 mg, 133.83 μmol) was added thereto, and the reaction mixture was stirred at 25°C for 16 hours. The reaction mixture was added with water (2 mL) and extracted with EA (5 mL × 3). The organic phases were combined, dried over anhydrous sodium sulfate, and concentrated under reduced pressure to obtain a crude product. The crude product was separated by preparative HPLC (column: Welch Xtimate C18, 100 × 40 mm × 3 μm; mobile phase: [water (trifluoroacetic acid)-acetonitrile]; gradient: 0%-30% acetonitrile over 8 minutes) to obtain a further crude product. The crude product was added with triethylamine (3 μL), and purified by preparative TLC (100% EA) to obtain compound **18.** MS m/z: 455.2 $[M+1]^+$; $^1H$ NMR (400 MHz, $CD_3OD$) δ ppm 7.64 (d, J=8.53 Hz, 1H), 7.06 - 7.16 (m, 2H), 3.76 (br d, J=12.55 Hz, 1H), 3.64 (s, 2H), 3.06 - 3.15 (m, 1H), 2.83 - 2.95 (m, 4H), 2.81 - 2.83 (m, 3H), 2.43 (s, 3H), 2.23 (td, *J*=11.54, 3.26 Hz, 1H), 1.92 - 2.00 (m, 2H), 1.64 - 1.78 (m, 1H), 1.22 - 1.27 (m, 1H).

**Example 19**

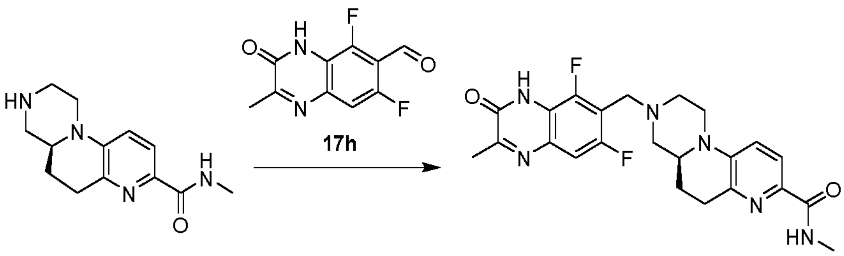


16i 19

Step 1: Synthesis of trifluoroacetate of compound **19**

Intermediate **16i** (12.5 mg, hydrochloride) was dissolved in DMSO (1 mL). Triethylamine (74.58 μmol, 10.38 μL) was added, and the reaction mixture was stirred at 20°C for 0.5 hours. **17h** (8.36 mg, 37.29 μmol) and the pH was adjusted to 6-7 using acetic acid. The mixture was stirred at 20°C for 2 hours. Subsequently, $NaBH(OAc)_3$ (15.81 mg, 74.58 μmol) was added, and the mixture was stirred for 16 hours. The reaction mixture was extracted with water (3 mL) and ethyl acetate (3 mL × 3). The organic phases were combined, washed with 3 mL of saturated brine, dried over anhydrous sodium sulfate, and filtered to obtain a crude product. The crude product was separated by preparative HPLC (column: Welch Xtimate C18, 100 × 40 mm × 3 μm; mobile phase: [water (trifluoroacetic acid)-acetonitrile]; gradient: 0%-30% acetonitrile over 8 minutes) to obtain the trifluoroacetate of compound **19.** MS m/z: 455.1 $[M+1]^+$; $^1H$ NMR (400 MHz, $CD_3OD$) δ ppm 1.63 - 1.77 (m, 1 H) 1.93 - 2.05 (m, 2 H) 2.18 - 2.31 (m, 1 H) 2.42 (s, 3 H) 2.81 (s, 3 H) 2.82 - 2.86 (m, 2 H) 2.93 (br t, J=10.42 Hz, 2 H) 3.07 (s, 2 H) 3.72 - 3.81 (m, 3 H) 7.09 (d, J=8.78 Hz, 1 H) 7.27 (dd, J=9.54, 1.76 Hz, 1 H) 7.59 - 7.68 (m, 1 H).

**Example 20**

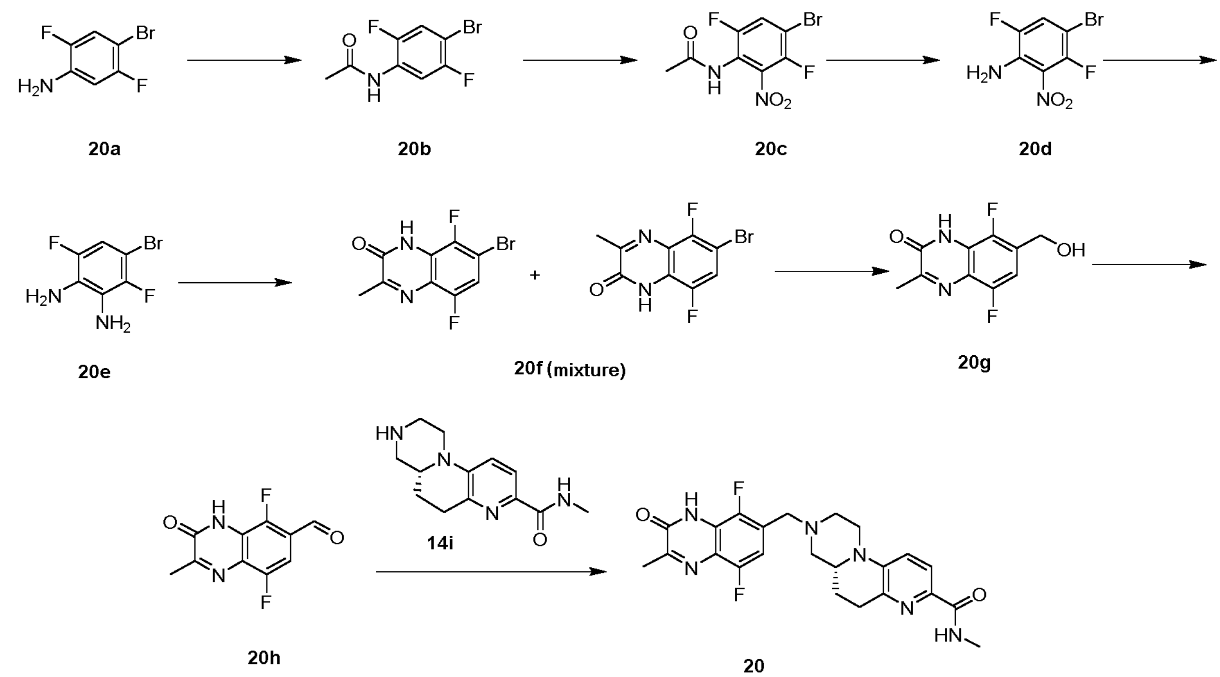

20a 20b 20c 20d

20e 20f (mixture) 20g

14i

20h 20

Step 1: Synthesis of intermediate **20b**

At 0°C, triethylamine (7.78 g, 76.92 mmol, 10.71 mL) was added dropwise to a solution of compound **20a** (4 g, 19.23 mmol) in DCM (20 mL). The mixture was stirred for 0.5 hours. Subsequently, acetyl chloride (2.42 g, 30.77 mmol, 2.19 mL) was slowly added dropwise thereto. The reaction mixture was then warmed to 18°C and reacted for 3 hours. The reaction mixture was slowly added dropwise into 100 mL of water. The mixture was extracted with dichloromethane (20 mL × 5). The organic phase was washed with 100 mL of saturated brine, dried over anhydrous sodium sulfate, and concentrated under reduced pressure to obtain a crude product. The crude product was purified by flash silica gel column chromatography (EA/PE = 0%-10%) to obtain intermediate **20b.** MS m/z: 250.0 $[M+1]^+$; $^1H$ NMR (400 MHz, DMSO-$d_6$) δ ppm 10.03 (br s, 1 H) 8.02 - 8.17 (m, 1 H) 7.77 (dd, *J*=10.16, 6.40 Hz, 1 H) 2.12 (s, 3 H).

Step 2: Synthesis of intermediate **20c**

At 0°C, intermediate **20b** (2.52 g, 10.08 mmol) was suspended in nitric acid (1.27 g, 20.16 mmol, 907.24 μL). Sulfuric acid (9.19 g, 93.73 mmol, 5.00 mL) was then slowly added dropwise to the mixture. After the dropwise addition was complete, the mixture was reacted at 25°C for 1 hour. The reaction mixture was added dropwise into water, resulting in the precipitation of a solid. The pH was adjusted to 6-7 using 2 M NaOH solution. The mixture was filtered, and the filter cake was dried to obtain intermediate **20c.** $^1H$ NMR (400 MHz, DMSO-$d_6$) δ ppm10.48 (s, 1 H) 8.21 - 8.38 (m, 1 H) 2.07 (s, 3 H).

Step **3:** Synthesis of intermediate **20d**

Intermediate **20c** (2.28 g, 7.73 mmol) was dissolved in sulfuric acid (22 mL) and stirred at 50°C for 2 hours. The reaction mixture was added into 400 mL of water, and the pH was adjusted to 6-7 using 2 M NaOH solution, resulting in the precipitation of a solid. The mixture was filtered to obtain the filter cake, and the filter cake was dried under vacuum to obtain intermediate **20d.** $^1H$ NMR (400 MHz, DMSO-$d_6$) δ ppm 7.76 - 7.89 (m, 1 H) 7.09 (s, 2 H).

Step 4: Synthesis of intermediate **20e**

Intermediate **20d** (1.49 g, 5.89 mmol) was dissolved in EtOH (15 mL). At 0°C, zinc powder (3.66 g, 55.95 mmol) and acetic acid (3.54 g, 58.89 mmol, 3.37 mL) were added in batches. The mixture was stirred at 40°C for 1 hour. The reaction mixture was filtered, and the filtrate was concentrated under reduced pressure to obtain a crude product. The crude product was purified by column chromatography (eluent: PE:EA = 100:0 to 90:10, v/v) to obtain intermediate **20e.** MS m/z: 223.0 $[M+1]^+$; $^1H$ NMR (400 MHz, DMSO-$d_6$) δ ppm 6.65 (dd, *J*=10.04, 6.02 Hz, 1 H) 5.06 (s, 2 H) 4.90 (s, 2 H).

Step 5: Synthesis of intermediate **20f**

Intermediate **20e** (1.14 g, 5.11 mmol) was dissolved in EtOH (12 mL), and pyruvic acid (540.17 mg, 6.13 mmol, 432.14 μL) was then added. The mixture was stirred at 100°C for 2 hours, then slowly cooled to 25°C to crystallize over 14 hours. The reaction mixture was filtered to obtain the filter cake, which was intermediate **20f** (mixture). MS m/z: 274.9 $[M+1]^+$.

Step 6: Synthesis of intermediate **20g**

Under a nitrogen atmosphere, intermediate **20f** (500 mg, 1.82 mmol) was dissolved in dioxane (5 mL). 1-(Tributylstannyl)methanol (700.42 mg, 2.18 mmol) and chloro(2-dicyclohexylphosphino-2',4',6'-triisoporpyl-1,1'-biphenyl)[2-(2'-amino-1,1'-biphenyl)] palladium (II) (71.51 mg, 90.89 μmol) were added. The reaction mixture was stirred at 110°C for 4 hours. The reaction mixture was concentrated under reduced pressure and purified by column chromatography (eluent: PE:EA = 90:10 to 50:50, V/V) to obtain a crude product. The crude product was purified by preparative HPLC (column: Welch Xtimate C18, 100 × 40 mm × 3 μm; mobile phase: [water (trifluoroacetic acid)-acetonitrile]; gradient: 0%-26% acetonitrile) to obtain intermediate **20g.** MS m/z: 226.8 $[M+1]^+$; $^1H$ NMR (400 MHz, DMSO-$d_6$) δ ppm 12.58 (br s, 1 H) 6.99 - 7.25 (m, 1 H) 4.62 (br d, J=3.51 Hz, 2 H) 2.43 (br s, 3 H).

Step 7: Synthesis of intermediate **20h**

Intermediate **20g** (100 mg, 442.13 μmol) was dissolved in DCM (2 mL). Dess-Martin periodinane (281.29 mg, 663.19 μmol, 205.47 μL) was added, and the reaction mixture was stirred at 20°C for 2 hours. The reaction mixture was added with 0.1899 g of calcium hydroxide and stirred at 20°C for 1 hour. The reaction mixture was filtered to obtain a crude product. The crude product was purified by preparative TLC (DCM:MeOH = 20:1) to obtain intermediate **20h.** $^1H$ NMR (400 MHz, DMSO-$d_6$) δ ppm 12.96 (br, s, 1H), 10.26 (s, 1H), 7.40 - 7.44 (m, 1H), 2.49 (s, 3H).

Step 8: Synthesis of compound **20**

Intermediate **14i** (13.25 mg, hydrochloride) was dissolved in DMSO (1 mL). Triethylamine (9.03 mg, 89.22 μmol, 12.42 μL) was added to the reaction mixture with stirring, and the mixture was stirred at 25°C for 30 minutes. Intermediate **20h** (10 mg, 44.61 μmol) was added to the reaction mixture, and the pH was adjusted to 6-7 using acetic acid. The mixture was stirred at 25°C for 2 hours. $NaBH(OAc)_3$ (18.91 mg, 89.22 μmol) was added thereto, and the reaction mixture was stirred at 25°C for 16 hours. The reaction mixture was added with water (2 mL) and extracted with EA (5 mL × 3). The organic phases were combined, dried over anhydrous sodium sulfate, and concentrated under reduced pressure to obtain a crude product. The crude product was separated by preparative HPLC (column: Welch Xtimate C18, 100 × 40 mm × 3 μm; mobile phase: [water (trifluoroacetic acid)-acetonitrile]; gradient: 0%-30% acetonitrile) to obtain a crude product. The crude product was added with triethylamine (3 μL), and purified by preparative TLC (100% EA) to obtain compound **20.** MS m/z: 455.2 $[M+1]^+$; $^1H$ NMR (400 MHz, $CD_3OD$) δ ppm 7.75 (d, *J*=8.78 Hz, 1H), 7.38 (dd, *J*=9.41, 1.63 Hz, 1H), 7.21 (d, *J*=8.78 Hz, 1H), 4.59 (s, 1H), 3.83 - 3.90 (m, 3H), 3.14 - 3.23 (m, 1H), 3.01 - 3.11 (m, 2H), 2.94 - 2.99 (m, 1H), 2.92 - 2.94 (m, 3H), 2.85 - 2.92 (m, 1H), 2.54 (s, 3H), 2.31 - 2.41 (m, 1H), 1.93 - 2.23 (m, 2H), 1.75 - 1.88 (m, 1H).

**Example 21**

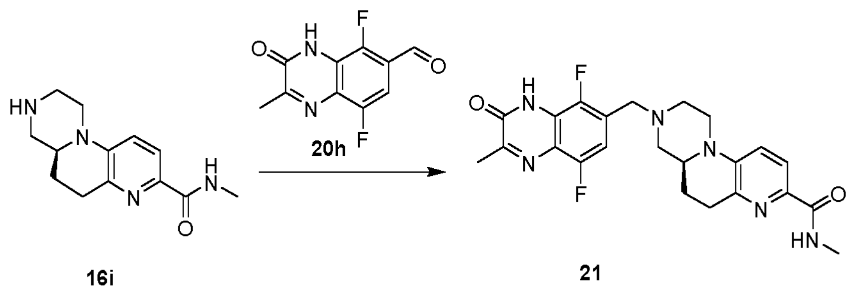

Step 1: Synthesis of compound **21**

Intermediate **16i** (15 mg, hydrochloride) was dissolved in DMSO (1 mL). Triethylamine (9.06 mg, 89.50 μmol, 12.46 μL) was added, and the reaction mixture was stirred at 20°C for 0.5 hours. Intermediate **20h** (12.54 mg, 44.75 μmol) was then added thereto and the pH was adjusted to 6-7 using acetic acid. The mixture was stirred at 20°C for 2 hours. Subsequently, $NaBH(OAc)_3$ (18.97 mg, 89.50 μmol) was added, and the mixture was stirred for 16 hours. The reaction mixture was extracted with water (3 mL) and ethyl acetate (3 mL × 3). The organic phases were combined, washed with 10 mL of saturated brine, then dried over anhydrous sodium sulfate, and filtered to obtain a crude product. The crude product was separated by preparative HPLC (column: Welch Xtimate C18, 100 × 40 mm × 3 μm; mobile phase: [water (trifluoroacetic acid)-acetonitrile]; gradient: 0%-30% acetonitrile over 8 minutes) to obtain a crude product. The crude product was added with 3 μL of triethylamine and purified by preparative TLC (EA = 100%) to obtain compound 21. MS m/z: 455.0 $[M+1]^+$; $^1H$ NMR (400 MHz, $CD_3OD$) δ ppm 7.58 - 7.69 (m, 1 H) 7.10 (d, J=8.78 Hz, 2 H) 3.76 (br d, *J*=11.80 Hz, 1 H) 3.64 (d, *J*=1.51 Hz, 2 H) 3.04 - 3.17 (m, 1 H) 2.82 - 2.96 (m, 5 H) 2.82 (s, 3 H) 2.43 (s, 3 H) 2.15 - 2.30 (m, 1 H) 1.88 - 2.01 (m, 2 H) 1.61 - 1.79 (m, 1 H).

**Example 22**

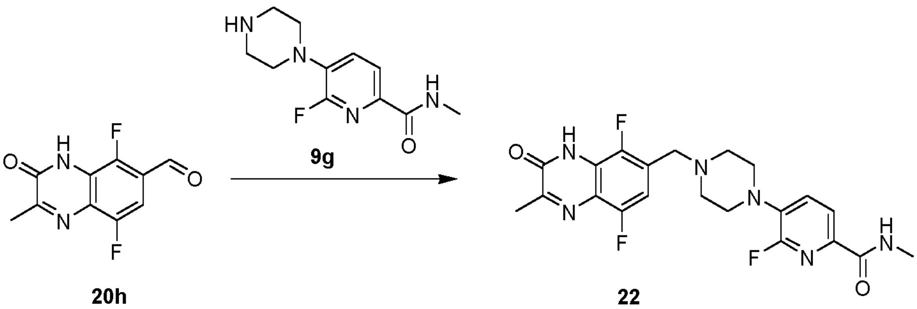

20h

22

Step 1: Synthesis of trifluoroacetate of compound **22**

Intermediate **9g** (7.29 mg, hydrochloride) was dissolved in DMSO (1 mL). Triethylamine (44.61 μmol, 6.21 μL) was added, and the reaction mixture was stirred at 20°C for 0.5 hours. Intermediate **20h** (5 mg, 22.31 μmol) was then added thereto and the pH was adjusted to 6-7 using acetic acid. The mixture was stirred for 2 hours. Subsequently, $NaBH(OAc)_3$ (9.45 mg, 44.61 μmol) was added, and the mixture was stirred for 4 hours. The reaction mixture was extracted with water (1 mL) and ethyl acetate (1 mL × 3). The organic phase was washed with 10 mL of saturated brine, dried, and filtered to obtain a crude product. The crude product was separated by preparative HPLC (column: Welch Xtimate C18, 100 × 40 mm × 3 μm; mobile phase: [water (trifluoroacetic acid)-acetonitrile]; gradient: 1%-31% acetonitrile over 8 minutes) to obtain the trifluoroacetate of compound **22.** MS m/z: 447.1 $[M+1]^+$; $^1H$ NMR (400 MHz, $CD_3OD$) δ ppm 7.92 - 7.99 (m, 1 H) 7.54 - 7.68 (m, 1 H) 7.27 (dd, *J*=10.16, 5.40 Hz, 1 H) 4.22 - 4.39 (m, 2 H) 3.42 - 3.51 (m, 4 H) 3.23 - 3.31 (m, 4 H) 2.93 (s, 3 H) 2.57 (s, 3 H).

**Example 23**

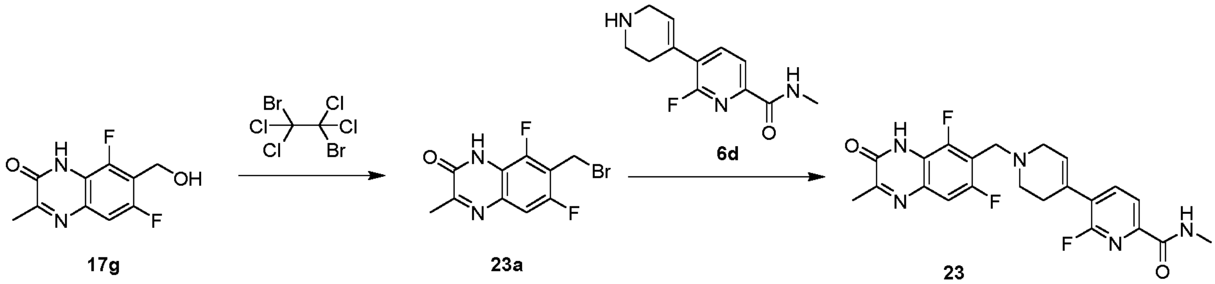

17g

23a

23

Step 1: Synthesis of intermediate **23a**

Compound **17g** (100.00 mg, 442.13 μmol) and 1,2-dibromo-1,1,2,2-tetrachloroethane (316.74 mg, 972.68 μmol) were dissolved in DCM (5 mL). At 0°C, tributylphosphine (178.90 mg, 884.25 μmol) was added thereto. After the addition was complete, the mixture was warmed to 25°C and stirred for 12 hours. The reaction mixture was filtered to obtain the filter cake, which was intermediate **23a.** $^1H$ NMR (400 MHz, DMSO-$d_6$) δ ppm 12.63 (br s, 1H), 7.33 - 7.72 (m, 1H), 4.71 - 4.94 (m, 2H), 2.43 (s, 3H).

Step 2: Synthesis of compound **23**

Triethylamine (21.00 mg, 207.56 μmol) was added to a solution of intermediate **6d** (25.64 mg, hydrochloride) in DMF (2 mL). The mixture was stirred at 25°C for 30 minutes. Intermediate **23a** (30 mg, 103.78 μmol) and KI (1.72 mg, 10.38 μmol) were then added, and the reaction mixture was stirred at 50°C for 2 hours. The reaction mixture was filtered, and the filtrate was concentrated to obtain a crude product. The crude product was purified by preparative HPLC (column: Welch Xtimate C18, 100 × 40 mm × 3 μm; mobile phase: [water (trifluoroacetic acid)-acetonitrile]; gradient: 0%-30% acetonitrile) to obtain a further crude product. The crude product was added with triethylamine (20 μL), and purified by preparative TLC (eluent ratio: DCM:MeOH = 10:1) to obtain compound **23.** MS m/z: 444.1 $[M+1]^+$; $^1H$ NMR (400 MHz, DMSO-$d_6$) δ ppm 12.54 (br s, 1H), 8.63 (br d, *J*=5.00 Hz, 1H), 8.05 (dd, *J*=9.82, 7.82 Hz, 1H), 7.91 (dd, *J*=7.69, 1.69 Hz, 1H), 7.46 (d, *J*=8.88 Hz, 1H), 6.14 - 6.27 (m, 1H), 3.80 (s, 2H), 3.13 - 3.26 (m, 3H), 2.79 (d, *J*=4.88 Hz, 3H), 2.60 - 2.76 (m, 3H), 2.44 (s, 3H).

**Example 24**

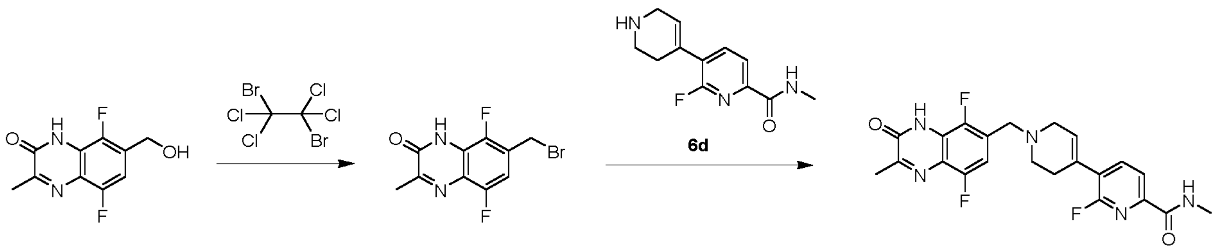


20g 24a 24

Step 1: Synthesis of intermediate **24a**

Compound **20g** (100.00 mg, 442.13 μmol) and 1,2-dibromo-1,1,2,2-tetrachloroethane (316.74 mg, 972.68 μmol) were dissolved in DCM (5 mL). At 0°C, tributylphosphine (178.90 mg, 884.25 μmol) was added thereto. After the addition was complete, the mixture was warmed to 25°C and stirred for 8 hours. The reaction mixture was filtered to obtain the filter cake, which was intermediate **24a.** $^1H$ NMR (400 MHz, DMSO-$d_6$) δ ppm 12.72 (br s, 1H), 7.32 (dd, J=10.54, 5.77 Hz, 1H), 4.76 (s, 2H), 2.44 (s, 3H).

Step 2: Synthesis of compound **24**

Triethylamine (21.00 mg, 207.56 μmol) was added to a solution of intermediate **6d** (25.64 mg, hydrochloride) in DMF (2 mL). The mixture was stirred at 25°C for 30 minutes. Intermediate **24a** (30 mg, 103.78 μmol) and KI (1.72 mg, 10.38 μmol) were then added, and the reaction mixture was stirred at 50°C for 2 hours. The reaction mixture was filtered, and the filtrate was concentrated to obtain a crude product. The crude product was purified by preparative HPLC (column: Welch Xtimate C18, 100 × 40 mm × 3 μm; mobile phase: [water (trifluoroacetic acid)-acetonitrile]; gradient: 0%-30% acetonitrile) to obtain a further crude product. The crude product was added with triethylamine (20 μL), and purified by preparative TLC (eluent ratio: DCM:MeOH = 10:1) to obtain compound **24.** MS m/z: 444.1 $[M+1]^+$; $^1H$ NMR (400 MHz, DMSO-$d_6$) δ ppm 12.63 (br s, 1H), 8.64 (br d, J=4.63 Hz, 1H), 8.01 - 8.17 (m, 1H), 7.92 (br d, J=7.38 Hz, 1H), 7.07 - 7.28 (m, 1H), 6.25 (br s, 1H), 3.75 (s, 2H), 3.18 - 3.25 (m, 3H), 2.80 (br d, J=4.63 Hz, 3H), 2.65 - 2.76 (m, 3H), 2.44 (s, 3H).

**Example 25**

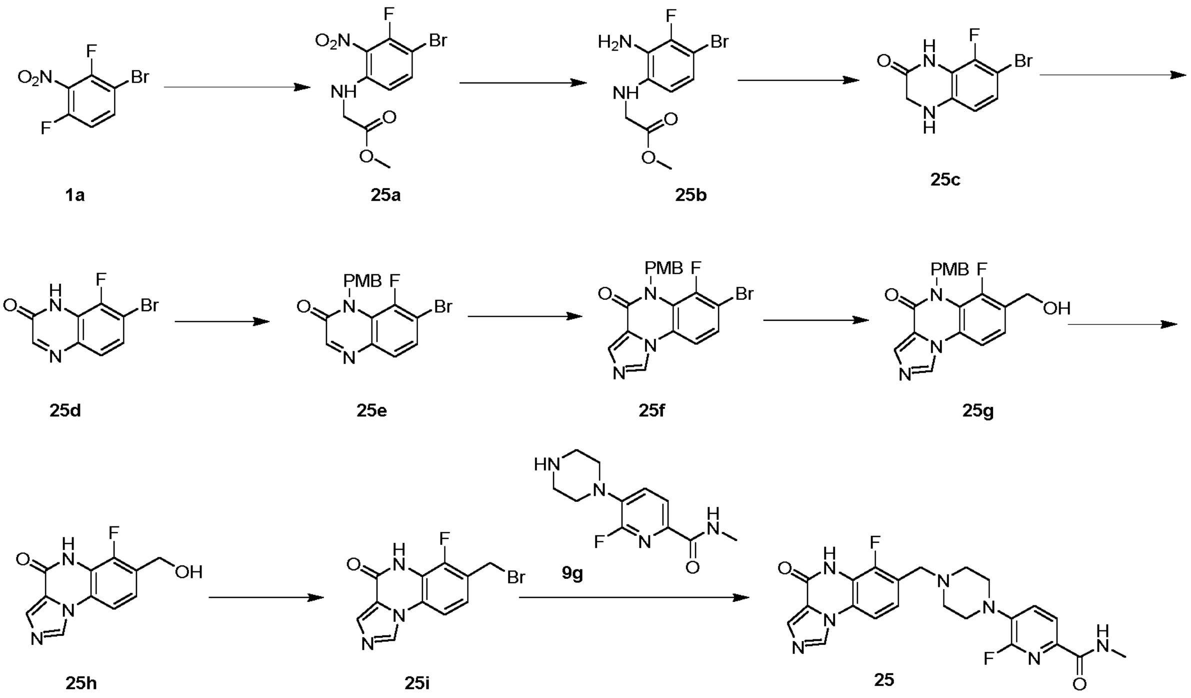

1a, 25a, 25b, 25c, 25d, 25e, 25f, 25g, 25h, 25i, 9g, 25

Step 1: Synthesis of intermediate **25a**

Compound **1a** (6.1 g, 25.63 mmol), methyl aminoacetate (3.54 g, 28.19 mmol, HCl), and N,N-diisopropylethylamine (9.94 g, 76.90 mmol) were added to DMF (60 mL). The reaction mixture was reacted at 25°C for 18 hours. The reaction mixture was poured into 300 mL of water and extracted with ethyl acetate (100 mL × 5). The organic phase was washed with 200 mL of saturated brine, dried over anhydrous sodium sulfate, filtered, and concentrated under reduced pressure to obtain a crude product. The crude product was purified by flash silica gel column chromatography (EA:PE = 0%-10%) to obtain intermediate **25a.** $^1H$ NMR (400 MHz, DMSO-$d_6$) δ ppm 7.54-7.68 (m, 2H), 6.70 (d, J=7.8 Hz, 1H), 4.20 (d, J=4.2 Hz, 2H), 3.68 (s, 3H).

Step **2:** Synthesis of intermediate **25b**

Intermediate **25a** (1.82 g, 5.93 mmol) and $NH_4Cl$ (2.54 g, 47.42 mmol) were added to a mixed solution of MeOH (26 mL) and $H_2O$ (0.468 mL). Zinc (1.98 g, 30.29 mmol) was then added. The reaction was carried out at 0°C for 2 hours. The reaction mixture was filtered, and 50 mL of water was added to the filtrate. The mixture was extracted with ethyl acetate (30 mL × 3). The organic phases were combined, washed with 50 mL of saturated brine, dried over anhydrous sodium sulfate, and filtered. The filtrate was concentrated under reduced pressure to obtain intermediate **25b.** MS m/z: 276.9, 278.9 $[M+1]^+$.

Step **3:** Synthesis of intermediate **25c**

At 0°C, intermediate **25b** (1.35 g, 4.87 mmol) was added to a mixed system of HCl/dioxane (4 M, 15.64 mL), EA (13.5 mL), and MeOH (13.5 mL). The reaction was carried out for 2 hours. The reaction mixture was directly concentrated under reduced pressure to obtain intermediate **25c.** MS m/z: 245.0, 247.0 $[M+1]^+$.

Step **4:** Synthesis of intermediate **25d**

At 0°C, DDQ (1.56 g, 6.86 mmol) was added to a solution of intermediate **25c** (1.4 g, 5.71 mmol) in DCM (14 mL). The mixture was warmed to 25°C and reacted for 4 hours. The reaction mixture was concentrated under reduced pressure, and 70 mL of saturated sodium bicarbonate solution was slowly added thereto. The mixture was stirred overnight. The mixture was extracted with ethyl acetate (50 mL × 3). The organic phases were combined, washed with 70 mL of saturated brine, dried over anhydrous sodium sulfate, filtered, and concentrated under reduced pressure to obtain a crude product. The crude product was purified by flash silica gel column chromatography (eluent ratio: MeOH/DCM = 0%-5%) to obtain intermediate **25d.** MS m/z: 243.0, 245.0 $[M+1]^+$. $^1H$ NMR (400 MHz, DMSO-$d_6$) δ ppm 12.75 (br s, 1H), 8.24 (s, 1H),7.53-7.60 (m, 2H).

Step **5:** Synthesis of intermediate **25e**

Intermediate **25d** (660 mg, 2.72 mmol) was dissolved in DMF (1 mL). Potassium carbonate (563.00 mg, 4.07 mmol), potassium iodide (67.62 mg, 407.35 μmol), and p-methoxybenzyl chloride (510.36 mg, 3.26 mmol) were added thereto, and the mixture was stirred at 20°C for 3 hours. The reaction system was added with water (15 mL), and extracted with ethyl acetate (50 mL). The phases were separated. The organic phase was washed with water, dried over anhydrous sodium sulfate, and filtered. The filtrate was concentrated to obtain a crude product. The crude product was purified by flash silica gel column chromatography (PE:EA= 3:1) to obtain intermediate **25e.** MS m/z: 362.8, 364.8 $[M+1]^+$; $^1H$ NMR (400 MHz, DMSO-$d_6$) δ ppm 8.43 (s, 1H), 7.50-7.64 (m, 2H),7.24 (d, J=4.2 Hz, 2H), 6.85 (d, J=4.2 Hz, 2H), 5.60 (s, 2H), 3.72 (s, 3H).

Step **6:** Synthesis of intermediate **25f**

At 0°C, a solution of p-toluenesulfonylmethyl isocyanide (451.57 mg, 2.31 mmol) in THF (10 mL) was added to sodium hydride (154.19 mg, 3.85 mmol). The mixture was stirred for 10 minutes, and intermediate **25e** (700.00 mg, 1.93 mmol) was then added thereto. The reaction mixture was stirred at 25°C for 2 hours. The reaction mixture was added with water (5 mL), and extracted with ethyl acetate (30 mL). The organic phase was dried over anhydrous sodium sulfate and filtered. The filtrate was concentrated to obtain a crude product. The crude product was purified by flash silica gel column chromatography (first using PE:EA = 3:1 to 1:1, then DCM:MeOH = 10:1) to obtain intermediate **25f.** MS m/z: 402.0, 404.0 $[M+1]^+$,$^1H$ NMR (400 MHz, $CDCl_3$) δ ppm 8.44 (s, 1H), 8.13 (s, 1H), 7.40-7.51 (m, 2H),7.22 (d, J=4.2 Hz, 2H), 6.85 (d, J=4.2 Hz, 2H), 5.61 (s, 2H), 3.79 (s, 3H).

Step 7: Synthesis of intermediate **25g**

1-(Tributylstannyl)methanol (191.59 mg, 596.69 μmol) and intermediate **25f** (200 mg, 497.24 μmol) were dissolved in 1,4-dioxane (5 mL). Chloro(2-dicyclohexylphosphino-2',4',6'-triisoporpyl-1,1'-biphenyl)[2-(2'-amino-1,1'-biphenyl)] palladium (II) (39.12 mg, 49.72 μmol) was added thereto, and the mixture was stirred at 80°C for 1.5 hours. 1-(Tributylstannyl)methanol (31.93 mg, 99.44 μmol) was then added thereto. The reaction mixture was stirred at 80°C for 2 hours, and heated to 100°C and stirred for another 2 hours. The reaction mixture was directly concentrated under reduced pressure to obtain a crude product. The crude product was purified by flash silica gel column chromatography (eluent ratio: PE:EA = 1:1; DCM:MeOH = 10:1) to obtain intermediate **25g.** MS m/z: 354.1 $[M+1]^+$.

Step **8:** Synthesis of intermediate **25h**

At 15 to 25°C, intermediate **25g** (80 mg, 226.41 μmol) was added to a reaction flask. Trifluoroacetic acid (3.07 g, 26.93 mmol, 2.00 mL) and trifluoromethanesulfonic acid (4.52 mmol, 400.00 μL) were added thereto. The reaction mixture was stirred for 2 hours. The reaction mixture was added with water (5 mL) and methanol (5 mL). The pH was adjusted to 10 with the addition of solid potassium carbonate. The mixture was stirred for another 1 hour. The reaction mixture was extracted with dichloromethane (20 mL). The organic phases were combined, washed with water (15 mL × 2), then dried and filtered. The filtrate was concentrated under reduced pressure to obtain intermediate **25h.** MS m/z: 234.1$[M+1]^+$.

Step **9:** Synthesis of intermediate **25i**

Intermediate **25h** (80 mg, 343.06 μmol) and 1,2-dibromotetrachloroethane (245.77 mg, 754.72 μmol) were added to DCM (1.5 mL). Tributylphosphine (138.81 mg, 686.11 μmol) was then added, and the mixture was stirred at 20°C for 3 hours. The reaction mixture was concentrated directly under reduced pressure to obtain the crude intermediate **25i.**

Step 10: Synthesis of trifluoroacetate of compound **25**

Intermediate **25i** (30 mg) and compound **9g** (28.97 mg, hydrochloride) were dissolved in DMF (1 mL). $Et_3N$ (10.25 mg, 101.32 μmol) and KI (16.82 mg, 101.32 μmol) were added thereto, and the mixture was stirred at 50°C for 2 hours. The reaction system was added with water (5 mL), and extracted with ethyl acetate (10 mL × 3). The organic phases were combined, dried over anhydrous sodium sulfate, and concentrated to obtain a crude product. The crude product was first purified by preparative TLC (eluent ratio: DCM:MeOH = 10:1), then separated by preparative HPLC (column: Welch Xtimate C18, 100 × 40 mm × 3 μm; mobile phase: [water (trifluoroacetic acid)-acetonitrile]; gradient: 0%-30% acetonitrile) to obtain the trifluoroacetate of compound **25.** MS m/z: 454.2$[M+1]^+$, $^1H$ NMR (400 MHz, $CD_3OD$) δ ppm 8.99 (s, 1H), 8.05 (d, *J*=4.4 Hz, 1H), 8.02 (s, 1H), 7.93 (d, *J*=4.4 Hz, 1H), 7.58-7.63 (m, 1H), 7.46-7.49 (m, 1H), 4.46 (s, 2H), 3.71-3.75 (m, 4H), 3.40-3.51 (m, 4H), 2.92 (s, 3H).

## Example 26

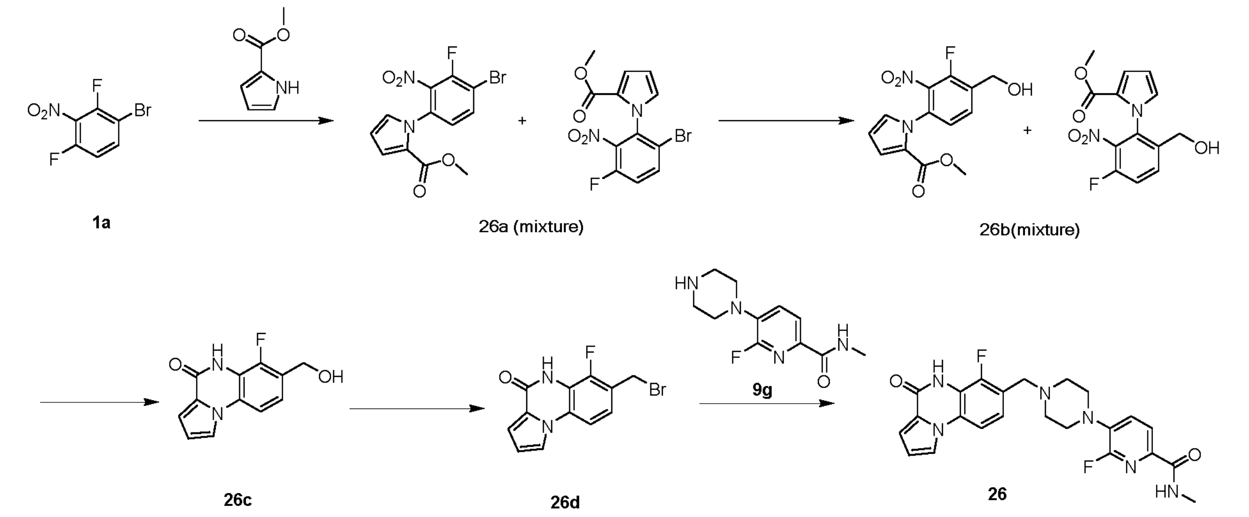


1a, 26a (mixture), 26b(mixture), 26c, 26d, 26

Step 1: Synthesis of intermediate **26a**

Methyl 2-pyrrolecarboxylate (262.88 mg, 2.10 mmol) and compound **1a** (500 mg, 2.10 mmol) were dissolved in DMF (10 mL). Sodium hydride (126.05 mg, 3.15 mmol, purity of 60%) was added in batches, and the mixture was stirred at room temperature (20°C) for 3 hours. The reaction mixture was slowly added dropwise into 20 mL of saturated ammonium chloride solution. The mixture was extracted with ethyl acetate (30 mL × 2). The organic phases were combined, washed with 20 mL of saturated brine, dried over anhydrous sodium sulfate, and filtered. The filtrate was concentrated under reduced pressure to obtain a crude product. The crude product was purified by flash silica gel column chromatography (eluent: PE:EA = 90:10, V/V) to obtain the mixture intermediate **26a** (in an approximate ratio of 2:1). MS m/z: 342.8, 344.8 $[M+1]^+$.

Step **2:** Synthesis of intermediate **26b**

The mixture intermediate compound **26a** (370 mg) was dissolved in dioxane (10 mL). 1-(Tributylstannyl) methanol (415.51 mg, 1.29 mmol) and chloro(2-dicyclohexylphosphino-2',4',6'-triisoporpyl-1,1'-biphenyl)[2-(2'-amino-1,1'-biphenyl)] palladium (II) (42.42 mg, 53.92 μmol) were added thereto, and the mixture was stirred at 110°C for 4 hours. The reaction mixture was concentrated under reduced pressure to obtain a crude product. The crude product was purified by flash silica gel column chromatography (gradient elution: PE:EA = 100:0 to 60:40) to obtain the mixture intermediate **26b.** MS m/z: 294.9 $[M+1]^+$.

Step **3:** Synthesis of intermediate **26c**

The mixture intermediate **26b** (185 mg) was dissolved in MeOH (3 mL) and purged with argon. Palladium hydroxide (8.83 mg, 62.87 μmol) was added. The reaction flask was purged with hydrogen, and the mixture was stirred at 30°C under 50 psi pressure for 16 hours. The reaction mixture was filtered, and the filtrate was concentrated under reduced pressure. DMF (2 mL) and 5 drops of triethylamine were added, and the mixture was stirred at 90°C for 16 hours. DMF was removed using an oil pump to obtain a crude product, and a mixed solution of DCM and MeOH (DCM:MeOH = 1:1, 3 mL) was added to the crude product. The mixture was filtered to obtain the filter cake, which was intermediate **26c.** MS m/z: 232.8 $[M+1]^+$. $^1$H NMR (400 MHz, DMSO-$d_6$) δ ppm 11.22 (br s, 1H), 8.18-8.19 (m, 1H), 7.87 (d, *J*=4.40 Hz 1H), 7.26 (t, *J*=8.20 Hz, 1H), 7.07 - 7.08 (m, 1H), 6.70-6.72 (m, 1H), 5.35 (t, J=5.60 Hz, 1H), 4.60 (d, *J*=2.80 Hz, 2H).

Step **4:** Synthesis of intermediate **26d**

Intermediate **26c** (20 mg, 86.13 μmol) and 1,2-dibromotetrachloroethane (28.05 mg, 86.13 μmol) were dissolved in DCM (0.5 mL). At 0°C, tributylphosphine (17.43 mg, 86.13 μmol) was added. After the addition was complete, the mixture was stirred at room temperature (20°C) for 16 hours. An additional portion of 1,2-dibromotetrachloroethane

(28.05 mg, 86.13 μmol) and tributylphosphine (17.43 mg, 86.13 μmol) was added thereto. The reaction mixture was stirred for another 1 hour. The reaction mixture was filtered to obtain the filter cake, which was intermediate **26d.** $^1$H NMR (400 MHz, DMSO-$d_6$) δ ppm 11.41 (br s, 1H), 8.20-8.21 (m, 1H), 7.85 (d, *J*=4.40 Hz 1H), 7.36 (t, J=8.20 Hz, 1H), 7.10 - 7.11 (m, 1H), 6.73-6.75 (m, 1H), 4.79 (s, 2H).

Step 5: Synthesis of compound **26**

Intermediate **9g** (11.83 mg, hydrochloride) was dissolved in DMF (1 mL). Triethylamine (17.43 mg, 172.26 μmol) was added, and the reaction mixture was stirred at 25°C for 0.5 hours. Intermediate **26d** (10 mg, 43.06 μmol) and potassium iodide (3.57 mg, 21.53 μmol) were then added. The mixture was stirred at 50°C for 2 hours. The reaction mixture was added with water (2 mL), and extracted with ethyl acetate (3 mL × 3). The organic phases were combined, dried over anhydrous sodium sulfate, and filtered. The filtrate was concentrated under reduced pressure to obtain a crude product. The crude product was purified by preparative TLC (DCM:MeOH = 30:1) to obtain compound 26. MS m/z: 453.0 [M+1]$^+$.

**Example 27**

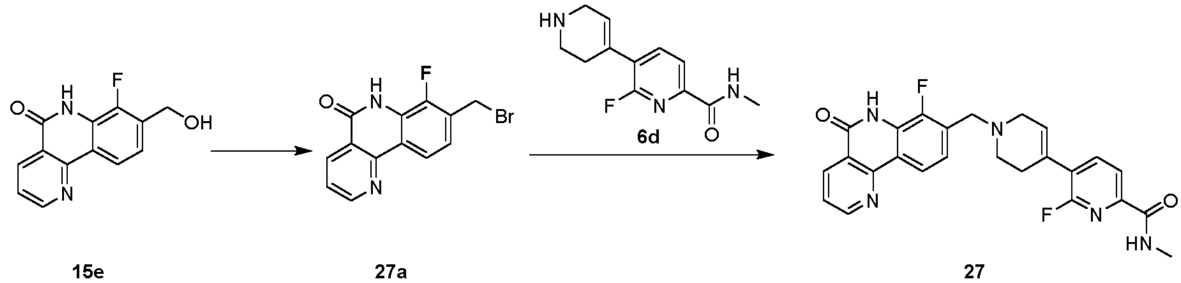

15e 27a 6d 27

Step 1: Synthesis of intermediate **27a**

Intermediate **15e** (61.9 mg, 253.46 μmol) and 1,2-dibromotetrachloroethane (82.54 mg, 253.46 μmol) were dissolved in DCM (2 mL). At 0°C, tributylphosphine (51.28 mg, 253.46 μmol) was added. After the addition was complete, the mixture was stirred at room temperature (20°C) for 19 hours. The reaction mixture was filtered to obtain the filter cake, which was intermediate **27a.** $^1$H NMR (400 MHz, DMSO-$d_6$) δ ppm 12.02 (s, 1 H) 9.05 - 9.13 (m, 1 H) 8.61 - 8.68 (m, 1 H) 8.41 - 8.49 (m, 1 H) 7.77 (br d, J=2.01 Hz, 1 H) 7.35 - 7.50 (m, 1 H) 4.94 (s, 2 H).

Step 2: Synthesis of compound **27**

Intermediate **6d** (21.23 mg, 78.15 μmol, HCl) was dissolved in DMF (2 mL). Triethylamine (19.77 mg, 195.36 μmol) was added, and the mixture was stirred at 25°C for 0.5 hours. Intermediate **27a** (30 mg, 97.68 μmol) and potassium iodide (1.62 mg, 9.77 μmol) were then added, and the mixture was stirred at 50°C for 2 hours. The reaction mixture was added with 2 mL of water, and extracted three times with 5 mL of a mixed solution (DCM:MeOH = 10:1). The organic phases were combined, dried over anhydrous sodium sulfate, and concentrated under reduced pressure to obtain a crude product. The crude product was stirred with EA (0.5 mL) for 10 minutes and filtered to obtain the filter cake, which was compound **27.** MS m/z: 462.1 [M+1]$^+$. $^1$H NMR (400 MHz, DMSO-$d_6$) δ ppm 11.90 (s, 1H), 9.09 (dd, J=4.82, 1.81 Hz, 1H), 8.65 (br dd, J=8.00, 1.88 Hz, 2H), 8.43 (d, J=8.50 Hz, 1H), 8.05 - 8.12 (m, 1H), 7.88 - 7.96 (m, 1H), 7.72 (dd, *J*=7.88, 4.88 Hz, 1H), 7.39 (br t, *J*=7.44 Hz, 1H), 6.26 (br s, 1H), 3.80 (s, 2H), 3.21 - 3.22 (m, 2H), 2.79 (d, *J*=4.75 Hz, 3H), 2.72 - 2.76 (m, 4H).

**Example 28**

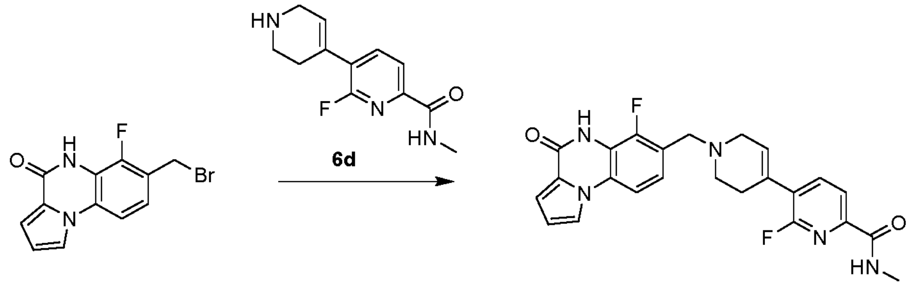


26d 28

Step 1: Synthesis of compound **28**

Compound **26d** (25 mg, 84.72 μmol) was dissolved in DMF (1 mL). Triethylamine (34.29 mg, 338.86 μmol) was added, and the mixture was stirred at 25°C for 0.5 hours. Compound **6d** (23 mg, hydrochloride) and potassium iodide (7.03 mg, 42.36 μmol) were then added, and the mixture was stirred at 50°C for 2 hours. The reaction mixture was added with water (2 mL), and extracted with ethyl acetate (3 mL × 3). The organic phases were combined, dried over anhydrous sodium sulfate, and filtered. The filtrate was concentrated under reduced pressure to obtain a crude product. The crude product was stirred with 0.5 mL of ethyl acetate for 10 minutes and filtered to obtain the filter cake, which was compound **28.** MS m/z: 450.0 $[M+1]^+$; $^1H$ NMR (400 MHz, DMSO-$d_6$) δ ppm 11.32 (s, 1 H), 8.64 (br d, J=4.77 Hz, 1 H), 8.20 (s, 1 H), 8.01 - 8.14 (m, 1 H), 7.83 - 7.97 (m, 2 H), 7.26 (t, J=7.78 Hz, 1 H), 7.09 (d, *J*=3.01 Hz, 1 H), 6.72 (t, J=3.26 Hz, 1 H), 6.25 (br s, 1 H), 3.72 (s, 2 H), 3.32 (s, 2 H), 3.13 - 3.22 (m, 2 H), 2.79 (d, J=4.77 Hz, 3 H), 2.66 - 2.75 (m, 2 H).

**Example 29**

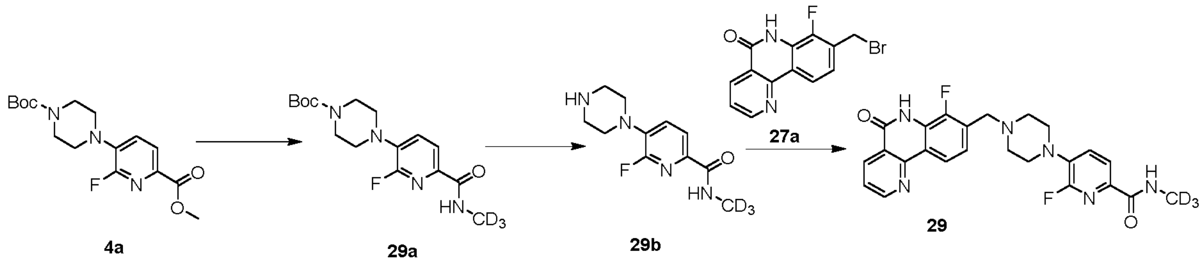


4a 29a 29b 29

Step 1: Synthesis of intermediate **29a**

Deuterated methylamine hydrochloride (124.71 mg, 1.77 mmol) was added to $H_2O$ (1 mL). Sodium tert-butoxide (84.95 mg, 884.01 μmol) was then added to the reaction mixture, and the mixture was stirred at 25°C for 1 hour. A solution of compound **4a** (30 mg, 88.40 μmol) in MeOH (2 mL) was added thereto. The reaction mixture was then heated to 60°C and reacted for 4 hours. The reaction mixture was directly concentrated under reduced pressure to obtain a crude product, which was intermediate **29a.** MS m/z: 342.1 $[M+1]^+$.

Step 2: Synthesis of hydrochloride of intermediate **29b**

Intermediate **29a** (20 mg) was dissolved in MeOH (0.6 mL). HCl/dioxane (4 M, 0.2 mL) was added thereto, and the mixture was stirred at 20°C for 2 hours. The reaction mixture was directly concentrated under reduced pressure to obtain the hydrochloride of intermediate **29b.** MS m/z: 242.2 $[M+1]^+$.

Step 3: Synthesis of compound **29**

Intermediate **29b** (19.22 mg, hydrochloride) was dissolved in DMF (1 mL). Triethylamine (162.80 μmol, 22.66 μL) was added, and the mixture was stirred at 25°C for 30 minutes. Compound **27a** (25 mg, 81.40 μmol) and potassium iodide (1.35 mg, 8.14 μmol) were then added, and the mixture was heated to 50°C and stirred for 3 hours. The reaction mixture was added with 2 mL of water, and filtered to obtain the filter cake, which was compound **29.** MS m/z: 468.2 $[M+1]^+$. $^1H$ NMR (400 MHz, DMSO-$d_6$) δ ppm 11.90 (s, 1H), 9.09 (dd, J=4.63, 1.75 Hz, 1H), 8.65 (dd, J=8.00, 1.75 Hz, 1H), 8.43 (d,

J=8.13 Hz, 1H), 8.38 (s, 1H), 7.85 (d, J=7.88 Hz, 1H), 7.72 (dd, J=8.00, 4.50 Hz, 1H), 7.57 (dd, *J*=10.57, 8.19 Hz, 1H), 7.37 (t, J=7.32 Hz, 1H), 3.75 (s, 2H), 3.15 - 3.22 (m, 4H), 2.62 - 2.65 (m, 4H).

**Example 30**

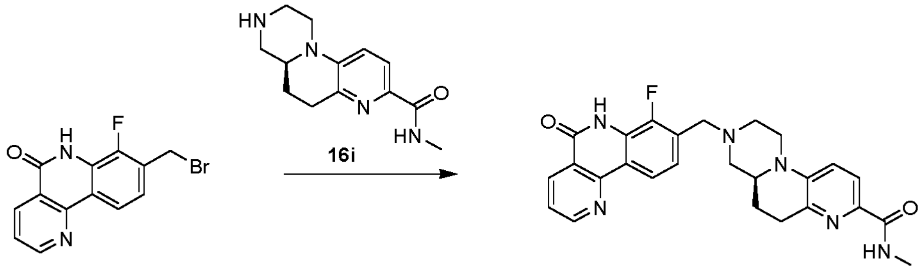


27a

30

Step 1: Synthesis of compound **30**

Intermediate **16i** (29.46 mg, hydrochloride) was dissolved in DMF (2 mL). Triethylamine (52.72 mg, 520.97 μmol) was added, and the reaction mixture was stirred at 25°C for 0.5 hours. Intermediate **27a** (40 mg, 130.24 μmol) and KI (21.62 mg, 130.24 μmol) were then added. The mixture was stirred at 50°C for 2 hours. The reaction mixture was added with 2 mL of water, and extracted with ethyl acetate (3 mL × 3). The organic phase was dried over anhydrous sodium sulfate, filtered, and concentrated under reduced pressure to obtain a crude product. The crude product was stirred with 0.5 mL of ethyl acetate for 10 minutes and filtered to obtain the filter cake. The filter cake was further purified by preparative TLC (DCM:MeOH = 20:1) to obtain compound **30.** MS m/z: 473.0[M+1]$^+$. $^1$H NMR (400 MHz, $CDCl_3$) δ ppm 8.95 - 9.02 (m, 1 H), 8.73 - 8.79 (m, 1 H), 8.68 (dd, J=8.16, 1.88 Hz, 1 H), 8.46 (br d, J=8.03 Hz, 1 H), 7.77 - 7.88 (m, 1 H), 7.65 - 7.75 (m, 1 H), 7.48 - 7.54 (m, 1 H), 7.34 - 7.43 (m, 1 H), 6.98 (d, J=8.53 Hz, 1 H), 3.63 - 3.86 (m, 3 H), 2.97 - 3.33 (m, 2 H), 2.92 (d, J=5.27 Hz, 3 H), 2.82 - 2.89 (m, 2 H), 2.03 - 2.43 (m, 1 H), 1.90 - 1.94 (m, 1 H), 1.68 - 1.83 (m, 1 H), 1.33 - 1.39 (m, 1 H), 1.23 - 1.31 (m, 2 H).

**Example 31**

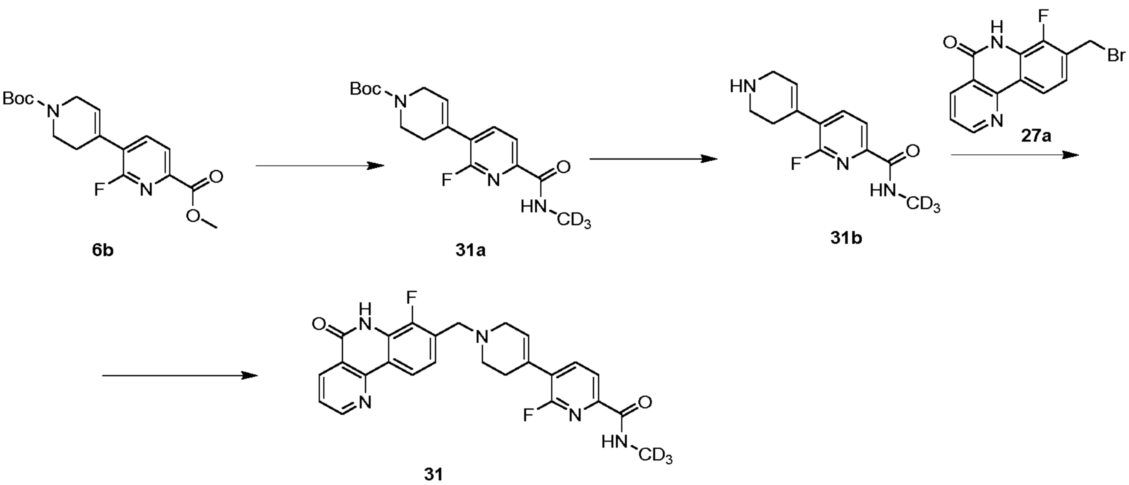


6b

31a

31b

31

Step 1: Synthesis of intermediate 31a

Deuterated methylamine hydrochloride (167.77 mg, 2.38 mmol) was added to $H_2O$ (1 mL). Potassium tert-butoxide (133.44 mg, 1.19 mmol) was then added to the reaction mixture, and the mixture was stirred at 25°C for 1 hour. A solution of compound **6b** (40 mg, 118.92 μmol) in MeOH (2 mL) was added thereto. The reaction mixture was then heated

to 60°C and reacted for 16 hours. The reaction mixture was directly concentrated under reduced pressure to obtain a crude product, which was intermediate **31a.** MS m/z: 339.1 [M+1]$^+$.

Step 2: Synthesis of hydrochloride of intermediate **31b**

Intermediate **31a** was dissolved in MeOH (1 mL). HCl/dioxane (4 M, 99.74 μL) was added thereto, and the mixture was stirred at 25°C for 2 hours. The reaction mixture was directly concentrated under reduced pressure to obtain the hydrochloride of intermediate **31b.** MS m/z: 238.9 [M+1]$^+$.

Step 3: Synthesis of compound **31**

Intermediate **31b** (15 mg, hydrochloride) was dissolved in DMF (1 mL). Triethylamine (27.62 mg, 272.99 μmol, 38.00 μL) was added, and the reaction mixture was stirred at 25°C for 0.5 hours. Intermediate **27a** (20.96 mg, 68.25 μmol) and potassium iodide (5.66 mg, 34.12 μmol) were then added. The mixture was stirred at 50°C for 2 hours. After cooling, the reaction mixture was added with 2 mL of water, resulting in the precipitation of a solid. The mixture was filtered to obtain the filter cake, which was compound **31.** MS m/z: 465.1 [M+1]$^+$; $^1$H NMR (400 MHz, DMSO-$d_6$) δ ppm 11.89 (br d, *J*=1.76 Hz, 1 H), 9.02 - 9.14 (m, 1 H), 8.56 - 8.70 (m, 2 H), 8.43 (d, *J*=8.03 Hz, 1 H), 8.04 - 8.14 (m, 1 H), 7.89 - 7.96 (m, 1 H), 7.72 (dd, *J*=8.03, 4.52 Hz, 1 H), 7.39 (t, *J*=7.53 Hz, 1 H), 6.26 (br s, 1 H), 3.80 (s, 2 H), 3.31 (s, 2 H), 3.18 - 3.24 (m, 2 H), 2.74 (br t, *J*=5.27 Hz, 2 H).

**Example 32**

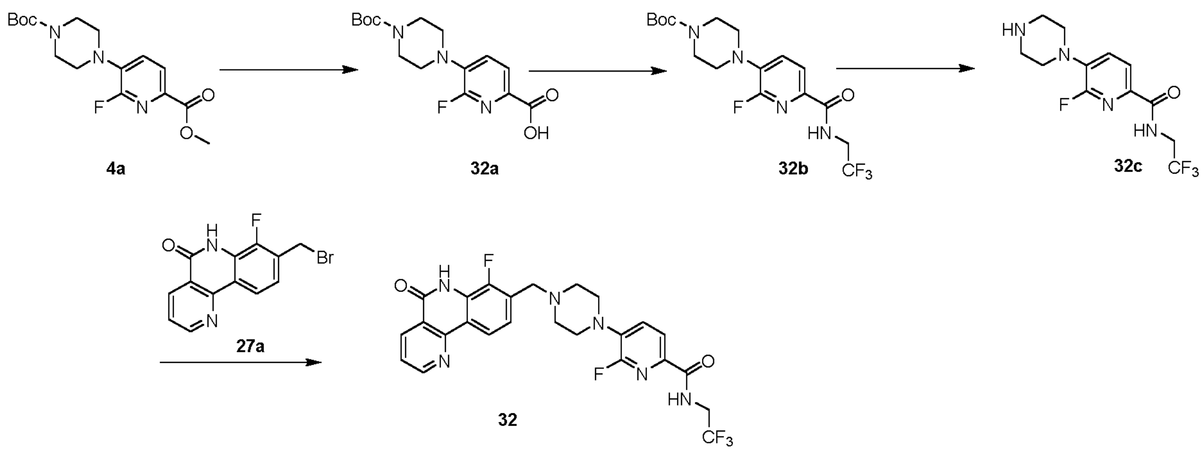
4a
32a
32b
32c
27a
32

Step 1: Synthesis of intermediate **32a**

Intermediate **4a** (0.2 g, 589.34 μmol) was added to a mixture of THF (5 mL) and $H_2O$ (1 mL). With stirring, lithium hydroxide monohydrate (123.65 mg, 2.95 mmol) was added thereto, and the reaction mixture was stirred at 20°C for 4 hours. The pH of the reaction mixture was adjusted to 3-4 using dilute hydrochloric acid (1 mol/L). The mixture was extracted with ethyl acetate (5 mL × 2). The organic phases were combined, dried over anhydrous sodium sulfate, concentrated under reduced pressure at 45°C to obtain intermediate **32a.** MS m/z: 347.9 [M+23]$^+$.

Step **2:** Synthesis of intermediate **32b**

Under a nitrogen atmosphere, intermediate **32a** (0.1 g, 307.38 μmol), 2,2,2-trifluoroethylamine (45.67 mg, 461.06 μmol), 1H-benzotriazol-1-yltetramethyluronium tetrafluoroborate (197.39 mg, 614.75 μmol), and diisopropylethylamine (119.18 mg, 922.13 μmol) were added to DMF (2 mL). The reaction mixture was stirred at 40°C for 4 hours. The reaction mixture was concentrated under reduced pressure to obtain a crude product. The crude product was purified by preparative TLC (PE/EA= 2/1) to obtain intermediate **32b.** MS m/z: 351.1 [M-56+1]$^+$.

Step 3: Synthesis of hydrochloride of intermediate **32c**

Intermediate **32b** (57.66 mg, 141.88 μmol) was added to MeOH (2 mL). With stirring, HCl/dioxane (4 M, 191.54 μL) was added thereto, and the reaction mixture was stirred at 25°C for 2 hours. The reaction mixture was concentrated under reduced pressure at 45°C to obtain a crude product, which was the hydrochloride of intermediate **32c.** MS m/z: 306.9 $[M+1]^+$

Step 4: Synthesis of compound **32**

Triethylamine (156.29 μmol, 21.75 μL) was added to a solution of intermediate **32c** (26.78 mg, hydrochloride) in DMF (1 mL). The mixture was stirred at 20°C for 0.5 hours. Intermediate **27a** (30 mg, 78.15 μmol) and potassium iodide (1.30 mg, 7.81 μmol) were added thereto. The reaction mixture was stirred at 50°C for 2 hours, and filtered. The filtrate was concentrated under reduced pressure at 60°C to obtain a crude product. The crude product was purified by preparative TLC (EA: 100%) to obtain compound **32.** MS m/z: 555.1 $[M+23]^+$. $^1H$ NMR (400 MHz, $CDCl_3$) δ ppm 8.92 - 9.04 (m, 1 H), 8.62 - 8.76 (m, 2 H), 8.44 (br d, *J*=8.25 Hz, 1 H), 7.93 (d, *J*=8.63 Hz, 1 H), 7.71 (s, 1 H), 7.45 - 7.55 (m, 1 H), 7.24 - 7.35 (m, 1 H), 3.92 - 4.07 (m, 2 H), 3.68 - 3.78 (m, 2 H), 3.21 (m, 4 H), 2.66 (m, 4 H).

**Example 33**

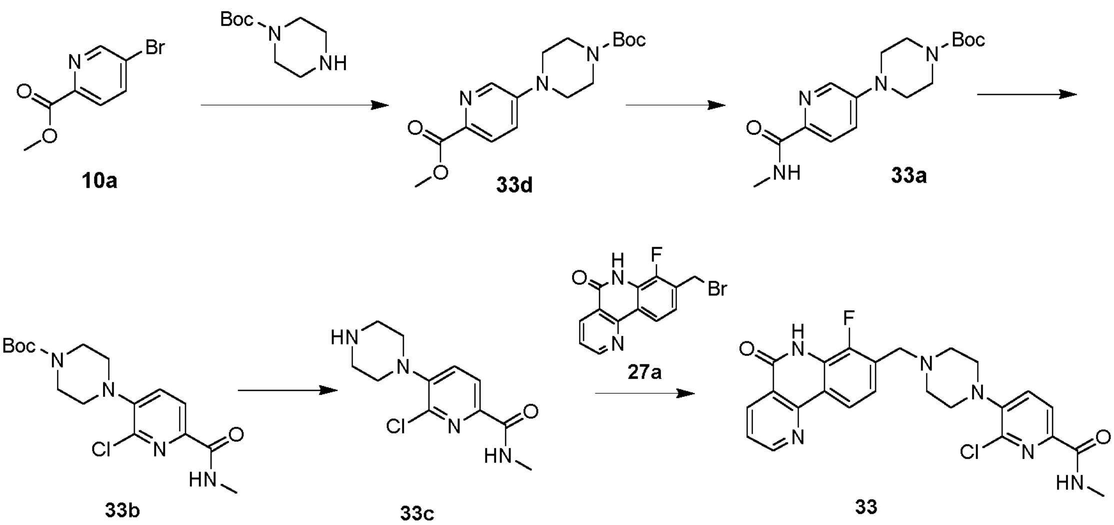

10a 33d 33a 33b 33c 27a 33

Step **1:** Synthesis of intermediate **33d**

**10a** (3 g, 13.89 mmol) was dissolved in toluene (30 mL). 1-(tert-Butoxycarbonyl)piperazine (2.59 g, 13.89 mmol), RuPhos (648.01 mg, 1.39 mmol), $Pd_2(dba)_3$ (381.49 mg, 416.61 μmol), and $Cs_2CO_3$ (13.57 g, 41.66 mmol) were added. The reaction mixture was stirred under a nitrogen atmosphere at 100°C for 16 hours. The reaction mixture was filtered, and the filtrate was concentrated under reduced pressure to obtain a crude product. The crude product was purified by column chromatography (eluent: PE:EA = 100:0 to 90:10 to 75:25, V/V) to obtain intermediate **33d.** MS m/z: 321.9 $[M+1]^+$.

Step 2: Synthesis of intermediate **33a**

At 25°C, intermediate **33d** (1 g, 3.11 mmol) was added to a solution of methylamine in ethanol (322.13 mg, 3.11 mmol). The reaction mixture was stirred at 25°C for 1 hour. The reaction mixture was concentrated under reduced pressure to obtain a crude product. The crude product was dissolved in 10 mL of dichloromethane and washed with 10 mL of water. The mixture was extracted, and the phases were separated to collect the organic phase. The organic phase was dried over anhydrous sodium sulfate, and filtered. The filtrate was concentrated under reduced pressure to obtain intermediate **33a.** MS m/z: 320.9 $[M+1]^+$. $^1H$ NMR (400 MHz, $CDCl_3$) δ ppm 8.16 (d, *J*=2.25 Hz, 1H), 8.08 (d, J=8.76 Hz, 1H), 7.85 (br s, 1H), 7.24 (dd, *J*=2.50, 8.75 Hz, 1H), 3.56-3.69 (m, 4H), 3.30 (br d, J=4.63 Hz, 4H), 3.01 (d, *J*=5.00 Hz, 3H), 1.49 (s, 9H).

Step **3:** Synthesis of intermediate **33b**

Compound **33a** (0.3 g, 936.37 μmol) was dissolved in DMF (1.5 mL). *N*-Chlorosuccinimide (137.54 mg, 1.03 mmol) was added at 20°C, and the mixture was stirred for 14 hours. The reaction mixture was added with water (5 mL) and extracted with ethyl acetate (20 mL × 2). The organic phases were combined, washed with water (20 mL), then dried over anhydrous sodium sulfate, and filtered. The filtrate was concentrated under reduced pressure to obtain compound **33b.** MS m/z: 355.1, 357.1 $[M+1]^+$. $^1$H NMR (400 MHz, $CDCl_3$) δ ppm 8.10 (d, *J*=4.2 Hz, 1 H), 7.69 (br, s, 1 H), 7.40 (d, *J*=4.2 Hz, 1 H), 3.63 - 3.66 (m, 4 H), 3.09 - 3.15 (m, 4 H), 3.03 (d, *J*=2.4 Hz, 3 H), 1.51 (s, 9 H).

Step 4: Synthesis of hydrochloride of intermediate **33c**

Intermediate **33b** (350 mg, 986.39 μmol) was dissolved in MeOH (6 mL). HCl/dioxane (4 M, 6 mL) was added, and the mixture was stirred at 20°C for 4 hours. The reaction mixture was concentrated under reduced pressure at 60°C to obtain the hydrochloride of intermediate **33c.** MS m/z: 255.1, 257.1 $[M+1]^+$.

Step 5: Synthesis of compound **33**

Intermediate **27a** (20 mg, 65.12 μmol), intermediate **33c** (18.96 mg, hydrochloride), potassium iodide (5.41 mg, 32.56 μmol), and triethylamine (26.36 mg, 260.49 μmol) were sequentially added to DMF (1 mL). The reaction mixture was heated to 50°C and stirred for 12 hours. The reaction mixture was added with water (3 mL), and extracted with ethyl acetate (10 mL × 2). The organic phases were combined, dried over anhydrous sodium sulfate, and filtered. The filtrate was concentrated under reduced pressure to obtain a crude product. The crude product was stirred with 1.2 mL of methanol for 10 minutes, and the mixture was filtered to obtain the filter cake, which was compound **33.** MS m/z: 481.1 $[M+1]^+$. $^1$H NMR (400 MHz, DMSO-$d_6$) δ ppm 11.90 (br s, 1 H), 9.08 - 9.09 (m, 1 H), 8.65 (d, *J*=2.40 Hz, 2 H), 8.43 (d, *J*=4.20 Hz, 1 H), 7.93 (d, *J*=3.92 Hz, 1 H), 7.66 -7.74 (m, 2 H), 7.33 - 7.39 (m, 1 H), 3.77 (s, 2H), 3.05-3.15 (m, 4 H), 2.79 (d, *J*=2.40 Hz, 3 H), 2.65-2.68 (m, 4 H).

**Example 34**

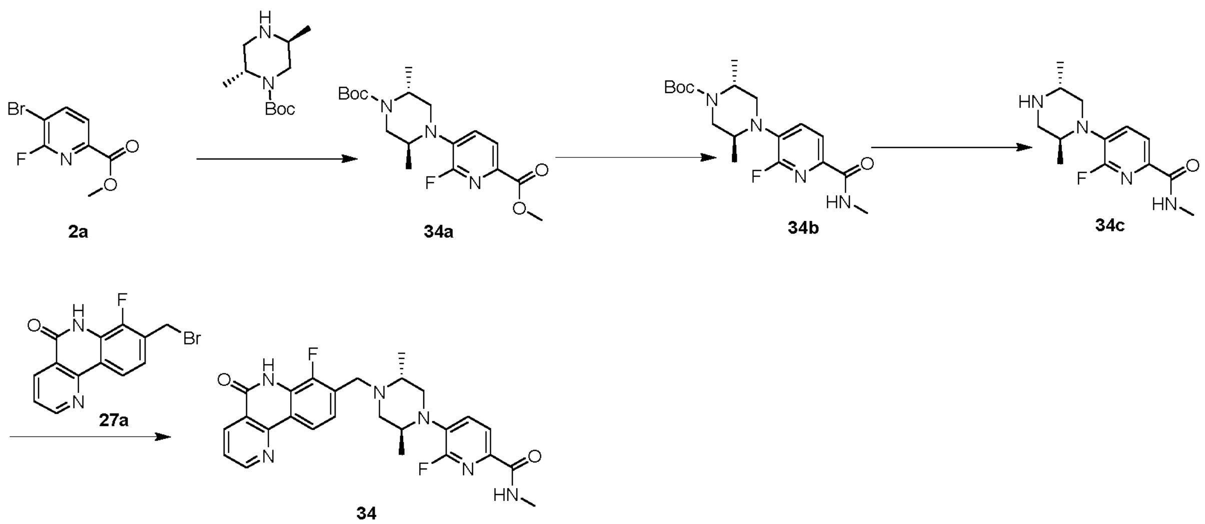

2a 34a 34b 34c 27a 34

Step 1: Synthesis of intermediate **34a**

Intermediate **2a** (200 mg, 854.62 μmol) and (2R,5S)-1-tert-butoxycarbonyl-2,5-dimethylpiperazine (183.15 mg, 854.62 μmol) were dissolved in toluene (5 mL). RuPhos (79.76 mg, 170.92 μmol), $Cs_2CO_3$ (556.90 mg, 1.71 mmol), and $Pd_2(dba)_3$ (78.26 mg, 85.46 μmol) were sequentially added. The reaction mixture was stirred under a nitrogen atmosphere at 100°C for 16 hours. The reaction mixture was filtered, and the filtrate was concentrated under reduced pressure to obtain a crude product. The crude product was purified by column chromatography (eluent: PE:EA = 100:0 to 90:10 to 75:25, v/v) to obtain intermediate **34a.** MS m/z: 368.0 $[M+1]^+$; $^1$H NMR (400 MHz, $CDCl_3$) δ ppm 7.97 (dd, J=8.03, 1.25 Hz, 1 H), 7.20 (dd, J=10.04, 8.03 Hz, 1 H), 4.38-4.45 (m, 1 H), 4.05 (br d, J=2.26 Hz, 1 H), 3.97 (s, 3 H), 3.74 - 3.83 (m, 1 H), 3.47-3.52 (m, 2

H), 3.10 (d, J=13.05 Hz, 1 H), 1.51 (s, 9 H), 1.29 - 1.32 (m, 3 H), 1.12 (d, J=5.27 Hz, 3 H).

Step 2: Synthesis of intermediate **34b**

Intermediate **34a** (187.4 mg, 510.05 μmol) was dissolved in EtOH (2 mL). A solution of methylamine in ethanol (792.03 mg, 7.63 mmol, 30%) was added, and the mixture was stirred at 25°C for 16 hours. The reaction mixture was directly concentrated under reduced pressure to obtain intermediate **34b.** MS m/z: 367.0 $[M+1]^+$, $^1$H NMR (400 MHz, $CDCl_3$) δ ppm 7.99 (dd, J=8.03, 1.26 Hz, 1 H), 7.47 - 7.54 (m, 1 H), 7.24 (dd, *J=10.16,* 8.16 Hz, 1 H), 4.39 - 4.52 (m, 1 H), 3.93 - 4.02 (m, 1 H), 3.73 - 3.78 (m, 1 H), 3.50 (dd, J=12.17, 3.89 Hz, 2 H), 2.95-3.02 (m, 4H), 1.51 (s, 9 H), 1.31 (d, J=6.78 Hz, 3 H), 1.09 (d, J=6.53 Hz, 3 H).

Step 3: Synthesis of hydrochloride of intermediate **34c**

Intermediate **34b** (170 mg, 463.94 μmol) was dissolved in MeOH (2 mL). HCl/dioxane (4 M, 0.62 mL) was added, and the mixture was stirred at 25°C for 3 hours. The reaction mixture was concentrated under reduced pressure to obtain the hydrochloride of intermediate **34c.** MS m/z: 266.9 $[M+1]^+$.

Step 4: Synthesis of trifluoroacetate of compound **34**

Intermediate **34c** (26.51 mg, hydrochloride) was dissolved in DMF (1 mL). Triethylamine (39.54 mg, 390.73 μmol) was added, and the mixture was stirred at 25°C for 0.5 hours. Intermediate **27a** (30 mg, 97.68 μmol) and potassium iodide (8.11 mg, 48.84 μmol) were then added, and the mixture was stirred at 50°C for 2 hours. The reaction mixture was filtered, and the filtrate was separated by preparative HPLC (column: Xtimate C18, 100 × 30 mm × 3 μm; mobile phase: [water (trifluoroacetic acid)-acetonitrile]; gradient: 14%-44% acetonitrile) to obtain the trifluoroacetate of compound **34.** MS m/z: 493.1 $[M+1]^+$. $^1$H NMR (400 MHz, $CD_3OD$) δ ppm 9.11 (dd, J=4.52, 1.76 Hz, 1 H), 8.78 (dd, J=8.03, 1.76 Hz, 1 H), 8.69 (d, J=8.53 Hz, 1 H), 8.01 (d, J=7.03 Hz, 1 H), 7.85 - 7.92 (m, 1 H), 7.76 (dd, J=8.03, 4.52 Hz, 1 H), 7.53-7.56 (m, 1 H), 4.98 (br d, J=16.56 Hz, 2 H), 4.47 (br d, J=14.31 Hz, 1 H), 3.74 - 3.90 (m, 1 H), 3.49 - 3.57 (m, 2 H), 3.07 - 3.20 (m, 2 H), 2.95 (s, 3 H), 1.65 (d, J=6.27 Hz, 3 H), 1.02 (d, J=6.02 Hz, 3 H).

## Example 35

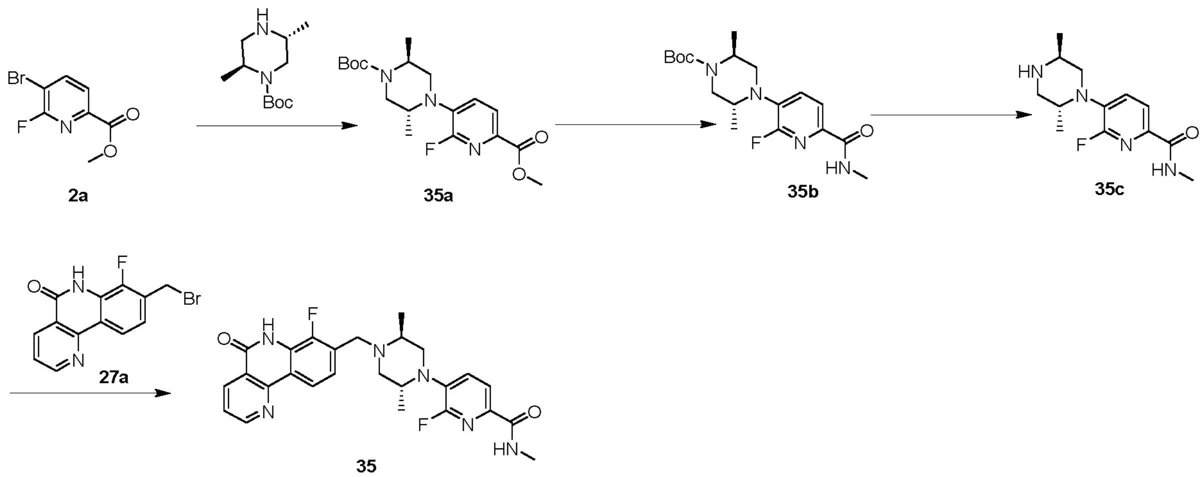

2a 35a 35b 35c 27a 35

Step 1: Synthesis of intermediate **35a**

(2S,5R)-1-tert-Butoxycarbonyl-2,5-dimethylpiperazine (180.00 mg, 839.93 μmol) and intermediate **2a** (196.56 mg, 839.93 μmol) were dissolved in toluene (10 mL). $Cs_2CO_3$ (821.00 mg, 2.52 mmol), RuPhos (39.19 mg, 83.99 μmol), and $Pd_2(dba)_3$ (38.46 mg, 42.00 μmol) were sequentially added. The reaction mixture was stirred under a nitrogen atmosphere at 100°C for 16 hours. The reaction mixture was filtered while hot, and the filtrate was concentrated under reduced pressure to obtain a crude product. The crude product was added with 2 mL of a mixture of petroleum ether and ethyl acetate (PE/EA = 5/1) and stirred at 20°C for 0.5 hours. The mixture was subjected to suction filtration, and the filter

cake was dried under reduced pressure to obtain intermediate **35a.** MS m/z: 368.1 [M+1]$^+$.

Step 2: Synthesis of intermediate **35b**

Intermediate **35a** (100 mg, 272.17 μmol) was dissolved in EtOH (2 mL). A solution of methylamine in ethanol (1.13 g, 10.89 mmol, 30%) was added, and the mixture was stirred at room temperature (25°C) for 16 hours. The reaction mixture was concentrated under reduced pressure to obtain intermediate **35b.** MS m/z: 367.1 [M+1]$^+$.

Step 3: Synthesis of hydrochloride of intermediate **35c**

Intermediate **35b** (100 mg, 272.90 μmol) was dissolved in MeOH (2 mL). HCl/dioxane (4 M, 0.38 mL) was added, and the mixture was stirred at 25°C for 2 hours. The reaction mixture was concentrated under reduced pressure to obtain the hydrochloride of intermediate **35c.** MS m/z: 267.0 [M+1]$^+$.

Step 4: Synthesis of trifluoroacetate of compound **35**

Triethylamine (15.8.2 mg, 156.29 μmol) was added to a solution of intermediate **35c** (23.66 mg, hydrochloride) in DMF (1 mL). The mixture was stirred at 20°C for 0.5 hours. Intermediate **27a** (30 mg, 78.15 μmol) and KI (1.30 mg, 7.815 μmol) were then added thereto, and the reaction mixture was stirred at 50°C for 0.5 hours. The reaction mixture was concentrated under reduced pressure to obtain a crude product. The crude product was purified by preparative HPLC (column: Xtimate C18, 100 × 30 mm × 3 μm; mobile phase: [water (trifluoroacetic acid)-acetonitrile]; gradient: 14%-44% acetonitrile) to obtain the trifluoroacetate of compound **35.** MS m/z: 493.2 [M+1]$^+$ $^1$H NMR (400 MHz, $CD_3OD$) δ ppm 9.11 (dd, *J*=4.52, 1.76 Hz, 1 H), 8.77 (dd, *J*=8.03, 1.76 Hz, 1 H), 8.69 (d, *J*=7.78 Hz, 1 H), 8.01 (d, *J*=7.78 Hz, 1 H), 7.86 - 7.93 (m, 1 H), 7.76 (dd, *J*=8.03, 4.77 Hz, 1 H), 7.55 (dd, *J*=8.28, 6.78 Hz, 1 H), 4.91-5.02(m, 2H), 4.50 (br d, *J*=13.55 Hz, 1 H), 3.75-3.83 (m, 1 H), 3.48 - 3.58 (m, 2 H), 3.10 - 3.23 (m, 2 H), 2.94 (s, 3 H), 1.66 (d, *J*=6.53 Hz, 3 H), 1.01 (d, *J*=6.02 Hz, 3 H).

**Example 36**

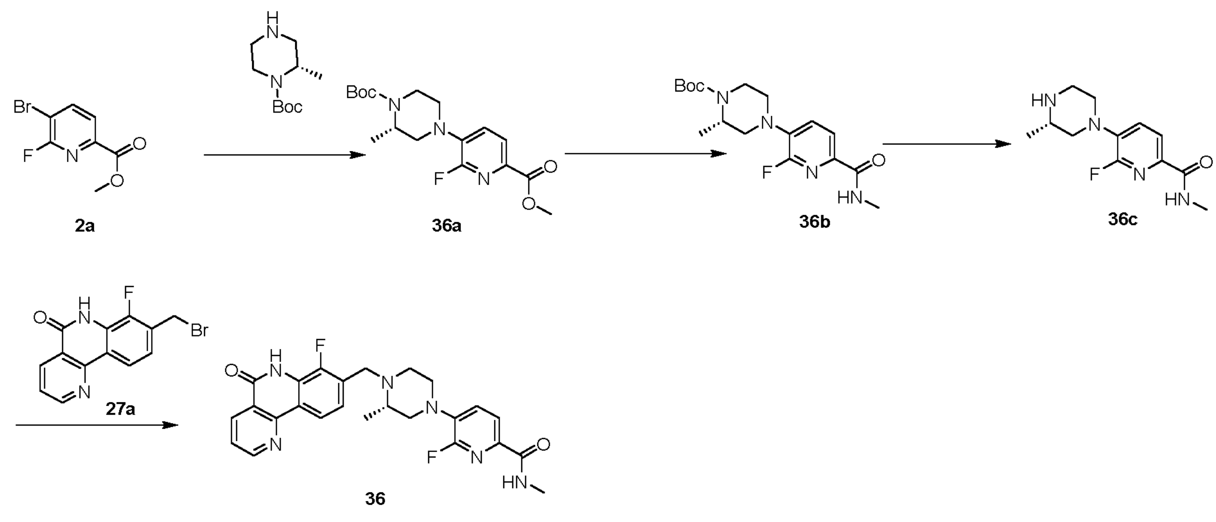

2a 36a 36b 36c 27a 36

Step 1: Synthesis of intermediate **36a**

(S)-1-*N*-tert-Butoxycarbonyl-2-methylpiperazine (128.37 mg, 640.96 μmol) and intermediate **2a** (150 mg, 640.96 μmol) were dissolved in toluene (10 mL). RuPhos (59.82 mg, 128.19 μmol), $Cs_2CO_3$ (417.68 mg, 1.28 mmol), and $Pd_2(dba)_3$ (58.69 mg, 64.10 μmol) were sequentially added. The reaction mixture was stirred under a nitrogen atmosphere at 100°C for 16 hours. The reaction mixture was filtered, and the filtrate was concentrated under reduced pressure to obtain a crude product. The crude product was purified by column chromatography (eluent: PE:EA = 90:10 to 75:25, v/v) to obtain intermediate **36a.** MS m/z: 354.1 [M+1]$^+$. $^1$H NMR (400 MHz, $CDCl_3$) δ ppm 7.99 (dd, *J*=8.03, 1.00 Hz, 1 H), 7.26 (dd, *J*=10.04, 8.28 Hz, 1 H), 4.38 (br d, *J*=5.02 Hz, 1 H), 4.02 (br s, 1 H), 3.98 (s, 3 H), 3.44 - 3.57 (m, 2 H), 3.31 (td, *J*=12.80, 3.26 Hz, 1 H), 3.02 (dd, *J*=11.92, 3.64 Hz, 1 H), 2.84 - 2.94 (m, 1 H), 1.51 (s, 9 H), 1.35 (d, *J*=6.78 Hz, 3 H).

Step 2: Synthesis of intermediate **36b**

Intermediate **36a** (100 mg, 272.17 μmol) was dissolved in EtOH (2 mL). A solution of methylamine in ethanol (0.65 g, 6.29 mmol, 30%) was added, and the mixture was stirred at room temperature (25°C) for 16 hours. The reaction mixture was directly concentrated under reduced pressure to obtain intermediate **36b.** MS m/z: 353.1 $[M+1]^+$. $^1$H NMR (400 MHz, $CDCl_3$) δ ppm 7.94 - 8.06 (m, 1 H), 7.52 (br d, J=4.27 Hz, 1 H), 7.29 - 7.34 (m, 1 H), 4.39 (br s, 1 H), 4.01 (br d, *J*=13.80 Hz, 1 H), 3.38 - 3.49 (m, 2 H), 3.30 (td, *J*=12.74, 3.39 Hz, 1 H), 3.02 (d, *J*=5.02 Hz, 3 H), 2.97 (dd, *J*=11.80, 3.51 Hz, 1 H), 2.81 - 2.89 (m, 1 H), 1.51 (s, 9 H), 1.36 (d, *J*=6.78 Hz, 3 H).

Step 3: Synthesis of hydrochloride of intermediate **36c**

Intermediate **36b** (51 mg, 428.49 μmol) was dissolved in MeOH (2 mL). HCl/dioxane (4 M, 0.54 mL) was added, and the mixture was stirred at 25°C for 2 hours. The reaction mixture was concentrated under reduced pressure to obtain the hydrochloride of intermediate **36c.** MS m/z: 253.0 $[M+1]^+$.

Step 4: Synthesis of compound **36**

Triethylamine (32.95 mg, 325.61 μmol) was added to a solution of intermediate **36c** (21.18 mg, hydrochloride) in DMF (1 mL). The mixture was stirred at 20°C for 0.5 hours. Intermediate **27a** (25 mg, 81.40 μmol) and KI (6.76 mg, 40.70 μmol) were then added thereto, and the reaction mixture was stirred at 50°C for 2 hours. The reaction mixture was added with water (2 mL), and extracted with ethyl acetate (3 mL × 3). The organic phases were combined, dried over anhydrous sodium sulfate, and filtered. The filtrate was concentrated under reduced pressure to obtain a crude product. The crude product was stirred with 1 mL of methanol for 10 minutes, and the mixture was filtered to obtain the filter cake, which was compound **36.** MS m/z: 479.0 $[M+1]^+$. $^1$H NMR (400 MHz, DMSO-*d6*) δ ppm 11.87 (s, 1 H), 9.08 (dd, *J*=4.64, 1.88 Hz, 1 H), 8.64 (dd, *J*=8.03, 1.76 Hz, 1 H), 8.34 - 8.49 (m, 2 H), 7.80 - 7.89 (m, 1 H), 7.71 (dd, *J*=8.03, 4.77 Hz, 1 H), 7.56 (dd, *J*=10.54, 8.28 Hz, 1 H), 7.33 - 7.44 (m, 1 H), 4.12 (d, *J*=13.55 Hz, 1 H), 3.50 (d, *J*=13.80 Hz, 1 H), 3.36 - 3.43 (m, 1 H), 2.87 - 2.98 (m, 1 H), 2.74 - 2.86 (m, 5 H), 2.65 - 2.73 (m, 1 H) 2.28 - 2.47 (m, 2 H) 1.22 (d, J=6.02 Hz, 3 H).

**Example 37**

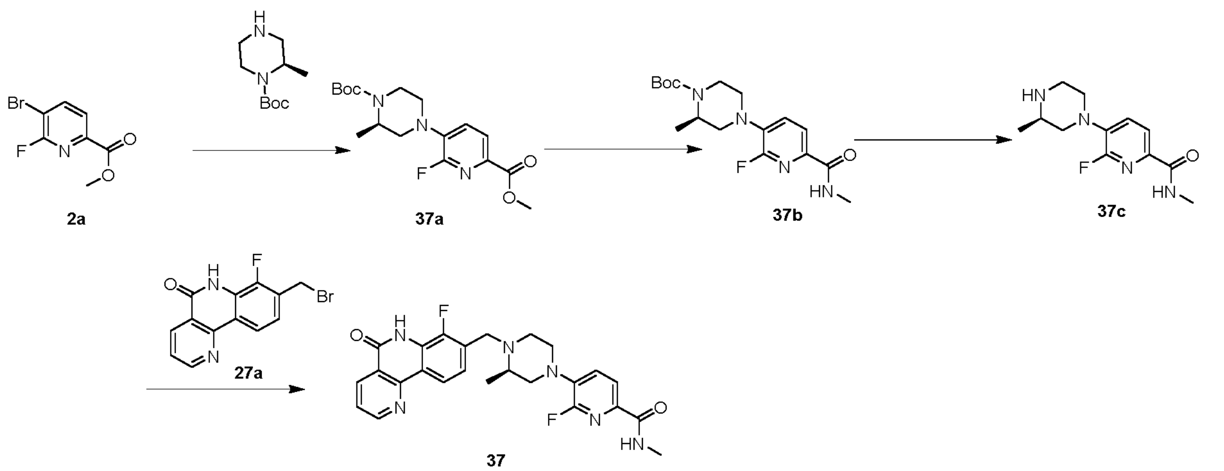

2a 37a 37b 37c 27a 37

Step 1: Synthesis of intermediate **37a**

(R)-1-*N*-tert-Butoxycarbonyl-2-methylpiperazine (0.13 g, 649.10 μmol) and intermediate **2a** (151.90 mg, 649.10 μmol) were dissolved in toluene (10 mL). $Cs_2CO_3$ (634.47 mg, 1.95 mmol), RuPhos (30.29 mg, 64.91 μmol), and $Pd_2(dba)_3$ (29.72 mg, 32.46 μmol) were sequentially added. The reaction mixture was stirred under a nitrogen atmosphere at 100°C for 16 hours. The reaction mixture was filtered while hot, and the filtrate was concentrated under reduced pressure to obtain a crude product. The crude product was stirred with 2 mL of a mixture of PE and EA (PE/EA= 5/1) at 20°C for 0.5 hours. The mixture was subjected to suction filtration under reduced pressure, and the filter cake was dried under reduced pressure to obtain intermediate **37a.** MS m/z: 376.1 $[M+23]^+$.

Step 2: Synthesis of intermediate **37b**

Intermediate **37a** (96.18 mg, 272.17 μmol) was dissolved in EtOH (2 mL). A solution of methylamine in ethanol (1.13 g, 10.89 mmol, 30%) was added, and the mixture was stirred at 20°C for 16 hours. The reaction mixture was concentrated under reduced pressure to obtain intermediate **37b.** MS m/z: 375.1 $[M+23]^+$.

Step 3: Synthesis of hydrochloride of intermediate **37c**

Intermediate **37b** (100 mg, 283.77 μmol) was dissolved in MeOH (2 mL). HCl/dioxane (4 M, 0.38 mL) was added, and the mixture was stirred at 25°C for 2 hours. The reaction mixture was concentrated under reduced pressure to obtain the hydrochloride of intermediate **37c.** MS m/z: 253.0 $[M+1]^+$.

Step 4: Synthesis of compound **37**

Triethylamine (10.54 mg, 104.19 μmol) was added to a solution of intermediate **37c** (15.04 mg, hydrochloride) in DMF (1 mL). The mixture was stirred at 20°C for 0.5 hours. Intermediate 27a (20 mg, 52.10 μmol) and KI (864.82 μg, 5.21 μmol) were then added thereto, and the reaction mixture was stirred at 50°C for 2 hours. The reaction mixture was concentrated under reduced pressure to obtain a crude product. The crude product was purified by preparative TLC (EA,100%) to obtain compound 37. MS m/z: 479.1 $[M+1]^+$.

**Example 38**

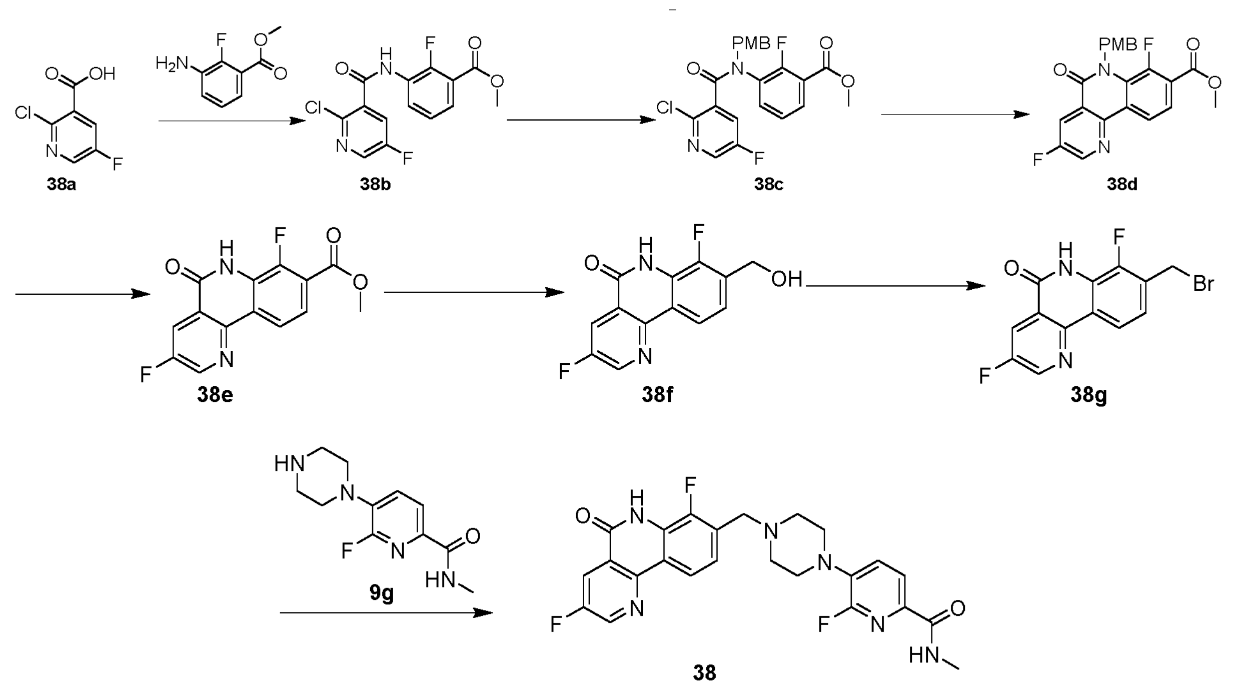

Step 1: Synthesis of intermediate **38b**

Compound **38a** (1 g, 5.70 mmol), methyl 3-amino-2-fluorobenzoate (867.23 mg, 5.13 mmol), and pyridine (2.70 g, 34.18 mmol, 2.76 mL) were added to DCM (10 mL). At 0 to 5°C, phosphorus oxychloride (960.81 mg, 6.27 mmol) was added, and the mixture was stirred at the same temperature for 2 hours. At 0 to 5°C, the reaction mixture was added with 10 mL of water, and extracted with DCM (10 mL × 2). The organic phases were combined and concentrated under reduced pressure to obtain a crude product. The crude product was purified by column chromatography (eluent: PE:EA = 100:0 to 90:10, v/v) to obtain intermediate **38b.** MS m/z: 327.0, 329.0 $[M+1]^+$; $^1H$ NMR (400 MHz, $CDCl_3$) δ ppm 8.91 (br s, 1H), 8.64 - 8.72 (m, 1H), 8.45 (d, *J*=3.00 Hz, 1H), 8.11 (dd, *J*=7.75, 3.00 Hz, 1H), 7.75-7.80 (m, 1H), 7.29 - 7.34 (m, 1H), 3.98 (s, 3H).

Step 2: Synthesis of intermediate **38c**

Intermediate **38b** (1.3 g, 3.98 mmol) and potassium carbonate (1.65 g, 11.94 mmol) were added to DMF (15 mL). p-Methoxybenzyl chloride (747.85 mg, 4.78 mmol) was then added, and the mixture was heated to 90°C and stirred for 2 hours. The reaction mixture was filtered. The filtrate was added with 15 mL of water, and extracted with EA (30 mL × 2). The organic phases were combined and concentrated under reduced pressure to obtain a crude product. The crude product was purified by column chromatography (eluent: PE:EA = 100:0 to 75:25, v/v) to obtain intermediate **38c.** MS m/z: 447.1, 449.1 $[M+1]^+$.

Step 3: Synthesis of intermediate **38d**

Under a nitrogen atmosphere, intermediate **38c** (300 mg, 671.40 μmol), tributylphosphine (135.83 mg, 671.40 μmol), 1,3-bis(diphenylphosphino)propane (110.76 mg, 268.56 μmol), $Pd(OAc)_2$ (60.29 mg, 268.56 μmol), and potassium carbonate (185.58 mg, 1.34 mmol) were added to DMF (8 mL). The reaction was carried out at 120°C under microwave irradiation for 10 minutes. The reaction mixture was filtered. The filter cake was washed with EA (30 mL), and the filtrate was concentrated to obtain a crude product. The crude product was purified by column chromatography (eluent: PE:EA = 100:0 to 90:10, v/v) to obtain intermediate **38d.** MS m/z: 411.0 $[M+1]^+$. $^1$H NMR (400 MHz, DMSO-$d_6$) δ ppm 9.22 (d, *J*=2.88 Hz, 1H), 8.69 (d, *J*=8.63 Hz, 1H), 8.51 (dd, *J*=8.38, 2.88 Hz, 1H), 7.80 (dd, *J*=8.38, 6.13 Hz, 1H), 7.15 (d, *J*=8.50 Hz, 2H), 6.87 (d, *J*=8.76 Hz, 2H), 5.60 (s, 2H), 3.86 (s, 3H), 3.71 (s, 3H).

Step 4: Synthesis of intermediate **38e**

Intermediate **38d** (140 mg, 341.16 μmol) was added to a mixed solution of trifluoroacetic acid (1 mL) and trifluoromethanesulfonic acid (0.2 mL). The reaction mixture was stirred at 25°C for 1 hour. The reaction mixture was added with water (4 mL), resulting in the precipitation of a solid. The mixture was filtered to obtain the filter cake, which was intermediate **38e.** $^1$H NMR (400 MHz, DMSO-$d_6$) δ ppm 12.23 (br s, 1H), 9.18 (d, *J*=2.26 Hz, 1H), 8.44-8.46 (m, 2H), 7.70 - 7.84 (m, 1H), 3.93 (s, 3H).

Step 5: Synthesis of intermediate **38f**

Under a nitrogen atmosphere at 0°C, $LiAlH_4$ (23.54 mg, 620.22 μmol) was added to a solution of intermediate **38e** (120 mg, 413.48 μmol) in THF (4 mL). The mixture was stirred at this temperature for 1 hour. To the reaction mixture, 24 μL of water, 24 μL of 15% sodium hydroxide solution, and 72 μL of ice water were slowly added in sequence to quench the reaction. The reaction mixture was added with water (2 mL) and extracted with EA (3 mL × 2). The organic phases were combined, dried, and concentrated to obtain a crude product. The crude product was stirred with 3 mL of a mixed solution (PE:EA = 1:1) for 10 minutes, and filtered to obtain the filter cake, which was intermediate **38f.** MS m/z: 263.1 $[M+1]^+$. $^1$H NMR (400 MHz, DMSO-$d_6$) δ ppm 12.01 (br s, 1H), 9.12 (d, *J*=2.76 Hz, 1H), 8.41 (dd, *J*=8.66, 2.89 Hz, 1H), 8.37 (d, *J*=8.03 Hz, 1H), 7.37 - 7.46 (m, 1H), 5.46 (t, *J*=5.65 Hz, 1H), 4.68 (d, *J*=5.27 Hz, 2H).

Step 6: Synthesis of intermediate **38g**

Intermediate **38f** (50 mg, 190.69 μmol) and 1,2-dibromotetrachloroethane (136.61 mg, 419.51 μmol) were dissolved in DCM (0.5 mL). At 0°C, tributylphosphine (77.16 mg, 381.37 μmol, 94.10 μL) was added. The mixture was warmed to room temperature (25°C) and stirred for 3 hours. The reaction mixture was filtered to obtain the filter cake, which was intermediate **38g.** $^1$H NMR (400 MHz, DMSO-$d_6$) δ ppm 12.15 (s, 1H), 9.14 (d, *J*=3.01 Hz, 1H), 8.42 (dd, *J*=8.53, 3.01 Hz, 1H), 8.36 (d, *J*=8.28 Hz, 1H), 7.38 - 7.51 (m, 1H), 4.84 (s, 2H).

Step 7: Synthesis of compound **38**

At 25°C, triethylamine (15.56 mg, 153.80 μmol) was added to a solution of intermediate **9g** (16.90 mg, hydrochloride) in DMF (1 mL). The mixture was stirred for 30 minutes. Intermediate **38g** (25 mg, 76.90 μmol) and KI (1.28 mg, 7.69 μmol) were then added, and the reaction mixture was stirred at 50°C for 3 hours. With stirring, the reaction mixture was added with 2 mL of water, and stirred for about 10 minutes. The mixture was filtered to obtain the filter cake, which was compound **38.** MS m/z: 483.1 $[M+1]^+$. $^1$H NMR (400 MHz, DMSO-$d_6$) δ ppm 12.05 (s, 1H), 9.14 (d, *J*=2.76 Hz, 1H), 8.35-8.43 (m, 3H), 7.81 - 7.88 (m, 1H), 7.57 (dd, *J*=10.54, 8.03 Hz, 1H), 7.35 - 7.41 (m, 1H), 3.74 (s, 2H) 3.15 - 3.23 (m, 4H), 2.76 (d, *J*=4.77 Hz, 3H), 2.60 - 2.65 (m, 4H).

**Example 39**

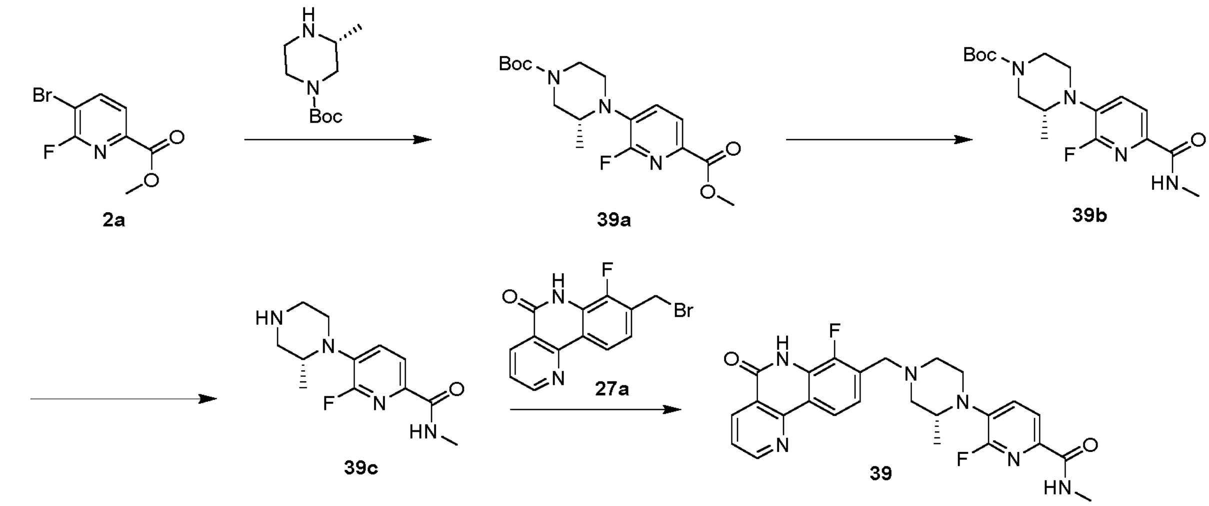

2a, 39a, 39b, 39c, 27a, 39

Step 1: Synthesis of intermediate **39a**

Compound **2a** (200 mg, 854.62 μmol) and (R)-4-tert-butoxycarbonyl-2-methylpiperazine (171.16 mg, 854.62 μmol) were dissolved in toluene (5 mL). RuPhos (79.76 mg, 170.92 μmol), $Cs_2CO_3$ (556.90 mg, 1.71 mmol), and $Pd_2(dba)_3$ (78.26 mg, 85.46 μmol) were sequentially added. The mixture was stirred under a nitrogen atmosphere at 100°C for 16 hours. The reaction mixture was filtered, and the filtrate was concentrated under reduced pressure to obtain a crude product. The crude product was purified by column chromatography (eluent: PE:EA = 100:0 to 90:10 to 75:25, V/V) to obtain intermediate **39a.** MS m/z: 353.9 $[M+1]^+$.

Step **2:** Synthesis of intermediate **39b**

Intermediate **39a** (150 mg, 424.46 μmol) was dissolved in EtOH (2 mL). A solution of methylamine in ethanol (659.13 mg, 21.22 mmol) was added, and the reaction mixture was stirred at 25°C for 16 hours. The reaction mixture was concentrated under reduced pressure to obtain intermediate **39b.** MS m/z: 353.0 $[M+1]^+$.

Step 3: Synthesis of hydrochloride of intermediate **39c**

Intermediate **39b** (150 mg, 425.65μmol) was dissolved in MeOH (3 mL). HCl/dioxane (4 M, 3 mL) was added, and the reaction mixture was stirred at room temperature (25°C) for 3 hours. The reaction mixture was concentrated under reduced pressure to obtain the hydrochloride of intermediate **39c.** MS m/z: 253.1 $[M+1]^+$

Step 4: Synthesis of trifluoroacetate of compound **39**

Intermediate **27a** (25 mg, 81.40 μmol), intermediate **39c** (20.54 mg, hydrochloride), KI (6.76 mg, 40.70μmol), and triethylamine (32.95 mg, 325.61 μmol) were dissolved in DMF (1 mL). The mixture was heated to 50°C and stirred for 2 hours. After cooling, the mixture was added with 3 mL of water and extracted with ethyl acetate (4 mL × 2). The organic phases were combined, dried over anhydrous sodium sulfate, and filtered. The filtrate was concentrated to obtain a crude product. The crude product was separated by preparative HPLC (column: C18, 100 × 40 mm; mobile phase: [water (trifluoroacetic acid)-acetonitrile]; gradient: 3%-33% acetonitrile) to obtain the trifluoroacetate of compound **39.** MS m/z: 479.2 $[M+1]^+$. $^1H$ NMR (400 MHz, $CD_3OD$) δ ppm 9.08 (dd, *J*=1.76, 4.77 Hz, 1H), 8.74 (d, *J*=4.82 Hz, 1 H), 8.64 (d, *J*=8.53 Hz, 1 H), 7.96 (d, *J*=8.03 Hz, 1H), 7.73 (dd, *J*=4.52, 8.03 Hz, 1H), 7.52 (dd, *J*=6.78, 8.28 Hz, 1H), 7.35 (d, *J*=8.03 Hz, 1H), 4.54 (s, 2 H), 3.85 - 4.12 (m, 2 H), 3.32 - 3.51 (m, 4 H), 3.23 - 3.28 (m, 1 H), 2.93 (s, 3 H), 1.13 (d, J=6.27 Hz, 3 H).

**Example 40**

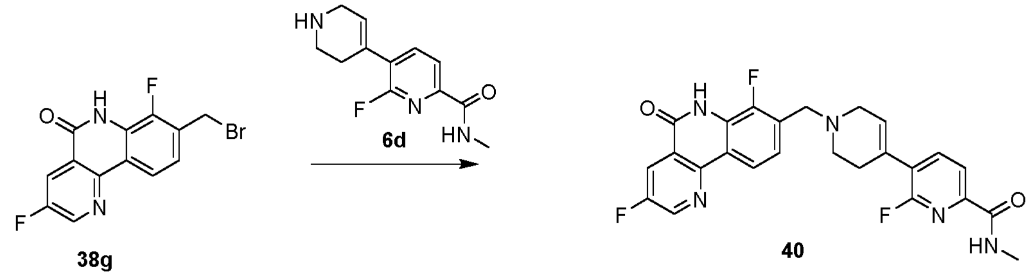

Step 1: Synthesis of trifluoroacetate of compound **40**

At 25°C, triethylamine (15.56 mg, 153.80 μmol) was added to a solution of intermediate **6d** (16.72 mg, hydrochloride) in DMF (1 mL). The mixture was stirred for 30 minutes. Intermediate **38g** (25 mg, 76.90 μmol) and KI (1.28 mg, 7.69 μmol) were then added, and the reaction mixture was stirred at 50°C for 3 hours. The reaction mixture was filtered, and the filtrate was separated by preparative HPLC (column: C18, 100 × 40 mm; mobile phase: [water (trifluoroacetic acid)-acetonitrile]; gradient: 6%-36% acetonitrile) to obtain the trifluoroacetate of compound **40.** MS m/z: 480.2 $[M+1]^+$. $^1H$ NMR (400 MHz, DMSO-$d_6$) δ ppm 12.29 (br s, 1H), 10.26 (s, 1H), 9.18 (d, *J*=2.63 Hz, 1H), 8.66 (d, *J*=5.13 Hz, 1H), 8.42 - 8.56 (m, 2H), 8.13 (t, *J*=8.63 Hz, 1H), 7.98 (d, *J*=7.63 Hz, 1H), 7.45 - 7.61 (m, 1H), 6.27 (s, 1H), 4.66 (s, 2H), 3.90 - 4.12 (m, 2H), 3.66 - 3.83 (m, 1H), 2.80 (d, *J*=4.75 Hz, 3H), 2.74 - 2.79 (m, 1H), 2.22 - 2.32 (m, 2H).

**Example 41**

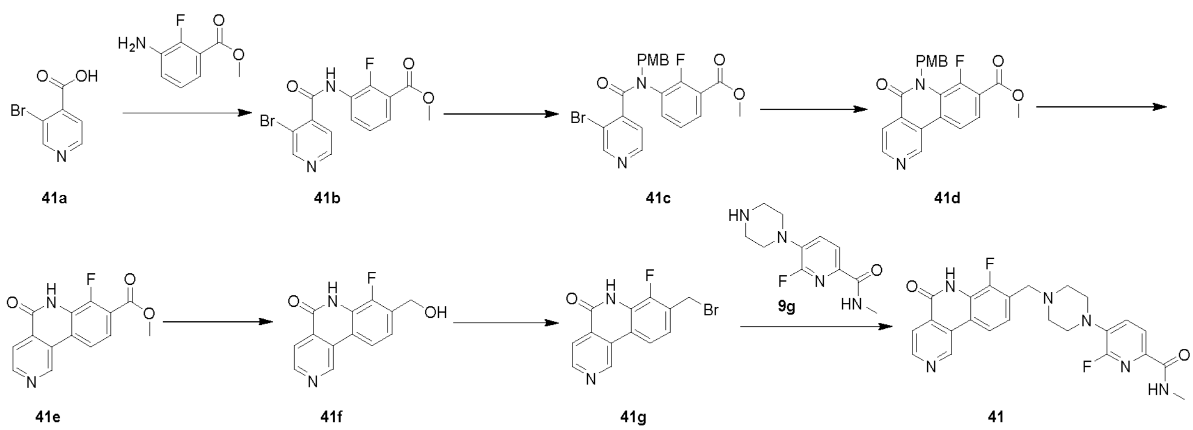

Step 1: Synthesis of intermediate **41b**

Compound **41a** (2 g, 9.90 mmol) and methyl 3-amino-2-fluorobenzoate (867.23 mg, 5.13 mmol) were added to DMF (20 mL). Triethylamine (3.01 g, 29.70 mmol) and HATU (4.52 g, 11.88 mmol) were then added, and the mixture was stirred at 25°C for 16 hours. The reaction mixture was added with water (20 mL) and extracted with EA (30 mL × 3). The organic phases were combined, dried over anhydrous sodium sulfate, and concentrated to obtain a crude product. The crude product was purified by silica gel column chromatography (eluent: DCM:MeOH = 100:0 to 99:1, v/v) to obtain intermediate **41b.** MS m/z: 352.9, 354.9 $[M+1]^+$; $^1H$ NMR (400 MHz, DMSO-$d_6$) δ ppm 10.67 - 10.84 (m, 1H), 8.89 (s, 1H), 8.70 (d, *J*=5.02 Hz, 1H), 8.06 - 8.12 (m, 1H), 7.74 - 7.78 (m, 1H), 7.65 (d, *J*=4.77 Hz, 1H), 7.38 (t, J=7.91 Hz, 1H), 3.88 (s, 3H).

Step 2: Synthesis of intermediate **41c**

Intermediate **41b** (1.8 g, 5.10 mmol) and potassium carbonate (2.11 g, 15.29 mmol) were added to DMF (20 mL). p-Methoxybenzyl chloride (957.90 mg, 6.12 mmol) was then added, and the mixture was heated to 90°C and stirred for 3 hours. The reaction mixture was filtered. The filtrate was added with 20 mL of water, and extracted with EA (30 mL × 2). The organic phases were combined and concentrated under reduced pressure to obtain a crude product. The crude product was purified by silica gel column chromatography (eluent: PE:EA = 2:1, v/v) to obtain intermediate **41c.** MS m/z: 473.0,

475.0 $[M+1]^+$.

Step 3: Synthesis of intermediate **41d**

Under a nitrogen atmosphere, intermediate **41c** (300 mg, 633.86 μmol), tributylphosphine (135.83 mg, 671.40 μmol), 1,3-bis(diphenylphosphino)propane (104.57 mg, 253.54 μmol), $Pd(OAc)_2$ (56.92 mg, 253.54 μmol), and potassium carbonate (175.21 mg, 1.27 mmol) were added to DMF (10 mL). The reaction was carried out at 140°C under microwave irradiation for 10 minutes. The reaction mixture was filtered. The filtrate was added with 20 mL of water and extracted with EA (30 mL) three times. The organic phases were combined, dried over anhydrous sodium sulfate, and concentrated to obtain a crude product. The crude product was purified by column chromatography (eluent: PE:EA = 100:0 to 60:40, v/v) to obtain intermediate **41d.** MS m/z: 393.1 $[M+1]^+$; $^1H$ NMR (400 MHz, DMSO-$d_6$) δ 9.99 (s, 1H), 8.95 (d, *J*=5.13 Hz, 1H), 8.66 (d, *J*=8.50 Hz, 1H), 8.22 (d, *J*=5.13 Hz, 1H), 7.76 (dd, *J*=8.38, 6.25 Hz, 1H), 7.13 (d, *J*=8.50 Hz, 2H), 6.86 (d, *J*=8.63 Hz, 2H), 5.58 (s, 2H), 3.86 (s, 3H), 3.71 (s, 3H).

Step 4: Synthesis of intermediate **41e**

Intermediate **41d** (250 mg, 637.14 μmol) was added to a mixed solution of trifluoroacetic acid (1 mL) and trifluoromethanesulfonic acid (0.2 mL). The mixture was stirred at 25°C for 1 hour. The reaction mixture was added with 2 mL of water and extracted with ethyl acetate (8 mL). The organic phase was dried over anhydrous sodium sulfate, and filtered. The filtrate was concentrated to obtain intermediate **41e.** MS m/z: 273.1 $[M+1]^+$; $^1H$ NMR (400 MHz, DMSO-$d_6$) δ ppm12.17 (br s, 1H), 9.95 (s, 1H), 8.93 (d, *J*=5.13 Hz, 1H), 8.52 (d, *J*=8.50 Hz, 1H), 8.19 (d, *J*=5.25 Hz, 1H), 7.72 (dd, *J*=8.44, 6.69 Hz, 1H), 3.93 (s, 3H).

Step 5: Synthesis of intermediate **41f**

Under a nitrogen atmosphere, at 0°C, $LiAlH_4$ (2.5 M in tetrahydrofuran solution, 205.71 μL) was added to a solution of intermediate **41e** (140 mg, 514.27 μmol) in THF (5 mL). The mixture was stirred at this temperature for 1 hour. To the reaction mixture, 30 μL of water, 30 μL of 15% sodium hydroxide solution, and 90 μL of ice water were slowly added in sequence to quench the reaction. The reaction mixture was added with water (10 mL) and extracted with EA (20 mL × 3). The organic phases were combined, dried, and concentrated to obtain a crude product, which was intermediate **41f.** MS m/z: 245.1 $[M+1]^+$. $^1H$ NMR (400 MHz, DMSO-$d_6$) δ ppm 11.96 (br s, 1H), 9.89 (s, 1H), 8.85 (d, *J*=5.02 Hz, 1H), 8.41 (d, *J*=8.53 Hz, 1H), 8.14 (d, *J*=5.02 Hz, 1H), 7.39 (t, *J*=7.65 Hz, 1H), 5.46 (t, *J*=5.77 Hz, 1H), 4.68 (d, *J*=4.77 Hz, 2H).

Step 6: Synthesis of intermediate **41g**

Intermediate **41f**(50 mg, 204.73 μmol) and 1,2-dibromotetrachloroethane (146.67 mg, 450.41 μmol) were dissolved in DCM (0.5 mL). At 0°C, tributylphosphine (82.84 mg, 409.47 μmol) was added. The mixture was warmed to room temperature (25°C) and stirred for 3 hours. The reaction mixture was filtered to obtain the filter cake, which was intermediate **41g.** $^1H$ NMR (400 MHz, DMSO-$d_6$) δ ppm 12.16 (s, 1H), 9.90 - 9.98 (m, 1H), 8.82 - 9.06 (m, 1H), 8.38 - 8.48 (m, 1H), 8.21 (d, *J*=5.02 Hz, 1H), 7.46 (t, *J*=7.65 Hz, 1H), 4.94 (s, 2H).

Step 7: Synthesis of trifluoroacetate of compound **41**

At 25°C, triethylamine (153.80 μmol, 21.41 μL) was added to a solution of intermediate **9g** (16.90 mg, hydrochloride) in DMF (1 mL). The mixture was stirred for 30 minutes. Intermediate **41g** (25 mg, 81.40 μmol) and KI (1.35 mg, 8.14 μmol) were then added, and the reaction mixture was stirred at 50°C for 2 hours. The reaction mixture was filtered, and the filtrate was purified by preparative liquid chromatography (column: C18, 100 × 40 mm; mobile phase: [water (trifluoroacetic acid)-acetonitrile]; gradient: 0%-30% acetonitrile) to obtain the trifluoroacetate of compound **41.** MS m/z: 465.2 $[M+1]^+$. $^1H$ NMR (400 MHz, $CD_3OD$) δ ppm 9.86 (s, 1H), 8.91 (d, J=5.27 Hz, 1H), 8.49 (d, J=8.53 Hz, 1H), 8.33 (d, J=5.27 Hz, 1H), 7.96 (d, J=8.28 Hz, 1H), 7.64 (dd, J=10.29, 8.28 Hz, 1H), 7.56 (dd, J=8.28, 7.03 Hz, 1H), 4.66 (s, 2H), 3.47 - 3.72 (m, 8H), 2.93 (s, 3H).

**Example 42**

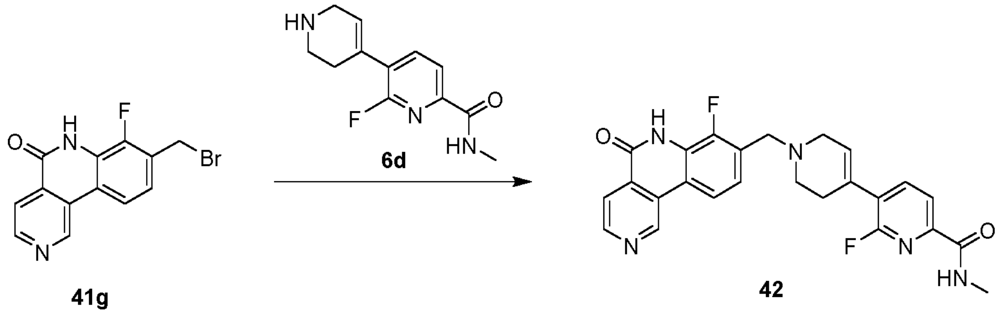

6d

41g

42

Step 1: Synthesis of trifluoroacetate of compound **42**

Triethylamine (16.47 mg, 162.80 μmol) was added to a solution of intermediate **6d** (17.69 mg, hydrochloride) in DMF (1 mL). The mixture was stirred at 25°C for 30 minutes. Intermediate **41g** (25 mg, 81.40 μmol) and KI (1.35 mg, 8.14 μmol) were then added, and the reaction mixture was heated to 50°C and stirred for 2 hours. The reaction mixture was filtered, and the filtrate was purified by preparative HPLC (column: C18, 100 × 40 mm; mobile phase: [water (trifluoroacetic acid)-acetonitrile]; gradient: 0%-30% acetonitrile) to obtain the trifluoroacetate of compound **42.** MS m/z: 462.1 $[M+1]^+$. $^1H$ NMR (400 MHz, $CD_3OD$) δ 9.86 (s, 1H), 8.91 (d, *J*=5.27 Hz, 1H), 8.50 (d, *J*=7.78 Hz, 1H), 8.34 (d, *J*=5.77 Hz, 1H), 8.08 - 8.15 (m, 1H), 8.02 - 8.07 (m, 1H), 7.58 (dd, *J*=8.28, 7.03 Hz, 1H), 6.31 (s, 1H), 4.74 (s, 2H), 4.11 (s, 2H), 3.58 - 3.85 (m, 2H), 2.96 - 3.01 (m, 2H), 2.96 (s, 3H).

**Example 43**

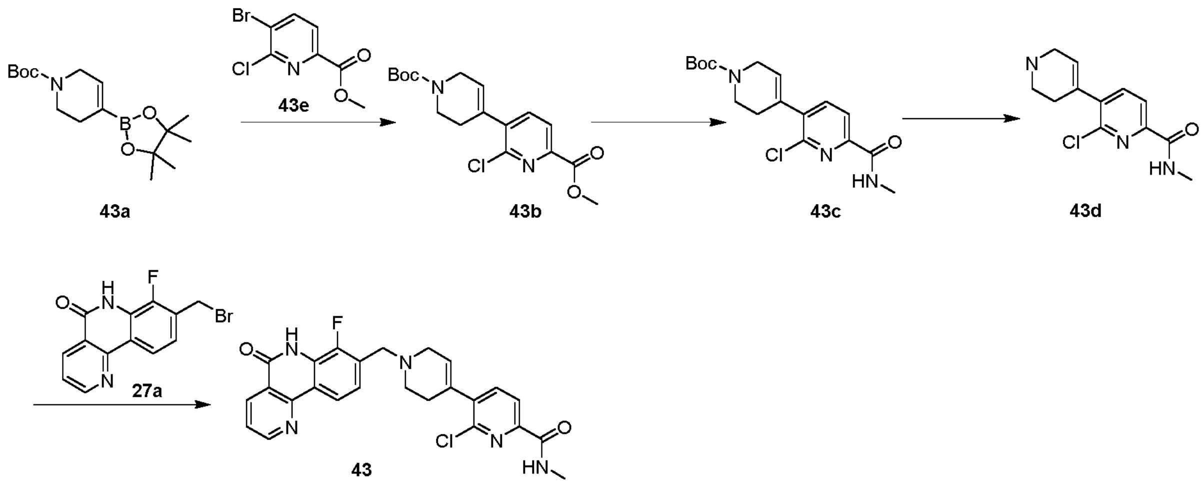

43e

43a 43b 43c 43d

27a

43

Step 1: Synthesis of intermediate **43b**

Under a nitrogen atmosphere, **43e** (800 mg, 3.19 mmol), compound **43a** (1.09 g, 3.51 mmol), $Pd(dppf)Cl_2$ (233.70 mg, 319.39 μmol), and potassium acetate (626.91 mg, 6.39 mmol) were added to a mixed solution of $H_2O$ (1.6 mL) and THF (8 mL). The mixture was heated to 70°C and stirred for 16 hours. The reaction mixture was filtered. The filter cake was rinsed with EA (15 mL), and the filtrate was concentrated under reduced pressure to obtain a crude product. The crude product was purified by flash column chromatography (eluent: PE:EA = 100:0 to 90:10, v/v) to obtain intermediate **43b.** MS m/z: 353.1 $[M+1]^+$. $^1H$ NMR (400 MHz, $CDCl_3$) δ ppm 8.07 (dd, J=7.78, 1.00 Hz, 1H), 7.64 - 7.76 (m, 1H), 5.83 (s, 1H), 4.11 (s, 2H), 3.67 (t, J=5.27 Hz, 2H), 2.50 (br s, 2H), 1.52 (s, 9H), 1.26 (s, 3H).

Step 2: Synthesis of intermediate **43c**

A solution of methylamine in ethanol (9.05 g, 87.42 mmol, 30%) was added to a solution of intermediate **43b** (300 mg, 850.31 μmol) in MeOH (1 mL). The mixture was stirred at 40°C for 16 hours. The reaction mixture was concentrated under reduced pressure to obtain intermediate **43c.** MS m/z: 352.1 $[M+1]^+$. $^1H$ NMR (400 MHz, $CD_3OD$) δ ppm7.92 (d,

J=7.78 Hz, 1H), 7.74 (d, J=7.53 Hz, 1H), 5.75 (s, 1H), 3.98 (s, 2H), 3.53 - 3.61 (m, 2H), 2.85 (s, 3H), 2.33 - 2.41 (m, 2H), 1.40 (s, 9H).

Step 3: Synthesis of hydrochloride of intermediate **43d**

HCl/dioxane (4 M, 1.06 mL) was added to a solution of intermediate **43c** (298 mg, 847.01 μmol) in MeOH (0.5 mL). The mixture was stirred at 25°C for 1 hour. HCl/dioxane (4 M, 635.26 μL) was further added. The mixture was stirred for another 1 hour. The reaction mixture was concentrated under reduced pressure to obtain the hydrochloride of intermediate **43d.** MS m/z: 252.1 $[M+1]^+$.

Step 4: Synthesis of compound **43**

Triethylamine (32.95 mg, 325.61 μmol) was added to a solution of intermediate **43d** (37.53 mg, hydrochloride) in DMF (1 mL). The mixture was stirred at 25°C for 30 minutes. Intermediate **27a** (50 mg, 162.80 μmol) and KI (2.70 mg, 16.28 μmol) were then added, and the mixture was heated to 50°C and stirred for 2.5 hours. The reaction mixture was added with DMF (2 mL), and with water (1 mL), resulting in the precipitation of a solid. The mixture was filtered to obtain the filter cake, which was compound **43.** MS m/z: 478.1 $[M+1]^+$. $^1H$ NMR (400 MHz, DMSO-$d_6$) δ ppm 11.90 (s, 1H), 9.09 (dd, *J*=4.57, 1.81 Hz, 1H), 8.65 (dd, J=8.07, 1.81 Hz, 1H), 8.61 (d, J=4.88 Hz, 1H), 8.43 (d, J=8.51 Hz, 1H), 7.88 - 7.93 (m, 2H), 7.72 (dd, J=8.07, 4.57 Hz, 1H), 7.36 - 7.43 (m, 1H), 5.86 (s, 1H), 3.82 (s, 2H), 3.67 - 3.74 (m, 2H), 3.18 (d, J=2.50 Hz, 2H), 2.81 (d, J=4.75 Hz, 3H), 2.45 (d, J=1.63 Hz, 2H).

**Example 44**

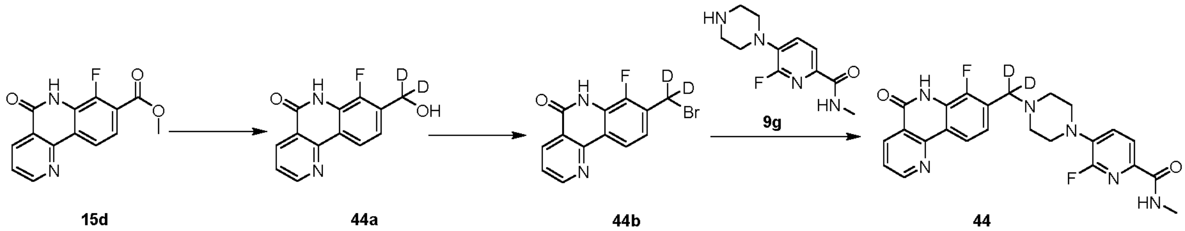


15d 44a 44b 44

Step 1: Synthesis of intermediate **44a**

Intermediate **15d** (100 mg, 367.34 μmol) was dissolved in THF (1 mL). At 0°C, deuterated lithium aluminum hydride powder (20.91 mg, 551.00 μmol) was added thereto, and the mixture was stirred at 0°C for 1 hour. The reaction mixture was added to saturated sodium potassium tartrate solution (5 mL). The mixture was then extracted with ethyl acetate (10 mL × 3). The organic phases were combined, dried over anhydrous sodium sulfate, and filtered. The filtrate was concentrated under reduced pressure at 45°C to obtain intermediate **44a.** MS m/z: 246.9 $[M+1]^+$. $^1H$ NMR (400 MHz, DMSO-$d_6$) δ ppm 11.87 (s, 1H), 9.08 (dd, *J* = 1.8, 4.5 Hz, 1H), 8.64 (dd, *J* = 1.9, 7.9 Hz, 1H), 8.43 (d, *J* = 7.5 Hz, 1H), 7.71 (dd, *J* = 4.8, 8.0 Hz, 1H), 7.40 (dd, *J* = 6.7, 8.2 Hz, 1H), 5.41 (s, 1H).

Step 2: Synthesis of intermediate **44b**

Intermediate **44a** (56 mg, 227.43 μmol) was dissolved in DCM (1 mL). 1,2-Dibromotetrachloroethane (162.93 mg, 500.34 μmol) was added, and at 0°C, tributylphosphine (92.02 mg, 454.85 μmol) was added. The mixture was stirred at 25°C for 3 hours. The reaction mixture was concentrated under reduced pressure, and methanol (2 mL) was added thereto. The mixture was stirred for 5 minutes and then filtered to obtain the filter cake, which was intermediate **44b.** $^1H$ NMR (400 MHz, DMSO-$d_6$) δ ppm 12.00 (s, 1H), 9.09 (d, *J* = 4.5 Hz, 1H), 8.71 - 8.61 (m, 1H), 8.49 - 8.38 (m, 1H), 7.80 - 7.69 (m, 1H), 7.51 - 7.36 (m, 1H).

Step 3: Synthesis of Compound **44**

Intermediate **9g** (31.99 mg, hydrochloride) was dissolved in DMF (1 mL). Triethylamine (58.92 mg, 582.28 μmol) was added, and the reaction mixture was stirred at 25°C for 0.5 hours. Intermediate **44b** (45 mg, 145.57 μmol) and KI (12.08 mg, 72.78 μmol) were then added. The mixture was stirred at 50°C for 2 hours. The reaction mixture was added with

water (1.5 mL), and stirred for 10 minutes, resulting in the precipitation of a solid. The mixture was filtered to obtain the filter cake, which was compound **44.** MS m/z: 489.0 [M+23]$^+$. $^1$H NMR (400 MHz, DMSO-$d_6$) δ ppm 11.90 (s, 1H), 9.09 (dd, *J* = 1.8, 4.5 Hz, 1H), 8.64 (dd, *J* = 1.8, 8.0 Hz, 1H), 8.47 - 8.37 (m, 2H), 7.84 (d, *J* = 7.8 Hz, 1H), 7.72 (dd, *J* = 4.5, 8.0 Hz, 1H), 7.57 (dd, *J* = 8.3, 10.5 Hz, 1H), 7.37 (dd, *J* = 6.7, 8.2 Hz, 1H), 3.17-3.20 (m, 4H), 2.76 (d, *J* = 4.8 Hz, 3H), 2.57-2.65 (m, 4H).

**Example 45**

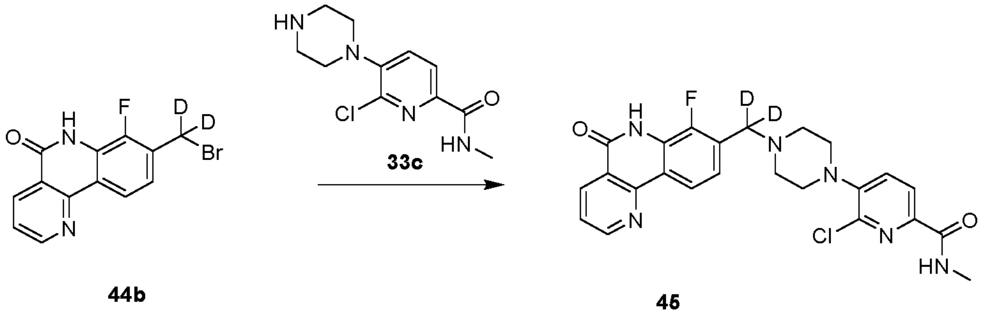

44b

33c

45

Step 1: Synthesis of compound 45

Intermediate **33c** (37.68 mg, hydrochloride) was dissolved in DMF (1 mL). Triethylamine (65.47 mg, 646.98 μmol) was added, and the reaction mixture was stirred at 25°C for 0.5 hours. Intermediate **44b** (50 mg, 161.74 μmol) and KI (13.42 mg, 80.87 μmol) were then added. The mixture was stirred at 50°C for 1 hour. After cooling, the reaction mixture was added with water (1 mL), and stirred for 10 minutes, resulting in the precipitation of a solid. The mixture was filtered to obtain the filter cake, which was compound **45.** MS m/z: 483.1 [M+1]$^+$. $^1$H NMR (400 MHz, DMSO-$d_6$) δ ppm 11.90 (s, 1H), 9.09 (dd, *J* = 1.8, 4.5 Hz, 1H), 8.65 (dd, *J* = 1.8, 8.0 Hz, 1H), 8.48 - 8.38 (m, 2H), 7.94 (d, *J* = 8.3 Hz, 1H), 7.72 (dd, *J* = 4.5, 8.0 Hz, 1H), 7.66 (d, *J* = 8.3 Hz, 1H), 7.37 (dd, *J* = 6.7, 7.9 Hz, 1H), 3.10-3.15 (m, 4H), 2.79 (d, *J* = 4.8 Hz, 3H), 2.62-2.67 (m, 4H).

**Example 46**

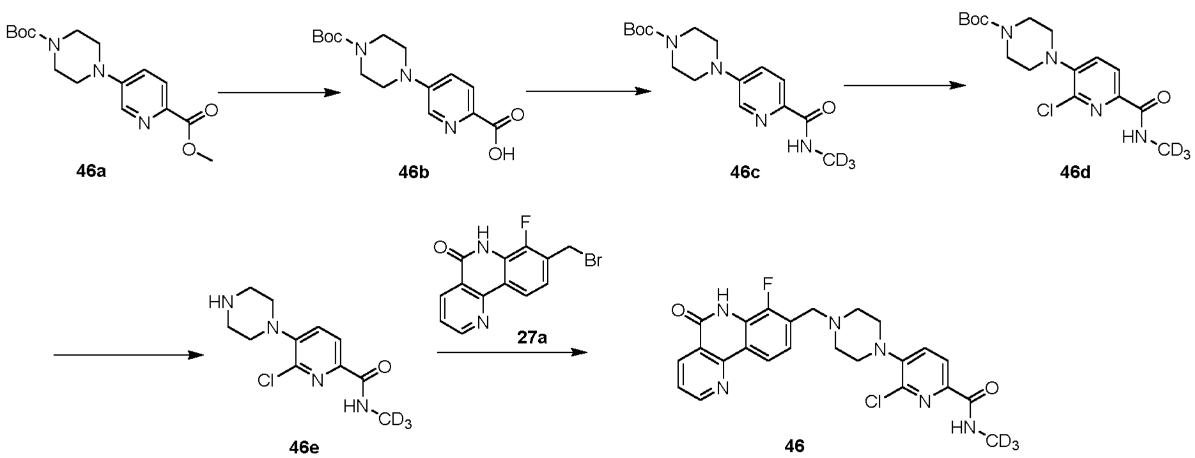

46a 46b 46c 46d 46e 27a 46

Step 1: Synthesis of intermediate **46b**

Intermediate **46a** (2 g, 6.22 mmol) was dissolved in THF (20 mL) and $H_2O$ (4 mL). Lithium hydroxide monohydrate (1.31 g, 31.12 mmol) was added thereto, and the mixture was stirred at room temperature (25°C) for 2 hours. The pH of the reaction mixture was adjusted to 3 with dilute hydrochloric acid (1 mol/L). Then the mixture was extracted with ethyl acetate (20 mL × 3). The organic phase was washed with 20 mL of saturated brine, dried over anhydrous sodium sulfate, and filtered. The filtrate was concentrated under reduced pressure to obtain intermediate **46b.** MS m/z: 307.9 [M+1]$^+$. $^1$H NMR (400 MHz, DMSO-$d_6$) δ ppm 8.37 (d, *J* = 2.8 Hz, 1H), 7.88 (d, *J* = 8.8 Hz, 1H), 7.36 (dd, *J* = 3.0, 8.8 Hz, 1H), 3.50 - 3.46 (m,

4H), 3.39 - 3.36 (m, 4H), 1.43 (s, 9H).

Step 2: Synthesis of intermediate **46c**

Intermediate **46b** (400 mg, 1.30 mmol) and deuterated methylamine hydrochloride (100.98 mg, 1.43 mmol) were dissolved in EA (4 mL). DIEA (336.41 mg, 2.60 mmol) and n-butyl phosphoric anhydride (50% ethyl acetate solution) (937.74 mg, 2.60 mmol) were added, and the mixture was stirred at 25°C for 3 hours. An additional 937.74 mg of n-butyl phosphoric anhydride was added, and the mixture was stirred for another 16 hours. The pH of the reaction mixture was adjusted to 10. The mixture was added with 5 mL of water and extracted with ethyl acetate (5 mL × 3). The organic phases were combined, dried over anhydrous sodium sulfate, and filtered. The filtrate was concentrated under reduced pressure to obtain intermediate 46c. MS m/z: 324.0 $[M+1]^+$. $^1H$ NMR (400 MHz, DMSO-$d_6$) δ ppm 8.43 - 8.37 (m, 1H), 8.28 (d, *J* = 2.8 Hz, 1H), 7.84 (d, *J* = 8.8 Hz, 1H), 7.41 (dd, *J* = 2.8, 8.8 Hz, 1H), 3.52 - 3.45 (m, 4H), 3.32 - 3.30 (m, 4H), 1.43 (s, 9H).

Step 3: Synthesis of intermediate **46d**

Intermediate **46c** (314 mg, 970.92 μmol) was dissolved in DMF (3.5 mL). N-Chlorosuccinimide (142.61 mg, 1.07 mmol) was added, and the reaction mixture was stirred at 50°C for 1 hour. The reaction mixture was added with 10 mL of water and extracted with ethyl acetate (10 mL × 3). The organic phases were combined, dried over anhydrous sodium sulfate, and filtered. The filtrate was concentrated under reduced pressure to obtain intermediate **46d.** MS m/z: 358.0 $[M+1]^+$, 380.0 $[M+23]^+$. $^1H$ NMR (400 MHz, DMSO-$d_6$) δ ppm 8.44 (s, 1H), 7.95 (d, *J* = 8.0 Hz, 1H), 7.68 (d, *J* = 8.0 Hz, 1H), 3.52 -3.47 (m, 4H), 3.09 - 3.01 (m, 4H), 1.43 (s, 9H).

Step 4: Synthesis of hydrochloride of intermediate **46e**

Intermediate **46d** (355 mg, 992.04 μmol) was dissolved in MeOH (4 mL). HCl/dioxane (4 M, 1.34 mL) was added, and the reaction mixture was stirred at 25°C for 2 hours. The reaction mixture was concentrated under reduced pressure to obtain the hydrochloride of intermediate **46e.** MS m/z: 257.9 $[M+1]^+$.

Step 5: Synthesis of compound **46**

Intermediate **46e** (38.32 mg, hydrochloride) was dissolved in DMF (1 mL). Triethylamine (65.90 mg, 651.22 μmol) was added, and the reaction mixture was stirred at 25°C for 0.5 hours. KI (13.51 mg, 81.40 μmol) and intermediate **27a** (50 mg, 162.80 μmol) were then added, and the mixture was stirred at 50°C for 1 hour. After cooling, the reaction mixture was stirred with 2 mL of water for 10 minutes, resulting in the precipitation of a solid. The mixture was filtered to obtain the filter cake, which was compound **46.** MS m/z: 484.0 $[M+1]^+$; 506.0$[M+23]^+$. $^1H$ NMR (400 MHz, DMSO-$d_6$) δ ppm 11.90 (s, 1H), 9.09 (dd, *J* = 1.8, 4.5 Hz, 1H), 8.64 (dd, *J* = 1.8, 8.0 Hz, 1H), 8.47 - 8.38 (m, 2H), 7.94 (d, *J* = 8.3 Hz, 1H), 7.72 (dd, *J* = 4.5, 8.0 Hz, 1H), 7.67 (d, *J* = 8.0 Hz, 1H), 7.37 (dd, *J* = 6.8, 8.0 Hz, 1H), 3.76 (s, 2H), 3.16-3.12 (m, 4H), 2.69-2.63 (m, 4H).

**Example 47**

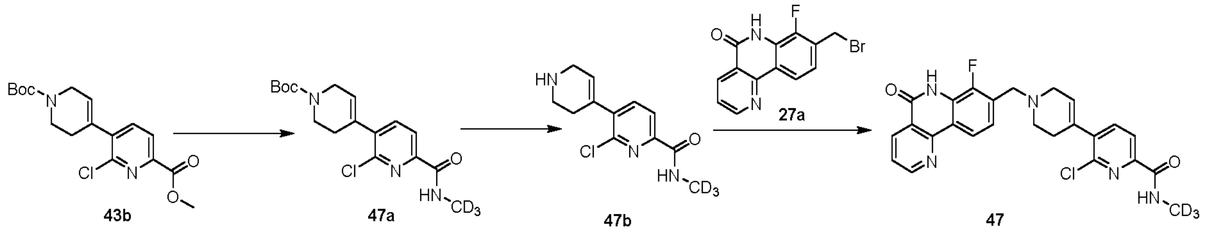

Step 1: Synthesis of intermediate **47a**

Deuterated methylamine hydrochloride (399.85 mg, 5.67 mmol) was added to $H_2O$ (2 mL). Sodium tert-butoxide (272.39 mg, 2.83 mmol) was then added. The mixture was stirred at 25°C for 1 hour. A solution of intermediate **43b** (100 mg, 283.44 μmol) in MeOH (4 mL) was added. The reaction mixture was heated to 60°C and stirred for 4 hours. After cooling to room temperature, the mixture was extracted with ethyl acetate (5 mL × 3). The organic phases were combined,

dried over anhydrous sodium sulfate, and concentrated under reduced pressure to obtain a crude product. The crude product was purified by preparative TLC (eluent: PE:EA=2:1) to obtain intermediate **47a.** MS m/z: 355.1, 357.1[M+1]$^+$.

Step 2: Synthesis of hydrochloride of intermediate **47b**

At 25°C, HCl/dioxane (4 M, 211.36 μL) was added to a solution of intermediate **47a** (60 mg, 169.09 μmol) in MeOH (0.5 mL). The mixture was stirred for 2 hours. The reaction mixture was concentrated under reduced pressure to obtain the hydrochloride of intermediate **47b.** MS m/z: 255.1, 257.1[M+1]$^+$. $^1$H NMR (400 MHz, DMSO-$d_6$) δ ppm 9.36 (br s, 2H), 8.63 (s, 1H), 8.03 (d, *J*=7.78 Hz, 1H), 7.89 (d, *J*=7.78 Hz, 1H), 5.93 (br s, 1H), 3.76 (br d, *J*=1.51 Hz, 2H), 3.30 (br d, *J*=4.52 Hz, 2H), 2.63 (br d, *J*=1.51 Hz, 2H).

Step 3: Synthesis of Compound **47**

Triethylamine (32.95 mg, 325.61 μmol) was added to a solution of intermediate **47b** (37.93 mg, hydrochloride) in DMF (1.5 mL). The mixture was stirred at 25°C for 0.5 hours. Intermediate **27a** (50 mg, 162.80 μmol) and KI (2.70 mg, 16.28 μmol) were then added, and the reaction mixture was stirred at 50°C for 2.5 hours. After cooling, the reaction mixture was added with 1.5 mL of water, resulting in the precipitation of a solid. The mixture was filtered to obtain the filter cake, which was compound **47.** MS m/z: 481.0, 483.0 [M+1]$^+$. $^1$H NMR (400 MHz, DMSO-$d_6$) δ ppm 11.89 (s, 1H), 9.09 (dd, *J*=4.50, 1.75 Hz, 1H), 8.65 (dd, *J*=8.00, 1.75 Hz, 1H), 8.57 (s, 1H), 8.44 (d, *J*=8.25 Hz, 1H), 7.95 - 7.99 (m, 1H), 7.88 - 7.93 (m, 1H), 7.72 (dd, *J*=8.00, 4.63 Hz, 1H), 7.36 - 7.43 (m, 1H), 5.87 (s, 1H), 3.82 (s, 2H), 3.19 (d, J=2.13 Hz, 2H), 2.72 - 2.78 (m, 2H), 2.44 - 2.47 (m, 2H).

**Example 48**

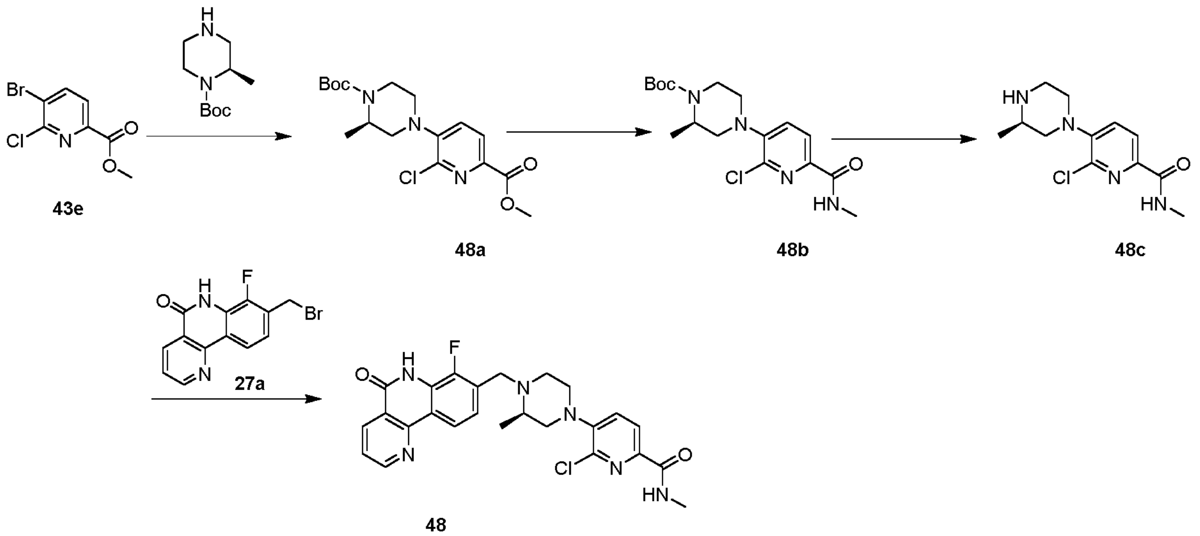

43e 48a 48b 48c 27a 48

Step 1: Synthesis of intermediate **48a**

(R)-1-tert-Butoxycarbonyl-2-methylpiperazine (0.2 g, 998.62 μmol), **43e** (250.13 mg, 998.62 μmol), $Cs_2CO_3$ (650.74 mg, 2.00 mmol), RuPhos (93.20 mg, 199.72 μmol), and $Pd_2(dba)_3$ (91.44 mg, 99.86 μmol) were sequentially added to toluene (4 mL). The mixture was purged with nitrogen three times and stirred at 100°C for 6 hours. The reaction mixture was filtered under reduced pressure while hot, and the filtrate was concentrated under reduced pressure at 50°C to obtain a crude product. The crude product was purified by column chromatography (eluent: PE/EA = 4/1, v/v) to obtain intermediate **48a.** MS m/z: 370.0, 372.0 [M+1]$^+$.

Step **2:** Synthesis of intermediate **48b**

Intermediate **48a** (183.15 mg, 495.21 μmol) was added to EtOH (2 mL). With stirring, a solution of methylamine in ethanol (2.27 g, 21.93 mmol) was added thereto, and the reaction mixture was stirred at 20°C for 16 hours. The reaction mixture was concentrated under reduced pressure at 45°C to obtain a crude product of intermediate **48b.** MS m/z: 312.9,

314.9 [M-56]⁺.

Step 3: Synthesis of hydrochloride of intermediate **48c**

Intermediate **48b** (100 mg, 271.11 μmol) was added to MeOH (2 mL). With stirring, HCl/dioxane (4 M, 383.08 μL) was added thereto, and the reaction mixture was stirred at 25°C for 2 hours. The reaction mixture was concentrated under reduced pressure at 45°C to obtain the hydrochloride of intermediate **48c.** MS m/z: 268.9,270.9 $[M+1]^+$.

Step 4: Synthesis of Compound **48**

Triethylamine (26.36 mg, 260.49 μmol) was added to a solution of intermediate **48c** (39.75 mg, hydrochloride) in DMF (2 mL). The mixture was stirred at 20°C for 0.5 hours. Intermediate **27a** (50 mg, 130.24 μmol) and KI (2.16 mg, 13.02 μmol) were then added thereto, and the reaction mixture was stirred at 50°C for 2 hours. The reaction mixture was concentrated under reduced pressure to obtain a crude product. The crude product was purified by preparative TLC (100% EA) to obtain compound **48.** MS m/z: 495.2, 497.2 $[M+1]^+$. $^1H$ NMR (400 MHz, DMSO-$d_6$) δ ppm 11.86 (s, 1 H), 9.08 (dd, J=4.57, 1.69 Hz, 1 H), 8.64 (dd, J=8.00, 1.75 Hz, 1 H), 8.36 - 8.48 (m, 2 H), 7.94 (d, J=8.13 Hz, 1 H), 7.60 - 7.76 (m, 2 H), 7.33 - 7.47 (m, 1 H), 4.12 (d, *J=13.38* Hz, 1 H), 3.53 (d, *J*=13.88 Hz, 1 H), 3.20-3.26 (m, 1 H), 2.85 - 2.95 (m, 1 H), 2.80 - 2.85 (m, 1 H), 2.79 (d, J=4.75 Hz, 3 H), 2.67 - 2.77 (m, 2 H), 2.38 - 2.45 (m, 2 H), 1.24 (d, *J*=5.50 Hz, 3 H).

## Example 49

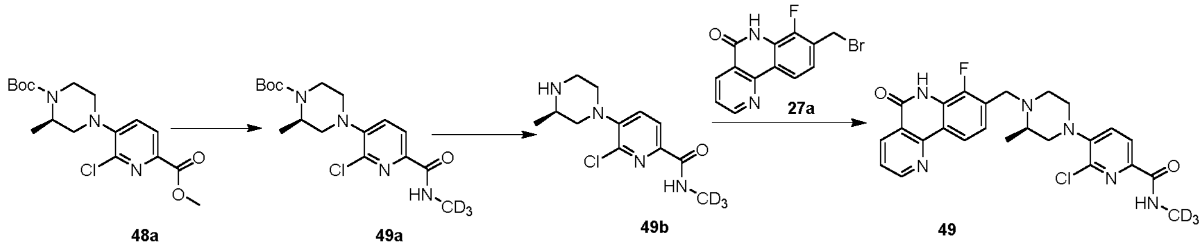


48a 49a 49b 49

Step 1: Synthesis of intermediate **49a**

Deuterated methylamine hydrochloride (190.72 mg, 2.70 mmol) was added to $H_2O$ (1 mL). Sodium tert-butoxide (129.92 mg, 1.35 mmol) was then added thereto. The reaction mixture was stirred at 25°C for 1 hour. A solution of intermediate **48a** (50 mg, 135.19 μmol) in MeOH (2 mL) was then added thereto, and the mixture was heated to 60°C and stirred for 4 hours. The reaction mixture was extracted with ethyl acetate (5 mL × 3). The organic phases were combined, dried over anhydrous sodium sulfate, and filtered. The filtrate was concentrated under reduced pressure to obtain intermediate **49a.** MS m/z: 394.1, 396.1 $[M+23]^+$.

Step 2: Synthesis of hydrochloride of intermediate **49b**

Intermediate **49a** (50 mg, 134.45 μmol) was added to MeOH (2 mL). With stirring, HCl/dioxane (4 M, 383.08 μL) was added thereto, and the reaction mixture was stirred at 25°C for 1 hour. The reaction mixture was concentrated under reduced pressure to obtain the hydrochloride of intermediate **49b.** MS m/z: 272.0, 274.0 $[M+1]^+$.

Step 3: Synthesis of compound **49**

Triethylamine (26.36 mg, 260.49 μmol) was added to a solution of intermediate **49b** (40.14 mg, hydrochloride) in DMF (2 mL). The mixture was stirred at 20°C for 0.5 hours. Intermediate **27a** (50 mg, 130.24 μmol) and KI (2.16 mg, 13.02 μmol) were added to the reaction mixture, and the mixture was stirred at 50°C for 2 hours. The mixture was concentrated under reduced pressure to obtain a crude product. The crude product was purified by preparative TLC (100% EA) to obtain compound **49.** MS m/z: 498.2, 500.2 $[M+1]^+$. $^1H$ NMR (400 MHz, DMSO-$d_6$) δ ppm 11.86 (s, 1 H), 9.08 (dd, J=4.57, 1.69 Hz, 1 H), 8.64 (dd, J=7.94, 1.81 Hz, 1 H), 8.33 - 8.50 (m, 2 H), 7.94 (d, J=8.25 Hz, 1 H), 7.58 - 7.78 (m, 2 H), 7.31 - 7.48 (m, 1 H), 4.12 (d, *J*=13.38 Hz, 1 H), 3.53 (d, J=13.76 Hz, 1 H), 3.24 (s, 1 H), 2.80 - 2.93 (m, 2 H), 2.70 - 2.79 (m, 2 H), 2.43 (t, *J*=9.01 Hz, 2 H), 1.24 (d, *J*=5.50 Hz, 3 H).

**Example 50**

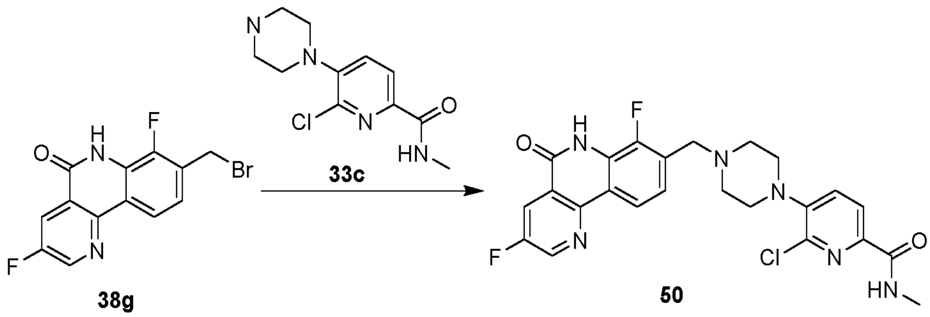


38g

50

Step 1: Synthesis of compound **50**

Triethylamine (31.12 mg, 307.59 μmol) was added to a solution of intermediate **33c** (35.83 mg, hydrochloride) in DMF (1 mL). The mixture was stirred at 25°C for 0.5 hours. Intermediate **38g** (50 mg, 153.80 μmol) and KI (2.55 mg, 15.38 μmol) were then added, and the reaction mixture was stirred at 50°C for 2.5 hours. After cooling, the reaction mixture was added with 1 mL of water, resulting in the precipitation of a solid. The mixture was directly filtered to obtain the filter cake, which was compound **50.** MS m/z: 499.1, 501.1 $[M+1]^+$. $^1H$ NMR (400 MHz, DMSO-$d_6$) δ ppm 12.03 (br s, 1H), 9.13 (d, *J*=2.88 Hz, 1H), 8.39 - 8.46 (m, 2H), 8.36 (d, *J*=8.25 Hz, 1H), 7.93 (d, *J*=8.25 Hz, 1H), 7.66 (d, *J*=8.25 Hz, 1H), 7.38 (dd, *J*=8.00, 6.75 Hz, 1H), 3.76 (s, 2H), 3.08 - 3.16 (m, 4H), 2.79 (d, *J*=4.88 Hz, 3H), 2.63 - 2.69 (m, 4H).

**Example 51**

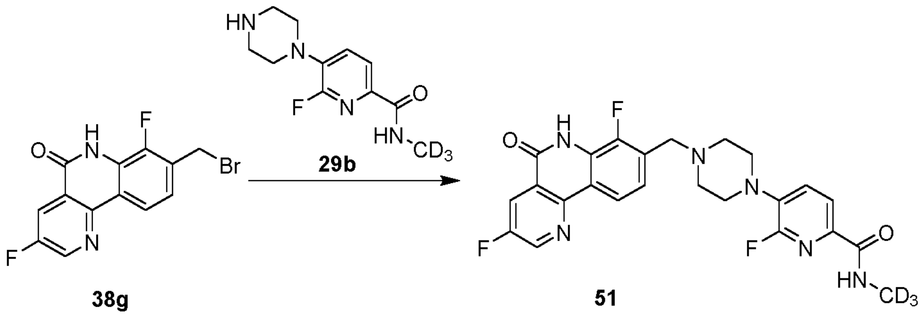


38g

51

Step 1: Synthesis of compound **51**

Triethylamine (18.67 mg, 184.55 μmol) was added to a solution of intermediate **29b** (23.07 mg, hydrochloride) in DMF (1 mL). The mixture was stirred at 25°C for 0.5 hours. Intermediate **38g** (30 mg, 92.28 μmol) and KI (1.53 mg, 9.23 μmol) were then added, and the reaction mixture was stirred at 50°C for 2.5 hours. After cooling to room temperature, the reaction mixture was added with 1 mL of water, resulting in the precipitation of a solid. The mixture was filtered to obtain the filter cake, which was compound **51.** MS m/z: 486.2 $[M+1]^+$. $^1H$ NMR (400 MHz, DMSO-$d_6$) δ ppm 12.05 (s, 1H), 9.13 (d, *J=3.00* Hz, 1H), 8.42 (dd, *J*=8.57, 2.94 Hz, 1H), 8.34 - 8.39 (m, 2H), 7.84 (d, *J*=7.50 Hz, 1H), 7.56 (dd, *J*=10.57, 8.19 Hz, 1H), 7.34 - 7.41 (m, 1H), 3.74 (s, 2H), 3.15 - 3.22 (m, 4H), 2.59 - 2.65 (m, 4H).

**Example 52**

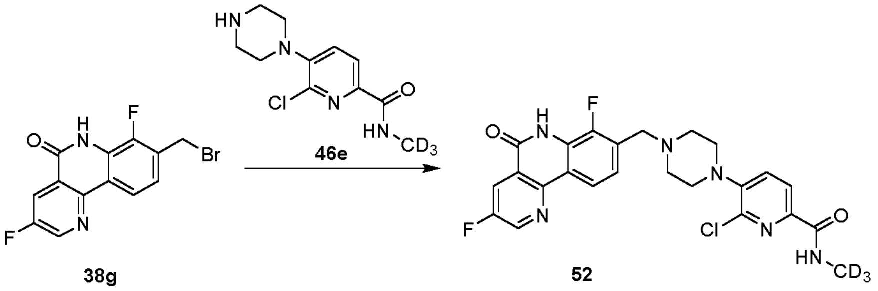


38g 52

Step 1: Synthesis of compound **52**

Intermediate **46e** (21.72 mg, hydrochloride) was dissolved in DMF (1 mL). Triethylamine (37.35 mg, 369.11 μmol) was added, and the reaction mixture was stirred at 25°C for 0.5 hours. KI (7.66 mg, 46.14 μmol) and intermediate **38e** (30 mg, 92.28 μmol) were then added, and the mixture was stirred at 50°C for 2 hours. After cooling to room temperature, the reaction mixture was added with 1 mL of water, resulting in the precipitation of a solid. The mixture was filtered to obtain the filter cake, which was compound **52.** MS m/z: 502.2 $[M+1]^+$. $^1H$ NMR (400 MHz, DMSO-$d_6$) δ ppm 12.06 (s, 1H), 9.14 (d, *J* = 3.0 Hz, 1H), 8.44 - 8.40 (m, 2H), 8.37 (d, *J* = 8.5 Hz, 1H), 7.94 (d, *J* = 8.0 Hz, 1H), 7.66 (d, *J* = 8.3 Hz, 1H), 7.39 (t, *J* = 7.5 Hz, 1H), 3.76 (s, 2H), 3.12 (m, 4H), 2.69 - 2.62 (m, 4H).

**Example 53**

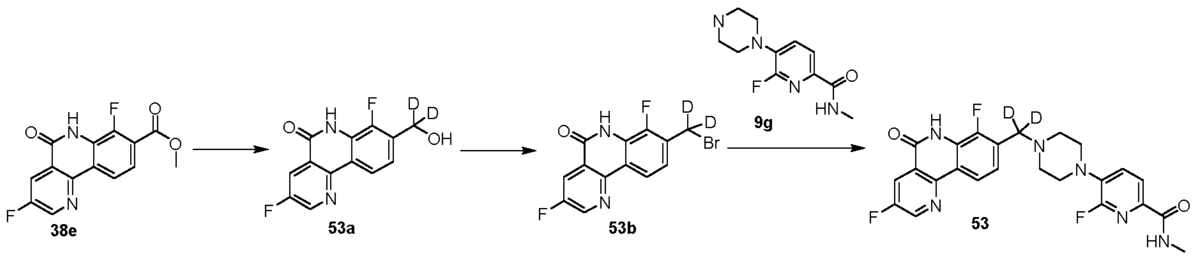


38e 53a 53b 53

Step 1: Synthesis of intermediate **53a**

Intermediate **38e** (150 mg, 516.85 μmol) was dissolved in THF (2 mL). At 0°C, deuterated lithium aluminum hydride powder (29.42 mg, 775.27 μmol) was added thereto, and the mixture was stirred at 0°C for 1 hour. To the reaction mixture, 30 μL of water, 30 μL of 15% sodium hydroxide solution, and 90 μL of ice water were slowly added in sequence to quench the reaction. The reaction mixture was added with water (5 mL) and extracted with ethyl acetate (10 mL). The organic phase was dried over anhydrous sodium sulfate, and filtered. The filtrate was concentrated under reduced pressure at 45°C to obtain intermediate **53a.** MS m/z: 265.1 $[M+1]^+$. $^1H$ NMR (400 MHz, DMSO-$d_6$) δ ppm 9.10 (d, *J* = 2.8 Hz, 1H), 8.34-8.41 (m,2H), 7.36 (t, *J* =8.0 Hz, 1H), 5.38 (s, 1H).

Step 2: Synthesis of intermediate **53b**

Intermediate **53a** (130 mg, 492.01 μmol) was dissolved in DCM (3 mL). 1,2-Dibromotetrachloroethane (352.48 mg, 1.08 mmol) was added, and at 0°C, tributylphosphine (199.09 mg, 984.01 μmol) was added. The mixture was stirred at 25°C for 3 hours. The reaction mixture was concentrated under reduced pressure, and methanol (5 mL) was added thereto. The mixture was stirred for 5 minutes and then filtered to obtain the filter cake, which was intermediate **53b.** $^1H$ NMR (400 MHz, DMSO-$d_6$) δ ppm 12.16 (s, 1H), 9.13 (d, *J* = 2.8 Hz, 1H), 8.41 - 8.43 (m, 1H), 8.34 - 8.37 (m, 1H), 7.44 (t, *J*=8.0 Hz, 1H).

Step 3: Synthesis of compound **53**

Intermediate **9g** (22.68 mg, hydrochloride) was dissolved in DMF (1 mL). Triethylamine (18.56 mg, 183.42 μmol) was added, and the reaction mixture was stirred at 25°C for 0.5 hours. Intermediate **53b** (30 mg, 91.71 μmol) and KI (1.52 mg, 9.17 μmol) were then added. The mixture was stirred at 50°C for 2 hours. The reaction mixture was added with water (1

mL), and stirred for 10 minutes, resulting in the precipitation of a solid. The mixture was filtered to obtain the filter cake, which was compound **53.** MS m/z: 485.1 $[M+1]^+$. $^1H$ NMR (400 MHz, DMSO-$d_6$) δ ppm 12.05 (s, 1H), 9.14 (d, *J*=3.01 Hz, 1H), 8.39 - 8.45 (m, 2H), 8.37 (d, *J*=8.03 Hz, 1H), 7.84 (d, *J*=7.28 Hz, 1H), 7.56 (dd, *J*=10.54, 8.28 Hz, 1H), 7.38 (t, *J*=7.15 Hz, 1H), 3.14 - 3.24 (m, 4H), 2.76 (d, *J*=4.77 Hz, 3H), 2.60 - 2.66 (m, 4H).

**Example 54**

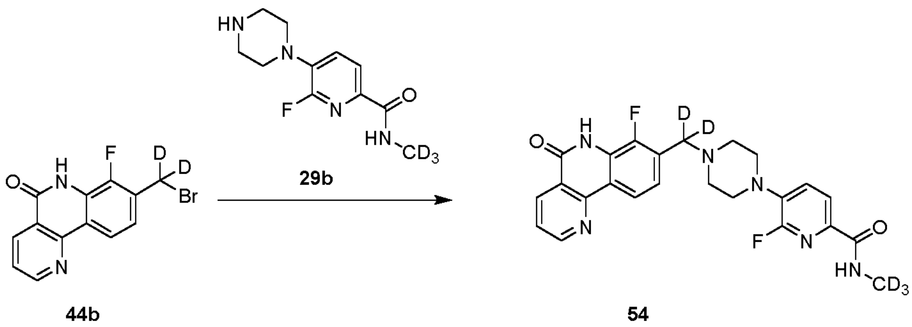

Step 1: Synthesis of compound **54**

Intermediate **29b** (23.87 mg, hydrochloride) was dissolved in DMF (1 mL). Triethylamine (43.49 mg, 429.80 μmol) was added, and the reaction mixture was stirred at 25°C for 0.5 hours. KI (8.92 mg, 53.73 μmol) and intermediate **44b** (33.22 mg, 107.45 μmol) were then added, and the mixture was stirred at 50°C for 1 hour. The reaction mixture was added with 1 mL of water, resulting in the precipitation of a solid. The mixture was filtered to obtain the filter cake, which was compound **54.** MS m/z: 470.2 $[M+1]^+$. $^1H$ NMR (400 MHz, DMSO-$d_6$) δ ppm 11.90 (s, 1H), 9.09 (dd, *J* = 1.8, 4.5 Hz, 1H), 8.64 (dd, *J* = 1.6, 7.9 Hz, 1H), 8.43 (d, *J* = 8.3 Hz, 1H), 8.38 (s, 1H), 7.84 (d, *J* = 8.5 Hz, 1H), 7.72 (dd, *J* = 4.5, 8.0 Hz, 1H), 7.57 (dd, *J* = 8.2, 10.7 Hz, 1H), 7.39 - 7.34 (m, 1H), 3.17-3.21 (m, 4H), 2.59-2.65 (m, 4H).

**Example 55**

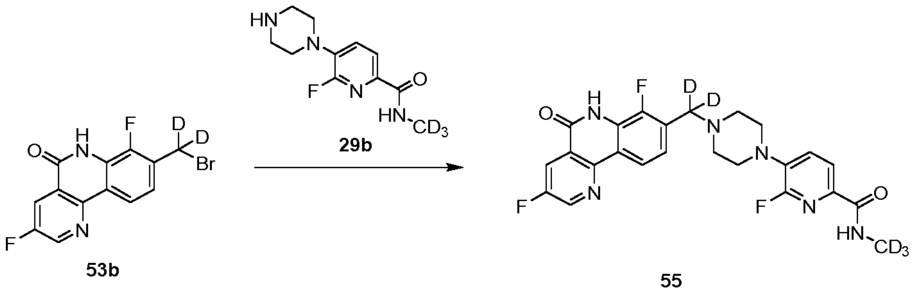

Step 1: Synthesis of compound **55**

Intermediate **29b** (25 mg, hydrochloride) was dissolved in DMF (1 mL). Triethylamine (40.48 mg, 400.05 μmol) was added, and the reaction mixture was stirred at 25°C for 0.5 hours. KI (8.30 mg, 50.01 μmol) and intermediate **53b** (32.72 mg, 100.01 μmol) were then added, and the mixture was stirred at 50°C for 1 hour. After cooling, the reaction mixture was added with 1 mL of water, resulting in the precipitation of a solid. The mixture was filtered to obtain the filter cake, which was compound **55.** MS m/z: 488.2 $[M+1]^+$. $^1H$ NMR (400 MHz, DMSO-$d_6$) δ ppm 12.06 (s, 1H), 9.14 (d, *J* = 2.8 Hz, 1H), 8.42 (dd, *J* = 3.0, 8.5 Hz, 1H), 8.39 - 8.34 (m, 2H), 7.84 (d, *J* = 8.0 Hz, 1H), 7.56 (dd, *J* = 8.0, 10.5 Hz, 1H), 7.38 (dd, *J* = 6.5, 8.3 Hz, 1H), 3.15-3.19 (m, 4H), 2.56-2.64 (m, 4H).

**Bioassay data:**

**Experimental example 1: Experiment on binding activity between compound and PARP1 using fluorescence polarization assay**

**Experimental methods:**

The fluorescence polarization assay was performed in 96-well black-walled plates (Greiner). The reaction buffer consisted of 50 mM Tris (pH 8), 0.001% Triton X100, 10 mM $MgCl_2$, and 150 mM NaCl. Both the fluorescent probe and His-Avi-tagged PARP1 protein were manufactured by WuXi AppTec (Shanghai). In a 100 μL reaction system, 8 nM PARP1, 5 nM fluorescent probe, and the compounds were added respectively. The compound concentration was prepared following the starting concentration and dilution gradient in Table 1. For example, compound 1 started from 100 nM with a 3-fold dilution. The test sample was incubated at room temperature for 4 hours, protected from light. Fluorescence polarization values were measured using an EnVision plate reader. The obtained fluorescence polarization values and compound concentrations were subjected to nonlinear fitting using GraphPad 8.0 software to determine the $IC_{50}$ values.

**Experimental results:**

Table 1. PARP1 binding activity assay for the compounds of the present disclosure

| Compound No. | PARP1 $IC_{50}$ (nM) | Starting concentration and dilution gradient for test compounds |
|---|---|---|
| Trifluoroacetate of compound **1** | 11 | starting concentration at 100 nM, 3-fold dilution gradient |
| Trifluoroacetate of compound **15** | 16 | starting concentration at 50 nM, 2-fold dilution gradient |
| Trifluoroacetate of compound **16** | 13 | starting concentration at 50 nM, 2-fold dilution gradient |
| Trifluoroacetate of compound **19** | 14 | starting concentration at 50 nM, 2-fold dilution gradient |
| Compound **21** | 22 | starting concentration at 50 nM, 2-fold dilution gradient |
| Trifluoroacetate of compound **22** | 14 | starting concentration at 50 nM, 2-fold dilution gradient |
| Compound **29** | 9.7 | starting concentration at 1000 nM, 3-fold dilution gradient |
| Compound **31** | 4.7 | starting concentration at 1000 nM, 3-fold dilution gradient |
| Compound **33** | 4.8 | starting concentration at 1000 nM, 3-fold dilution gradient |
| Compound **38** | 6.8 | starting concentration at 1000 nM, 3-fold dilution gradient |
| Compound **44** | 10 | starting concentration at 1000 nM, 3-fold dilution gradient |
| Compound **46** | 6.8 | starting concentration at 1000 nM, 3-fold dilution gradient |
| Compound **50** | 7.9 | starting concentration at 1000 nM, 3-fold dilution gradient |
| Compound **51** | 5.2 | starting concentration at 100 nM, 3-fold dilution gradient |
| Compound **52** | 5.5 | starting concentration at 100 nM, 3-fold dilution gradient |
| Compound **53** | 4.5 | starting concentration at 100 nM, 3-fold dilution gradient |
| Compound **54** | 6.0 | starting concentration at 100 nM, 3-fold dilution gradient |
| Compound **55** | 5.2 | starting concentration at 100 nM, 3-fold dilution gradient |

Conclusion: The compounds of the present disclosure exhibit excellent binding activity to PARP1.

**Experimental example 2: Experiment on binding activity between compound and PARP2 using fluorescence polarization assay**

**Experimental methods:**

The fluorescence polarization assay was performed in 96-well black-walled plates (Greiner). The reaction buffer

consisted of 50 mM Tris (pH 8), 0.001% Triton X100, 10 mM $MgCl_2$, and 150 mM NaCl. Both the fluorescent probe and His-Avi-tagged PARP2 protein were manufactured by WuXi AppTec (Shanghai). In a 100 μL reaction system, 3 nM PARP2, 5 nM fluorescent probe, and the compounds were added respectively. The compound concentration was prepared following the starting concentration and dilution gradient in Table 2. The test sample was incubated at room temperature for 4 hours, protected from light. Fluorescence polarization values were measured using an EnVision plate reader. The obtained fluorescence polarization values and compound concentrations were subjected to nonlinear fitting using GraphPad 8.0 software to determine the $IC_{50}$ values.

**Experimental results:**

Table 2. PARP2 binding activity assay for the compounds of the present disclosure

| Compound No. | PARP2 $IC_{50}$ (μM) | Starting concentration and dilution gradient for test compounds |
|---|---|---|
| Trifluoroacetate of compound **1** | >20 | starting concentration at 20 μM, 3-fold dilution gradient |
| Trifluoroacetate of compound **15** | >50 | starting concentration at 50 μM, 3-fold dilution gradient |
| Trifluoroacetate of compound **16** | >50 | starting concentration at 50 μM, 3-fold dilution gradient |
| Trifluoroacetate of compound **19** | >50 | starting concentration at 50 μM, 3-fold dilution gradient |
| Compound **21** | >50 | starting concentration at 50 μM, 3-fold dilution gradient |
| Trifluoroacetate of compound **22** | >50 | starting concentration at 50 μM, 3-fold dilution gradient |
| Compound **29** | >20 | starting concentration at 20 μM, 3-fold dilution gradient |
| Compound **31** | >20 | starting concentration at 20 μM, 3-fold dilution gradient |
| Compound **33** | >20 | starting concentration at 20 μM, 3-fold dilution gradient |
| Compound **38** | >20 | starting concentration at 20 μM, 3-fold dilution gradient |
| Compound **44** | >20 | starting concentration at 20 μM, 3-fold dilution gradient |
| Compound **46** | >20 | starting concentration at 20 μM, 3-fold dilution gradient |
| Compound **50** | >20 | starting concentration at 20 μM, 3-fold dilution gradient |

Conclusion: The compounds of the present disclosure exhibit relatively weak binding activity to PARP2.

**Experimental example 3: Anti-proliferation assay of compounds on MDA-MB-436 cells (BRCA1 mutation)**

**Experimental methods:**

MDA-MB-436 cells (BRCA1 mutation) were seeded in black 96-well plates with clear bottoms. Each well contained 135 μL of cell suspension with 3,500 MDA-MB-436 cells (BRCA1 mutation). The cell plate was incubated overnight in a $CO_2$ incubator. A 400X stock solution of the test compound was prepared. The test compounds were 5-fold diluted with a multi-channel pipette to the 9th concentration, i.e., diluted from 4 mM to 104 nM, and duplicate experiment was set. 78 μL of medium was added to an intermediate plate. Subsequently, 2 μL of the serially diluted compound per well was transferred to the corresponding wells of the intermediate plate. 2 μL of DMSO was added to the vehicle control and blank control. After mixing well, 15 μL of the mixture per well was then transferred to the cell plate. Concentrations of compounds transferred to the cell plate ranged from 10 μM to 0.26 nM, with a final DMSO concentration of 0.25%. The cell plate was incubated for 7 days in a $CO_2$ incubator. The cell plate was removed and equilibrated to room temperature for 30 minutes. 75 μL of chemiluminescent cell viability assay reagent was added to each well. The cell plate was shaken on an orbital shaker for 3 minutes to induce cell lysis, followed by 10 minutes of incubation at room temperature to stabilize the luminescent signal. Luminescent signals were detected using a 2104 EnVision plate reader. The inhibition rate (IR) of the test compounds was calculated using the following formula: IR (%) = (1 - (RLU compound - RLU blank control) / (RLU vehicle control - RLU blank control)) × 100% The inhibition rates of compounds at different concentrations were calculated in Excel, and the inhibition curves were plotted and related parameters were calculated using GraphPad Prism software.

**Experimental results:** As shown in Table 3:

Table 3. Proliferation inhibition assay on MDA-MB-436 cells (BRCA1 mutation)

| Compound No. | $IC_{50}$ (nM) |
|---|---|
| Trifluoroacetate of compound **5** | 1.2 |
| Trifluoroacetate of compound **15** | 1.58 |
| Trifluoroacetate of compound **22** | 5.73 |
| Compound **27** | 0.69 |
| Compound **28** | 0.37 |
| Compound **29** | 1.14 |
| Compound **31** | 0.75 |
| Compound **33** | 0.41 |
| Compound **37** | 4.54 |
| Compound **38** | 0.56 |
| Compound **41** | 2.92 |
| Compound **44** | 0.65 |
| Compound **45** | 0.27 |
| Compound **46** | 0.36 |
| Compound **50** | 0.41 |

**Experimental conclusion:** The compounds of the present disclosure exhibit excellent proliferation inhibitory activity against MDA-MB-436 cells (BRCA1 mutation).

**Experimental example 4: Anti-proliferation assay of compounds on DLD1 cells (BRCA2 KO)**

**Experimental methods:**

DLD1 (BRCA2 KO) cells were seeded in white a 96-well plate with 80 μL of cell suspension per well, containing 1,000 DLD1 (BRCA2 KO) cells per well. The cell plate was incubated overnight in a $CO_2$ incubator. The test compounds were 5-fold diluted with a multi-channel pipette to the 8th concentration, i.e., diluted from 2 mM to 0.0256 μM, and duplicate experiment was set. 78 μL of medium was added to an intermediate plate. Subsequently, 2 μL of the serially diluted compound per well was transferred to the corresponding wells of the intermediate plate and mixed well. 20 μL of the mixture per well was then transferred to the cell plate. The concentration of the compound transferred to the cell plate ranged from 10 μM to 0.128 nM. The cell plate was incubated for 7 days in a $CO_2$ incubator. An additional cell plate was prepared to read the signal value on the day of compound addition, which served as the maximum value (Max in the following equation) for data analysis. The cell plate was added with 25 μL of chemiluminescent cell viability assay reagent per well and incubated at room temperature for 10 minutes to stabilize the luminescent signal. A multimode microplate reader was used for reading. After the incubation of the cell plate with the compound, the cell plate was added with 25 μL of chemiluminescent cell viability assay reagent per well and incubated at room temperature for 10 minutes to stabilize the luminescent signal. A multimode microplate reader was used for reading. Using the equation (Sample - Min) / (Max - Min) × 100% to convert the raw data into inhibition rate, the $IC_{50}$ value can be obtained by curve fitting with four parameters (obtained by "log (inhibitor) vs. response -- Variable slope" mode in GraphPad Prism).

**Experimental results:** as shown in Table 4.

Table 4. Anti-proliferation assay on DLD1 (BRCA2 KO) cells

| Compound No. | $IC_{50}$ (nM) |
|---|---|
| Trifluoroacetate of compound **15** | 1.71 |
| Trifluoroacetate of compound **16** | 13.05 |
| Trifluoroacetate of compound **19** | 24.18 |
| Compound **21** | 10.01 |
| Trifluoroacetate of compound **22** | 4.43 |

**Experimental conclusion:** The compounds of the present disclosure exhibit good antiproliferative inhibitory effects on DLD1 (BRCA2 KO) cells.

**Experimental example 5: *In vivo* pharmacokinetic evaluation and brain penetration evaluation of the compounds of the present disclosure in mice**

**Experimental methods:**

The trifluoroacetate of compound **16** was evaluated using female BALB/C mice, and the trifluoroacetate of compound **15** was evaluated using male CD-1 mice. A clear solution of the test compound (0.4 mg/mL) in 10% 2-hydroxypropyl-β-cyclodextrin and 90% water was administered to the mice (fasted overnight, aged 7 to 9 weeks) via tail vein injection at a dose of 2 mg/kg. A clear solution of the test compound (1 mg/mL) in 0.5% methylcellulose was administered to the mice (fasted overnight, aged 7 to 9 weeks) via oral gavage at a dose of 10 mg/kg. Blood samples (about 30 μL) were collected from the two groups of animals at the following time points: from the jugular vein at 0.0833, 0.25, 0.5, 1.0, 2.0, 4.0, 8.0, and 24 hours post-administration, and from the tail vein at 0.25, 0.5, 1.0, 2.0, 4.0, 6.0, 8.0, and 24 hours post-administration. The samples were placed in anticoagulant tubes containing EDTA-K2, followed by centrifugation to separate the plasma. For the trifluoroacetate of compound **15,** one group received intravenous administration (IV, 2 mg/kg) with blood samples collected at the above time points, and another group received oral administration (PO, 10 mg/kg) with blood samples collected at the above time points. Additionally, for the oral administration group (PO, 10 mg/kg), plasma and brain tissue homogenate samples were collected at the 2-hour time point. Plasma concentrations were determined by LC-MS/MS method, and relevant pharmacokinetic parameters were calculated using WinNonlin™ Version 6.3 (Pharsight, Mountain View, CA) pharmacokinetic software with non-compartmental model linear-log trapezoidal method.

**Experimental results:** The pharmacokinetic data of mice are shown in Table 5, and the brain penetration data are shown in Table 6.

Table 5. Pharmacokinetic data of mice

| | Trifluoroacetate of compound **16** | | Trifluoroacetate of compound **15** | |
|---|---|---|---|---|
| | **IV 2 mg/kg** | **PO 10 mg/kg** | **IV 2 mg/kg** | **PO 10 mg/kg** |
| $C_0$ (nM) or $C_{max}$ (nM) | 14017 | 42673 | 4210 | 13700 |
| $T_{max}$ (h) | - | 0.5 | - | 0.25 |
| $T_{1/2}$ (h) | 0.25 | 2 | 3.7 | 4.1 |
| $Vd_{ss}$ (L/kg) | 1.54 | - | 2.1 | - |
| Cl (mL/min/kg) | 4.0 | - | 7.7 | - |
| $T_{last}$ (h) | 24.0 | 24.0 | 24.0 | 24.0 |
| $AUC_{0-last}$ (nM.h) | 17915 | 173382 | 9230 | 61600 |

Note: "-" indicates that the parameter could not be calculated. $C_0$ represents initial concentration; $C_{max}$ represents peak concentration; $T_{max}$ represents time to reach peak concentration; $T_{1/2}$ represents elimination half-life; $Vd_{ss}$ represents apparent volume of distribution at steady state; Cl represents total clearance; $T_{last}$ represents time point of the last quantifiable drug concentration; $AUC_{0-last}$ represents area under the plasma concentration-time curve from time 0 to the last quantifiable time point.

Table 6. Brain penetration data of the compound of the present disclosure in mice

| | **PO 10 mg/kg Drug concentrations at the 2-hour time point** | | |
|---|---|---|---|
| | $C_{2h}$(nM) Plasma drug concentration | $C_{2h}$(nM) Brain drug concentration | Brain-to-plasma drug concentration ratio |
| Trifluoroacetate of compound **15** | 8467 | 5814 | 0.7 |

**Experimental conclusion:** The compounds of the present disclosure exhibit excellent metabolic stability *in vivo* and excellent drug exposure following oral administration. Oral administration result in superior drug concentration in brain tissue, with a high brain-to-plasma concentration ratio.

**Experimental example 6: Permeability evaluation of the compounds of the present disclosure**

**Cell line:**

This experiment used the MDR1-MDCK II cell line, authorized by the Piet Borst Laboratory at the Netherlands Cancer Institute, as the *in vitro* model for permeability evaluation experiment. Cells were seeded at a density of $2.3 \times 10^5$ cells/cm$^2$ in a Transwell-96 well plate. After being cultured for 4 to 7 days in a $CO_2$ incubator, the cells were used for transport experiments.

**Experimental conditions:**

Test compound concentration: 2.00 μM;
Testing directions and replicates: Bidirectional (A (apical) -B (basolateral) and B-A), with 2 replicates;
Transport buffer (TB): HBSS solution containing 10 mM HEPES (pH 7.40 ± 0.05)
Incubation conditions: 37 ± 1°C, 5% $CO_2$, incubated for 150 minutes
Control compounds: Nadolol and Metoprolol were used as control compounds for low and high permeability, respectively. Digoxin was used as a substrate for P-glycoprotein. The administration concentration of nadolol and metoprolol was 2.00 μM, and that of digoxin was 10.0 μM.

Monolayer cell membrane integrity test:

After the transport experiment, the integrity of the MDR1-MDCK II cell layer was assessed using the Lucifer Yellow Rejection Assay. The remaining solution in the apical and basolateral wells was removed, followed by the addition of 75 μL of TB containing 100 μM Lucifer Yellow to the apical wells and 250 μL of TB to the basolateral wells. The cell plates were incubated in a cell incubator at 37 ± 1°C, 5% $CO_2$, and saturated humidity for 30 minutes. After incubation, 20 μL of the sample was collected from the apical side and mixed with 60 μL of TB, while 80 μL of the sample was collected from the basolateral side. The relative fluorescence intensity (RFU) was measured using a microplate reader at 425/528 nm (excitation/emission).

**Sample analysis:**

In this experiment, sample analysis for the test compounds and the control compounds-nadolol, metoprolol, and digoxin-was conducted using liquid chromatographytandem mass spectrometry (LC-MS/MS). The retention time, chromatogram acquisition, and chromatogram integration of the analytes and internal standards were processed using Analyst software (Sciex, Framingham, MA, USA). The semi-quantitative determination of the sample analysis was performed by calculating the ratio of the peak area of the analyte to that of the internal standard.

**Data analysis:**

$$P_{app} = \frac{V_R}{Area \times Time} \times \frac{[drug]_{receiver}}{[drug]_{initial,\ donor}} = \frac{V_R}{Area \times Time} \times$$

$$Efflux\ Ratio = \frac{P_{app}(B\ to\ A)}{P_{app}(A\ to\ B)}$$

$$\%Solution\ Recovery = \frac{C_R \times V_R + C_D \times V_D}{C_0 \times V_D} \times 100$$

$V_R$ represents the volume of the solution at the receiving side (0.075 mL for the apical side and 0.25 mL for the basolateral side). Area represents the relative surface area of the cell monolayer (0.0804 $cm^2$). Time represents the incubation time (9000 s). $C_0$ represents the peak area ratio of the compound on the donor side. $V_D$ is the volume on the donor side (0.075 mL for the apical side and 0.25 mL for the basolateral side). $C_D$ and $C_R$ represent the peak area ratios of the compound on the donor and receiving sides, respectively.

The permeability of Lucifer Yellow (%Lucifer Yellow) was calculated using the following formula:

$$\%\ Lucifer\ Yellow = \frac{V_{Basolateral} \times RFU_{Basolateral}}{V_{Apical} \times RFU_{Apical} + V_{Basolateral} \times RFU_{Basolateral}} \times 100$$

$RFU_{Apical}$ and $RFU_{Basolateral}$ are the relative fluorescence intensities of Lucifer Yellow on the apical and basolateral sides, respectively. $V_{Apical}$ and $V_{Basolateral}$ are the loading volumes on the apical and basolateral sides, which are 0.0750 mL and 0.250 mL, respectively.

**Experimental results:**

The permeability test results of the compounds of the present disclosure on the MDR1-MDCK II cell line are summarized in Table 7.

Table 7: Summary of permeability evaluation of the compounds in the present disclosure

| Compound | Permeability $P_{app}$ ($10^{-6}$ cm/s) | | Efflux ratio | Permeability conclusion |
|---|---|---|---|---|
| | A to B | B to A | | |
| Trifluoroacetate of compound **15** | 25.2 | 17.7 | 0.703 | High permeability |
| Compound **33** | 10.6 | 7.2 | 0.679 | High permeability |

Note: low permeability: $P_{app} \leq 1.0$ ($\times 10^{-6}$ cm/s); moderate permeability: $1.0 < P_{app} < 5.5$ ($\times 10^{-6}$ cm/s); high permeability: $P_{app} \geq 5.5$ ($\times 10^{-6}$ cm/s).

**Conclusion:** The compounds of the present disclosure demonstrate excellent membrane permeability in the cell membrane permeability study.

**Experimental example 7: Pharmacokinetic evaluation and brain penetration evaluation of the compounds of the present disclosure in rats**

**Experimental methods:**

A 0.2 mg/mL clear solution of the test compound in 10% 2-hydroxypropyl-β-cyclodextrin and 90% water was administered to male SD rats (fasted overnight, 200 to 230 g) via tail vein injection at a dose of 1 mg/kg. A 0.5 mg/mL solution of the test compound in 10% 2-hydroxypropyl-β-cyclodextrin and 90% water was administered to male SD rats (fasted overnight, 200 to 230 g) via oral gavage at a dose of 5 mg/kg. Blood samples (about 30 μL) were collected from the two groups of animals at the following time points: from the jugular vein at 0.0833, 0.25, 0.5, 1.0, 2.0, 4.0, 8.0, and 24 hours

post-administration, and from the tail vein at 0.25, 0.5, 1.0, 2.0, 4.0, 8.0, and 24 hours post-administration. The samples were placed in anticoagulant tubes containing EDTA-K2, followed by centrifugation to separate the plasma. For the trifluoroacetate of compound 15, one group received intravenous administration (IV, 1 mg/kg) with blood samples collected at the above time points, and another group received oral administration (PO, 5 mg/kg) with blood samples collected at the above time points. Additionally, for the oral administration group (PO, 5 mg/kg), plasma and brain tissue homogenate samples were collected at the 2-hour time point. Plasma concentrations were determined by LC-MS/MS method, and relevant pharmacokinetic parameters were calculated using WinNonlin™ Version 6.3 (Pharsight, Mountain View, CA) pharmacokinetic software with non-compartmental model linear-log trapezoidal method.

**Experimental results:** The pharmacokinetic data of rats are shown in Table 8, and the brain penetration data are shown in Table 9.

Table 8. Pharmacokinetic data of rats

| | Trifluoroacetate of compound **15** | |
|---|---|---|
| | **IV 1 mg/kg** | **PO 5 mg/kg** |
| $C_0$ (nM) or $C_{max}$ (nM) | 2120 | 2830 |
| $T_{max}$ (h) | - | 4 |
| $T_{1/2}$ (h) | 5.2 | 3.3 |
| $Vd_{ss}$ (L/kg) | 2.1 | - |
| Cl (mL/min/kg) | 6.0 | - |
| $T_{last}$ (h) | 24.0 | 24.0 |
| $AUC_{0-last}$ (nM.h) | 5820 | 22900 |
| F (%) | | 78% |

Note: "-" indicates that the parameter could not be calculated; $C_0$ represents initial concentration; $C_{max}$ represents peak concentration; $T_{max}$ represents time to reach peak concentration; $T_{1/2}$ represents elimination half-life; $Vd_{ss}$ represents apparent volume of distribution at steady state; Cl represents total clearance; $T_{last}$ represents time point of the last quantifiable drug concentration; $AUC_{0-last}$ represents area under the plasma concentration-time curve from time 0 to the last quantifiable time point; F(%) represents bioavailability, calculated using $AUC_{0-last}$.

Table 9. Brain penetration data of the compound of the present disclosure in rats

| | **PO 5 mg/kg Drug concentrations at the 2-hour time point** | | |
|---|---|---|---|
| | $C_{2h}$(nM) Plasma drug concentration | $C_{2h}$(nM) Brain drug concentration | Brain-to-plasma drug concentration ratio |
| Trifluoroacetate of compound **15** | 8467 | 5814 | 0.7 |

**Experimental conclusion:** The compounds of the present disclosure exhibit excellent metabolic stability *in vivo* and excellent drug exposure following oral administration. Oral administration result in superior drug concentration in brain tissue, with a high brain-to-plasma concentration ratio.

**Experimental example 8: *In vitro* liver microsomal stability of the compound of the present disclosure**

**Experimental objective:** To investigate the metabolic stability of the compound of the present disclosure in humans through *in vitro* metabolic stability testing using human liver microsomes.

**Experimental conditions:** The compound (1 μM) was incubated with human liver microsomes supplemented with an NADPH regeneration system at 37°C for a specified duration, lasting up to 60 minutes. The concentration of the compound in the samples was determined by LC-MS/MS.

**Experimental process:** The incubation was performed using 96-well incubation plates, with individual plates labeled as T0, T5, T15, T30, T45, T60, Blank60, and NCF60. The reaction time points corresponding to the incubation plates were set at 0, 5, 15, 30, 45, and 60 minutes. In the Blank60 plate, no test compound or control compound was added, and the sample was collected after 60 minutes of incubation. In the NCF60 plate, potassium phosphate buffer was used in place of the NADPH regeneration system and incubated for 60 minutes. All time point samples were taken from individual

wells.

For the T0, T5, T15, T30, T45, T60, and NCF60 plates, 5 μL of either the test or control compound working solution and 100 μL of microsomal working solution (with a liver microsomal protein concentration of 0.5 mg/mL) were added. Only microsomal working solution was added to the Blank60 well. In the Blank60 plate, only the microsomal working solution was added. Then, the incubation plates, except for T0 and NCF60, including Blank60, T5, T15, T30, T45, and T60, were pre-incubated in a 37°C water bath for approximately 10 minutes.

For samples in the T0 plate, 180 μL of stop solution (where the stop solution was a solution of acetonitrile and methanol (95:5, V/V) containing 100 ng/mL tolbutamide) was added before adding the NADPH regeneration system working solution.

50 μL of potassium phosphate buffer was added to each well of the NCF60 plate and incubated for 60 minutes.

After the pre-incubation of Blank60, T5, T15, T30, T45, and T60 incubation plates, 44 μL of NADPH regeneration system working solution was added to each sample well to initiate the reaction. Therefore, for sample wells containing either the test compound or the control compound working solutions, the final reaction concentration was 1 μM, with the concentration of liver microsomes at 0.5 mg/mL. The final concentrations of DMSO and acetonitrile in the reaction system were 0.01% (v/v) and 0.99% (v/v), respectively.

After incubation for an appropriate period (e.g., 5, 15, 30, 45, and 60 minutes), 180 μL of stop solution was added to each test compound and control compound sample well in the Blank60, T5, T15, T30, T45, T60, and NCF60 plates to stop the reaction.

All sample plates were shaken thoroughly and centrifuged for 10 minutes. A volume of 80 μL of the supernatant from each test compound was diluted to 240 μL with a solution of acetonitrile (1:9, v/v) containing 0.1% formic acid for LC-MS/MS analysis.

The half-life ($T_{1/2}$) and clearance ($CL_{int(liver)}$) of the compound in human liver microsomes were determined by calculating the percentage of the compound remaining at each corresponding time point.

$$C_t = C_0 \bullet e^{-k_e \bullet t}$$

when

$$C_t = \frac{1}{2} C_0 ,$$

$$T_{1/2} = \frac{Ln2}{k_e} = \frac{0.693}{k_e}$$

$$CL_{int(mic)} = \frac{0.693}{\text{In vitro } T_{1/2}} \bullet \frac{1}{\text{mg / mL microsomal protein in reaction system}}$$

$$CL_{int(liver)} = CL_{int(mic)} \bullet \frac{\text{mg microsomes}}{\text{g liver}} \bullet \frac{\text{g liver}}{\text{kg body weight}}$$

**Experimental results:** As shown in Table 10.

Table 10: *In vitro* metabolic stability study results of the compounds of the present disclosure in human and mouse liver microsomes

| | Human liver microsomes | | Mouse liver microsomes | |
|---|---|---|---|---|
| | $CL_{int(liver)}$ (mL/min/kg) | $T_{1/2}$ (minutes) | $CL_{int(liver)}$ (mL/min/kg) | $T_{1/2}$ (minutes) |
| Trifluoroacetate of compound **1** | <8.6 | >145 | <38 | >145 |

(continued)

| | Human liver microsomes | | Mouse liver microsomes | |
|---|---|---|---|---|
| | $CL_{int(liver)}$ (mL/min/kg) | $T_{1/2}$ (minutes) | $CL_{int(liver)}$ (mL/min/kg) | $T_{1/2}$ (minutes) |
| Compound **15** | <8.6 | >145 | 74 | 77 |
| Compound **27** | 9.5 | 132 | NA | NA |
| Compound **29** | NA | NA | <38 | >145 |
| Compound **31** | <8.6 | >145 | NA | NA |
| Compound **37** | NA | NA | 64 | 85 |
| NA: Not tested | | | | |

**Conclusion:** The compounds of the present disclosure demonstrate outstanding stability in both human and mouse liver microsomes *in vitro.*

**Experimental example 9: *In vivo* pharmacodynamic study of the compounds of the present disclosure in a human breast cancer MDA-MB-436 cell subcutaneous xenograft tumor model using BALB/c nude mice**

**Experimental objective:**

To investigate the inhibitory effect of the compound of the present disclosure on the *in vivo* growth of tumors in a human breast cancer MDA-MB-436 xenograft model in nude mice.

**Experimental methods:**

1) Cell culture:

Routine cell culture was performed under 5% $CO_2$ at 37°C in MEM medium containing 10% fetal bovine serum. The cells were passaged using 0.25% trypsin digestion. Depending on the growth condition of the cells, the cells were passaged two to three times per week at a ratio of 1:3 to 1:6.

2) Preparation of animal model

Animals: Female BALB/c nude mice, 6 to 8 weeks of age.

MDA-MB-436 cells in the logarithmic growth phase were collected, counted, and then resuspended in a mixture of 50% serum-free MEM medium and 50% Matrigel to adjust the cell concentration to $5.0 \times 10^7$ cells/mL. After dispersing the cells evenly with a pipette, the suspension was transferred into a 50 mL centrifuge tube, which was placed in an icebox. Using a 1 mL syringe, the cell suspension was injected subcutaneously into the right axilla of each nude mouse, with 200 μL ($1.1 \times 10^7$ cells/mouse) injected per animal, establishing the MDA-MB-436 nude mouse xenograft model. After inoculation, the animals were regularly observed for their condition and tumor growth. Tumor diameters were measured using an electronic caliper, and the data were directly input into an Excel spreadsheet to calculate tumor volume. When the tumor volume reached 100 to 200 $mm^3$, healthy animals with similar tumor sizes were selected and grouped (n = 6) based on a randomized block design, while ensuring that the average body weight of each group remained consistent. The day of grouping was considered as the first day of the experiment (D1). After the experiment commenced, the tumor diameter was measured twice weekly, and tumor volume was calculated, while the body weight of the animals was weighed and recorded.

The formula used for calculating tumor volume (TV) is as follows: TV ($mm^3$) = $a \times w^2/2$; where a represents the long diameter of the tumor (mm) and w represents the short diameter of the tumor (mm).

3) Dose: 1.0 mg/kg; Administration route: Oral gavage (p.o.), once daily (QD)

4) Data recording

The formula for calculating Relative Tumor Volume (RTV) is as follows: RTV = TVt/TVinitial; where TVinitial is the tumor volume measured at the time of grouping and dosing, and TVt is the tumor volume measured at each time point

during administration.

The formula for calculating the Relative Tumor Proliferation Rate (%T/C) is: %T/C =100% × (RTVT/RTVC); where RTVT represents the RTV of the treatment group, and RTVC represents the RTV of the vehicle control group.

The formula for calculating Tumor Growth Inhibition (TGI) is: TGI =100% × [1 - (TVt(T)- TVinitial(T))/(TVt(C)-TVinitial(C))]; where TVt(T) is the tumor volume of the treatment group at each measurement, TVinitial(T) is the tumor volume of the treatment group at the time of grouping and administration, TVt(C) is the tumor volume of the vehicle group at each measurement, and TVinitial(C) is the tumor volume of the vehicle group at the time of grouping and administration.

**Experimental results:** Tumor growth volumes are shown in FIG. 1. The body weight changes of mice during the administration are shown in FIG. 2. Tumor growth inhibition rates and relative tumor proliferation rates are shown in Table 11.

Table 11. Summary of tumor growth inhibition rate and relative tumor proliferation rate in MDA-MB-436

| | Dose (p.o.) | TGI (%) (tumor growth inhibition rate) D24 | T/C (%) (relative tumor proliferation rate) D24 | p-value[1] |
|---|---|---|---|---|
| Trifluoroacetate of compound **15** | 1.0 mg/kg | 104% | 14% | **** |
| [1]: Comparisons between the two groups were analyzed by T-test. A p-value < 0.05 was considered statistically significant: *p < 0.05, **p < 0.01, ***p < 0.001, ****p < 0.0001. | | | | |

**Experimental conclusion:** The compounds of the present disclosure exhibit significant anti-tumor activity.

## Claims

1. A compound of formula (XII) or a pharmaceutically acceptable salt thereof,

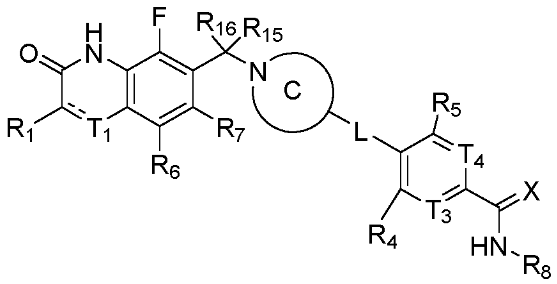

**(XII)**

,

wherein

X is selected from O and S;
the structural moiety

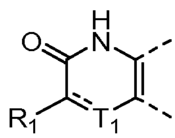

is selected from

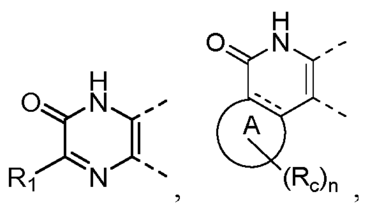

and

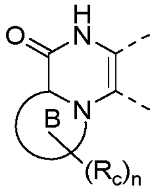

;

ring A is selected from phenyl and 6-membered heteroaryl;
ring B is selected from 6-membered heteroaryl;
L is selected from a single bond, and ring C is selected from

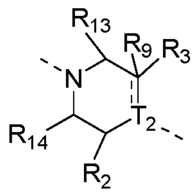

;

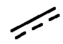

is selected from a single bond or a double bond;
$T_2$ is selected from C, N, and CH;
$T_3$ and $T_4$ are each independently selected from N and $CR_{10}$;
$R_1$ is selected from $C_{1-3}$ alkyl and $C_{3-5}$ cycloalkyl, and the $C_{1-3}$ alkyl and $C_{3-5}$ cycloalkyl are each independently and optionally substituted by 1, 2, or 3 halogens;
$R_2$ is selected from H and $C_{1-3}$ alkyl, and the $C_{1-3}$ alkyl is optionally substituted by 1, 2, or 3 halogens;
$R_3$ is selected from H and halogen;
$R_9$ is absent, or selected from H and halogen;
$R_4$ is selected from H, halogen, CN, $C_{1-3}$ alkyl, and $C_{1-3}$ alkoxy;
$R_5$ is selected from H, halogen, CN, $C_{1-3}$ alkyl, and $C_{1-3}$ alkoxy;
$R_6$ and $R_7$ are each independently selected from H, halogen, $C_{1-3}$ alkyl, and $C_{1-3}$ alkoxy, and the $C_{1-3}$ alkyl and $C_{1-3}$ alkoxy are each independently and optionally substituted by 1, 2, or 3 halogens;
$R_8$ is selected from $C_{1-3}$ alkyl and $C_{3-5}$ cycloalkyl, and the $C_{1-3}$ alkyl and $C_{3-5}$ cycloalkyl are each independently and optionally substituted by 1, 2, or 3 R;
$R_{10}$ is selected from H and halogen;
$R_{13}$ and $R_{14}$ are each independently selected from H, halogen, $C_{1-3}$ alkyl, $C_{1-3}$ alkoxy, and $C_{3-5}$ cycloalkyl, and the $C_{1-3}$ alkyl, $C_{1-3}$ alkoxy, and $C_{3-5}$ cycloalkyl are each independently and optionally substituted by 1, 2, or 3 halogens;
$R_{15}$ and $R_{16}$ are each independently selected from H, D, $C_{1-3}$ alkyl, and $C_{1-3}$ alkoxy, and the $C_{1-3}$ alkyl and $C_{1-3}$ alkoxy are each independently and optionally substituted by 1, 2, or 3 halogens;
or, $R_2$ and $R_4$ form a ring, so that the structural moiety

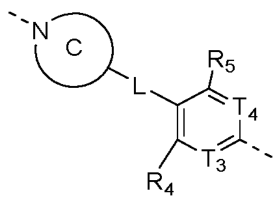

is selected from

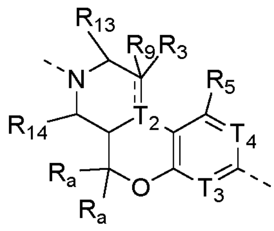 and 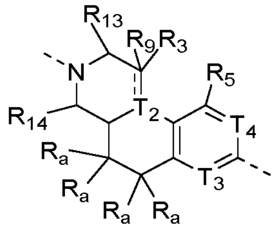 ;

or, $R_3$ and $R_5$ form a ring, so that the structural moiety

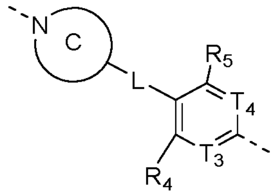

is selected from

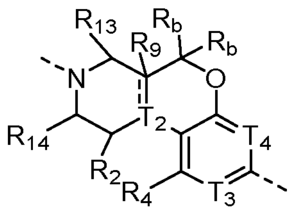 and 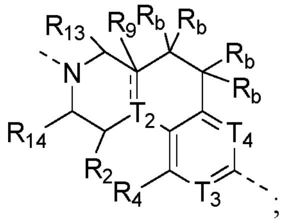 ;

each $R_a$ is independently selected from H, halogen, $C_{1-3}$ alkyl, $C_{1-3}$ alkoxy, and $C_{3-5}$ cycloalkyl, and the $C_{1-3}$ alkyl, $C_{1-3}$ alkoxy, and $C_{3-5}$ cycloalkyl are optionally substituted by 1, 2, or 3 halogens;
or, two $R_a$ on adjacent atoms, together with the atom to which they are attached, form a double bond or a cyclopropyl group;
each $R_b$ is independently selected from H, halogen, $C_{1-3}$ alkyl, $C_{1-3}$ alkoxy, and $C_{3-5}$ cycloalkyl, and the $C_{1-3}$ alkyl, $C_{1-3}$ alkoxy, and $C_{3-5}$ cycloalkyl are optionally substituted by 1, 2, or 3 halogens;
each $R_c$ is independently selected from H, halogen, $C_{1-3}$ alkyl, $C_{1-3}$ alkoxy, and $C_{3-5}$ cycloalkyl, and the $C_{1-3}$ alkyl, $C_{1-3}$ alkoxy, and $C_{3-5}$ cycloalkyl are optionally substituted by 1, 2, or 3 halogens;
each R is independently selected from halogen and D;
n is selected from 0, 1, 2, 3, and 4;
given that when the structural moiety

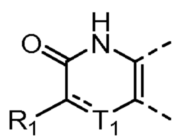

is selected from

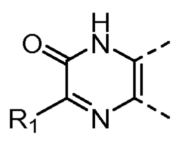 ,

L is selected from a single bond, and ring C is selected from

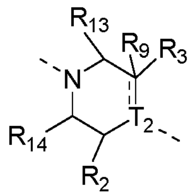

, then $R_2$ and $R_4$ form a ring, or $R_3$ and $R_5$ form a ring;
the halogen represents F, Cl, Br, and I atoms;
"hetero" in the 5- to 6-membered heteroaryl represents 1, 2, 3, or 4 heteroatoms or heteroatom groups, each independently selected from -O-, -S-, and -N-.

2. The compound or the pharmaceutically acceptable salt thereof according to claim 1, wherein the compound has a structure of formula (XII-1),

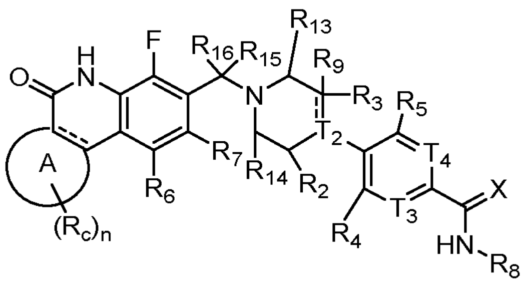

**(XII-1)** ,

wherein

ring A is selected from 6-membered heteroaryl;
$R_2$, $R_3$, $R_4$, $R_5$, $R_6$, $R_7$, $R_8$, $R_9$, $R_{13}$, $R_{14}$, $R_{15}$, $R_{16}$, $R_c$, X, $T_2$, $T_3$, $T_4$, n, and

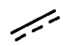

are as defined in claim 1.

3. The compound or the pharmaceutically acceptable salt thereof according to claim 1, wherein X is selected from O.

4. The compound or the pharmaceutically acceptable salt thereof according to claim 1, wherein each $R_a$, each $R_b$, and each $R_c$ are independently selected from H, F, and $CH_3$.

5. The compound or the pharmaceutically acceptable salt thereof according to claim 1, wherein $T_3$ is selected from N, CH, and CF; or $T_3$ is selected from N.

6. The compound or the pharmaceutically acceptable salt thereof according to claim 1, wherein $T_4$ is selected from N and CH.

7. The compound or the pharmaceutically acceptable salt thereof according to claim 1, wherein $R_1$ is selected from $CH_3$ and $CH_2CH_3$; or $R_1$ is selected from $CH_3$.

8. The compound or the pharmaceutically acceptable salt thereof according to claim 1, wherein $R_2$ is selected from H and $CH_3$.

9. The compound or the pharmaceutically acceptable salt thereof according to claim 1, wherein $R_6$ and $R_7$ are each independently selected from H, F, and $CH_3$; or $R_6$ and $R_7$ are each independently selected from H and F.

10. The compound or the pharmaceutically acceptable salt thereof according to claim 1, wherein $R_4$ and $R_5$ are each

independently selected from H, F, Cl, CN, and $CH_3$.

**11.** The compound or the pharmaceutically acceptable salt thereof according to claim 1, wherein $R_8$ is selected from $CH_3$, $CH_2CH_3$, $CH_2CF_3$, cyclopropyl, and $CD_3$.

**12.** The compound or the pharmaceutically acceptable salt thereof according to claim 1, wherein $R_3$ and $R_9$ are each independently selected from H and F.

**13.** The compound or the pharmaceutically acceptable salt thereof according to claim 1, wherein $R_{10}$ is selected from H and F.

**14.** The compound or the pharmaceutically acceptable salt thereof according to claim 1, wherein $R_{13}$ and $R_{14}$ are each independently selected from H and $CH_3$.

**15.** The compound or the pharmaceutically acceptable salt thereof according to claim 1, wherein $R_{15}$ and $R_{16}$ are each independently selected from H, D, and $CH_3$.

**16.** The compound or the pharmaceutically acceptable salt thereof according to claim 1, wherein ring A is selected from phenyl, pyridyl, pyrazinyl, and pyrimidinyl.

**17.** The compound or the pharmaceutically acceptable salt thereof according to claim 1, wherein ring B is selected from pyridyl, pyrazinyl, and pyrimidinyl.

**18.** The compound or the pharmaceutically acceptable salt thereof according to claim 1, wherein the structural moiety

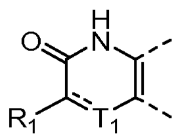

is selected from

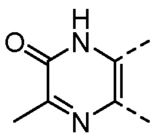, 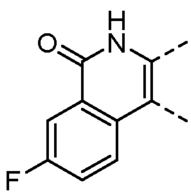,

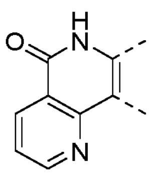, 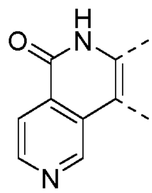, 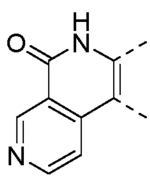, and 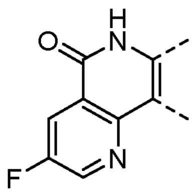.

**19.** The compound or the pharmaceutically acceptable salt thereof according to claim 1, wherein the structural moiety

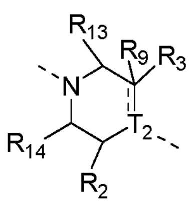

is selected from

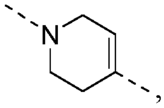, 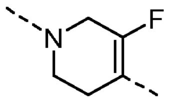,

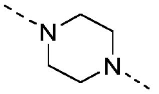, 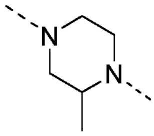, 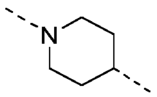, 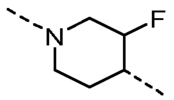, 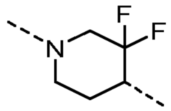, 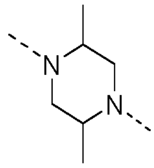,

and

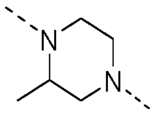.

**20.** The compound or the pharmaceutically acceptable salt thereof according to claim 1, wherein the structural moiety

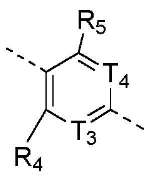

is selected from

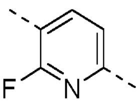, 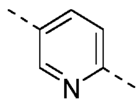,

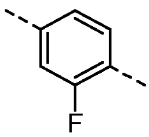, 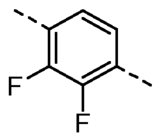, 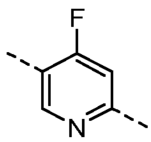, 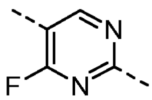, 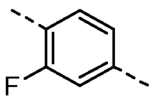, 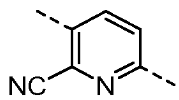,

and

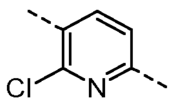.

**21.** The compound or the pharmaceutically acceptable salt thereof according to claim 1, wherein L is selected from a single bond, and the structural moiety

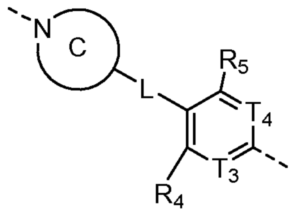 is

selected from

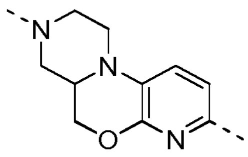 , 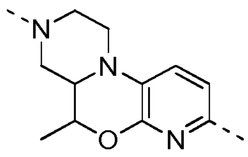 , 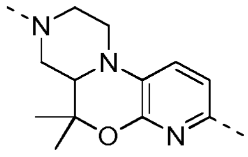 ,

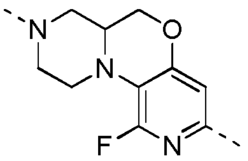 , 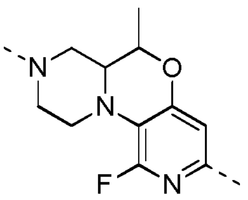 , 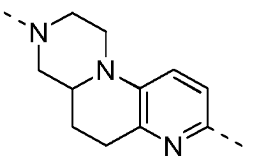 , 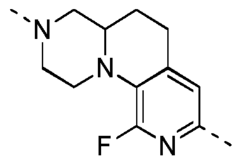 ,

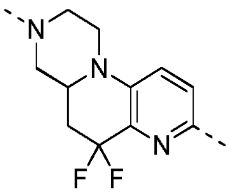 , 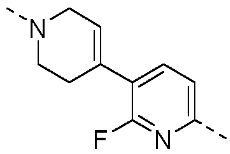 , 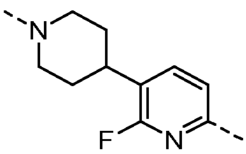 , 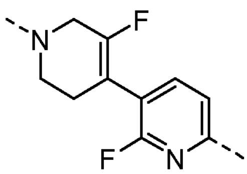 ,

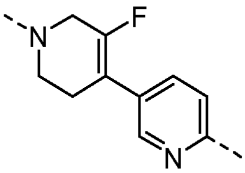 , 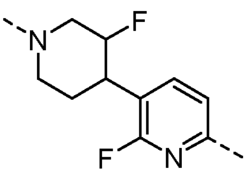 , 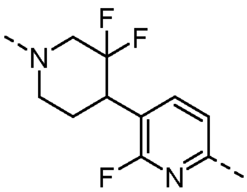 , 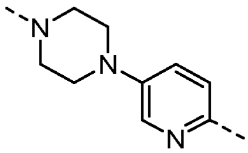 ,

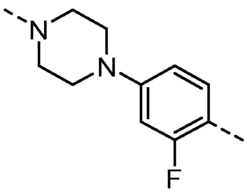 , 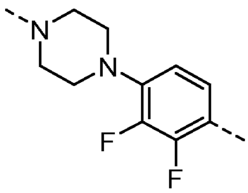 , 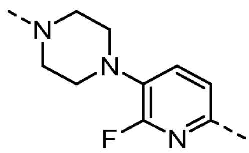 , 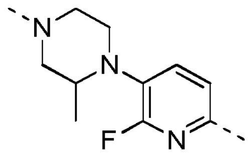 ,

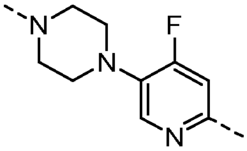 , 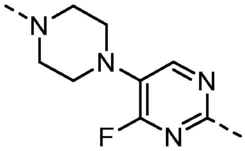 , 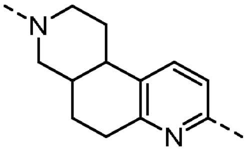 , 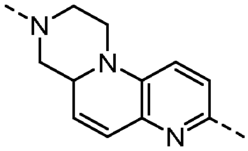 ,

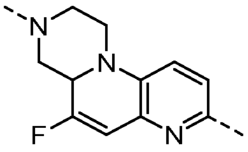, 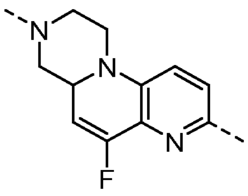, 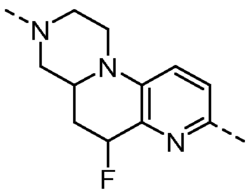, 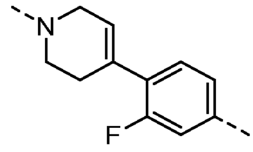,

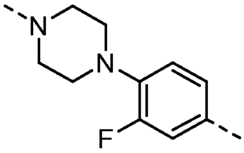, 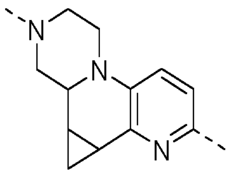, 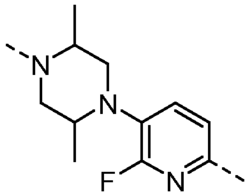, 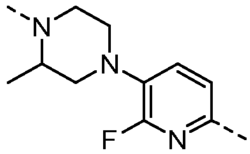,

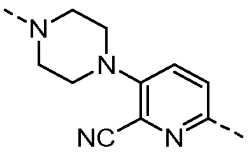, 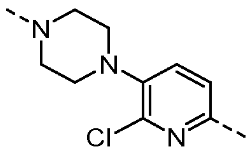, 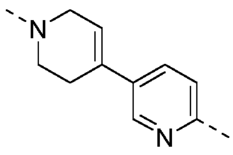, 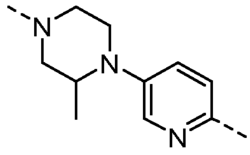,

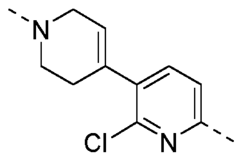, and 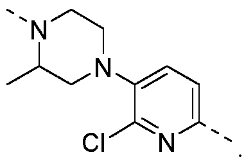.

**22.** The compound or the pharmaceutically acceptable salt thereof according to claim 1, wherein the compound has a structure of formula (P-1),

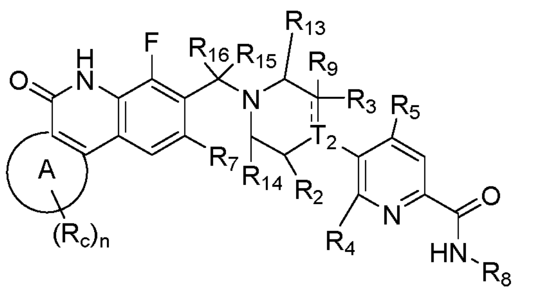

**(P-1)**

,

wherein ring A is selected from pyridyl;

$R_2$, $R_3$, $R_4$, $R_5$, $R_7$, $R_8$, $R_9$, $R_{13}$, $R_{14}$, $R_{15}$, $R_{16}$, $R_c$, $T_2$, n, and 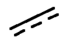 are as defined in claim 1.

**23.** The compound or the pharmaceutically acceptable salt thereof according to claim 1, wherein the compound has a structure of formula (P-2),

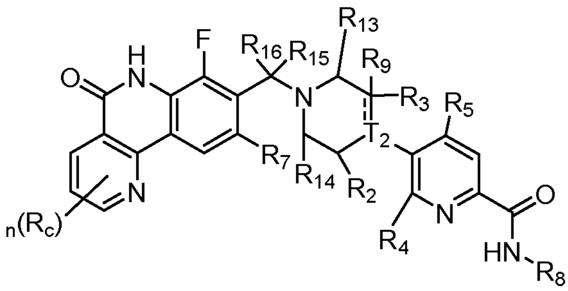

**(P-2)**

,

wherein

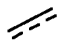

is selected from a single bond or a double bond;

$T_2$ is selected from C and N;

$R_2$ is selected from H and $C_{1-3}$ alkyl, and the $C_{1-3}$ alkyl is optionally substituted by 1, 2, or 3 halogens;

$R_3$ is selected from H and halogen;

$R_4$ is selected from H, halogen, CN, $C_{1-3}$ alkyl, and $C_{1-3}$ alkoxy;

$R_5$ is selected from H, halogen, CN, $C_{1-3}$ alkyl, and $C_{1-3}$ alkoxy;

$R_9$ is absent, or selected from H and halogen;

$R_7$, $R_8$, $R_{13}$, $R_{14}$, $R_{15}$, $R_{16}$, $R_c$, and n are as defined in claim 1.

**24.** A compound as shown below or a pharmaceutically acceptable salt thereof,

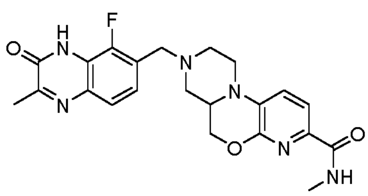 , 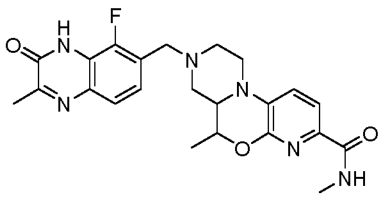 , 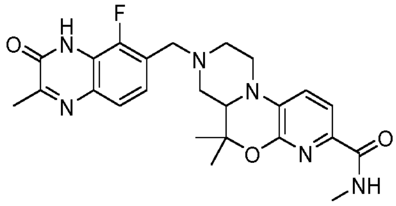 ,

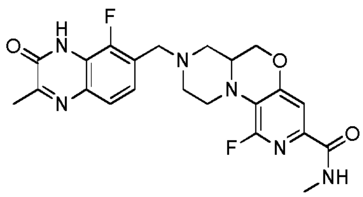 , 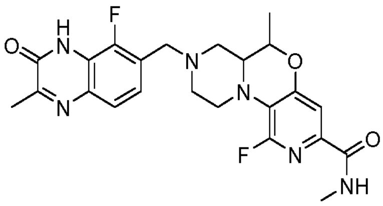 , 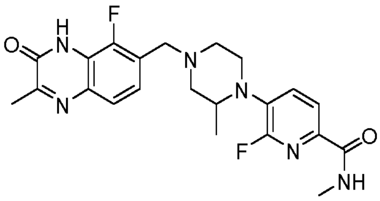 ,

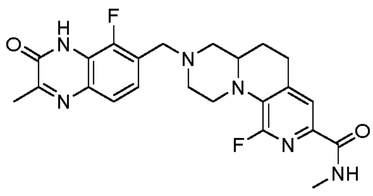 , 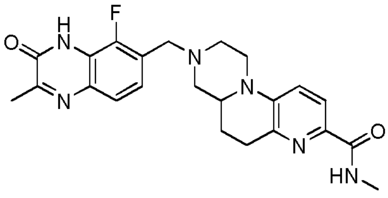 , 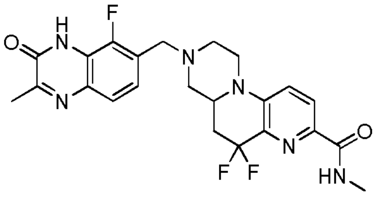 ,

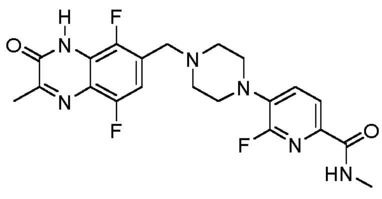 ,
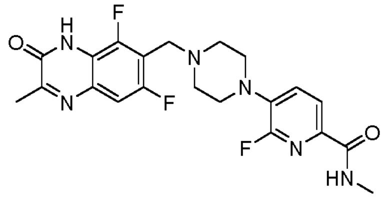 ,
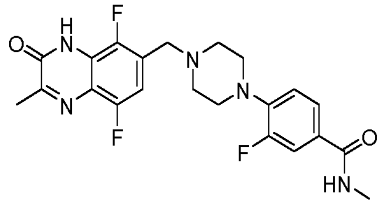 ,

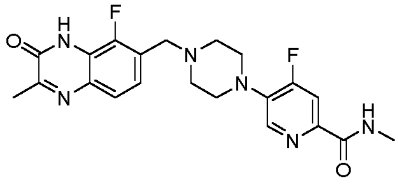 ,
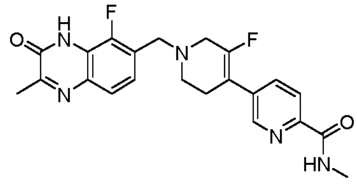 ,
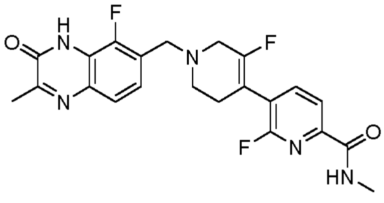 ,

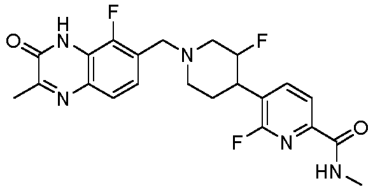 ,
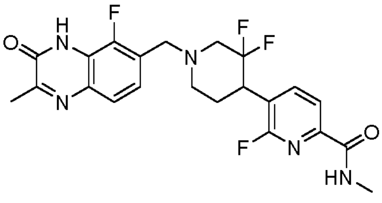 ,
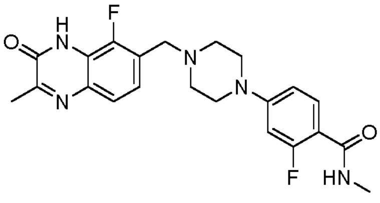 ,

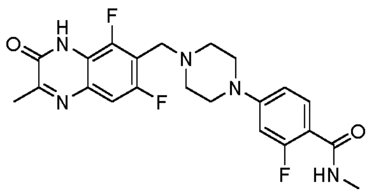 ,
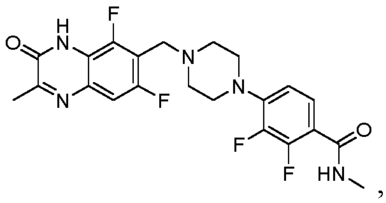 ,
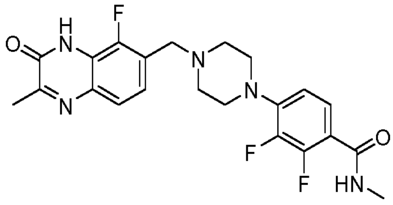 ,

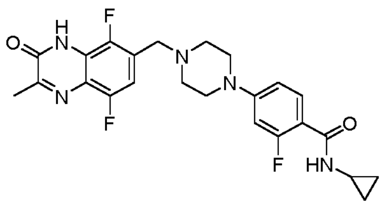 ,
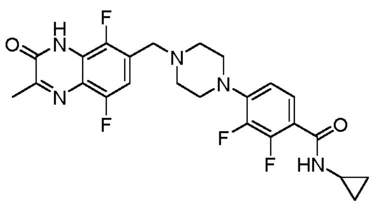 ,
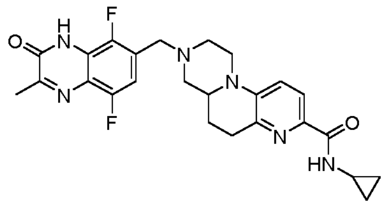 ,

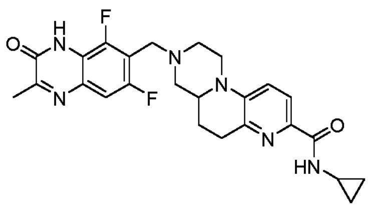 ,
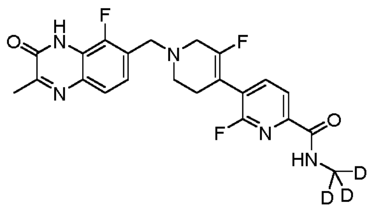 ,
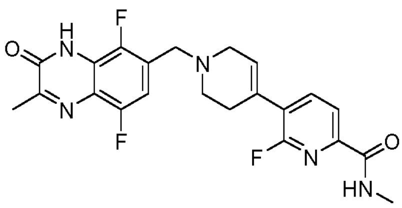 ,

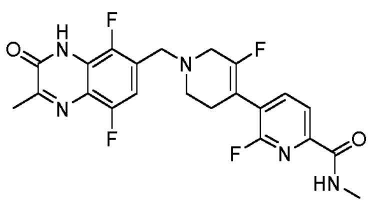 ,
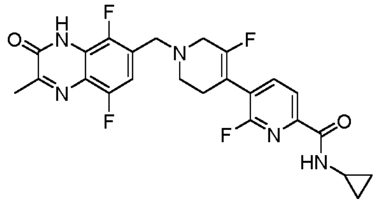 ,
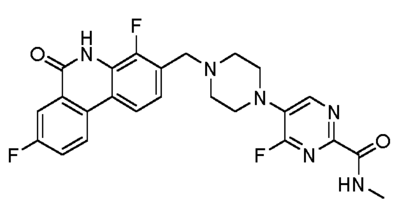 ,

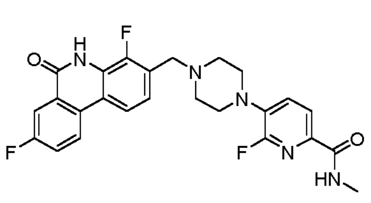 , 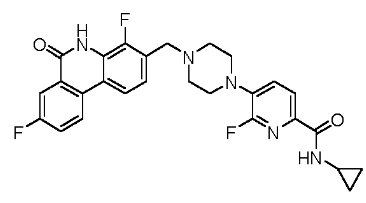 , 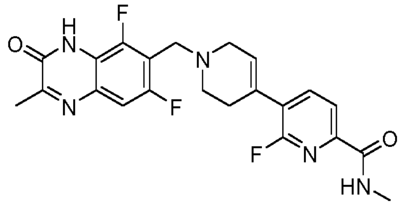 ,

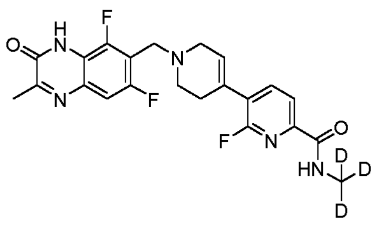 , 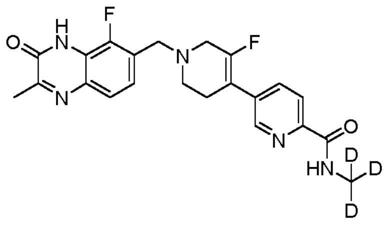 , 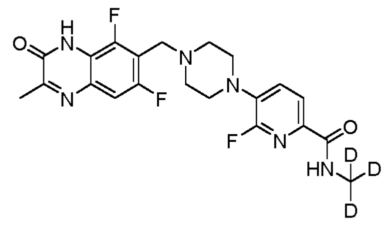 ,

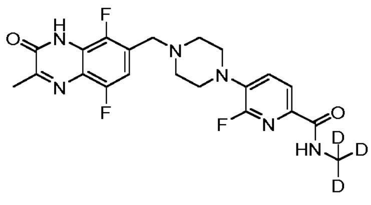 , 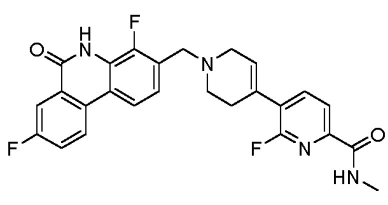 , 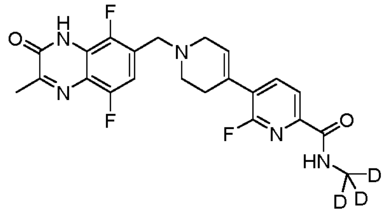 ,

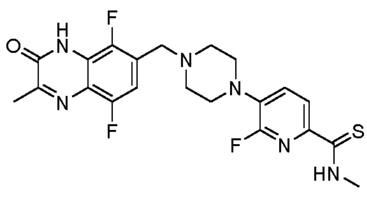 , 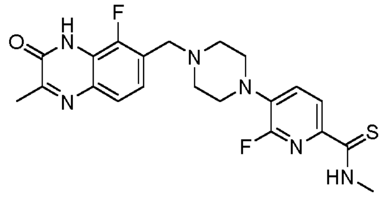 , 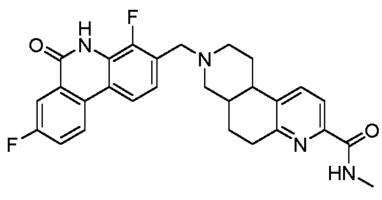 ,

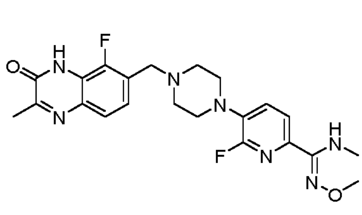 , 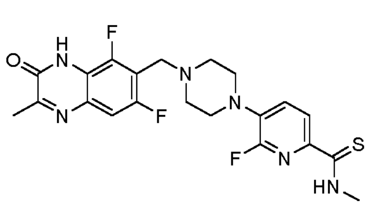 , 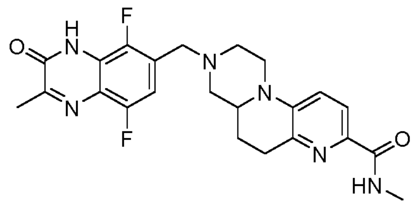 ,

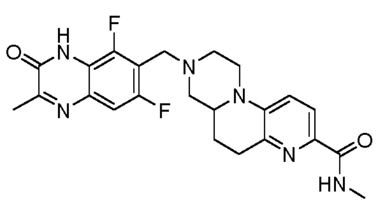 , 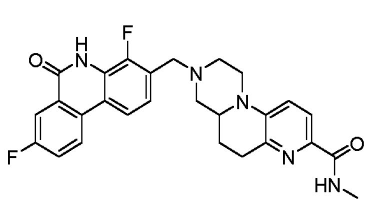 , 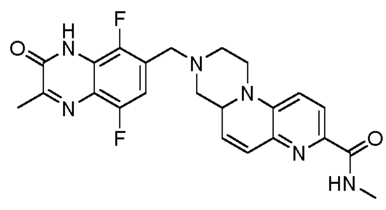 ,

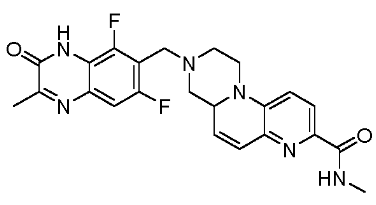 , 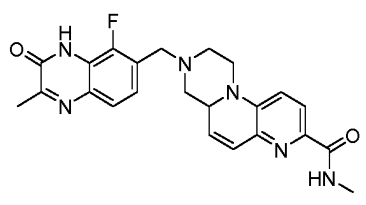 , 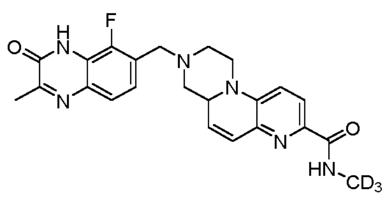 ,

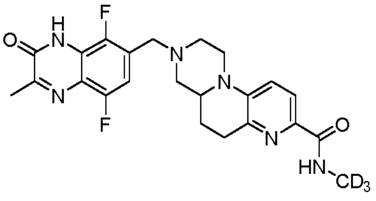 , 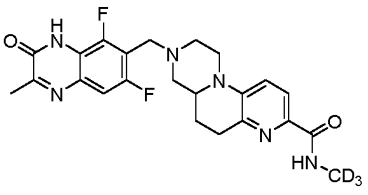 , 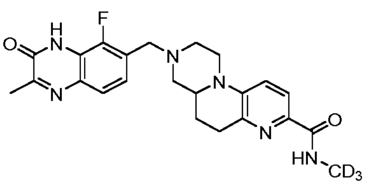 ,

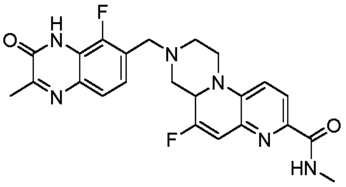 , 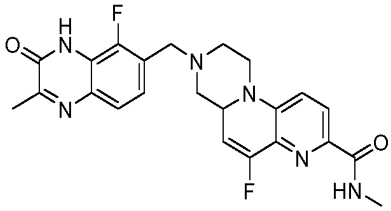 , 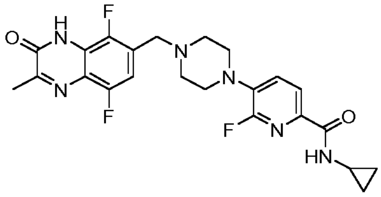 ,

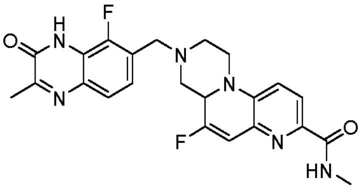 , 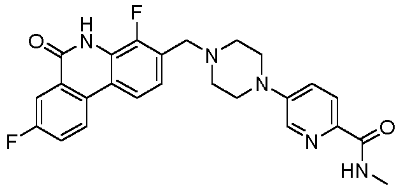 , 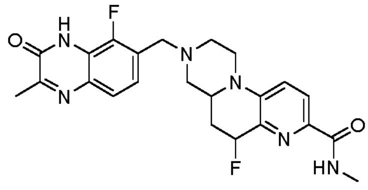 ,

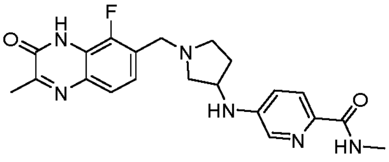 , 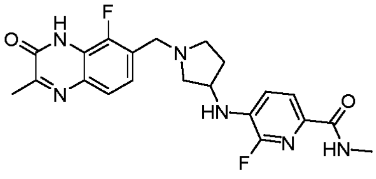 , 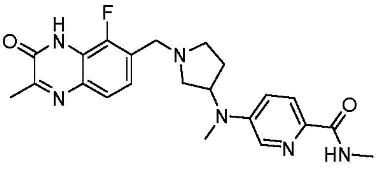 ,

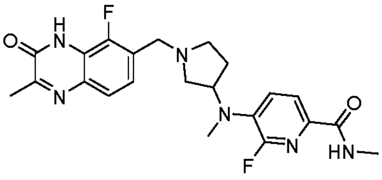 , 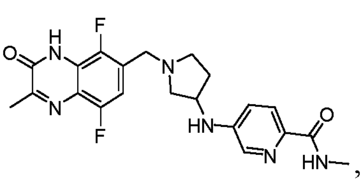 , 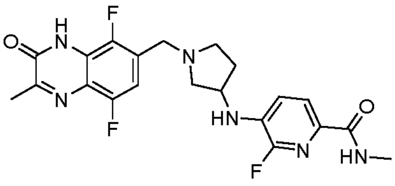 ,

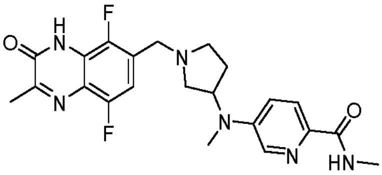 , 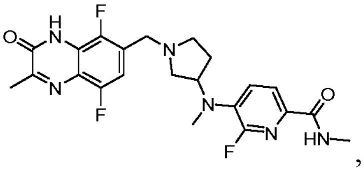 , 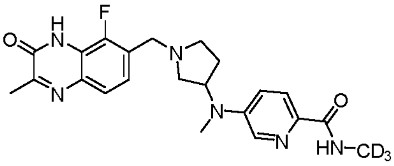 ,

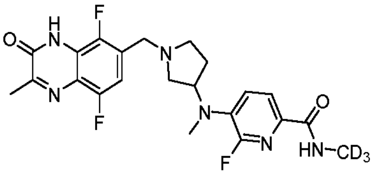 , 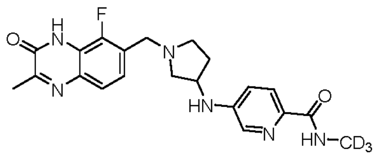 , 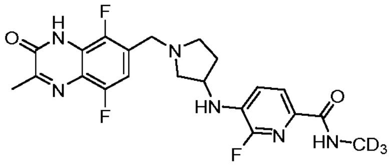 ,

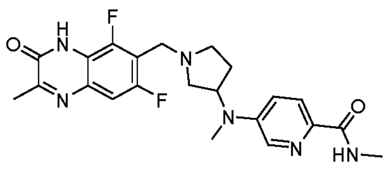, 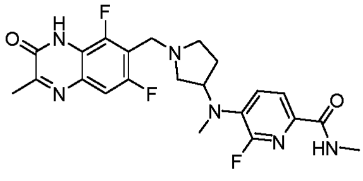, 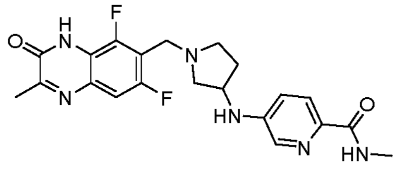,

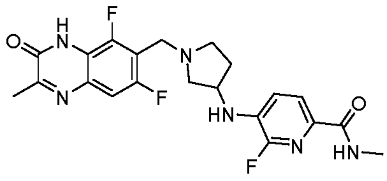, 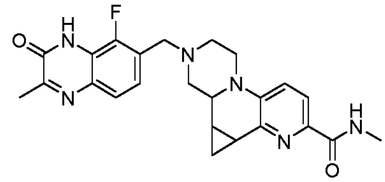, 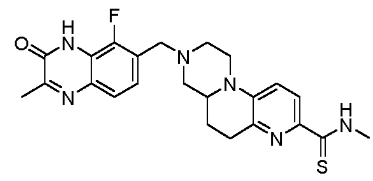,

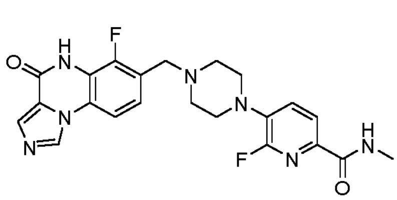, 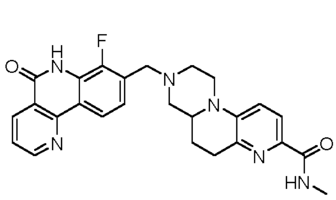, 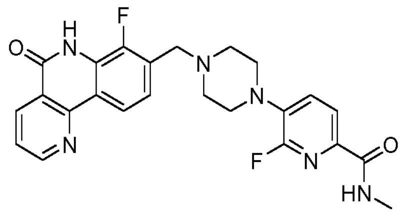,

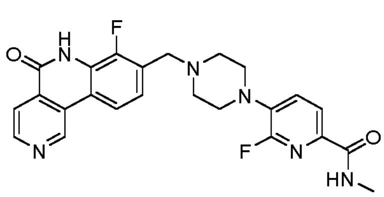, 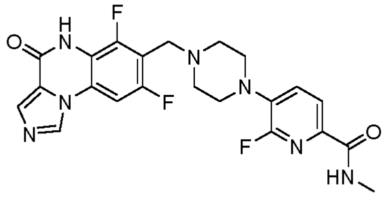, 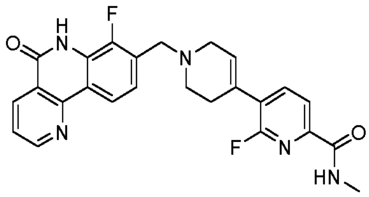,

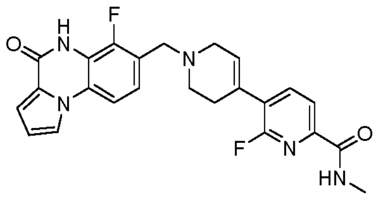, 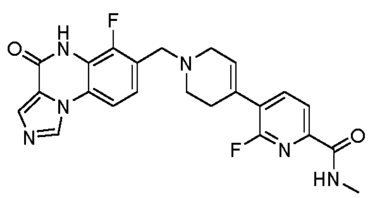, 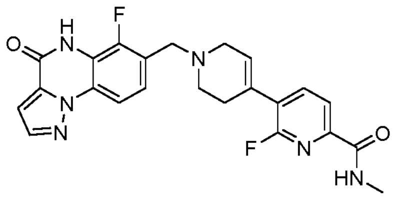,

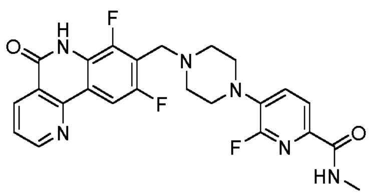, 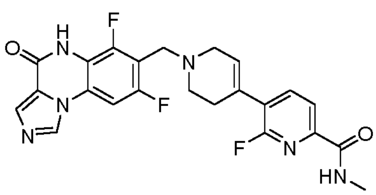, 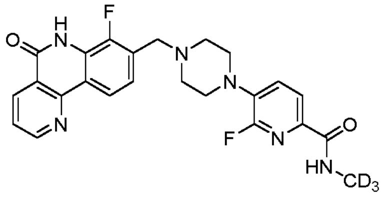,

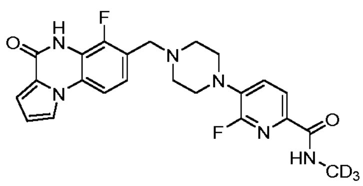, 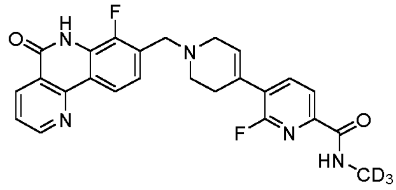, 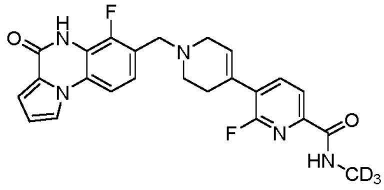,

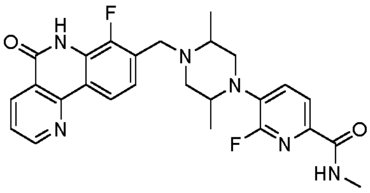 ,
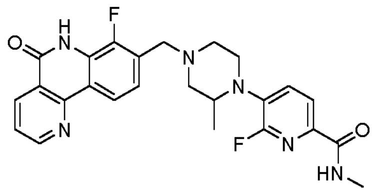 ,
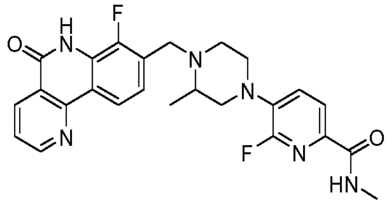 ,

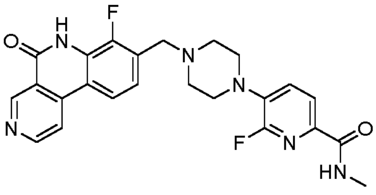 ,
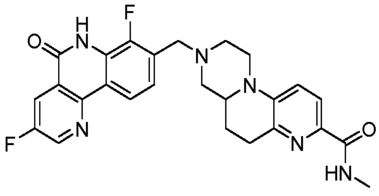 ,
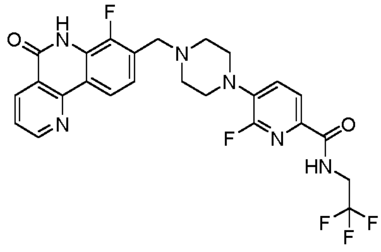 ,

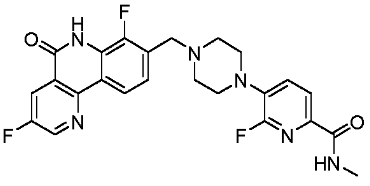 ,
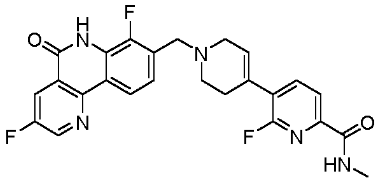 ,
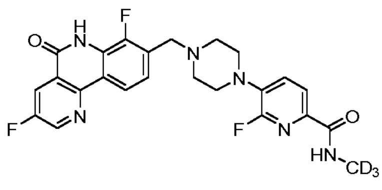 ,

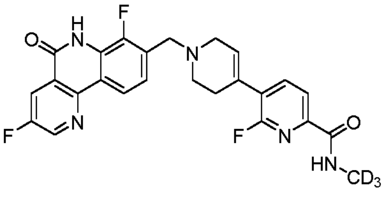 ,
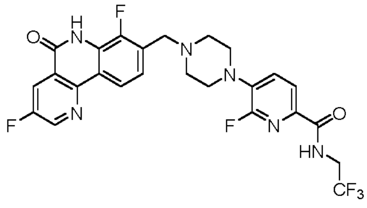 ,
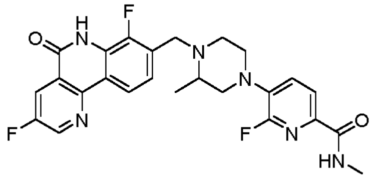 ,

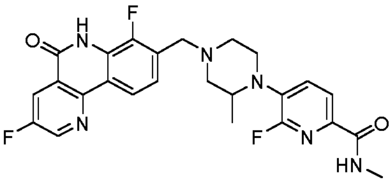 ,
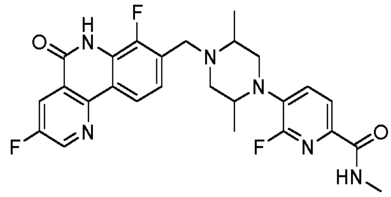 ,

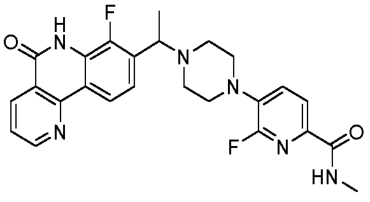 ,
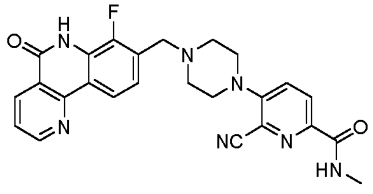 ,
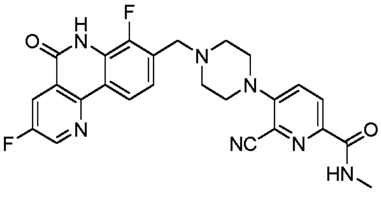 ,

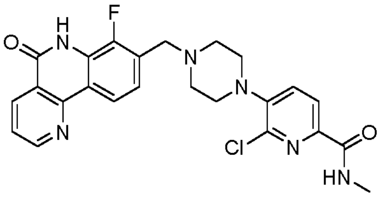 ,
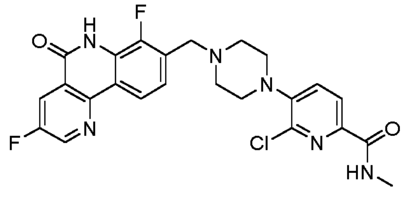 ,
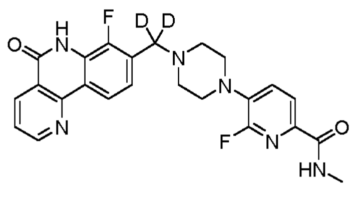 ,

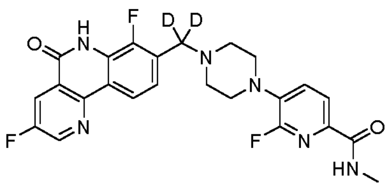,

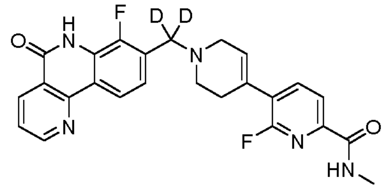,

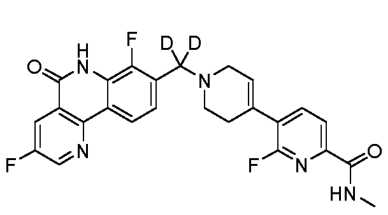,

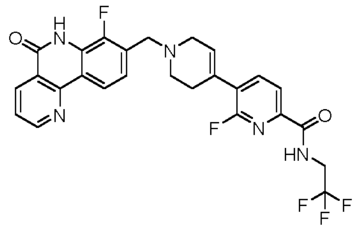,

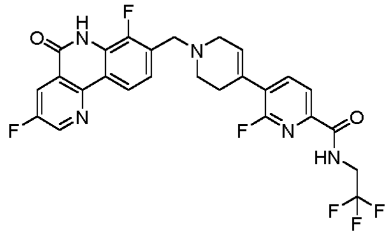,

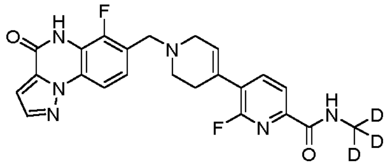,

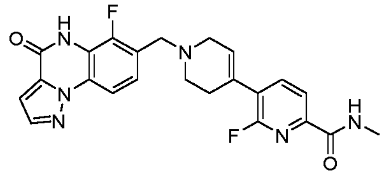,

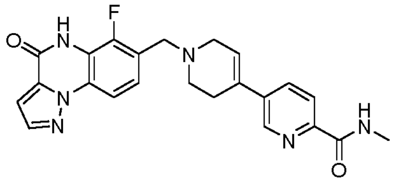,

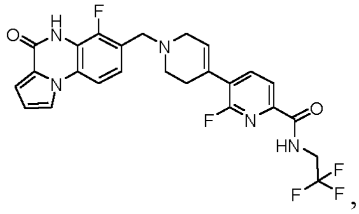,

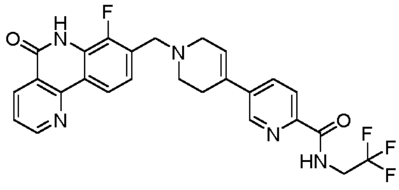,

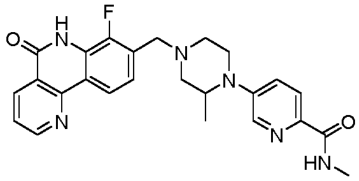,

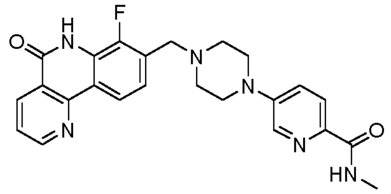,

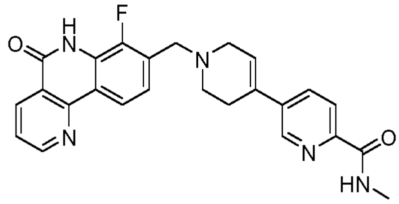,

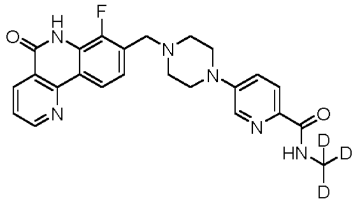,

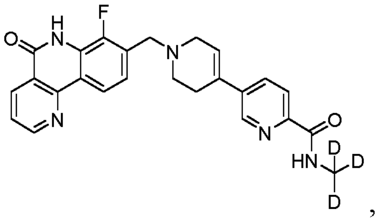,

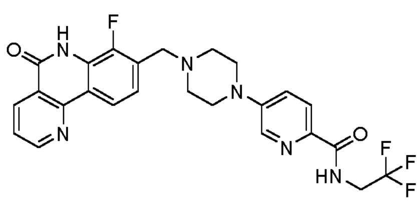,

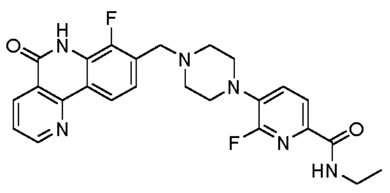,

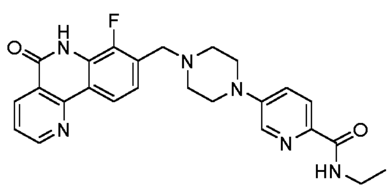,

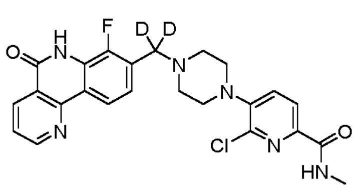,

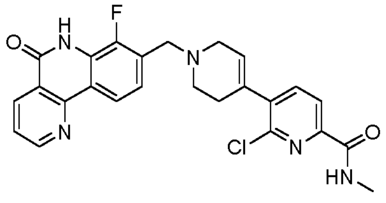,
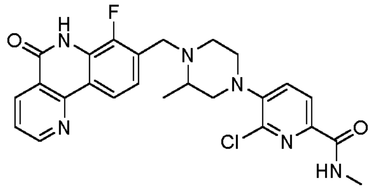,
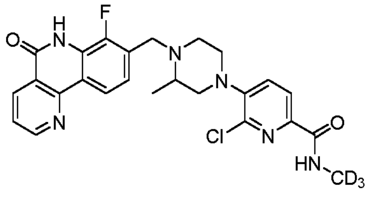,

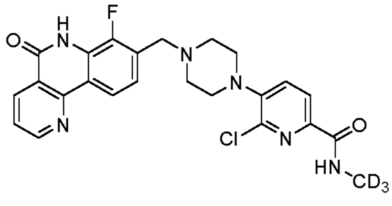,
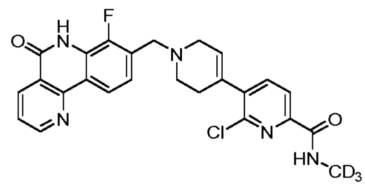,
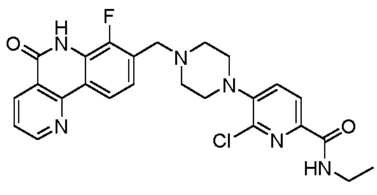,

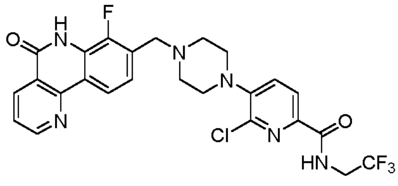,
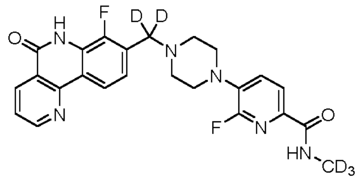,
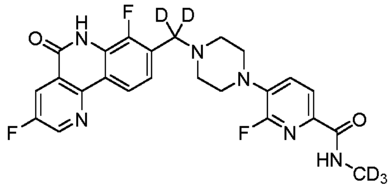,

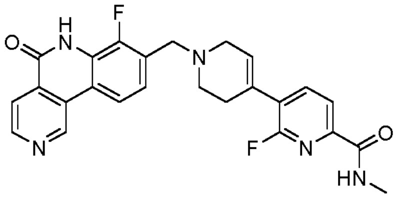, and
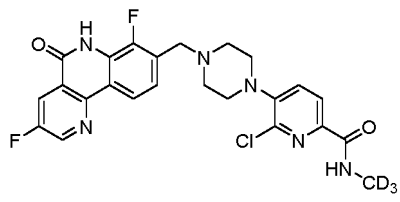.

**25.** The compound or the pharmaceutically acceptable salt thereof according to claim 24, wherein the compound is selected from:

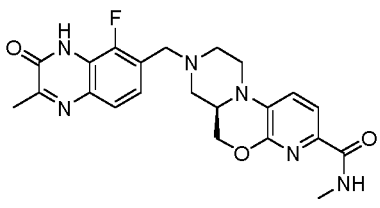,
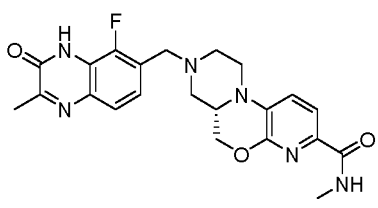,
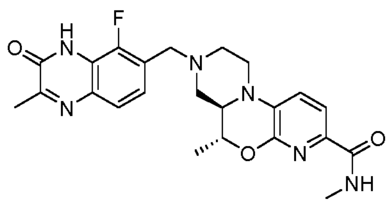,

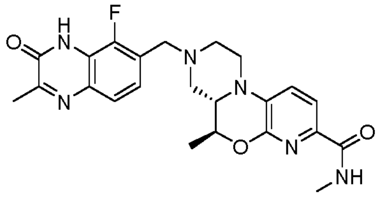,
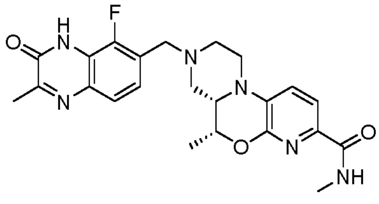,
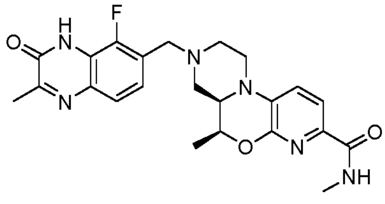,

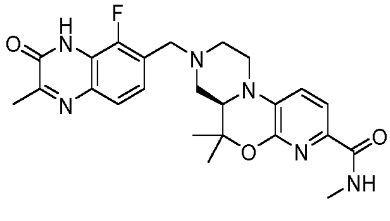,
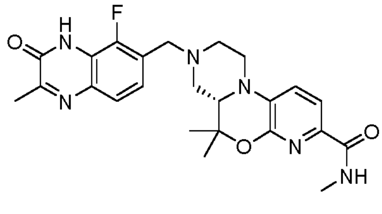,
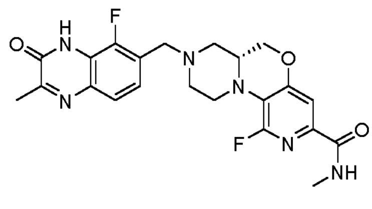,

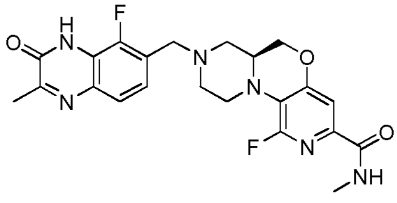,
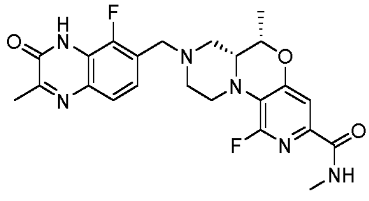,
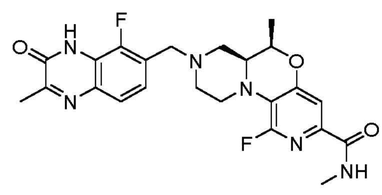,

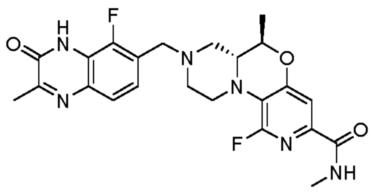,
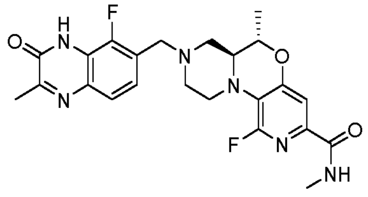,
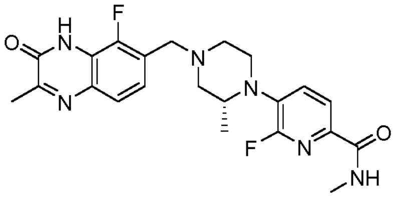,

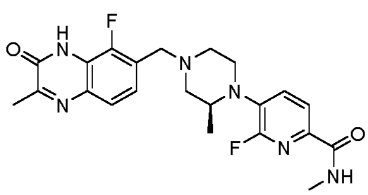,
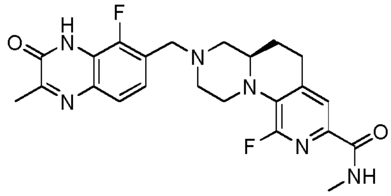,
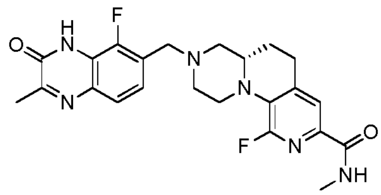,

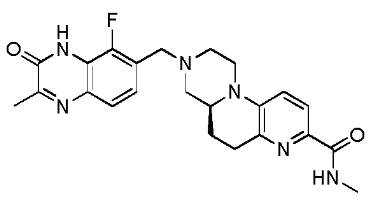,
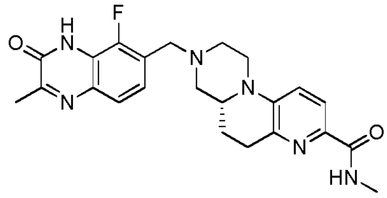,
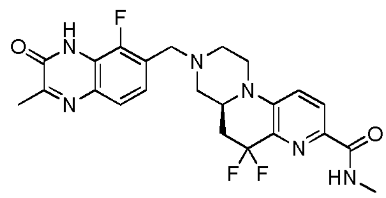,

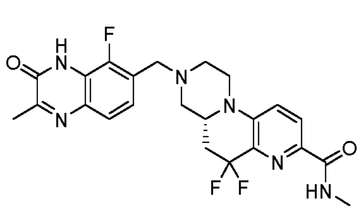,
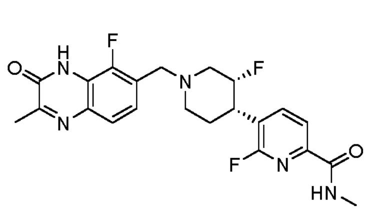,
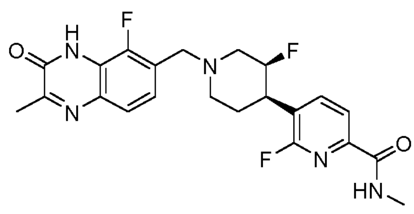,

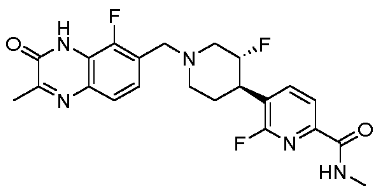,
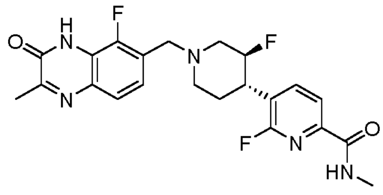,
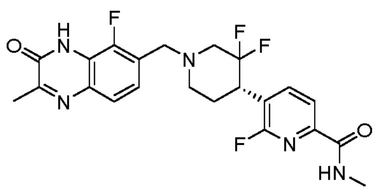,

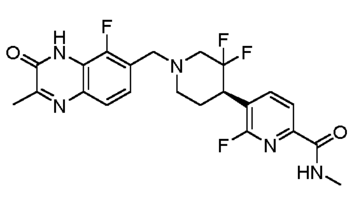,
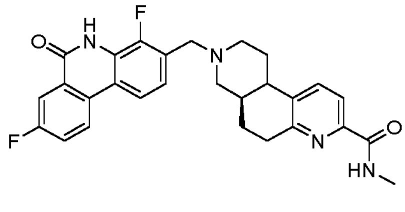,
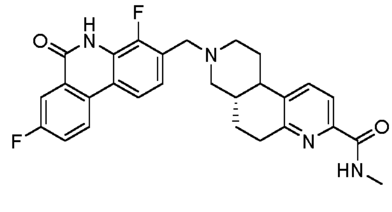,

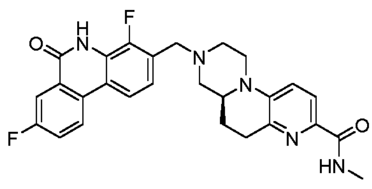,
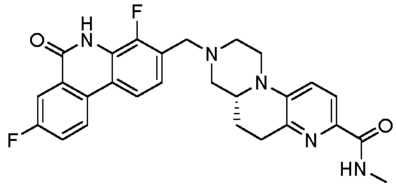,
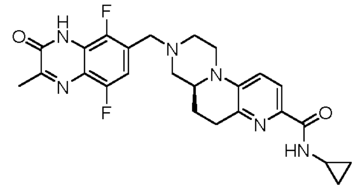,

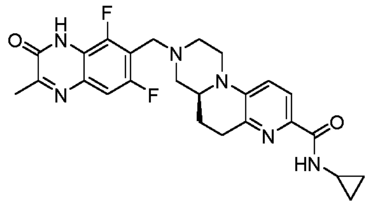,
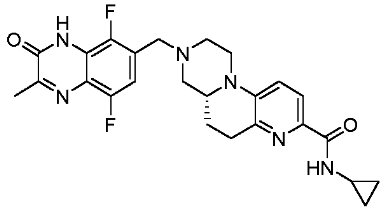,
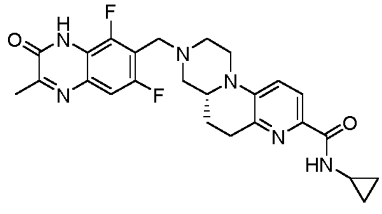,

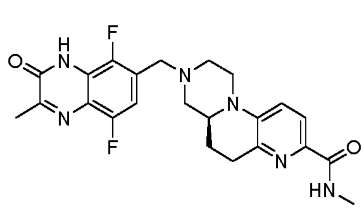,
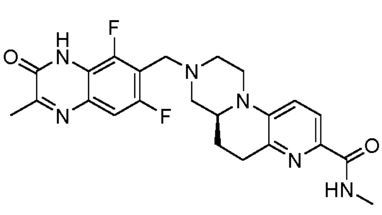,
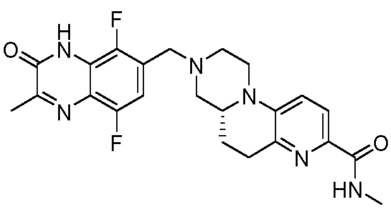,

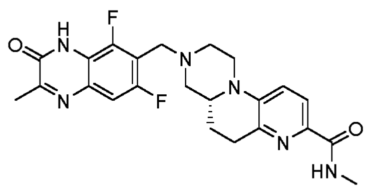,
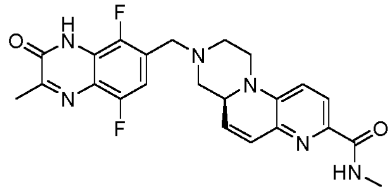,
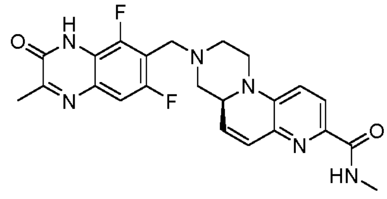,

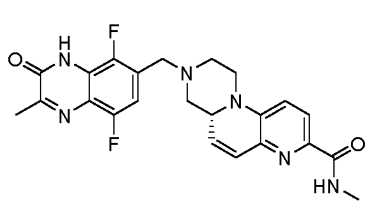,
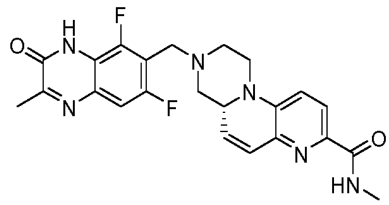,
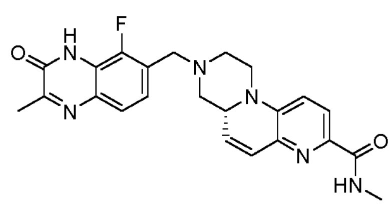,

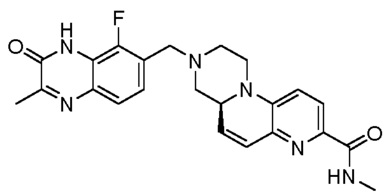,
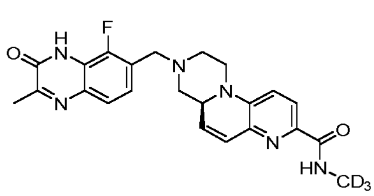,
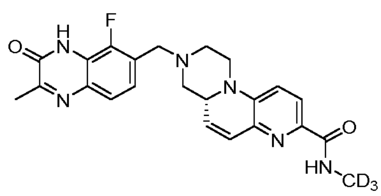,

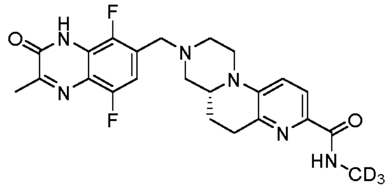,
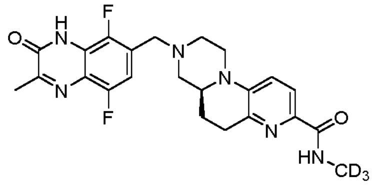,
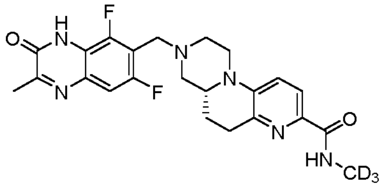,

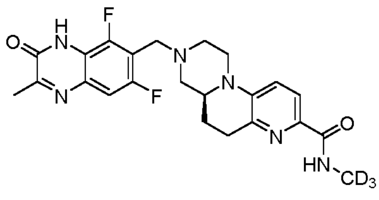, 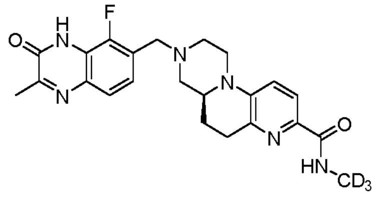, 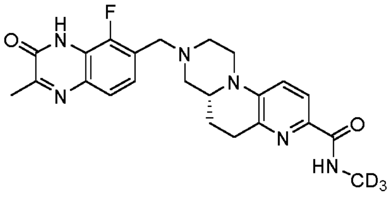,

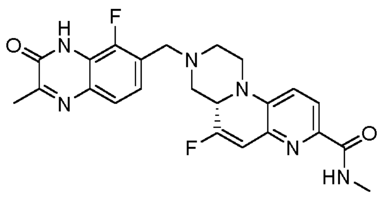, 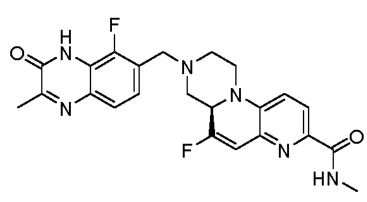, 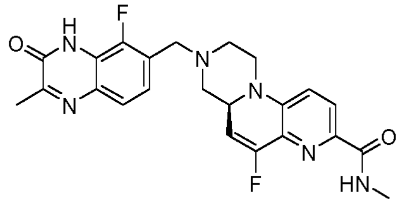,

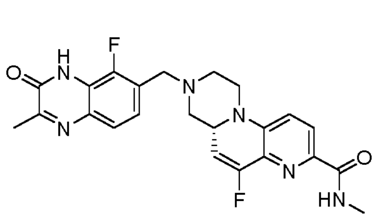, 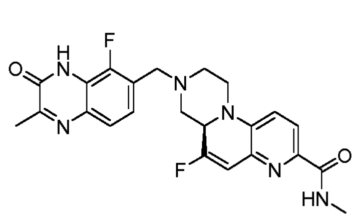, 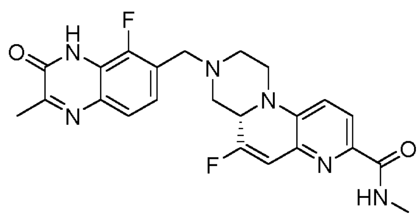,

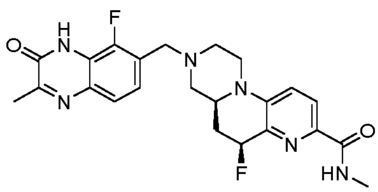, 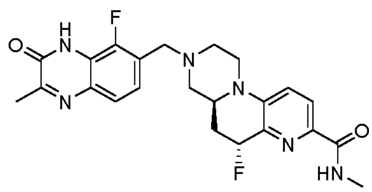, 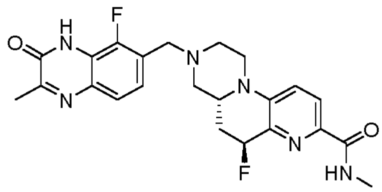,

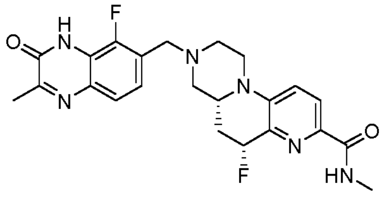, 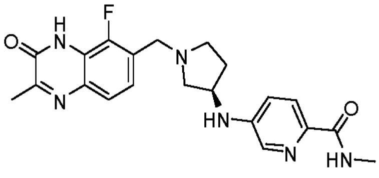, 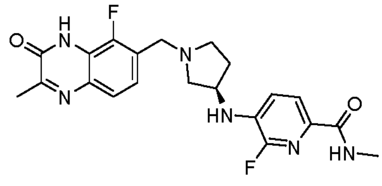,

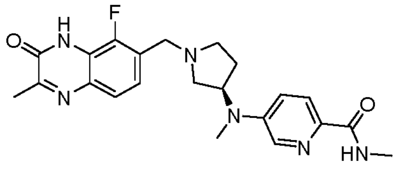, 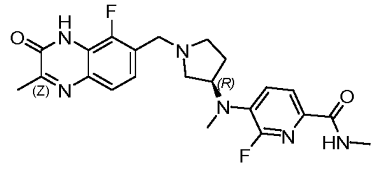, 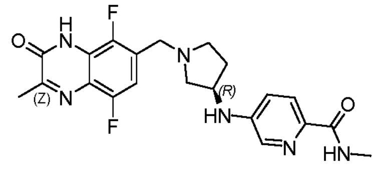,

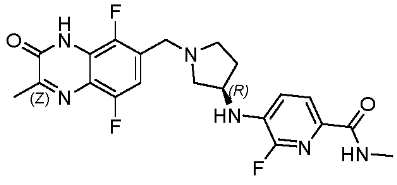, 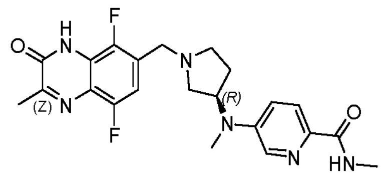, 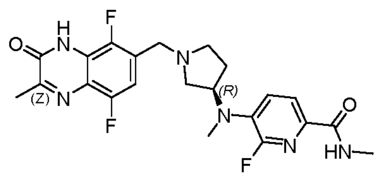,

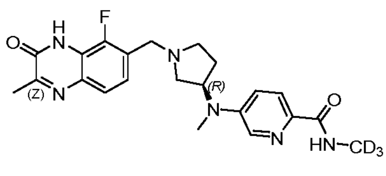 , 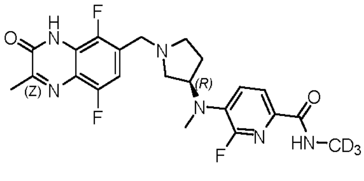 , 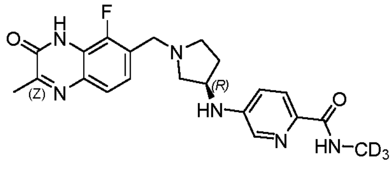 ,

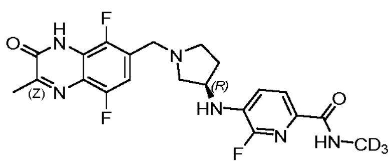 , 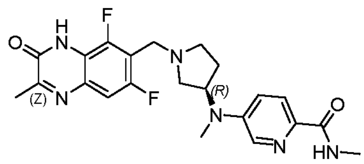 , 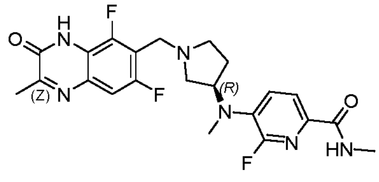 ,

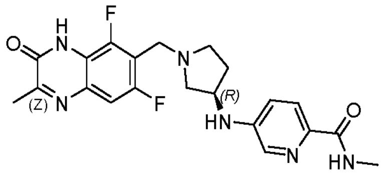 , 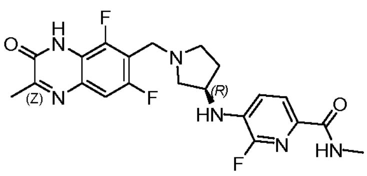 , 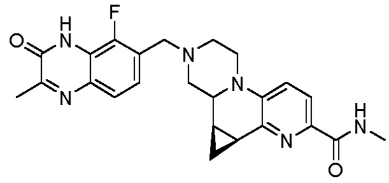 ,

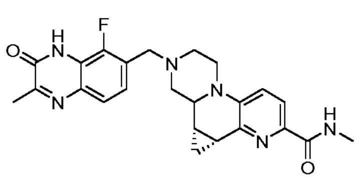 , 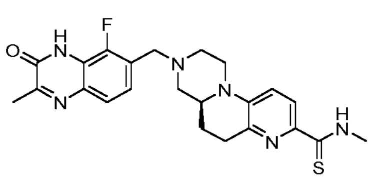 , 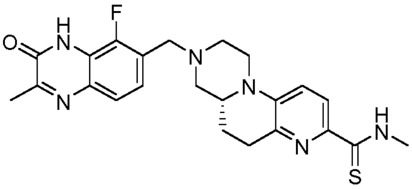 ,

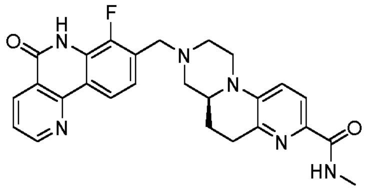 , 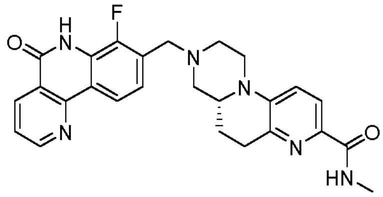 , 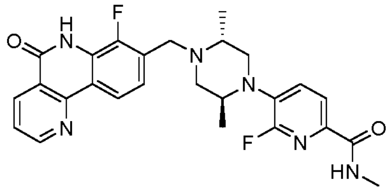 ,

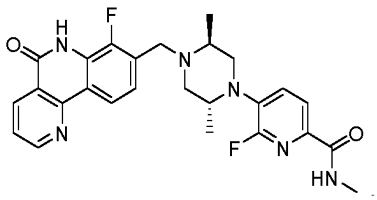 , 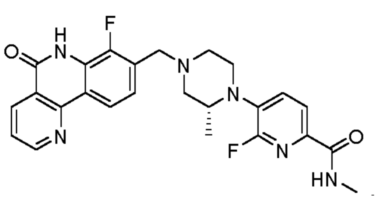 , 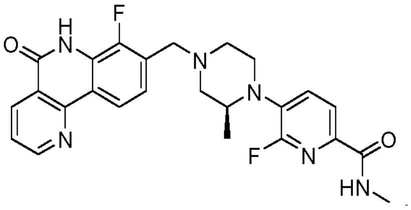 ,

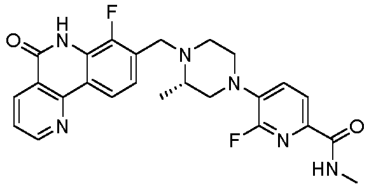 , 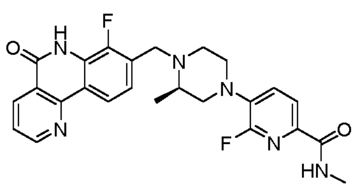 , 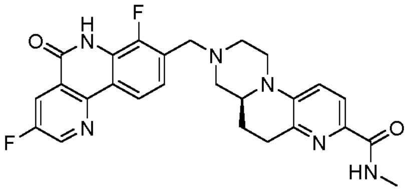 ,

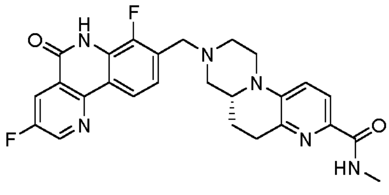, 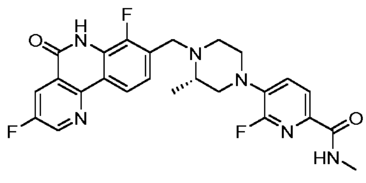, 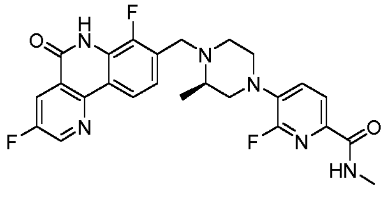,

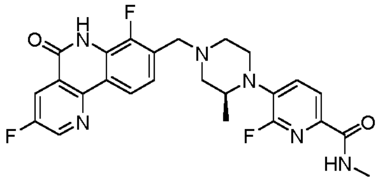, 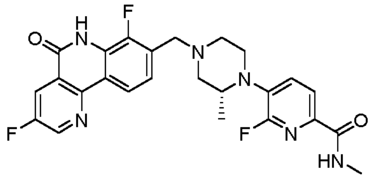, 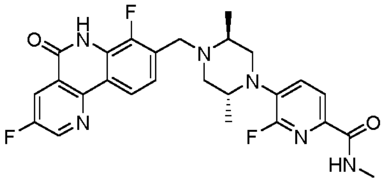,

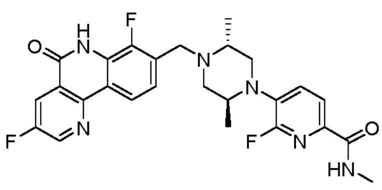, 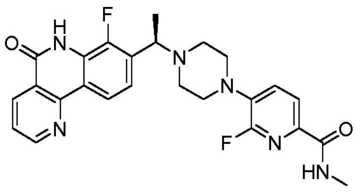, 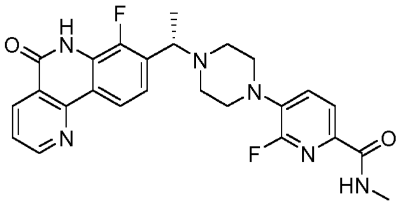,

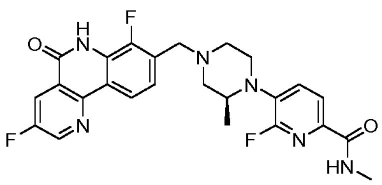, 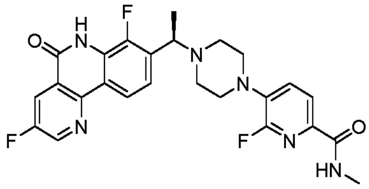, 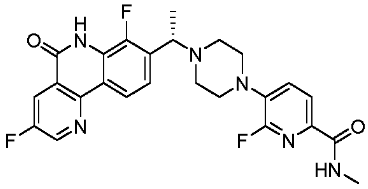,

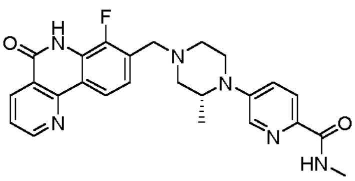, 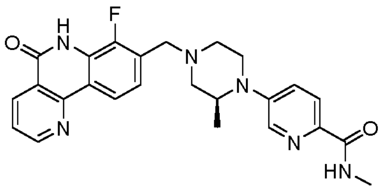, 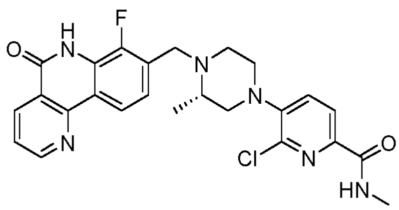,

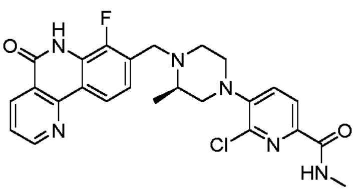, 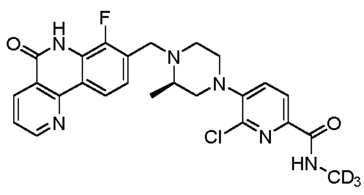, and 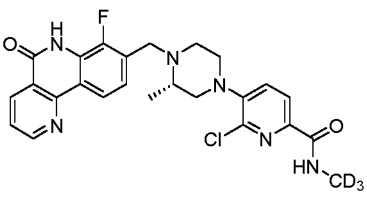.

**26.** A pharmaceutical composition comprising a therapeutically effective amount of the compound or the pharmaceutically acceptable salt thereof according to any one of claims 1 to 25 as an active ingredient, and a pharmaceutically acceptable carrier, diluent, or excipient.

**27.** A use of the compound or the pharmaceutically acceptable salt thereof according to any one of claims 1 to 25 or the composition according to claim 26 in the manufacture of a medicament for treating solid tumors.

**28.** The use according to claim 27, wherein the solid tumors refer to solid tumors such as ovarian cancer, breast cancer, prostate cancer, and glioma.

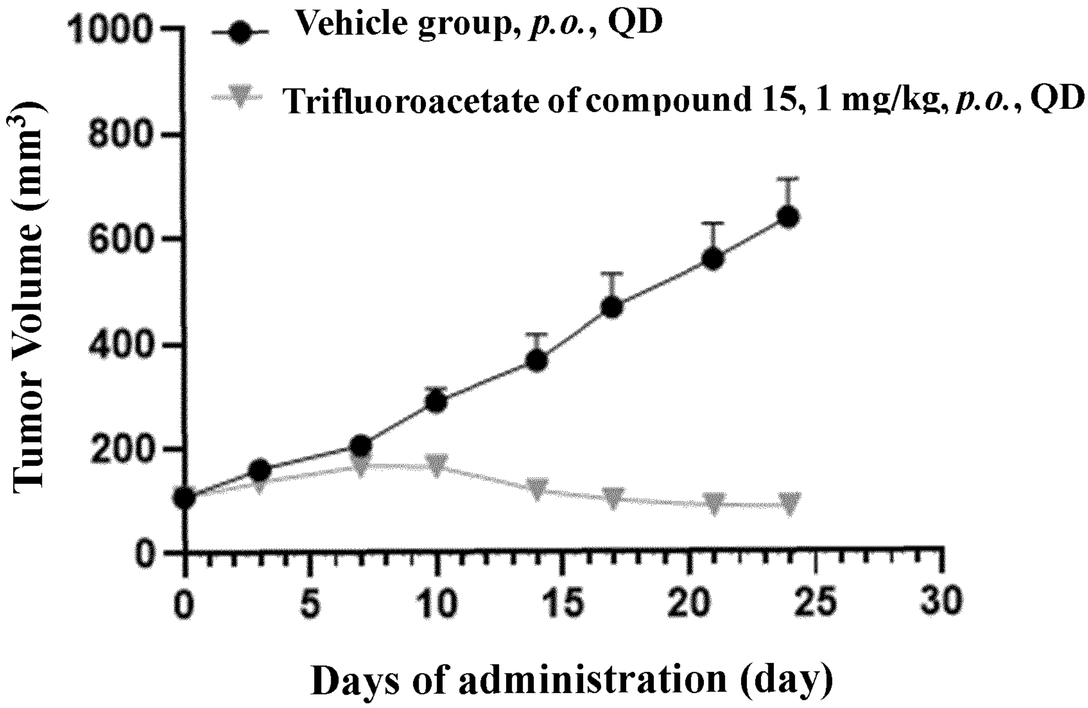
Vehicle group, *p.o.*, QD
Trifluoroacetate of compound 15, 1 mg/kg, *p.o.*, QD
Tumor Volume (mm3)
1000
800
600
400
200
0
0
5
10
15
20
25
30
Days of administration (day)

FIG. 1

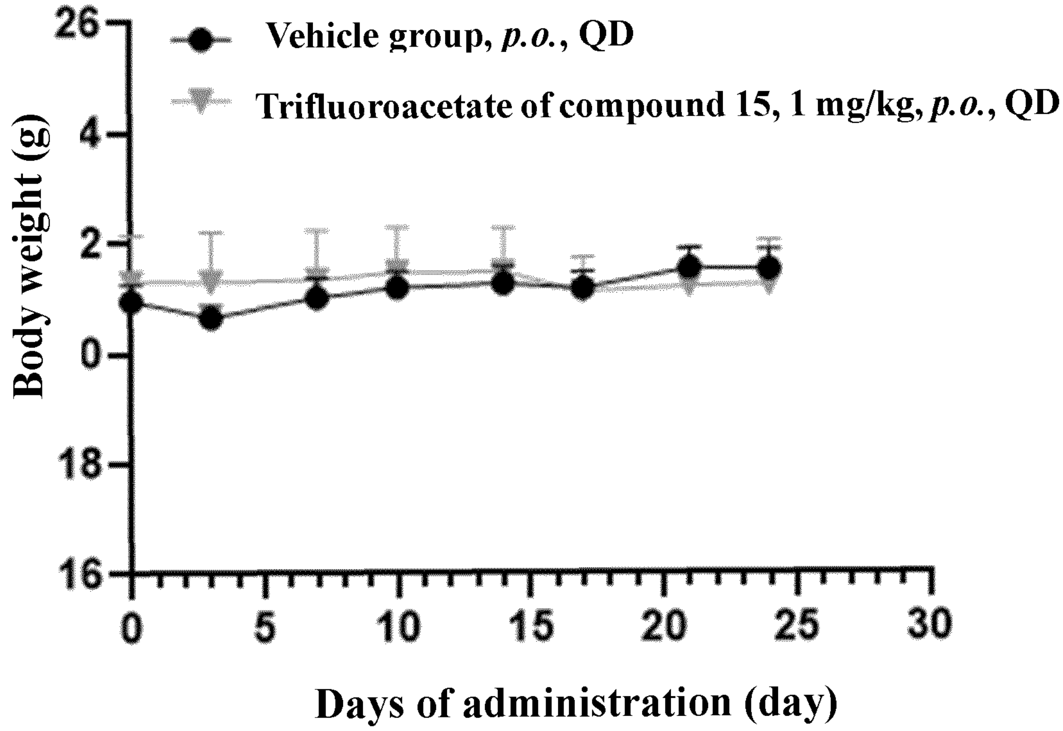
Vehicle group, *p.o.*, QD
Trifluoroacetate of compound 15, 1 mg/kg, *p.o.*, QD
Body weight (g)
26
4
2
0
18
16
0
5
10
15
20
25
30
Days of administration (day)

FIG. 2

**INTERNATIONAL SEARCH REPORT**

International application No.
**PCT/CN2023/092572**

**A. CLASSIFICATION OF SUBJECT MATTER**

C07D498/14(2006.01)i； C07D487/04(2006.01)i； C07D471/04(2006.01)i； C07D471/14(2006.01)i； C07D401/14(2006.01)i； C07D401/02(2006.01)i； A61K31/498(2006.01)i； A61K31/4985(2006.01)i； A61K31/4375(2006.01)i； A61K31/435(2006.01)i； A61P35/00(2006.01)i

According to International Patent Classification (IPC) or to both national classification and IPC

**B. FIELDS SEARCHED**

Minimum documentation searched (classification system followed by classification symbols)

IPC： C07D498/-， C07D487/-， C07D471/-， C07D401/-， A61P35/-

Documentation searched other than minimum documentation to the extent that such documents are included in the fields searched

Electronic data base consulted during the international search (name of data base and, where practicable, search terms used)

CNTXT, DWPI, VEN, WPABSC, CNKI, REGISTRY(STN), CAPLUS(STN): 喹喔啉, 酮, PARP, BRCA, 三环, 根据式(XII)的结构式检索, search according to structural formula (XII)

**C. DOCUMENTS CONSIDERED TO BE RELEVANT**

| Category* | Citation of document, with indication, where appropriate, of the relevant passages | Relevant to claim No. |
|---|---|---|
| PA | WO 2023025307 A1 (IMPACT THERAPEUTICS SHANGHAI INC.) 02 March 2023 (2023-03-02)<br>entire document | 1-28 |
| PA | WO 2022222995 A1 (MEDSHINE DISCOVERY INC.) 27 October 2022 (2022-10-27)<br>entire document | 1-28 |
| A | CN 107849040 A (JE IL PHARMACEUTICAL CO., LTD.) 27 March 2018 (2018-03-27)<br>entire document | 1-28 |
| A | CN 114144413 A (ASTRAZENECA AB) 04 March 2022 (2022-03-04)<br>entire document | 1-28 |
| A | WO 2021260092 A1 (ASTRAZENECA AB) 30 December 2021 (2021-12-30)<br>entire document | 1-28 |

☐ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **17 August 2023** | **21 August 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/092572**

**Box No. III Observations where unity of invention is lacking (Continuation of item 3 of first sheet)**

This International Searching Authority found multiple inventions in this international application, as follows:

The present application sets forth a compound represented by formula (XII) or a pharmaceutically acceptable salt thereof (claims 1-25), a pharmaceutical composition thereof (claim 26), and a pharmaceutical use thereof (claims 27-28).

On the basis of the content disclosed in the description, the technical solutions described in the claims of the present application can be divided into the following 10 groups:

group 1: a compound of formula (XII) in claims 1-28 having a structure, wherein is , and is or a pharmaceutically acceptable salt thereof, a pharmaceutical composition thereof, and a pharmaceutical use thereof;

group 2: a compound of formula (XII) in claims 1-28 having a structure, wherein is , and is or a pharmaceutically acceptable salt thereof, a pharmaceutical composition thereof, and a pharmaceutical use thereof;

group 3: a compound of formula (XII) in claims 1-28 having a structure, wherein is , and is or a pharmaceutically acceptable salt thereof, a pharmaceutical composition thereof, and a pharmaceutical use thereof;

group 4: a compound of formula (XII) in claims 1-28 having a structure, wherein is , and is or a pharmaceutically acceptable salt thereof, a pharmaceutical composition thereof, and a pharmaceutical use thereof;

group 5: a compound of formula (XII) in claims 1-28, wherein is , and ring A is phenyl, or a pharmaceutically acceptable salt thereof, a pharmaceutical composition thereof, and a pharmaceutical use thereof;

groups 6-8: a compound of formula (XII) in claims 1-28, wherein is , and ring A is pyridyl fused at different positions (see strctures 3-5 in claim 18), or a pharmaceutically acceptable salt thereof, a pharmaceutical composition thereof, and a pharmaceutical use thereof;

group 9: a compound of formula (XII) in claims 1-28, wherein is or a pharmaceutically acceptable salt thereof, a pharmaceutical composition thereof, and a pharmaceutical use thereof; and

group 10: a compound (for example, compounds in lines 4-7 in claim 24, etc.) of formula (XII) in claims 24-28, wherein is , L is a single bond, and ring C does not form a fused ring structure with the rings where $T_3$ and $T_4$ are located, and a pharmaceutically acceptable salt thereof, a pharmaceutical composition thereof, or a pharmaceutical use thereof.

When the Markush grouping is for alternatives of chemical compounds, they shall be regarded as being of a similar nature where the following criteria are fulfilled: (A) all alternatives have a common property or activity, and (B)(1) a common structure is present, that is, a significant structural element is shared by all of the alternatives, or (B)(2) in cases where the common structure cannot be the unifying criteria, all alternatives belong to a recognized class of chemical compounds in the art to which the invention pertains.

The definitions for the groups of the compound of formula (XII) in claim 1 of the present application are broad, and the common structure thereof is not a significant structural unit, for example, see document WO 2021260092 A1 (publication date: 30 December 2021), which discloses a compound having the same use, i.e., . In addition, when the structure where T1 is located, i.e., is selected from different groups as follows: , or and/or is not a fused structure (corresponding to some of the compounds of claims 24-25), or is selected from different structures: , , or (hereinafter referred to as B5), the compounds of formula (XII) represented thereby do not belong to the same class of chemical compounds well-recognized in the art to which the invention pertains.

Therefore, the compounds of the 10 groups of inventions cannot meet the above-mentioned criteria. Therefore, these inventions lack unity of invention and do not comply with PCT Rule 13.

Form PCT/ISA/210 (continuation of first sheet) (July 2022)

**INTERNATIONAL SEARCH REPORT**

International application No.
**PCT/CN2023/092572**

**Box No. III Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☑ As only some of the required additional search fees were timely paid by the applicant, this international search report covers only those claims for which fees were paid, specifically claims Nos.: **The relevant technical solutions in claims 1-28 that relate to inventions 1, 2 and 6-8.**

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
**Information on patent family members**

International application No.
**PCT/CN2023/092572**

| Patent document cited in search report | Publication date (day/month/year) | Patent family member(s) | Publication date (day/month/year) |
|---|---|---|---|
| WO 2023025307 A1 | 02 March 2023 | None | |
| WO 2022222995 A1 | 27 October 2022 | CN 115702156 A | 14 February 2023 |
| | | TW 111115317 A | 21 April 2022 |
| CN 107849040 A | 27 March 2018 | MY 194590 A | 05 December 2022 |
| | | RU 2017146130 A | 16 July 2019 |
| | | RU 2017146130 A3 | 17 July 2019 |
| | | RU 2715413 C2 | 28 February 2020 |
| | | PL 3312177 T3 | 02 November 2021 |
| | | DK 3312177 T3 | 05 July 2021 |
| | | AU 2016276806 A1 | 04 January 2018 |
| | | AU 2016276806 B2 | 14 February 2019 |
| | | AU 2016276806 B9 | 21 February 2019 |
| | | KR 20160144916 A | 19 December 2016 |
| | | KR 101837047 B1 | 09 March 2018 |
| | | JP 2019163287 A | 26 September 2019 |
| | | JP 6806969 B2 | 06 January 2021 |
| | | JP 2018521033 A | 02 August 2018 |
| | | CL 2017003138 A1 | 15 June 2018 |
| | | ZA 201708663 B | 24 April 2019 |
| | | PT 3312177 T | 17 May 2021 |
| | | SA 517390509 B1 | 02 June 2021 |
| | | NZ 738187 A | 29 March 2019 |
| | | PH 12017502228 A1 | 11 June 2018 |
| | | CA 2990277 A1 | 15 December 2016 |
| | | CA 2990277 C | 26 October 2021 |
| | | ES 2870203 T3 | 26 October 2021 |
| | | MX 2017016013 A | 20 April 2018 |
| | | WO 2016200101 A2 | 15 December 2016 |
| | | WO 2016200101 A3 | 02 February 2017 |
| | | US 2018162834 A1 | 14 June 2018 |
| | | US 10464919 B2 | 05 November 2019 |
| | | EP 3312177 A2 | 25 April 2018 |
| | | EP 3312177 A4 | 05 December 2018 |
| | | EP 3312177 B1 | 14 April 2021 |
| CN 114144413 A | 04 March 2022 | AU 2020318599 A1 | 10 March 2022 |
| | | US 2021040084 A1 | 11 February 2021 |
| | | US 11325906 B2 | 10 May 2022 |
| | | CR 20220070 A | 21 March 2022 |
| | | UY 38793 A | 26 February 2021 |
| | | ECSP 22012826 A | 31 March 2022 |
| | | BR 112022000534 A2 | 10 May 2022 |
| | | MX 2022000711 A | 23 February 2022 |
| | | AR 119424 A1 | 15 December 2021 |
| | | IL 289534 A | 01 March 2022 |
| | | KR 20220035941 A | 22 March 2022 |
| | | CL 2022000110 A1 | 20 September 2022 |
| | | JP 2022541483 A | 26 September 2022 |
| | | JOP 20220008 A1 | 30 January 2023 |
| | | DOP 2022000006 A | 15 March 2022 |
| | | US 2022227768 A1 | 21 July 2022 |

Form PCT/ISA/210 (patent family annex) (July 2022)

**INTERNATIONAL SEARCH REPORT**
**Information on patent family members**

International application No.
**PCT/CN2023/092572**

| Patent document cited in search report | Publication date (day/month/year) | Patent family member(s) | Publication date (day/month/year) |
|---|---|---|---|
| | | PE 20221339 A1 | 13 September 2022 |
| | | CO 2022001590 A2 | 18 March 2022 |
| | | TW 202116750 A | 01 May 2021 |
| | | WO 2021013735 A1 | 28 January 2021 |
| | | EP 3999506 A1 | 25 May 2022 |
| | | CA 3145644 A1 | 28 January 2021 |
| WO 2021260092 A1 | 30 December 2021 | AU 2021295423 A1 | 16 February 2023 |
| | | KR 20230026450 A | 24 February 2023 |
| | | IL 299123 A | 01 February 2023 |
| | | CL 2022003711 A1 | 23 June 2023 |
| | | US 2022009901 A1 | 13 January 2022 |
| | | TW 202218664 A | 16 May 2022 |
| | | CA 3186996 A1 | 30 December 2021 |
| | | ECSP 23005059 A | 28 February 2023 |
| | | UY 39296 A | 31 December 2021 |
| | | CO 2023000186 A2 | 17 April 2023 |
| | | BR 112022025435 A2 | 24 January 2023 |
| | | EP 4172152 A1 | 03 May 2023 |
| | | DOP 2022000288 A | 15 January 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210495459
- CN 202210693545X
- CN 202211204558
- CN 202211351783
- CN 202211408423
- CN 202211593592
- CN 202310088997X
- CN 2023101237901
- CN 2023102323675
- CN 2023103034259
- CN 2023103408613
- CN 202310388951X


### Non-patent literature cited in the description


- *CHEMICAL ABSTRACTS*, 87413-09-0;
- *CHEMICAL ABSTRACTS*, 19172-47-5